(19) Europäisches Patentamt
European Patent Office
Office européen des brevets

(11) EP 2 663 656 B1

(12) **EUROPEAN PATENT SPECIFICATION**

(45) Date of publication and mention of the grant of the patent:
**24.08.2016 Bulletin 2016/34**

(21) Application number: **12734103.0**

(22) Date of filing: **12.01.2012**

(51) Int Cl.:
***C12Q 1/68*** *(2006.01)*

(86) International application number:
**PCT/IS2012/050001**

(87) International publication number:
**WO 2012/095872 (19.07.2012 Gazette 2012/29)**

(54) **GENETIC VARIANTS AS MARKERS FOR USE IN URINARY BLADDER CANCER RISK ASSESSMENT**

GENETISCHE VARIANTEN ALS MARKER ZUR VERWENDUNG BEI DER BESTIMMUNG EINES HARNBLASENKREBSRISIKOS

VARIANTS GÉNÉTIQUES COMME MARQUEURS À UTILISER DANS L'ÉVALUATION DU RISQUE DU CANCER DE LA VESSIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2011 IS 50004**

(43) Date of publication of application:
**20.11.2013 Bulletin 2013/47**

(73) Proprietors:
- **Decode Genetics EHF**
  **101 Reykjavik (IS)**
- **Illumina Inc.**
  **San Diego, CA 92121 (US)**

(72) Inventors:
- **RAFNAR, Thorunn**
  **IS-108 Reykjavík (IS)**
- **SULEM, Patrick**
  **IS-105 Reykjavik (IS)**

(74) Representative: **Arnason Faktor Intellectual Property Consulting**
**Gudridarstig 2-4**
**113 Reykjavik (IS)**

(56) References cited:

| | |
|---|---|
| **EP-A1- 2 138 848** | **WO-A1-2009/035402** |
| **WO-A1-2011/104730** | **WO-A1-2011/104731** |
| **WO-A2-2010/004590** | **US-A1- 2003 113 758** |
| **US-A1- 2005 009 072** | **US-A1- 2009 305 257** |
| **US-A1- 2009 307 179** | |

- **VAN RHIJN B W G ET AL: "Recurrence and Progression of Disease in Non-Muscle-Invasive Bladder Cancer: From Epidemiology to Treatment Strategy", EUROPEAN UROLOGY, ELSEVIER BV, NL, vol. 56, no. 3, 1 September 2009 (2009-09-01), pages 430-442, XP026379565, ISSN: 0302-2838 [retrieved on 2009-06-26]**
- **CLAUDIO PELUCCHI ET AL: "Mechanisms of Disease: the epidemiology of bladder cancer", NATURE CLINICAL PRACTICE UROLOGY, vol. 3, no. 6, 1 June 2006 (2006-06-01), pages 327-340, XP055179594, ISSN: 1743-4270, DOI: 10.1038/ncpuro0510**
- **KIEMENEY LAMBERTUS A ET AL: "Sequence variant on 8q24 confers susceptibility to urinary bladder cancer", NATURE GENETICS, NATURE PUBLISHING GROUP, US, vol. 40, no. 11, 1 November 2008 (2008-11-01), pages 1307-1312, XP002547167, ISSN: 1546-1718, DOI: 10.1038/NG.229 [retrieved on 2008-09-14]**
- **WANG MEILIN ET AL: "Common genetic variants on 8q24 contribute to susceptibility to bladder cancer in a Chinese population", CARCINOGENESIS, OXFORD UNIVERSITY PRESS, GB, vol. 30, no. 6, 1 June 2009 (2009-06-01), pages 991-996, XP002547168, ISSN: 0143-3334, DOI: 10.1093/CARCIN/BGP091**
- **GARCIA-CLOSAS ET AL.: 'A genome-wide association study of bladder cancer identifies a new susceptibility locus within SLC14A1, a urea transporter gene on chromosome 18q12.3' HUMAN MOLECULAR GENETICS vol. 20, no. 21, 2011, pages 4282 - 4289, XP055009600**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- RAFNAR ET AL.: 'European genome-wide association study identifies SLC14A1 as a new urinary bladder cancer susceptibility gene' HUMAN MOLECULAR GENETICS vol. 20, no. 21, 2011, pages 4268 - 4281, XP055092612
- WANG ET AL.: 'Common genetic variants on 8q24 contribute to susceptibility to bladder cancer in a Chinese population' CARCINOGENESIS vol. 30, no. 6, 2009, pages 991 - 996, XP002547168
- XIFENG ET AL.: 'Genetic polymorphism in bladder cancer' FRONTIERS IN BIOSCIENCE vol. 12, 2007, pages 192 - 213, XP002547170
- SMITH ET AL.: 'Genomic Organization of the Mammalian SLC14A2 Urea Transporter Genes' JOURNAL OF MEMBRANE BIOLOGY vol. 212, no. 2, 2007, pages 109 - 117, XP019489235

## Description

## BACKGROUND OF THE INVENTION

Urinary bladder cancer (UBC) is the 6th most common type of cancer in the United States with approximately 67,000 new cases and 14,000 deaths from the disease in 2007. UBC tends to occur most commonly in individuals over 60 years of age. Exposure to certain industrially used chemicals (derivatives of compounds called arylamines) is strong risk factor for the development of bladder cancers. Tobacco use (specifically cigarette smoking) is thought to cause 50% of bladder cancers discovered in male patients and 30% of those found in female patients. Thirty percent of bladder tumors probably result from occupational exposure in the workplace to carcinogens such as benzidine. Occupations at risk are metal industry workers, rubber industry workers, workers in the textile industry and people who work in printing. Certain drugs such as cyclophosphamide and phenacetin are known to predispose to bladder cancer. Chronic bladder irritation (infection, bladder stones, catheters, and bilharzia) predisposes to squamous cell carcinoma of the bladder.

Familial clustering of UBC cases suggests that there is a genetic component to the risk of the disease (Aben, K.K. et al. "Familial aggregation of urothelial cell carcinoma". Int J Cancer 98, 274-8 (2002); Amundadottir, L.T. et al. "Cancer as a Complex Phenotype: Pattern of Cancer Distribution within and beyond the Nuclear Family." PLoS Med 1, e65 Epub 2004 Dec 28 (2004); Murta-Nascimento, C. et al. "Risk of bladder cancer associated with family history of cancer: do low-penetrance polymorphisms account for the increase in risk?" Cancer Epidemiol Biomarkers Prev 16, 1595-600 (2007)). Genetic segregation analyses have suggested that this component is multifactorial with many genes conferring small risks (Aben, K.K. et al. "Segregation analysis of urothelial cell carcinoma." Eur J Cancer 42, 1428-33 (2006)). Many epidemiological studies have evaluated potential associations between sequence variants in candidate genes and bladder cancer, but the most consistent risk association to the disease is found for variations in the NAT2 gene. (Sanderson, S. et al., "Joint effects of the N-acetyltransferase 1 and 2 (NAT1 and NAT2) genes and smoking on bladder carcinogenesis: a literature-based systematic HuGE review and evidence synthesis." Am J Epidemiol 166, 741-51 (2007)).

Majority (>90%) of bladder cancers are transitional cell carcinomas (TCC) and arise from the urothelium. Other bladder cancer types include squamous cell carcinoma, adenocarcinoma, sarcoma, small cell carcinoma and secondary deposits from cancers elsewhere in the body.

TCCs are often multifocal, with 30-40% of patients having a more than one tumor at diagnosis. The pattern of growth of TCCs can be papillary, sessile (flat) or carcinoma-in-situ (CIS). Superficial tumors are defined as tumors that either do not invade, or those that invade but stay superficial to the deep muscle wall of the bladder. At initial diagnosis, 70% of patients with bladder cancers have superficial disease. Tumors that are clinically superficial are composed of three distinctive pathologic types. The majority of superficial urothelial carcinomas present as noninvasive, papillary tumors (pathologic stage pTa (or Ta)). 70% of these superficial papillary tumors will recur over a prolonged clinical course, causing significant morbidity. In addition, 5-10% of these papillary lesions will eventually progress to invasive carcinomas. These tumors are pathologically graded as either low malignant potential, low grade or high grade. High grade tumors have a higher risk of progression. Flat urothelial carcinoma in situ (CIS) are highly aggressive lesions and progress more rapidly than the papillary tumors. A minority of tumors invade only superficially into the lamina propria. These tumors recur 80% of the time, and eventually invade the detrusor muscle in 30% of cases. Approximately 30% of urothelial carcinomas invade the detrusor muscle at presentation. These cancers are highly aggressive. Those invasive tumors may spread by way of the lymph and blood systems to invade bone, liver, and lungs and have high morbidity (Kaufman, D.S. Ann Oncol 17, v106-112 (2006)).

The treatment of transitional cell or urothelial carcinoma is different for superficial tumors and muscle invasive tumors. Superficial bladder cancers can be managed without cystectomy (removing the bladder). The standard initial treatment of superficial tumors includes cystoscopy with trans-urethral resection of the tumor (TUR). The cystoscope allows visualization and entire removal of a bladder tumor. Adjuvant intravesical drug therapy after TUR is commonly prescribed for patients with tumors that are large, multiple, high grade or superficially invasive. Intravesical therapy consists of drugs placed directly into the bladder through a urethral catheter, in an attempt to minimize the risk of tumor recurrence and progression. About 50-70% of patients with superficial bladder cancer have a very good response to intravesical therapy. The current standard of care consists of urethro-cystoscopy and urine cytology every 3-4 months for the first two years and at a longer interval in subsequent years.

Cystectomy is indicated when bladder cancer is invasive into the muscle wall of the bladder or when patients with superficial tumors have frequent recurrences that are not responsive to intravesical therapy. The benefits of surgically removing the bladder are disease control, eradication of symptoms associated with bladder cancer, and long-term survival. For advanced bladder cancer that has extended beyond the bladder wall, radiation and chemotherapy are treatment options. Local lymph nodes are frequently radiated as part of the therapy to treat the microscopic cancer cells which may have spread to the nodes. Current treatment of advanced bladder cancer can involve a combination of radiation and chemotherapy.

Early detection can improve prognosis, treatment options as well as quality of life of the patient. If screening methods could detect bladder cancers destined to become muscle invading while they are still superficial it is likely that a significant reduction in morbidity and mortality would result.

Cystoscopic examination is costly and causes substantial discomfort for the patient. Urine cytology has poor sensitivity in detecting low-grade disease and its accuracy can vary between pathology labs. Many urine-based tumor markers have been developed for detection and surveillance of the disease and some of these are used in routine patient care (Lokeshwar, V.B. et al. Urology 66, 35-63 (2005); Friedrich, M.G. et al. BJU Int 92, 389-92 (2003); Ramakumar, S. et al. J Urol 161, 388-94 (1999); Sozen, S. et al. Eur Urol 36, 225-9 (1999); Heicappell, R. et al. Urol Int 65, 181-4 (2000)).

However, no biomarker reported to date has shown sufficient sensitivity and specificity for detecting all types of bladder cancers in the clinic. It should be remembered that efficiency of screening increases with the disease's prevalence in the screened population. Therefore, the efficiency of the test could be increased by limiting the screening program to people at high risk. For bladder cancer, this may mean restricting participation to people with occupational exposure to known bladder carcinogens or individuals with known cancer predisposing variants.

There is clearly a need for improved diagnostic procedures that would facilitate early-stage bladder cancer detection and prognosis, as well as aid in preventive and curative treatments of the disease. In addition, there is a need to develop tools to better identify those patients who are more likely to have aggressive forms of bladder cancer from those patients that are diagnosed with the superficial disease. This would help to avoid invasive and costly procedures for patients not at significant risk.

Genetic risk is conferred by subtle differences in the genome among individuals in a population. Variations in the human genome are most frequently due to single nucleotide polymorphisms (SNPs), although other variations are also important. SNPs are located on average every 1000 base pairs in the human genome. Accordingly, a typical human gene containing 250,000 base pairs may contain 250 different SNPs. Only a minor number of SNPs are located in exons and alter the amino acid sequence of the protein encoded by the gene. Most SNPs may have little or no effect on gene function, while others may alter transcription, splicing, translation, or stability of the mRNA encoded by the gene. Additional genetic polymorphisms in the human genome are caused by insertions, deletions, translocations or inversion of either short or long stretches of DNA. Genetic polymorphisms conferring disease risk may directly alter the amino acid sequence of proteins, may increase the amount of protein produced from the gene, or may decrease the amount of protein produced by the gene.

As genetic polymorphisms conferring risk of common diseases are uncovered, genetic testing for such risk factors is becoming increasingly important for clinical medicine. Examples are apolipoprotein E testing to identify genetic carriers of the apoE4 polymorphism in dementia patients for the differential diagnosis of Alzheimer's disease, and of Factor V Leiden testing for predisposition to deep venous thrombosis. More importantly, in the treatment of cancer, diagnosis of genetic variants in tumor cells is used for the selection of the most appropriate treatment regime for the individual patient. In breast cancer, genetic variation in estrogen receptor expression or heregulin type 2 (Her2) receptor tyrosine kinase expression determine if anti-estrogenic drugs (tamoxifen) or anti-Her2 antibody (Herceptin) will be incorporated into the treatment plan. In chronic myeloid leukemia (CML) diagnosis of the Philadelphia chromosome genetic translocation fusing the genes encoding the Bcr and Abl receptor tyrosine kinases indicates that Gleevec (STI571), a specific inhibitor of the Bcr-Abl kinase should be used for treatment of the cancer. For CML patients with such a genetic alteration, inhibition of the Bcr-Abl kinase leads to rapid elimination of the tumor cells and remission from leukemia. Furthermore, genetic testing services are now available, providing individuals with information about their disease risk based on the discovery that certain SNPs have been associated with risk of many of the common diseases.

*Loci Associated with Bladder Cancer*

The genetic polymorphisms in a number of metabolic enzymes and other genes have been found as the modulators of bladder cancer risk. The most studied polymorphisms in connection with bladder cancer risk are polymorphisms in genes for some important enzymes, especially N-acetyl-transferases (NATs), glutathione S-transferases (GSTs), DNA repair enzymes, and many others. An improved understanding of the molecular biology of urothelial malignancies is helping to define more clearly the role of new prognostic indices and multidisciplinary treatment for this disease.

It has been suggested that some of the NAT variants modify individual susceptibility to cancer. Slow NAT2 acetylation capacity has been suggested as conferring an increased risk of bladder, breast, liver and lung cancers, and a decreased risk of colon cancer, whereas a prominent change in the NAT1 gene, putatively associated with increased NAT1 activity, has been suggested as increasing the risk of bladder and colon cancer, and decreasing that of lung cancer (A. Hirvonen, IARC Sci Publ 148 (1999), pp. 251-270). NAT1 polymorphisms may affect the individual bladder cancer risk by interacting with environmental factors and interacting with the NAT2 gene (Cascorbi I, et al. Cancer Res 61:5051-6).

Glutathione S-transferases (GST) comprise a major group of enzymes that play a key role in detoxification of carcinogenic compounds. At least five GST families have been identified, and the effects of polymorphisms in these genes have been studied in bladder cancer. The results from these studies are contradictory but association between GSTM1 null genotype and bladder cancer is fairly constant (Wu, X. et al. Front Biosci 12, 192-213 (2007)).

Polymorphisms in genes coding for other metabolic enzymes such as NQO1, MPO or the CYP enzyme superfamily have also in some studies been found to be associated with bladder cancer but the results are controversial (Wu, X. *et al. supra*). Since bladder cancer has strong environmental risk factors, polymorphisms in DNA repair genes have been studied in bladder cancer patients. These include genes for Xeroderma pigmentosum (XP) and X-ray repair cross-complementing (XRCC) genes. Many different polymorphisms have been tested but larger sample size and better matching between cases and controls is needed to conclude the effects of these variants on bladder cancer risks.

Recently performed genome-wide association studies of UBC have resulted in the identification of genetic variants associated with UBC in several distinct locations (Kiemeney, LA, et al. Nat Genet 40:1307-12 (2008); Wu, X. et al. Nat Genet 41:991-5 (2009); Rafnar, T. et al. Nat Genet 41:221-7 (2009) and Kiemeney, LA, et al. Nat Genet 42:415-419 (2010)). These loci, however, only explain a portion of the genetic risk of UBC in the human population. Thus, it is clear that additional genetic risk factors for UBC remain to be found. It is likely that these genetic risk factors will include a relatively high number of low-to-medium risk genetic variants. These low-to-medium risk genetic variants may, however, be responsible for a substantial fraction of bladder cancer, and their identification, therefore, a great benefit for public health. The present invention provides such additional genetic risk factors of UBC.

## SUMMARY OF THE INVENTION

The present invention is based on the finding that certain genetic variants are associated with risk of urinary bladder cancer (UBC). The invention provides diagnostic applications based on this surprising finding, including methods, kits, media and apparati useful for determining UBC risk.

In a first aspect, the invention provides a method of determining a susceptibility to urinary bladder cancer in a human individual, the method comprising analyzing nucleic acid sequence data from a human individual for at least one polymorphic marker in the human *SLC14A1* gene, wherein different alleles of the at least one polymorphic marker are associated with different susceptibilities to Bladder Cancer in humans, and determining a susceptibility to Bladder Cancer from the nucleic acid sequence data. In one embodiment, the nucleic acid sequence data is obtained from a biological sample containing nucleic acid from the human individual.

In another aspect, the invention provides a method of determining a susceptibility to Bladder Cancer, the method comprising obtaining amino acid sequence data about at least one encoded SLC14A1 protein in a human individual, and analyzing the amino acid sequence data to determine whether at least one amino acid substitution predictive of increased susceptibility of Bladder Cancer is present, wherein a determination of the presence of the at least one amino acid substitution is indicative of increased susceptibility of Bladder Cancer for the individual, and wherein a determination of the absence of the at least one amino acid substitution is indicative of the individual not having the increased susceptibility.

As a consequence of the foregoing, the invention in another aspect provides a method of determining a susceptibility to Bladder Cancer, the method comprising analyzing amino acid sequence data about at least one encoded SLC14A1 protein in a human individual, and/or nucleic acid sequence data about at least one polymorphic marker in the human *SLC14A1* gene, wherein different alleles of the at least one polymorphic marker and/or at least one amino acid substitution are associated with different susceptibilities to Bladder Cancer in humans, and determining a susceptibility to Bladder Cancer from the nucleic acid sequence data and/or the amino acid sequence data.

Also disclosed is a method of identification of a marker for use in assessing susceptibility to urinary bladder cancer in human individuals, the method comprising (a) identifying at least one polymorphic marker in the human *SLC14A1* gene; (b) obtaining sequence information about the at least one polymorphic marker in a group of individuals diagnosed with urinary bladder cancer; and (c) obtaining sequence information about the at least one polymorphic marker in a group of control individuals; wherein determination of a significant difference in frequency of at least one allele in the at least one polymorphism in individuals diagnosed with urinary bladder cancer as compared with the frequency of the at least one allele in the control group is indicative of the at least one polymorphism being useful for assessing susceptibility to urinary bladder cancer. In one embodiment, an increase in frequency of the at least one allele in the at least one polymorphism in individuals diagnosed with urinary bladder cancer, as compared with the frequency of the at least one allele in the control group, is indicative of the at least one polymorphism being useful for assessing increased susceptibility to urinary bladder cancer, and wherein a decrease in frequency of the at least one allele in the at least one polymorphism in individuals diagnosed with urinary bladder cancer, as compared with the frequency of the at least one allele in the control group, is indicative of the at least one polymorphism being useful for assessing decreased susceptibility to, or protection against, urinary bladder cancer.

Also disclosed is a method of assessing a subject's risk for urinary bladder cancer, the method comprising (a) obtaining sequence information about the individual identifying at least one allele of at least one polymorphic marker in the genome of the individual; (b) representing the sequence information as digital genetic profile data; (c) transforming the digital genetic profile data on a computer processor to generate risk assessment report of urinary bladder cancer for the subject; and (d) displaying the risk assessment report on an output device; wherein the at least one polymorphic marker is a marker within the human *SLC14A1* gene that is predictive of risk of urinary bladder cancer in humans.

Also disclosed is a method of determining whether an individual is at increased risk of developing bladder cancer, the method comprising steps of (i) obtaining a biological sample containing nucleic acid from the individual; (ii) determining, in the biological sample, nucleic acid sequence about the SLC14A1 gene; and (iii) comparing the sequence information to the wild-type nucleic acid sequence of SLC14A1 (SEQ ID NO:134); wherein an identification of a mutation in SLC14A1 in the individual is indicative that the individual is at increased risk of developing bladder cancer.

Also disclosed is a method of determining the recurrence risk of an individual diagnosed with urinary bladder cancer, the method comprising steps of (a) obtaining sequence data about a human individual who has been diagnosed with urinary bladder cancer, identifying at least one allele of at least one polymorphic marker, wherein different alleles of the at least one polymorphic marker are associated with different recurrence risk of urinary bladder cancer in humans, and (b) determining the recurrence risk of urinary bladder cancer for the human individual from the sequence data; wherein the at least one polymorphic marker is a marker in the human *SLC14A1* gene, wherein different alleles of the at least one polymorphic marker are associated with different recurrence risk of urinary bladder cancer in humans.

Further disclosed is a method of predicting prognosis of an individual diagnosed with urinary bladder cancer, the method comprising obtaining sequence data about a human individual identifying at least one allele of at least one polymorphic marker in the human *SLC14A1* gene, wherein different alleles of the at least one polymorphic marker are predictive of different prognosis of urinary bladder cancer in humans, and predicting prognosis of urinary bladder cancer from the sequence data.

It may be useful to be able to determine which individuals are suitably for further diagnostic evaluation of urinary bladder cancer. Thus also disclosed is a method for identifying a subject who is a candidate for further diagnostic evaluation for urinary bladder cancer, comprising the steps of (a) determining, in the genome of a human subject, the allelic identity of at least one polymorphic marker in the human *SLC14A1* gene, and/or the identity of at least one amino acid at a variant amino acid position in an encoded SLC14A1 protein, wherein different alleles of the at least one marker and/or the identity of the at least one amino acid are associated with different susceptibilities to urinary bladder cancer in humans; and (b) identifying the subject as a subject who is a candidate for further diagnostic evaluation for urinary bladder cancer based on the allelic identity at the at least one polymorphic marker and/or the identity of the at least one amino acid. In a preferred embodiment, the further diagnostic evaluation comprises urine cytology, cystoscopy and/or a Hematuria test.

Assessment of genetic risk can be reported in a risk assessment report. Therefore also disclosed is a risk assessment report comprising (a) at least one personal identifier, and (b) representation of at least one risk assessment measure of urinary bladder cancer for the human subject for at least one polymorphic marker or at least one amino acid variation.

Kits are also disclosed. In one example, a kit for assessing susceptibility to urinary bladder cancer in humans is disclosed, the kit comprising reagents for selectively detecting at least one at-risk variant for Bladder Cancer in the individual, wherein the at least one risk variant is a marker in the human *SLC14A1* gene or an amino acid variant in an encoded SLC14A1 protein, and a collection of data comprising correlation data between the at least one marker and susceptibility to urinary bladder cancer. The at least one marker is in one embodiment selected from the group consisting of rs1058396, and markers in linkage disequilibrium therewith.

Also disclosed are diagnostic reagents. In one such aspect, the use of an oligonucleotide probe in the manufacture of a diagnostic reagent for diagnosing and/or assessing a susceptibility to urinary bladder cancer in humans is disclosed, wherein the probe is capable of hybridizing to a segment of the human *SLC14A1* gene with sequence as given by SEQ ID NO:134, and wherein the segment is 15-400 nucleotides in length. In a suitable embodiment, the segment of the nucleic acid to which the probe is capable of hybridizing comprises a polymorphic site. The polymorphic site is suitably selected from the group consisting of the markers rs1058396, rs11877062, rs2298720, rs2298719, and markers in linkage disequilibrium therewith.

The invention also provides computer-implemented aspects. As is known in the art, sequence data can conveniently be stored and analyzed in digital format, and either such sequence data (*e.g.,* genotype data) or results derived therefrom (*e.g.,* disease-risk estimates) can be provided in digital format to an end-user.

One such aspect discloses a computer-readable medium having computer executable instructions for determining susceptibility to urinary bladder cancer in humans, the computer readable medium comprising (i) sequence data identifying at least one allele of at least one polymorphic marker in the individual; and (ii) a routine stored on the computer readable medium and adapted to be executed by a processor to determine risk of developing Bladder Cancer for the at least one polymorphic marker; wherein the at least one polymorphic marker is a marker in the human *SLC14A1* gene, or an amino acid variant in an encoded SLC14A1 protein, that is predictive of susceptibility of Bladder Cancer in humans.

Another computer-implemented aspect relates to an apparatus for determining a genetic indicator for urinary bladder cancer in a human individual, comprising (i) a processor; and (ii) a computer readable memory having computer executable instructions adapted to be executed on the processor to analyze marker information for at least one marker in the human *SLC14A1* gene that is predictive of susceptibility to Bladder Cancer in humans, or at least one amino acid variation in an encoded SLC14A1 protein, and generate an output based on the marker or amino acid information, wherein the output comprises at least one measure of susceptibility to Bladder Cancer for the human individual.

In one embodiment, the computer readable memory further comprises data indicative of the risk of developing urinary bladder cancer associated with at least one allele of at least one polymorphic marker, and wherein a risk measure for the human individual is based on a comparison of the marker information for the human individual to the risk of urinary bladder cancer associated with the at least one allele of the at least one polymorphic marker.

In certain embodiments, the polymorphic marker is suitably selected from the group consisting of the markers rs1058396, rs11877062, rs2298720, rs2298719, and markers in linkage disequilibrium therewith. In certain embodiments, the amino acid variation is selected from the group consisting of an asparagine to aspartic acid substitution at position 336, an arginine to tryptophan substitution at position 4, a lysine to glutamic acid substitution at position 100 and a methionine to valine substitution at position 223, all in a protein with sequence as set forth in SEQ ID NO: 133.The invention also provides risk assessment reports. One such aspect relates to a risk assessment report of urinary bladder cancer for a human individual, comprising (i) at least one personal identifier, and (ii) representation of at least one risk assessment measure of urinary bladder cancer for the human subject for at least one polymorphic marker in the human *SLC14A1* gene, wherein different alleles of the at least one polymorphic marker are associated with different susceptibilities to Bladder Cancer in humans. Such reports may be provided in any suitable format, including electronic format (*e.g.,* on a computer-readable medium) or a paper format (*e.g.,* a reported printed or written on paper).

Further disclosed is the providing use of variants for selecting individuals for administration of therapeutic agents for treating urinary bladder cancer. One such aspect provides use of an agent for treating urinary bladder cancer in a human individual that has been tested for the presence of at least one allele of at least one risk marker of urinary bladder cancer, as described herein.

It should be understood that all combinations of features described herein are contemplated, even if the combination of feature is not specifically found in the same sentence or paragraph herein. This includes in particular the use of all markers disclosed herein, alone or in combination, for analysis individually or in haplotypes, in all aspects of the invention as described herein.

## BRIEF DESCRIPTION OF THE DRAWINGS

The foregoing and other objects, features and advantages of the invention will be apparent from the following more particular description of preferred embodiments of the invention.

**FIG 1** shows a schematic view of an exemplary computer system for implementing the invention.

**FIG 2** shows a diagram illustrating a system comprising computer implemented methods utilizing risk variants as described herein.

**FIG 3** shows an exemplary system for determining risk of cancer as described further herein.

**FIG 4** shows a system for selecting a treatment protocol for a subject diagnosed with a cancer.

## DETAILED DESCRIPTION

*Definitions*

Unless otherwise indicated, nucleic acid sequences are written left to right in a 5' to 3' orientation. Numeric ranges recited within the specification are inclusive of the numbers defining the range and include each integer or any non-integer fraction within the defined range. Unless defined otherwise, all technical and scientific terms used herein have the same meaning as commonly understood by the ordinary person skilled in the art to which the invention pertains.

The following terms shall, in the present context, have the meaning as indicated:

A "polymorphic marker", sometime referred to as a "marker", as described herein, refers to a genomic polymorphic site. Each polymorphic marker has at least two sequence variations characteristic of particular alleles at the polymorphic site. Thus, genetic association to a polymorphic marker implies that there is association to at least one specific allele of that particular polymorphic marker. The marker can comprise any allele of any variant type found in the genome, including SNPs, mini- or microsatellites, translocations and copy number variations (insertions, deletions, duplications). Polymorphic markers can be of any measurable frequency in the population. For mapping of disease genes, polymorphic markers with population frequency higher than 5-10% are in general most useful. However, polymorphic markers may also have lower population frequencies, such as 1-5% frequency, or even lower frequency, in particular copy number variations (CNVs). The term shall, in the present context, be taken to include polymorphic markers with any population frequency.

An "allele" refers to the nucleotide sequence of a given locus (position) on a chromosome. A polymorphic marker allele thus refers to the composition (i.e., sequence) of the marker on a chromosome. Genomic DNA from an individual contains two alleles (*e.g.,* allele-specific sequences) for any given polymorphic marker, representative of each copy of the marker on each chromosome. Sequence codes for nucleotides used herein are: A = 1, C = 2, G = 3, T = 4. For microsatellite alleles, the CEPH sample (Centre d'Etudes du Polymorphisme Humain, genomics repository, CEPH sample 1347-02) is used as a reference, the shorter allele of each microsatellite in this sample is set as 0 and all other alleles in other samples are numbered in relation to this reference. Thus, *e.g.,* allele 1 is 1 bp longer than the shorter allele in the CEPH sample, allele 2 is 2 bp longer than the shorter allele in the CEPH sample, allele 3 is 3 bp longer than the lower allele in the CEPH sample, etc., and allele -1 is 1 bp shorter than the shorter allele in the CEPH sample, allele -2 is 2 bp shorter than the shorter allele in the CEPH sample, etc.

Sequence conucleotide ambiguity as described herein is as proposed by IUPAC-IUB. These codes are compatible with the codes used by the EMBL, GenBank, and PIR databases.

| IUB code | Meaning |
|---|---|
| A | Adenosine |
| C | Cytidine |
| G | Guanine |
| T | Thymidine |
| R | G or A |
| Y | Tor C |
| K | G or T |
| M | A or C |
| S | G or C |
| W | A or T |
| B | C, G or T |
| D | A, G or T |
| H | A, C or T |
| V | A, C or G |
| N | A, C, G or T (Any base) |

A nucleotide position at which more than one sequence is possible in a population (either a natural population or a synthetic population, *e.g.,* a library of synthetic molecules) is referred to herein as a "polymorphic site".

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

A "variant", as described herein, refers to a segment of DNA that differs from the reference DNA. A "marker" or a "polymorphic marker", as defined herein, is a variant. Alleles that differ from the reference are referred to as "variant" alleles.

A "microsatellite" is a polymorphic marker that has multiple small repeats of bases that are 2-8 nucleotides in length (such as CA repeats) at a particular site, in which the number of repeat lengths varies in the general population. An "indel" is a common form of polymorphism comprising a small insertion or deletion that is typically only a few nucleotides long.

A "haplotype," as described herein, refers to a segment of genomic DNA that is characterized by a specific combination of alleles arranged along the segment. For diploid organisms such as humans, a haplotype comprises one member of the pair of alleles for each polymorphic marker or locus along the segment. In a certain embodiment, the haplotype can comprise two or more alleles, three or more alleles, four or more alleles, or five or more alleles.

Allelic identities are described herein in the context of the marker name and the particular allele of the marker, *e.g.,* "1 rs1058396" refers to the 1 allele of marker rs1058396, and is equivalent to "rs1058396 allele 1". Furthermore, allelic codes are as for individual markers, i.e. 1 = A, 2 = C, 3 = G and 4=T.

The term "susceptibility", as described herein, refers to the proneness of an individual towards the development of a certain state (*e.g.,* a certain trait, phenotype or disease), or towards being less able to resist a particular state than the average individual. The term encompasses both increased susceptibility and decreased susceptibility. Thus, particular alleles at polymorphic markers and/or haplotypes comprising such markers, including those described herein, may be characteristic of increased susceptibility (i.e., increased risk) of urinary bladder cancer, as characterized by a relative risk (RR) or odds ratio (OR) of greater than one for the particular allele or haplotype. Alternatively, the markers and/or haplotypes of the invention are characteristic of decreased susceptibility (i.e., decreased risk) of urinary bladder cancer, as characterized by a relative risk of less than one.

The term "and/or" shall in the present context be understood to indicate that either or both of the items connected by it are involved. In other words, the term herein shall be taken to mean "one or the other or both".

The term "look-up table", as described herein, is a table that correlates one form of data to another form, or one or more forms of data to a predicted outcome to which the data is relevant, such as phenotype or trait. For example, a look-up table can comprise a correlation between allelic data for at least one polymorphic marker and a particular trait or phenotype, such as a particular disease diagnosis, that an individual who comprises the particular allelic data is likely to display, or is more likely to display than individuals who do not comprise the particular allelic data. Look-up tables can be multidimensional, *i.e.* they can contain information about multiple alleles for single markers simultaneously, or they can contain information about multiple markers, and they may also comprise other factors, such as particulars about diseases diagnoses, racial information, biomarkers, biochemical measurements, therapeutic methods or drugs, etc.

A "computer-readable medium", is an information storage medium that can be accessed by a computer using a commercially available or custom-made interface. Exemplary computer-readable media include memory (*e.g.,* RAM, ROM, flash memory, etc.), optical storage media (*e.g.,* CD-ROM), magnetic storage media (*e.g.,* computer hard drives, floppy disks, etc.), punch cards, or other commercially available media. Information may be transferred between a system of interest and a medium, between computers, or between computers and the computer-readable medium for storage or access of stored information. Such transmission can be electrical, or by other available methods, such as IR links, wireless connections, etc.

A "nucleic acid sample" as described herein, refers to a sample obtained from an individual that contains nucleic acid (DNA or RNA). In certain embodiments, i.e. the detection of specific polymorphic markers and/or haplotypes, the nucleic acid sample comprises genomic DNA. Such a nucleic acid sample can be obtained from any source that contains genomic DNA, including a blood sample, sample of amniotic fluid, sample of cerebrospinal fluid, or tissue sample from skin, muscle, buccal or conjunctival mucosa, placenta, gastrointestinal tract or other organs.

The term "UBC therapeutic agent" refers to an agent that can be used to ameliorate or prevent symptoms associated with urinary bladder cancer (UBC).

The term "UBC-associated nucleic acid", as described herein, refers to a nucleic acid that has been found to be associated to urinary bladder cancer. This includes, but is not limited to, the markers and haplotypes described herein and markers and haplotypes in strong linkage disequilibrium (LD) therewith.

The term "antisense agent" or "antisense oligonucleotide" refers, as described herein, to molecules, or compositions comprising molecules, which include a sequence of purine and pyrimidine heterocyclic bases, supported by a backbone, which are effective to hydrogen bond to a corresponding contiguous bases in a target nucleic acid sequence. The backbone is composed of subunit backbone moieties supporting the purine and pyrimidine hetercyclic bases at positions which allow such hydrogen bonding. These backbone moieties are cyclic moieties of 5 to 7 atoms in size, linked together by phosphorous-containing linkage units of one to three atoms in length. In certain preferred embodiments, the antisense agent comprises an oligonucleotide molecule.

The term *"SLC14A1",* as described herein, refers to the Solute Carrier Family 14 (urea transporter), member 1 gene on chromosome 18q12 (pos 41,558,113-41,586,483 in NCBI Build 36 of the human genome assembly). The sequence of the coding region of the gene (Accession No. NM_001128588) is set forth in SEQ ID NO:134.

*Variants conferring risk of urinary bladder cancer*

The present inventors have found that a non-synonymous polymorphic marker rs1058396 (D280N) in the SLC14A gene on chromosome 18q12.3 is predictive of risk of Urinary Bladder Cancer. The A allele of rs1058396 is associated with risk of Bladder cancer with an OR value of 0.87 (95% CI; 0.81-0.93) and a P-value of $1.3x10^{-04}$. Thus, the alternate G allele of rs1058396, which encodes an Aspartic acid (D) at position 280 in the encoded protein (splice variant 1 a shown in SEQ ID NO:209; position 336 in splice variant 2 as shown in SEQ ID NO: 133), is an at-risk allele for Bladder Cancer. The association was replicated in sample sets from the UK, Italy, Belgium, Sweden, Germany and Eastern Europe. The results from the combined analysis of the discovery set and the replication sample sets showed an OR for the A allele of rs1058396 of 0.90 (95% CI; 0.85-0.94), corresponding to an OR of 1.11 for the G allele, and a P-value of $3.7x10^{-05}$.

Association was also noted between UBC and missense mutations within the same gene, SLC14A, namely rs11877062 (R4W) and rs2298720 (K44E in SEQ ID NO:209 E100K in SEQ ID NO: 133). A fourth risk variant was identified in Icelandic samples, rs2298719 (M223V in SEQ ID NO: 133; M167V in SEQ ID NO:209 For these three additional missense variants, the latter amino acid recited (W, E and V for R4W, K44E and M223V, respectively) denotes the amino acid that correlates with increased risk of bladder cancer.

The *SLC14A1* human gene consists of 28393 bases and contains 9 coding Exons. The protein encoded by the *SLC14A1* gene is a membrane transporter that mediates urea transport in erythrocytes but also forms the basis for the Kidd blood group system that is responsible for the inherited blood types. Thus, the Kidd blood group antigens (called JK) are the product of the *SLC14A1* gene. All four markers, i.e. rs1058396, rs11877062, rs2298719 and rs2298720, are missense variants located within the human *SLC14A1* gene. It is possible that these variants affect the physiological function of the SLC14A1 gene product. Thus, these variants, or other missense, nonsense, splice site or truncating variants of SLC14A1 may affect SLC14A1 function. For example, these variants may alter the sequence and thus function of the Kidd blood group antigen, thus affecting the blood group status. Variants in the SLC14A1 gene may also result in JK-null variants that lack expression of the protein on blood cells. Variants in SLC14A may also affect the urea transporting properties of the expressed protein, resulting in impaired capacity of carriers of such variants to concentrate urea in the urine.

*Methods of determining susceptibility to urinary bladder cancer*

Accordingly, the present invention in one aspect provides a method of determining a susceptibility to urinary bladder cancer in a human individual, the method comprising steps of (a) analyzing nucleic acid sequence data from a human individual for at least one polymorphic marker in the human *SLC14A1* gene, wherein different alleles of the at least one polymorphic marker are associated with different susceptibilities to Bladder Cancer in humans, and (b) determining a susceptibility to Bladder Cancer from the nucleic acid sequence data. In certain embodiments, the at least one polymorphic marker is selected from the group consisting of rs1058396, rs11877062, rs2298720 and rs2298719, and markers in linkage disequilibrium therewith. In one preferred embodiments, the at least one polymorphic marker is selected from the group consisting of rs1058396, and markers in linkage disequilibrium therewith. In another preferred embodiments, the at least one polymorphic marker is selected from the group consisting of rs1058396, and markers in linkage disequilibrium therewith, characterized by values of the linkage disequilibrium correlation measure $r^2$ of greater than 0.2 to rs1058396.

The G allele of rs1058396, the C allele of rs11877062, the G allele of rs2298720, and the A allele of rs2298720 are indicative of an increased risk of Bladder Cancer in humans. Thus, in certain embodiment, determination of the presence of at least one allele selected from the group consisting of the G allele of rs1058396, the C allele of rs11877062, the G allele of rs2298720, and the A allele of rs2298720 is indicative of increased risk of Bladder Cancer for the individual. Determination of the absence of any one of these alleles is indicative that the individual does not have the increased risk conferred by the allele.

In certain embodiments of the invention, the allele that is detected can be the allele of the complementary strand of DNA, such that the nucleic acid sequence data includes the identification of at least one allele which is complementary to any of the alleles of the polymorphic markers referenced above. For example, the allele that is detected may be the complementary C allele of the at-risk G allele of rs1058396, the complementary G allele of the at-risk C allele of ras11877062, the complementary C allele of the at-risk G allele of rs2298720, or the complementary T allele to the at-risk A allele of rs2298720.

In certain embodiments, the nucleic acid sequence data is obtained from a biological sample containing nucleic acid from the human individual. The nucleic acids sequence may suitably be obtained using a method that comprises at least one procedure selected from (i) amplification of nucleic acid from the biological sample; (ii) hybridization assay using a nucleic acid probe and nucleic acid from the biological sample; (iii) hybridization assay using a nucleic acid probe and nucleic acid obtained by amplification of the biological sample, and (iv) sequencing, in particular high-throughput sequencing. The nucleic acid sequence data may also be obtained from a preexisting record. For example, the preexisting record may comprise a genotype dataset for at least one polymorphic marker. In certain embodiments, the determining comprises comparing the sequence data to a database containing correlation data between the at least one polymorphic marker and susceptibility to Bladder Cancer.

It is contemplated that in certain embodiments of the invention, it may be convenient to prepare a report of results of risk assessment. Thus, certain embodiments of the methods of the invention comprise a further step of preparing a report containing results from the determination, wherein said report is written in a computer readable medium, printed on paper, or displayed on a visual display. In certain embodiments, it may be convenient to report results of susceptibility to at least one entity selected from the group consisting of the individual, a guardian of the individual, a genetic service provider, a physician, a medical organization, and a medical insurer.

Surrogate markers in linkage disequilibrium with particular key markers can in general be selected based on any particular numerical values of the linkage disequilibrium measures D' and $r^2$, as described further herein. For example, markers that are in linkage disequilibrium with rs1058396 are exemplified by the markers listed in Table 1 herein, but the skilled person will appreciate that other markers in linkage disequilibrium with rs1058396 marker may also be used in the diagnostic applications described herein. As appreciated by the skilled person, other markers in linkage disequilibrium with rs1058396, ras11877062, rs2298720 and/or rs2298719, for example from public databases comprising information about SNP markers in the human genome, may also be selected to realize the present invention. Further, as also described in more detail herein, the skilled person will appreciate that since linkage disequilibrium is a continuous measure, certain values of the LD measures D' and $r^2$ may be suitably chosen to define markers that are useful as surrogate markers in LD with the markers described herein. Numeric values of D' and $r^2$ may thus in certain embodiments be used to define marker subsets that fulfill certain numerical cutoff values of D' and/or $r^2$. In one embodiment, markers in linkage disequilibrium with a particular anchor marker (e.g., rs1058396) are in LD with the anchor marker characterized by numerical values of D' of greater than 0.8 and/or numerical values of $r^2$ of greater than 0.2. In one embodiment, markers in linkage disequilibrium with a particular anchor marker are in LD with the anchor marker characterized by numerical values of $r^2$ of greater than 0.2. For example, the markers provided in Table 1 provide exemplary markers that fulfill this criterion. In other embodiments, markers in linkage disequilibrium with a particular anchor marker are in LD with the anchor marker characterized by numerical values of $r^2$ of greater than 0.3, greater than 0.4, greater than 0.5, greater than 0.6, greater than 0.7, greater than 0.8, greater than 0.9, greater than 0.95. Other numerical values of $r^2$ and/or D' may also be suitably selected to select markers that are in LD with the anchor marker. The stronger the LD, the more similar the association signal and/or the predictive risk by the surrogate marker will be to that of the anchor marker. Markers with values of $r^2 = 1$ to the anchor marker are perfect surrogates of the anchor marker and will provide identical association and risk prediction data. In one preferred embodiment, surrogate markers of rs1058396 are those markers that have values of $r^2$ to rs1058396 of greater than 0.2. In another preferred embodiment, surrogate markers of rs1058396 are those markers that have values of $r^2$ to rs1058396 of greater than 0.5. In another preferred embodiment, surrogate markers of rs1058396 are those markers that have values of $r^2$ to rs1058396 of greater than 0.8 Further, as described in more detail in the following, LD may be determined in samples from any particular population. In one embodiment, LD is determined in Caucasian samples. In another embodiment, LD is determined in European samples. In another embodiment, LD is determined in Icelandic samples. In other embodiments, LD is determined in African American samples, in Asian samples, or the LD may be suitably determined in samples of any other population.

**Table 1.** Surrogate markers of marker rs1058396 on Chromosome 18q12.3. Markers were identified based on sequence data from an Icelandic dataset comprising whole-genome sequences of approximately 300 individuals (A) and from an additional 800 individuals (B). Shown are the marker names, their location in NCBI build 36, numerical values of the linkage disequilibrium measure $r^2$ to rs1058396, the risk alleles for the surrogate markers, i.e. alleles that are correlated with the at-risk G allele of the anchor marker rs1058396, and the other allele, and lastly a sequence listing number, identifying the flanking sequence of each particular surrogate marker.

| **A** | | | | | |
|---|---|---|---|---|---|
| **Surrogate marker** | **Pos in NCBI Build 36** | **$R^2$** | **Risk allele** | **Other allele** | **Seq ID NO** |
| rs7233769 | 41560679 | 0.923 | A | G | 1 |
| rs3819177 | 41570108 | 0.953 | T | C | 2 |
| rs692899 | 41570268 | 0.537 | C | T | 3 |
| rs1058396 | 41573517 | 1 | G | A | 4 |
| rs11660575 | 41574436 | 0.829 | A | G | 5 |
| rs7234986 | 41574602 | 0.874 | A | G | 6 |
| rs564409 | 41574712 | 0.461 | C | G | 7 |
| rs565153 | 41574769 | 0.497 | T | G | 8 |

(continued)

| A | | | | | |
|---|---|---|---|---|---|
| **Surrogate marker** | **Pos in NCBI Build 36** | **$R^2$** | **Risk allele** | **Other allele** | **Seq ID NO** |
| ras11082468 | 41575026 | 0.873 | A | G | 9 |
| rs11082469 | 41575267 | 0.879 | A | G | 10 |
| rs8086499 | 41575410 | 0.859 | A | G | 11 |
| rs12454680 | 41575415 | 0.81 | T | C | 12 |
| rs8086631 | 41575440 | 0.894 | C | T | 13 |
| rs56044725 | 41575561 | 0.895 | A | G | 14 |
| rs8087320 | 41575759 | 0.932 | G | C | 15 |
| rs12962485 | 41576408 | 0.44 | T | C | 16 |
| rs28897968 | 41576485 | 0.606 | G | C | 17 |
| rs493262 | 41577622 | 0.475 | G | A | 18 |
| rs6507640 | 41578062 | 0.663 | A | G | 19 |
| rs1682392 | 41579157 | 0.472 | G | A | 20 |
| rs4890588 | 41581292 | 0.469 | C | T | 21 |
| rs474270 | 41582293 | 0.464 | A | G | 22 |
| rs2282616 | 41582632 | 0.486 | G | A | 23 |
| rs2282615 | 41582748 | 0.463 | G | C | 24 |
| rs6507641 | 41583196 | 0.515 | A | G | 25 |
| rs17142 | 41583308 | 0.478 | A | G | 26 |
| rs3745006 | 41583588 | 0.687 | T | G | 27 |
| rs9954521 | 41584894 | 0.604 | T | A | 28 |
| rs1135980 | 41585281 | 0.433 | C | T | 29 |
| rs28903070* | 41585749 | 0.436 | T | A | 30 |
| rs3087560 | 41586253 | 0.63 | C | T | 31 |
| rs3178156 | 41586478 | 0.635 | A | C | 32 |
| rs11662680 | 41587183 | 0.585 | A | G | 33 |
| rs7359740 | 41587911 | 0.63 | G | A | 34 |
| rs1944336 | 41588324 | 0.477 | G | T | 35 |
| rs8090136 | 41588762 | 0.497 | C | T | 36 |
| rs8090390 | 41588774 | 0.494 | G | T | 37 |
| rs8090267 | 41588803 | 0.477 | C | T | 38 |
| rs11874337 | 41589256 | 0.395 | T | A | 39 |
| rs9953451 | 41589691 | 0.445 | T | C | 40 |
| rs9966818 | 41589897 | 0.458 | C | T | 41 |
| rs534637 | 41590141 | 0.489 | A | G | 42 |
| rs900970 | 41590556 | 0.442 | T | C | 43 |
| rs9947769 | 41591565 | 0.519 | G | T | 44 |

(continued)

| A | | | | | |
|---|---|---|---|---|---|
| **Surrogate marker** | **Pos in NCBI Build 36** | **$R^2$** | **Risk allele** | **Other allele** | **Seq ID NO** |
| rs4890300 | 41591993 | 0.459 | G | A | 45 |
| rs9951207 | 41592624 | 0.511 | G | A | 46 |
| rs9963415 | 41592825 | 0.502 | T | C | 47 |
| rs8096228 | 41593025 | 0.523 | C | T | 48 |
| rs59932916 | 41593069 | 0.429 | C | A | 49 |
| rs8096392 | 41593219 | 0.447 | A | G | 50 |
| rs572858 | 41594516 | 0.523 | G | A | 51 |
| rs2005378 | 41595420 | 0.569 | G | C | 52 |
| rs550201 | 41595770 | 0.526 | A | C | 53 |
| rs576687 | 41595792 | 0.569 | C | G | 54 |
| rs10502870 | 41596131 | 0.569 | A | G | 55 |
| rs3018180 | 41596964 | 0.268 | T | C | 56 |
| rs1625960 | 41597012 | 0.526 | G | A | 57 |
| rs1625985 | 41597016 | 0.532 | T | C | 58 |
| rs1626743 | 41597080 | 0.491 | A | G | 59 |
| rs1789558 | 41597184 | 0.538 | A | G | 60 |
| rs475584 | 41597415 | 0.559 | C | G | 61 |
| rs517221 | 41597628 | 0.557 | A | G | 62 |
| rs502339 | 41597987 | 0.523 | A | G | 63 |
| rs505060 | 41598280 | 0.545 | T | C | 64 |
| rs9959600 | 41600337 | 0.206 | G | T | 65 |
| rs9960093 | 41600802 | 0.212 | G | T | 66 |
| rs9959923 | 41600815 | 0.234 | C | A | 67 |
| rs2187408 | 41600839 | 0.208 | C | T | 68 |
| rs7237369 | 41601336 | 0.203 | C | T | 69 |
| rs7237823 | 41601433 | 0.217 | G | A | 70 |
| rs1115074 | 41612293 | 0.257 | A | G | 71 |
| rs1944333 | 41613122 | 0.232 | A | G | 72 |
| rs7506509 | 41613925 | 0.238 | T | C | 73 |
| rs1789553 | 41614138 | 0.237 | C | T | 74 |
| rs2187405 | 41617419 | 0.236 | C | G | 75 |
| rs539249 | 41618120 | 0.247 | A | C | 76 |
| rs4890301 | 41622267 | 0.25 | C | T | 77 |
| rs7241939 | 41623325 | 0.294 | G | A | 78 |
| rs7241734 | 41623487 | 0.275 | A | G | 79 |
| rs8083889 | 41623910 | 0.284 | G | T | 80 |

(continued)

| A | | | | | |
|---|---|---|---|---|---|
| Surrogate marker | Pos in NCBI Build 36 | $R^2$ | Risk allele | Other allele | Seq ID NO |
| rs4890592 | 41624961 | 0.298 | G | A | 81 |
| rs495078 | 41663873 | 0.273 | T | C | 82 |
| rs7230514 | 41626329 | 0.228 | T | A | 83 |
| rs2156610 | 41626469 | 0.237 | A | G | 84 |
| rs1944340 | 41626853 | 0.266 | G | C | 85 |
| rs4362470 | 41627762 | 0.282 | G | A | 86 |
| rs9953356 | 41628618 | 0.209 | G | T | 87 |
| rs9955590 | 41628958 | 0.264 | A | T | 88 |
| rs9956040 | 41629414 | 0.215 | A | G | 89 |
| rs11082474 | 41630283 | 0.277 | T | A | 90 |
| rs11082475 | 41630288 | 0.274 | G | A | 91 |
| rs7233627 | 41630717 | 0.252 | T | C | 92 |
| rs12457954 | 41631576 | 0.222 | A | G | 93 |
| rs12457989 | 41631771 | 0.282 | A | C | 94 |
| rs8084937 | 41633341 | 0.299 | A | G | 95 |
| rs8085076 | 41633410 | 0.271 | A | G | 96 |
| rs4890593 | 41634005 | 0.281 | C | T | 97 |
| rs559774 | 41634834 | 0.261 | A | G | 98 |
| rs4890594 | 41635463 | 0.257 | C | G | 99 |
| rs4890302 | 41635510 | 0.285 | G | A | 100 |
| rs7237600 | 41636812 | 0.267 | T | G | 101 |
| rs8095840 | 41637106 | 0.261 | G | A | 102 |
| rs6507646 | 41637703 | 0.254 | G | A | 103 |
| rs4890596 | 41638223 | 0.237 | C | T | 104 |
| rs1944339 | 41639040 | 0.292 | C | A | 105 |
| rs1944338 | 41639170 | 0.264 | G | A | 106 |
| rs484914 | 41639267 | 0.235 | T | C | 107 |
| rs563386 | 41640930 | 0.246 | C | G | 108 |
| rs9966999 | 41641160 | 0.247 | T | C | 109 |
| rs536784 | 41641545 | 0.276 | A | C | 110 |
| rs576309 | 41642213 | 0.244 | G | A | 111 |
| rs546739 | 41643152 | 0.207 | T | C | 112 |
| rs545070 | 41643303 | 0.232 | T | A | 113 |
| rs553007 | 41643483 | 0.254 | C | T | 114 |
| rs521133 | 41643641 | 0.231 | A | G | 115 |
| rs515762 | 41644205 | 0.241 | A | T | 116 |

(continued)

| A | | | | | |
|---|---|---|---|---|---|
| Surrogate marker | Pos in NCBI Build 36 | $R^2$ | Risk allele | Other allele | Seq ID NO |
| rs693488 | 41644637 | 0.225 | A | G | 117 |
| rs1789557 | 41644754 | 0.209 | A | C | 118 |
| rs1789556 | 41645163 | 0.282 | A | C | 119 |
| rs9966400 | 41646511 | 0.257 | T | A | 120 |
| rs9748917 | 41646575 | 0.225 | T | C | 121 |
| rs532613 | 41647517 | 0.27 | A | G | 122 |
| rs504030 | 41648373 | 0.226 | A | G | 123 |
| rs573463 | 41648521 | 0.212 | T | C | 124 |
| rs498545 | 41649711 | 0.245 | A | T | 125 |
| rs538405 | 41653433 | 0.238 | A | C | 126 |
| rs503331 | 41654092 | 0.241 | A | G | 127 |
| rs489653 | 41658448 | 0.218 | C | T | 128 |
| rs495078 | 41663873 | 0.279 | T | C | 129 |
| B | | | | | |
| Marker | Build36 Position | $R^2$ | Risk allele | Other allele | Seq ID NO |
| rs10460033 | 41557200 | 0.229969 | A | T | 135 |
| rs10460072 | 41557208 | 0.225427 | G | A | 136 |
| rs12963143 | 41557685 | 0.32201 | A | C | 137 |
| rs16978469 | 41559783 | 0.923926 | G | A | 138 |
| rs7234310 | 41560791 | 0.920508 | G | A | 139 |
| rs10432193 | 41561070 | 0.923207 | T | C | 140 |
| ras11877062 | 41561244 | 0.921977 | C | T | 130 |
| rs11877086 | 41561336 | 0.918935 | C | A | 141 |
| rs9304321 | 41562186 | 0.921852 | C | T | 142 |
| rs9304322 | 41562196 | 0.921757 | G | T | 143 |
| rs9304323 | 41562313 | 0.923237 | A | G | 144 |
| rs2170974 | 41562808 | 0.923788 | A | T | 145 |
| rs9967412 | 41562887 | 0.924013 | C | G | 146 |
| rs4479340 | 41562953 | 0.923288 | C | T | 147 |
| rs4316845 | 41563133 | 0.923826 | G | T | 148 |
| rs4310958 | 41563353 | 0.922973 | C | G | 149 |
| rs8087241 | 41563701 | 0.921248 | G | A | 150 |
| rs8088163 | 41563807 | 0.921677 | T | C | 151 |
| rs17674580 | 41563909 | 0.489916 | T | C | 152 |
| rs8090908 | 41564185 | 0.923912 | A | G | 153 |
| rs3819178 | 41564843 | 0.922224 | G | C | 154 |

(continued)

| B | | | | | |
|---|---|---|---|---|---|
| Marker | Build36 Position | $R^2$ | Risk allele | Other allele | Seq ID NO |
| rs12963324 | 41564849 | 0.488927 | A | G | 155 |
| rs17674709 | 41565378 | 0.92356 | C | G | 156 |
| rs10460034 | 41565397 | 0.923926 | C | T | 157 |
| rs10460036 | 41565564 | 0.923423 | A | G | 158 |
| rs8099449 | 41565694 | 0.921686 | T | C | 159 |
| rs7229967 | 41566366 | 0.923509 | G | A | 160 |
| rs7229753 | 41566458 | 0.923926 | A | G | 161 |
| rs7230298 | 41566517 | 0.924184 | G | A | 162 |
| rs9946832 | 41566788 | 0.922155 | A | G | 163 |
| rs9946998 | 41566820 | 0.923729 | C | T | 164 |
| rs12455090 | 41567365 | 0.924213 | C | T | 165 |
| rs8096571 | 41568471 | 0.923711 | T | A | 166 |
| rs8095657 | 41568543 | 0.922352 | C | T | 167 |
| rs8083653 | 41569025 | 0.922692 | T | C | 168 |
| rs28898869 | 41569589 | 0.961109 | A | C | 169 |
| rs2298718 | 41570536 | 0.813038 | A | G | 170 |
| rs7238033 | 41570964 | 0.817394 | T | C | 171 |
| rs493363 | 41571247 | 0.531684 | A | G | 172 |
| rs10775480 | 41571280 | 0.819409 | T | C | 173 |
| rs11082466 | 41571526 | 0.817711 | C | T | 174 |
| rs10853535 | 41571545 | 0.817664 | C | T | 175 |
| rs11877028 | 41571864 | 0.999609 | C | G | 176 |
| rs11877630 | 41571975 | 0.999789 | A | G | 177 |
| rs17675121 | 41572183 | 0.997932 | T | C | 178 |
| rs11877720 | 41572253 | 0.999008 | A | G | 179 |
| rs11082467 | 41572642 | 0.99972 | T | C | 180 |
| rs9955503 | 41572692 | 0.660708 | A | T | 181 |
| rs11665385 | 41572744 | 0.999865 | G | A | 182 |
| rs473429 | 41573058 | 0.658081 | C | T | 183 |
| rs55723051 | 41585321 | 0.229219 | C | A | 184 |
| rs11082471 | 41599614 | 0.23525 | C | T | 185 |
| rs11082472 | 41599703 | 0.307919 | T | A | 186 |
| rs35702919 | 41599705 | 0.331755 | T | A | 187 |
| rs2156611 | 41599862 | 0.235398 | C | T | 188 |
| rs2156612 | 41599877 | 0.23274 | C | T | 189 |
| rs2187406 | 41600017 | 0.233978 | G | T | 190 |
| rs2187407 | 41600038 | 0.234041 | C | T | 191 |

(continued)

| B | | | | | |
|---|---|---|---|---|---|
| **Marker** | **Build36 Position** | **$R^2$** | **Risk allele** | **Other allele** | **Seq ID NO** |
| rs12457207 | 41600113 | 0.234179 | G | C | 192 |
| rs9959453 | 41600347 | 0.230845 | C | T | 193 |
| rs9948723 | 41600462 | 0.2353 | T | G | 194 |
| rs9959480 | 41600467 | 0.235028 | A | G | 195 |
| rs9948733 | 41600486 | 0.235677 | T | C | 196 |
| rs2187409 | 41600896 | 0.231358 | T | C | 197 |
| rs7237218 | 41601280 | 0.225405 | C | T | 198 |
| rs7237722 | 41630942 | 0.278823 | T | C | 199 |
| rs7236814 | 41630943 | 0.268958 | G | A | 200 |
| rs7236744 | 41631138 | 0.30923 | A | C | 201 |
| rs7237029 | 41631229 | 0.311759 | C | G | 202 |
| rs6507645 | 41635799 | 0.363613 | T | C | 203 |
| rs9954025 | 41646268 | 0.320274 | G | A | 204 |
| rs1440822 | 41659790 | 0.216897 | T | C | 205 |
| rs515373 | 41660929 | 0.214746 | G | A | 206 |
| rs12326162 | 41663680 | 0.234552 | C | T | 207 |
| rs4890303 | 41675437 | 0.205304 | C | T | 208 |
| *Also known as rs544373 | | | | | |

Measures of susceptibility or risk include measures such as relative risk (RR), odds ratio (OR), and absolute risk (AR), as described in more detail herein.

In certain embodiments, increased susceptibility refers to a risk with values of RR or OR of at least 1.10, at least 1.11, at least 1.12, at least 1.13, at least 1.14, at least 1.15, at least 1.16, at least 1.17, at least 1.18, at least 1.19, at least 1.20, at least 1.21, at least 1.22, at least 1.23, at least 1.24, at least 1.25, at least 1.30, at least 1.35, at least 1.40, at least 1.45, at least 1.50, at least 1.55, at least 1.60, at least 1.65, at least 1.70, at least 1.75, and/or at least 1.80. Other numerical non-integer values greater than unity are also possible to characterize the risk, and such numerical values are also within scope of the invention. Certain embodiments relate to homozygous individuals for a particular marker, *i.e.* individuals who carry two copies of the same allele in their genome. One preferred embodiment relates to individuals who are homozygous carriers of the A allele of rs1058396, or a marker allele in linkage disequilibrium therewith.

In certain other embodiments, determination of the presence of particular marker alleles or particular haplotypes is predictive of a decreased susceptibility of urinary bladder cancer in humans. For SNP markers with two alleles, the alternate allele to an at-risk allele will be in decreased frequency in patients compared with controls. Thus, determination of the presence of the alternate allele is indicative of a decreased susceptibility of urinary bladder cancer. Individuals who are homozygous for the alternate (protective) allele are at particularly decreased susceptibility or risk.

To identify markers that are useful for assessing susceptibility to urinary bladder cancer, it may be useful to compare the frequency of markers alleles in individuals with urinary bladder cancer to control individuals. The control individuals may be a random sample from the general population, i.e. a population cohort. The control individuals may also be a sample from individuals that are disease-free, e.g. individuals who have been confirmed not to have urinary bladder cancer. In one embodiment, an increase in frequency of at least one allele in at least one polymorphism in individuals diagnosed with urinary bladder cancer, as compared with the frequency of the at least one allele in the control group is indicative of the at least one allele being useful for assessing increased susceptibility to urinary bladder cancer. In another embodiment, a decrease in frequency of at least one allele in at least one polymorphism in individuals diagnosed with urinary bladder cancer, as compared with the frequency of the at least one allele in the control sample is indicative of the at least one allele being useful for assessing decreased susceptibility to, or protection against, urinary bladder cancer.

In general, sequence data can be obtained by analyzing a sample from an individual, or by analyzing information about specific markers in a database or other data collection, for example a genotype database or a sequence database. The sample is in certain embodiments a nucleic acid sample, or a sample that contains nucleic acid material. Analyzing a sample from an individual may in certain embodiments include steps of isolating genomic nucleic acid from the sample, amplifying a segment of the genomic nucleic acid that contains at least one polymorphic marker, and determine sequence information about the at least one polymorphic marker. Amplification is preferably performed by Polymerase Chain Reaction (PCR) techniques. In certain embodiments, sequence data can be obtained through nucleic acid sequence information or amino acid sequence information from a preexisting record. Such a preexisting record can be any documentation, database or other form of data storage containing such information.

Determination of a susceptibility or risk of a particular individual in general comprises comparison of the genotype information (sequence information) to a record or database providing a correlation about particular polymorphic marker(s) and susceptibility to disease, such as urinary bladder cancer. Thus, in specific embodiments, determining a susceptibility comprises comparing the sequence data to a database containing correlation data between the at least one polymorphic marker and susceptibility to urinary bladder cancer. In certain embodiments, the database comprises at least one measure of susceptibility to urinary bladder cancer for the at least one polymorphic marker. In certain embodiments, the database comprises a look-up table comprising at least one measure of susceptibility to urinary bladder cancer for the at least one polymorphic marker. The measure of susceptibility may in the form of relative risk (RR), absolute risk (AR), percentage (%) or other convenient measure for describing genetic susceptibility of individuals.

In certain embodiments of the invention, more than one polymorphic marker is analyzed. In certain embodiments, at least two polymorphic markers are analyzed. Thus, in certain embodiments, sequence data about at least two polymorphic markers is obtained.

In certain embodiments, a further step of analyzing at least one haplotype comprising two or more polymorphic markers is included. Any convenient method for haplotype analysis known to the skilled person may be employed in such embodiments.

One aspect of the invention relates to a method for determining a susceptibility to urinary bladder cancer in a human individual, comprising determining the presence or absence of at least one allele of at least one polymorphic marker in a nucleic acid sample obtained from the individual, or in a genotype dataset from the individual, wherein the at least one polymorphic marker is selected from the group consisting of rs1058396, and markers in linkage disequilibrium therewith, and wherein determination of the presence of the at least one allele is indicative of a susceptibility to urinary bladder cancer. Determination of the presence of an allele that correlates with urinary bladder cancer is indicative of an increased susceptibility to urinary bladder cancer. Individuals who are homozygous for such alleles are particularly susceptible (*i.e.,* at particularly high risk) to urinary bladder cancer. On the other hand, individuals who do not carry such at-risk alleles are at a decreased susceptibility of developing urinary bladder cancer, as compared with a random sample from the population. For SNPs, such individuals will be homozygous for the alternate (protective) allele of the polymorphism.

Determination of susceptibility is in some embodiments reported by a comparison with non-carriers of the at-risk allele(s) of polymorphic markers. In certain embodiments, susceptibility is reported based on a comparison with the general population, *e.g.* compared with a random selection of individuals from the population.

Another aspect of the methods of the invention relates to a method of determining susceptibility to bladder cancer, the method comprising analyzing nucleic acid sequence data representative of at least one allele of the SLC14A1 gene in a human subject, wherein different alleles of the SLC14A1 gene are associated with different susceptibilities to bladder cancer in humans, and determining a susceptibility to bladder cancer for the human subject from the data. In certain embodiments, the analyzing nucleic acid sequence data comprises analyzing a biological sample from the human subject to obtain information selected from the group consisting of (a) nucleic acid sequence information, wherein the nucleic acid sequence information comprises sequence sufficient to identify the presence or absence of at least one allele of the SLC14A1 gene in the subject; (b) nucleic acid sequence information, wherein the nucleic acid sequence information identifies at least one allele of a polymorphic marker in linkage disequilibrium (LD) with an SLC14A1 allele associated with bladder cancer in humans, wherein the LD is characterized by a value for $r^2$ of at least 0.2; (c) measurement of the quantity or length of SLC14A1 mRNA, wherein the measurement is indicative of the presence or absence of the allele; (d) measurement of the quantity of SLC14A1 protein, wherein the measurement is indicative of the presence or absence of the allele; and (e) measurement of SLC14A1 activity, wherein the measurement is indicative of the presence or absence of the allele. The SLC14A1 activity may for example be urea transport or urea binding activity. The SLC14A1 activity may also be identity of the Kidd blood group status of an individual expressing SLC14A1 protein containing the allele. In certain embodiments, the method further comprises obtaining a biological sample comprising nucleic acid from the human subject. In certain embodiments, the analyzing data may comprise analyzing data from a preexisting record about the human subject.

*Obtaining nucleic acid sequence data*

Sequence data can be nucleic acid sequence data or protein sequence data, which may be obtained by means known in the art. Nucleic acid sequence data is suitably obtained from a biological sample of genomic DNA, RNA, or cDNA (a "test sample") from an individual ("test subject). For example, nucleic acid sequence data may be obtained through direct analysis of the sequence of the polymorphic position (allele) of a polymorphic marker. Suitable methods, some of which are described herein, include, for instance, whole genome sequencing methods, whole genome analysis using SNP chips (e.g., Infinium HD BeadChip), cloning for polymorphisms, non-radioactive PCR-single strand conformation polymorphism analysis, denaturing high pressure liquid chromatography (DHPLC), DNA hybridization, computational analysis, single-stranded conformational polymorphism (SSCP), restriction fragment length polymorphism (RFLP), automated fluorescent sequencing; clamped denaturing gel electrophoresis (CDGE); denaturing gradient gel electrophoresis (DGGE), mobility shift analysis, restriction enzyme analysis; heteroduplex analysis, chemical mismatch cleavage (CMC), RNase protection assays, use of polypeptides that recognize nucleotide mismatches, such as E. coli mutS protein, allele-specific PCR, and direct manual and automated sequencing. These and other methods are desribed in the art (see, for instance, Li et al., Nucleic Acids Research, 28(2): e1 (i-v) (2000); Liu et al., Biochem Cell Bio 80:17-22 (2000); and Burczak et al., Polymorphism Detection and Analysis, Eaton Publishing, 2000; Sheffield et al., Proc. Natl. Acad. Sci. USA, 86:232-236 (1989); Orita et al., Proc. Natl. Acad. Sci. USA, 86:2766-2770 (1989); Flavell et al., Cell, 15:25-41 (1978); Geever et al., Proc. Narl. Acad. Sci. USA, 78:5081-5085 (1981); Cotton et al., Proc. Natl. Acad. Sci. USA, 85:4397-4401 (1985); Myers et al., Science 230:1242-1246 (1985); Church and Gilbert, Proc. Natl. Acad. Sci. USA, 81:1991-1995 (1988); Sanger et al., Proc. Natl. Acad. Sci. USA, 74:5463-5467 (1977); and Beavis et al., U.S. Patent No. 5,288,644).

Recent technological advances have resulted in technologies that allow massive parallel sequencing to be performed in relatively condensed format. These technologies share sequencing-by-synthesis principle for generating sequence information, with different technological solutions implemented for extending, tagging and detecting sequences. Exemplary technologies include 454 pyrosequencing technology (Nyren, P. et al. Anal Biochem 208:171-75 (1993); http://www.454.com), Illumina Solexa sequencing technology (Bentley, D.R. Curr Opin Genet Dev 16:545-52 (2006); http://www.illumina.com), and the SOLiD technology developed by Applied Biosystems (ABI) (http://www.appliedbiosystems.com; see also Strausberg, R.L., et al. Drug Disc Today 13:569-77 (2008)). Other sequencing technologies include those developed by Pacific Biosciences (http://www.pacificbiosciences.com), Complete Genomics (http://www.completegenomics.com), Intelligen Bio-Systems (http://www.intelligentbiosystems.com), Genome Corp (http://www.genomecorp.com), ION Torrent Systems (http://www.iontorrent.com) and Helicos Biosciences (http://www.helicosbio.som). It is contemplated that sequence data useful for performing the present invention may be obtained by any such sequencing method, or other sequencing methods that are developed or made available. Thus, any sequence method that provides the allelic identity at particular polymorphic sites (*e.g.,* the absence or presence of particular alleles at particular polymorphic sites) is useful in the methods described and claimed herein.

Alternatively, hybridization methods may be used (see Current Protocols in Molecular Biology, Ausubel et al., eds., John Wiley & Sons, including all supplements). For example, a biological sample of genomic DNA, RNA, or cDNA (a "test sample") may be obtained from a test subject. The subject can be an adult, child, or fetus. The DNA, RNA, or cDNA sample is then examined. The presence of a specific marker allele can be indicated by sequence-specific hybridization of a nucleic acid probe specific for the particular allele. The presence of more than one specific marker allele or a specific haplotype can be indicated by using several sequence-specific nucleic acid probes, each being specific for a particular allele. A sequence-specific probe can be directed to hybridize to genomic DNA, RNA, or cDNA. A "nucleic acid probe", as used herein, can be a DNA probe or an RNA probe that hybridizes to a complementary sequence. One of skill in the art would know how to design such a probe so that sequence specific hybridization will occur only if a particular allele is present in a genomic sequence from a test sample.

To diagnose a susceptibility to Bladder Cancer, a hybridization sample can be formed by contacting the test sample, such as a genomic DNA sample, with at least one nucleic acid probe. A nonlimiting example of a probe for detecting mRNA or genomic DNA is a labeled nucleic acid probe that is capable of hybridizing to mRNA or genomic DNA sequences described herein. The nucleic acid probe can be, for example, a full-length nucleic acid molecule, or a portion thereof, such as an oligonucleotide of at least 10, 15, 30, 50, 100, 250 or 500 nucleotides in length that is sufficient to specifically hybridize under stringent conditions to appropriate mRNA or genomic DNA. For example, the nucleic acid probe can comprise all or a portion of the nucleotide sequence of the *SLC14A1* gene, or the probe can be the complementary sequence of such a sequence. Hybridization can be performed by methods well known to the person skilled in the art (see, e.g., Current Protocols in Molecular Biology, Ausubel et al., eds., John Wiley & Sons, including all supplements). In one embodiment, hybridization refers to specific hybridization, i.e., hybridization with no mismatches (exact hybridization). In one embodiment, the hybridization conditions for specific hybridization are high stringency.

Specific hybridization, if present, is detected using standard methods. If specific hybridization occurs between the nucleic acid probe and the nucleic acid in the test sample, then the sample contains the allele that is complementary to the nucleotide that is present in the nucleic acid probe.

Additionally, or alternatively, a peptide nucleic acid (PNA) probe can be used in addition to, or instead of, a nucleic acid probe in the hybridization methods described herein. A PNA is a DNA mimic having a peptide-like, inorganic backbone, such as N-(2-aminoethyl)glycine units, with an organic base (A, G, C, T or U) attached to the glycine nitrogen via a methylene carbonyl linker (see, for example, Nielsen et al., Bioconjug. Chem. 5:3-7 (1994)). The PNA probe can be designed to specifically hybridize to a molecule in a sample suspected of containing one or more of the marker alleles or haplotypes that are associated with eosinophilia, asthma, myocardial infarction, and/or hypertension.

In one embodiment of the invention, a test sample containing genomic DNA obtained from the subject is collected and the polymerase chain reaction (PCR) is used to amplify a fragment comprising one or more polymorphic marker. As described herein, identification of particular marker alleles can be accomplished using a variety of methods. In another embodiment, determination of a susceptibility is accomplished by expression analysis, for example using quantitative PCR (kinetic thermal cycling). This technique can, for example, utilize commercially available technologies, such as TaqMan® (Applied Biosystems, Foster City, CA) . The technique can for example assess the presence of an alteration in the expression or composition of a polypeptide or splicing variant(s) that is encoded by a nucleic acid associated described herein. Alternatively, this technique may assess expression levels of genes or particular splice variants of genes, that are affected by one or more of the variants described herein. Further, the expression of the variant(s) can be quantified as physically or functionally different.

Allele-specific oligonucleotides can also be used to detect the presence of a particular allele in a nucleic acid. An "allele-specific oligonucleotide" (also referred to herein as an "allele-specific oligonucleotide probe") is an oligonucleotide of any suitable size, for example an oligonucleotide of approximately 10-50 base pairs or approximately 15-30 base pairs, that specifically hybridizes to a nucleic acid which contains a specific allele at a polymorphic site (e.g., a polymorphic marker). An allele-specific oligonucleotide probe that is specific for one or more particular alleles at polymorphic markers can be prepared using standard methods (see, e.g., *Current Protocols in Molecular Biology, supra*). PCR can be used to amplify the desired region. Specific hybridization of an allele-specific oligonucleotide probe to DNA from a subject is indicative of the presence of a specific allele at a polymorphic site (see, e.g., Gibbs et al., Nucleic Acids Res. 17:2437-2448 (1989) and WO 93/22456).

With the addition of analogs such as locked nucleic acids (LNAs), the size of primers and probes can be reduced to as few as 8 bases. LNAs are a novel class of bicyclic DNA analogs in which the 2' and 4' positions in the furanose ring are joined via an O-methylene (oxy-LNA), S-methylene (thio-LNA), or amino methylene (amino-LNA) moiety. Common to all of these LNA variants is an affinity toward complementary nucleic acids, which is by far the highest reported for a DNA analog. For example, particular all oxy-LNA nonamers have been shown to have melting temperatures (Tm) of 64°C and 74°C when in complex with complementary DNA or RNA, respectively, as opposed to 28°C for both DNA and RNA for the corresponding DNA nonamer. Substantial increases in Tm are also obtained when LNA monomers are used in combination with standard DNA or RNA monomers. For primers and probes, depending on where the LNA monomers are included (e.g., the 3' end, the 5' end, or in the middle), the Tm could be increased considerably. It is therefore contemplated that in certain embodiments, LNAs are used to detect particular alleles at polymorphic sites associated with particular vascular conditions, as described herein.

In certain embodiments, arrays of oligonucleotide probes that are complementary to target nucleic acid sequence segments from a subject, can be used to identify polymorphisms in a nucleic acid. For example, an oligonucleotide array can be used. Oligonucleotide arrays typically comprise a plurality of different oligonucleotide probes that are coupled to a surface of a substrate in different known locations. These arrays can generally be produced using mechanical synthesis methods or light directed synthesis methods that incorporate a combination of photolithographic methods and solid phase oligonucleotide synthesis methods, or by other methods known to the person skilled in the art (see, e.g., Bier et al., Adv Biochem Eng Biotechnol 109:433-53 (2008); Hoheisel, Nat Rev Genet 7:200-10 (2006); Fan et al., Methods Enzymol 410:57-73 (2006); Raqoussis & Elvidge, Expert Rev Mol Diagn 6:145-52 (2006); Mockler et al., Genomics 85:1-15 (2005), and references cited therein). Many additional descriptions of the preparation and use of oligonucleotide arrays for detection of polymorphisms can be found, for example, in US 6,858,394, US 6,429,027, US 5,445,934, US 5,700,637, US 5,744,305, US 5,945,334, US 6,054,270, US 6,300,063, US 6,733,977, US 7,364,858, EP 619 321, and EP 373 203.

Also, standard techniques for genotyping can be used to detect particular marker alleles, such as fluorescence-based techniques (*e.g.,* Chen et al., Genome Res. 9(5): 492-98 (1999); Kutyavin et al., Nucleic Acid Res. 34:e128 (2006)), utilizing PCR, LCR, Nested PCR and other techniques for nucleic acid amplification. Specific commercial methodologies available for SNP genotyping include, but are not limited to, TaqMan genotyping assays and SNPlex platforms (Applied Biosystems), gel electrophoresis (Applied Biosystems), mass spectrometry (e.g., MassARRAY system from Sequenom), minisequencing methods, real-time PCR, Bio-Plex system (BioRad), CEQ and SNPstream systems (Beckman), array hybridization technology(e.g., Affymetrix GeneChip; Perlegen ), BeadArray Technologies (e.g., Illumina GoldenGate and Infinium assays), array tag technology (*e.g.,* Parallele), and endonuclease-based fluorescence hybridization technology (Invader; Third Wave).

Suitable biological sample in the methods described herein can be any sample containing nucleic acid (e.g., genomic DNA) and/or protein from the human individual. For example, the biological sample can be a blood sample, a serum sample, a leukapheresis sample, an amniotic fluid sample, a cerbrospinal fluid sample, a hair sample, a tissue sample from skin, muscle, buccal, or conjuctival mucosa, placenta, gastrointestinal tract, or other organs, a semen sample, a urine sample, a saliva sample, a nail sample, a tooth sample, and the like. Preferably, the sample is a blood sample, a salive sample or a buccal swab.

*Protein analysis*

Missense nucleic acid variations may lead to an altered amino acid sequence, as compared to the non-variant (e.g., wild-type) protein, due to one or more amino acid substitutions, deletions, or insertions, or truncation (due to, e.g., splice variation). In such instances, detection of the amino acid substitution of the variant protein may be useful. This way, nucleic acid sequence data may be obtained through indirect analysis of the nucleic acid sequence of the allele of the polymorphic marker, *i.e.* by detecting a protein variation. Methods of detecting variant proteins are known in the art. For example, direct amino acid sequencing of the variant protein followed by comparison to a reference amino acid sequence can be used. Alternatively, SDS-PAGE followed by gel staining can be used to detect variant proteins of different molecular weights. Also, Immunoassays, e.g., immunofluorescent immunoassays, immunoprecipitations, radioimmunoasays, ELISA, and Western blotting, in which an antibody specific for an epitope comprising the variant sequence among the variant protein and non-variant or wild-type protein can be used. In certain embodiments of the present invention, the identity of amino acids at particular position in an encoded SLC14A1 protein is determined. In certain embodiments, identity of amino acids at one or more of positions 4, 44, 167 and 280 in a SLC14A1 protein with sequence as set forth in SEQ ID NO: 133 is determined. In certain embodiments, determination of the presence or absence an amino acid selected from the group consisting of Tryptophan at position 4, Glutamic Acid at position 100, Valine at position 167 and Aspartic Acid at position 280 is indicative of risk of Bladder Cancer. In certain embodiments, determination of the presence of an amino acid selected from the group consisting of Tryptophan at position 4, Glutamic Acid at position 100, Valine at position 167 and Aspartic Acid at position 280 is indicative of increased risk of Bladder Cancer. The detection may be suitably performed using any of the methods described in the above.

Thus, one aspect of the invention relates to a method of determining a susceptibility to bladder cancer, the method comprising obtaining amino acid sequence data about at least one encoded SLC14A1 protein in a human individual; and analyzing the amino acid sequence data to determine whether at least one amino acid substitution predictive of increased susceptibility of bladder cancer is present, wherein a determination of the presence of the at least one amino acid substitution is indicative of increased susceptibility of bladder cancer for the individual, and wherein a determination of the absence of the at least one amino acid substitution is indicative of the individual not having the increased susceptibility.

In certain embodiments, the method further comprises obtaining a biological sample containing protein from the individual, and obtain amino acid sequence data about SLC14A1 protein from the sample. Sequence information about the SLC14A1 protein may suitably obtained using a method selected from protein sequencing and antibody assay methods.

In some cases, a variant protein has altered (e.g., upregulated or downregulated) biological activity, in comparison to the non-variant or wild-type protein. The biological activity can be, for example, a binding activity or enzymatic activity. In this instance, altered biological activity may be used to detect a variation in protein encoded by a nucleic acid sequence variation. Methods of detecting binding activity and enzymatic activity are known in the art and include, for instance, ELISA, competitive binding assays, quantitative binding assays using instruments such as, for example, a Biacore® 3000 instrument, chromatographic assays, e.g., HPLC and TLC.

Alternatively or additionally, a protein variation encoded by a genetic variation could lead to an altered expression level, e.g., an increased expression level of an mRNA or protein, a decreased expression level of an mRNA or protein. In such instances, nucleic acid sequence data about the allele of the polymorphic marker, or protein sequence data about the protein variation, can be obtained through detection of the altered expression level. Methods of detecting expression levels are known in the art. For example, ELISA, radioimmunoassays, immunofluorescence, and Western blotting can be used to compare the expression of protein levels. Alternatively, Northern blotting can be used to compare the levels of mRNA. These processes are described in Sambrook et al., Molecular Cloning: A Laboratory Manual, 3rd ed. Cold Spring Harbor Laboratory Press, Cold Spring Harbor, New York (2001).

Any of these methods may be performed using a nucleic acid (e.g., DNA, mRNA) or protein of a biological sample obtained from the human individual for which a susceptibility is being determined. The biological sample can be any nucleic acid or protein containing sample obtained from the human individual. For example, the biological sample can be any of the biological samples described herein.

*Number of Polymorphic Markers/Genes Analyzed*

With regard to the methods of determining a susceptibility described herein, the methods can comprise obtaining sequence data about any number of polymorphic markers and/or about any number of genes. For example, the method can comprise obtaining sequence data for about at least 1, 2, 3, 4, 5, 6, 7, 8, 9, 10, 15, 20, 25, 30, 40, 50, 100, 500, 1000, 10,000 or more polymorphic markers. In certain embodiments, the sequence data is obtained from a microarray comprising probes for detecting a plurality of markers. The markers can be independent of rs1058396, rs11877062, rs2298720 and/or rs2298719 and/or the markers may be in linkage disequilibrium with any one of rs1058396, rs11877062, rs2298720 and/or rs2298719. The polymorphic markers can be the ones of the group specified herein or they can be different polymorphic markers that are not listed herein. In a specific embodiment, the method comprises obtaining sequence data about at least two polymorphic markers. In certain embodiments, each of the markers may be associated with a different gene. For example, in some instances, if the method comprises obtaining nucleic acid data about a human individual identifying at least one allele of a polymorphic marker, then the method comprises identifying at least one allele of at least one polymorphic marker. Also, for example, the method can comprise obtaining sequence data about a human individual identifying alleles of multiple, independent markers, which are not in linkage disequilibrium.

*Linkage Disequilibrium*

Linkage Disequilibrium (LD) refers to a non-random assortment of two genetic elements. For example, if a particular genetic element (*e.g.,* an allele of a polymorphic marker, or a haplotype) occurs in a population at a frequency of 0.50 (50%) and another element occurs at a frequency of 0.50 (50%), then the predicted occurrance of a person's having both elements is 0.25 (25%), assuming a random distribution of the elements. However, if it is discovered that the two elements occur together at a frequency higher than 0.25, then the elements are said to be in linkage disequilibrium, since they tend to be inherited together at a higher rate than what their independent frequencies of occurrence (e.g., allele or haplotype frequencies) would predict. Roughly speaking, LD is generally correlated with the frequency of recombination events between the two elements. Allele or haplotype frequencies can be determined in a population by genotyping individuals in a population and determining the frequency of the occurence of each allele or haplotype in the population. For populations of diploids, *e.g.,* human populations, individuals will typically have two alleles for each genetic element (*e.g.,* a marker, haplotype or gene).

Many different measures have been proposed for assessing the strength of linkage disequilibrium (LD; reviewed in Devlin, B. & Risch, N., Genomics 29:311-22 (1995)). Most capture the strength of association between pairs of biallelic sites. Two important pairwise measures of LD are $r^2$ (sometimes denoted $\Delta^2$) and |D'| (Lewontin, R., Genetics 49:49-67 (1964); Hill, W.G. & Robertson, A. Theor. Appl. Genet. 22:226-231 (1968)). Both measures range from 0 (no disequilibrium) to 1 ('complete' disequilibrium), but their interpretation is slightly different. |D'| is defined in such a way that it is equal to 1 if just two or three of the possible haplotypes are present, and it is < 1 if all four possible haplotypes are present. Therefore, a value of |D'| that is <1 indicates that historical recombination may have occurred between two sites (recurrent mutation can also cause |D'| to be <1, but for single nucleotide polymorphisms (SNPs) this is usually regarded as being less likely than recombination). The measure $r^2$ represents the statistical correlation between two sites, and takes the value of 1 if only two haplotypes are present.

The $r^2$ measure is arguably the most relevant measure for association mapping, because there is a simple inverse relationship between $r^2$ and the sample size required to detect association between susceptibility loci and SNPs. These measures are defined for pairs of sites, but for some applications a determination of how strong LD is across an entire region that contains many polymorphic sites might be desirable (*e.g.,* testing whether the strength of LD differs significantly among loci or across populations, or whether there is more or less LD in a region than predicted under a particular model). Measuring LD across a region is not straightforward, but one approach is to use the measure r, which was developed in population genetics. Roughly speaking, r measures how much recombination would be required under a particular population model to generate the LD that is seen in the data. This type of method can potentially also provide a statistically rigorous approach to the problem of determining whether LD data provide evidence for the presence of recombination hotspots.

For the methods described herein, a significant $r^2$ value between sites can be at least 0.1 such as at least 0.1, 0.15, 0.2, 0.25, 0.3, 0.35, 0.4, 0.45, 0.5, 0.55, 0.6, 0.65, 0.7, 0.75, 0.8, 0.85, 0.9, 0.91, 0.92, 0.93, 0.94, 0.95, 0.96, 0.97, 0.98, 0.99 or 1.0. In one specific embodiment of invention, the significant $r^2$ value can be at least 0.2. In another specific embodiment of invention, the significant $r^2$ value can be at least 0.5. In one specific embodiment of invention, the significant $r^2$ value can be at least 0.8. Alternatively, linkage disequilibrium as described herein, refers to linkage disequilibrium characterized by values of $r^2$ of at least 0.2, such as 0.3, 0.4, 0.5, 0.6, 0.7, 0.8, 0.85, 0.9, 0.95, 0.96, 0.97, 0.98, 0.99. Thus, linkage disequilibrium represents a correlation between alleles of distinct markers. It is measured by correlation coefficient or |D'| ($r^2$ up to 1.0 and |D'| up to 1.0). Linkage disequilibrium can be determined in a single human population, as defined herein, or it can be determined in a collection of samples comprising individuals from more than one human population. In one embodiment of the invention, LD is determined in a sample from one or more of the HapMap populations. These include samples from the Yoruba people of Ibadan, Nigeria (YRI), samples from individuals from the Tokyo area in Japan (JPT), samples from individuals Beijing, China (CHB), and samples from U.S. residents with northern and western European ancestry (CEU), as described (The International HapMap Consortium, Nature 426:789-796 (2003)). In one such embodiment, LD is determined in the Caucasian CEU population of the HapMap samples. In another embodiment, LD is determined in the African YRI population. In yet another embodiment, LD is determined in samples from the Icelandic population.

If all polymorphisms in the genome were independent at the population level (*i.e.,* no LD between polymorphisms), then every single one of them would need to be investigated in association studies, to assess all different polymorphic states. However, due to linkage disequilibrium between polymorphisms, tightly linked polymorphisms are strongly correlated, which reduces the number of polymorphisms that need to be investigated in an association study to observe a significant association. Another consequence of LD is that many polymorphisms may give an association signal due to the fact that these polymorphisms are strongly correlated.

Genomic LD maps have been generated across the genome, and such LD maps have been proposed to serve as framework for mapping disease-genes (Risch, N. & Merkiangas, K, Science 273:1516-1517 (1996); Maniatis, N., et al., Proc Natl Acad Sci USA 99:2228-2233 (2002); Reich, DE et al, Nature 411:199-204 (2001)).

It is now established that many portions of the human genome can be broken into series of discrete haplotype blocks containing a few common haplotypes; for these blocks, linkage disequilibrium data provides little evidence indicating recombination (see, *e.g.,* Wall., J.D. and Pritchard, J.K., Nature Reviews Genetics 4:587-597 (2003); Daly, M. et al., Nature Genet. 29:229-232 (2001); Gabriel, S.B. et a/., Science 296:2225-2229 (2002); Patil, N. et al., Science 294:1719-1723 (2001); Dawson, E. et al., Nature 418:544-548 (2002); Phillips, M.S. et al., Nature Genet. 33:382-387 (2003)).

Haplotype blocks (LD blocks) can be used to map associations between phenotype and haplotype status, using single markers or haplotypes comprising a plurality of markers. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

It has thus become apparent that for any given observed association to a polymorphic marker in the genome, it is likely that additional markers in the genome also show association. This is a natural consequence of the uneven distribution of LD across the genome, as observed by the large variation in recombination rates. The markers used to detect association thus in a sense represent "tags" for a genomic region (i.e., a haplotype block or LD block) that is associating with a given disease or trait, and as such are useful for use in the methods and kits of the invention.

By way of example, the markers rs1058396, ras11877062, rs2298720 and rs2298719 may be detected directly to determine risk of Bladder Cancer. Alternatively, any marker in linkage disequilibrium with these markers may be detected to determine risk.

The present invention thus refers to the rs1058396, rs11877062, rs2298720 and rs2298719 markers used for detecting association to Bladder Cancer, as well as markers in linkage disequilibrium with these markers. Thus, in certain embodiments of the invention, markers that are in LD with these markers and/or haplotypes of the invention, as described herein, may be used as surrogate markers.

Suitable surrogate markers may be selected using public information, such as from the International HapMap Consortium (http://www.hapmap.org) and the International 1000genomes Consortium (http://www.l000genomes.org). The stronger the linkage disequilibrium to the anchor marker, the better the surrogate, and thus the mores similar the association detected by the surrogate is expected to be to the association detected by the anchor marker. Markers with values of $r^2$ equal to 1 are perfect surrogates for the at-risk variants, i.e. genotypes for one marker perfectly predicts genotypes for the other. In other words, the surrogate will, by necessity, give exactly the same association data to any particular disease as the anchor marker. Markers with smaller values of $r^2$ than 1 can also be surrogates for the at-risk anchor variant.

The present invention encompasses the assessment of such surrogate markers for the markers as disclosed herein. Such markers are annotated, mapped and listed in public databases, as well known to the skilled person, or can alternatively be readily identified by sequencing the region or a part of the region identified by the markers of the present invention in a group of individuals, and identify polymorphisms in the resulting group of sequences. As a consequence, the person skilled in the art can readily and without undue experimentation identify and select appropriate surrogate markers.

*Association analysis*

For single marker association to a disease, the Fisher exact test can be used to calculate two-sided p-values for each individual allele. Correcting for relatedness among patients can be done by extending a variance adjustment procedure previously described (Risch, N. & Teng, J. Genome Res., 8:1273-1288 (1998)) for sibships so that it can be applied to general familial relationships. The method of genomic controls (Devlin, B. & Roeder, K. Biometrics 55:997 (1999)) can also be used to adjust for the relatedness of the individuals and possible stratification.

For both single-marker and haplotype analyses, relative risk (RR) and the population attributable risk (PAR) can be calculated assuming a multiplicative model (haplotype relative risk model) (Terwilliger, J.D. & Ott, J., Hum. Hered. 42:337-46 (1992) and Falk, C.T. & Rubinstein, P, Ann. Hum. Genet. 51 (Pt 3):227-33 (1987)), i.e., that the risks of the two alleles/haplotypes a person carries multiply. For example, if RR is the risk of A relative to a, then the risk of a person homozygote AA will be RR times that of a heterozygote Aa and $RR^2$ times that of a homozygote aa. The multiplicative model has a nice property that simplifies analysis and computations - haplotypes are independent, *i.e.,* in Hardy-Weinberg equilibrium, within the affected population as well as within the control population. As a consequence, haplotype counts of the affecteds and controls each have multinomial distributions, but with different haplotype frequencies under the alternative hypothesis. Specifically, for two haplotypes, $h_i$ and $h_j$, risk($h_i$)/risk($h_j$) = $(f_i/p_i)/(f_j/p_j)$, where $f$ and $p$ denote, respectively, frequencies in the affected population and in the control population. While there is some power loss if the true model is not multiplicative, the loss tends to be mild except for extreme cases. Most importantly, p-values are always valid since they are computed with respect to null hypothesis.

An association signal detected in one association study may be replicated in a second cohort, for example a cohort from a different population (e.g., different region of same country, or a different country) of the same or different ethnicity. The advantage of replication studies is that the number of tests performed in the replication study is usually quite small, and hence the less stringent the statistical measure that needs to be applied. For example, for a genome-wide search for susceptibility variants for a particular disease or trait using 300,000 SNPs, a correction for the 300,000 tests performed (one for each SNP) can be performed. Since many SNPs on the arrays typically used are correlated *(i.e.,* in LD), they are not independent. Thus, the correction is conservative. Nevertheless, applying this correction factor requires an observed P-value of less than 0.05/300,000 = $1.7 \times 10^{-7}$ for the signal to be considered significant applying this conservative test on results from a single study cohort. Obviously, signals found in a genome-wide association study with P-values less than this conservative threshold *(i.e.,* more significant) are a measure of a true genetic effect, and replication in additional cohorts is not necessary from a statistical point of view. Importantly, however, signals with P-values that are greater than this threshold may also be due to a true genetic effect. The sample size in the first study may not have been sufficiently large to provide an observed P-value that meets the conservative threshold for genome-wide significance, or the first study may not have reached genome-wide significance due to inherent fluctuations due to sampling. Since the correction factor depends on the number of statistical tests performed, if one signal (one SNP) from an initial study is replicated in a second case-control cohort, the appropriate statistical test for significance is that for a single statistical test, *i.e.,* P-value less than 0.05. Replication studies in one or even several additional case-control cohorts have the added advantage of providing assessment of the association signal in additional populations, thus simultaneously confirming the initial finding and providing an assessment of the overall significance of the genetic variant(s) being tested in human populations in general.

The results from several case-control cohorts can also be combined to provide an overall assessment of the underlying effect. The methodology commonly used to combine results from multiple genetic association studies is the Mantel-Haenszel model (Mantel and Haenszel, J Natl Cancer Inst 22:719-48 (1959)). The model is designed to deal with the situation where association results from different populations, with each possibly having a different population frequency of the genetic variant, are combined. The model combines the results assuming that the effect of the variant on the risk of the disease, a measured by the OR or RR, is the same in all populations, while the frequency of the variant may differ between the populations. Combining the results from several populations has the added advantage that the overall power to detect a real underlying association signal is increased, due to the increased statistical power provided by the combined cohorts. Furthermore, any deficiencies in individual studies, for example due to unequal matching of cases and controls or population stratification will tend to balance out when results from multiple cohorts are combined, again providing a better estimate of the true underlying genetic effect.

*Risk assessment and Diagnostics*

Within any given population, there is an absolute risk of developing a disease or trait, defined as the chance of a person developing the specific disease or trait over a specified time-period. For example, a woman's lifetime absolute risk of breast cancer is one in nine. That is to say, one woman in every nine will develop breast cancer at some point in their lives. Risk is typically measured by looking at very large numbers of people, rather than at a particular individual. Risk is often presented in terms of Absolute Risk (AR) and Relative Risk (RR). Relative Risk is used to compare risks associating with two variants or the risks of two different groups of people. For example, it can be used to compare a group of people with a certain genotype with another group having a different genotype. For a disease, a relative risk of 2 means that one group has twice the chance of developing a disease as the other group. The risk presented is usually the relative risk for a person, or a specific genotype of a person, compared to the population with matched gender and ethnicity. Risks of two individuals of the same gender and ethnicity could be compared in a simple manner. For example, if, compared to the population, the first individual has relative risk 1.5 and the second has relative risk 0.5, then the risk of the first individual compared to the second individual is 1.5/0.5 = 3.

*Risk Calculations*

The creation of a model to calculate the overall genetic risk involves two steps: i) conversion of odds-ratios for a single genetic variant into relative risk and ii) combination of risk from multiple variants in different genetic loci into a single relative risk value.

*Deriving risk from odds-ratios*

Most gene discovery studies for complex diseases that have been published to date in authoritative journals have employed a case-control design because of their retrospective setup. These studies sample and genotype a selected set of cases (people who have the specified disease condition) and control individuals. The interest is in genetic variants (alleles) which frequency in cases and controls differ significantly.

The results are typically reported in odds ratios, that is the ratio between the fraction (probability) with the risk variant (carriers) versus the non-risk variant (non-carriers) in the groups of affected versus the controls, i.e. expressed in terms of probabilities conditional on the affection status:

$$OR = (Pr(c|A)/Pr(nc|A)) / (Pr(c|C)/Pr(nc|C))$$

Sometimes it is however the absolute risk for the disease that we are interested in, i.e. the fraction of those individuals carrying the risk variant who get the disease or in other words the probability of getting the disease. This number cannot be directly measured in case-control studies, in part, because the ratio of cases versus controls is typically not the same as that in the general population. However, under certain assumption, we can estimate the risk from the odds ratio.

It is well known that under the rare disease assumption, the relative risk of a disease can be approximated by the odds ratio. This assumption may however not hold for many common diseases. Still, it turns out that the risk of one genotype variant relative to another can be estimated from the odds ratio expressed above. The calculation is particularly simple under the assumption of random population controls where the controls are random samples from the same population as the cases, including affected people rather than being strictly unaffected individuals. To increase sample size and power, many of the large genome-wide association and replication studies use controls that were neither age-matched with the cases, nor were they carefully scrutinized to ensure that they did not have the disease at the time of the study. Hence, while not exactly, they often approximate a random sample from the general population. It is noted that this assumption is rarely expected to be satisfied exactly, but the risk estimates are usually robust to moderate deviations from this assumption.

Calculations show that for the dominant and the recessive models, where we have a risk variant carrier, "c", and a non-carrier, "nc", the odds ratio of individuals is the same as the risk ratio between these variants:

$$OR = Pr(A|c)/Pr(A|nc) = r$$

And likewise for the multiplicative model, where the risk is the product of the risk associated with the two allele copies, the allelic odds ratio equals the risk factor:

$$OR = Pr(A|aa)/Pr(A|ab) = Pr(A|ab)/Pr(A|bb) = r$$

Here "a" denotes the risk allele and "b" the non-risk allele. The factor "r" is therefore the relative risk between the allele types.

For many of the studies published in the last few years, reporting common variants associated with complex diseases, the multiplicative model has been found to summarize the effect adequately and most often provide a fit to the data superior to alternative models such as the dominant and recessive models.

The person skilled in the art will appreciate that for markers with two alleles present in the population being studied (such as SNPs), and wherein one allele is found in increased frequency in a group of individuals with a trait or disease in the population, compared with controls, the other allele of the marker will be found in decreased frequency in the group of individuals with the trait or disease, compared with controls. In such a case, one allele of the marker (the one found in increased frequency in individuals with the trait or disease) will be the at-risk allele, while the other allele will be a protective allele.

*Database*

Determining susceptibility can alternatively or additionally comprise comparing nucleic acid sequence data and/or genotype data to a database containing correlation data between polymorphic markers and susceptibility to Bladder Cancer. The database can be part of a computer-readable medium as described herein.

In a specific aspect of the invention, the database comprises at least one measure of susceptibility to the condition for the polymorphic markers. For example, the database may comprise risk values associated with particular genotypes at such markers. The database may also comprise risk values associated with particular genotype combinations for multiple such markers.

In another specific aspect of the invention, the database comprises a look-up table containing at least one measure of susceptibility to the condition for the polymorphic markers.

*Further steps*

The methods disclosed herein can comprise additional steps which may occur before, after, or simultaneously with one of the aforementioned steps of the method of the invention. In a specific embodiment of the invention, the method of determining a susceptibility to Bladder Cancer further comprises reporting the susceptibility to at least one entity selected from the group consisting of the individual, a guardian of the individual, a genetic service provider, a physician, a medical organization, and a medical insurer. The reporting may be accomplished by any of several means. For example, the reporting can comprise sending a written report on physical media or electronically or providing an oral report to at least one entity of the group, which written or oral report comprises the susceptibility. Alternatively, the reporting can comprise providing the at least one entity of the group with a login and password, which provides access to a report comprising the susceptibility posted on a password-protected computer system.

*Study population*

In a general sense, the methods and kits described herein can be utilized from samples containing nucleic acid material (DNA or RNA) from any source and from any individual, or from genotype or sequence data derived from such samples. In preferred embodiments, the individual is a human individual. The individual can be an adult, child, or fetus. The nucleic acid source may be any sample comprising nucleic acid material, including biological samples, or a sample comprising nucleic acid material derived therefrom. The present invention also provides for assessing markers in individuals who are members of a target population. Such a target population is in one embodiment a population or group of individuals at risk of developing Bladder Cancer, based on other genetic factors, biophysical parameters, family history, etc.).

The Icelandic population is a Caucasian population of Northern European ancestry. A large number of studies reporting results of genetic linkage and association in the Icelandic population have been published in the last few years. Many of those studies show replication of variants, originally identified in the Icelandic population as being associating with a particular disease, in other populations (Sulem, P., et al. Nat Genet May 17 2009 (Epub ahead of print); Rafnar, T., et al. Nat Genet 41:221-7 (2009); Gretarsdottir, S., et al. Ann Neurol 64:402-9 (2008); Stacey, S.N., et al. Nat Genet 40:1313-18 (2008); Gudbjartsson, D.F., et al. Nat Genet 40:886-91 (2008); Styrkarsdottir, U., et al. N Engl J Med 358:2355-65 (2008); Thorgeirsson, T., et al. Nature 452:638-42 (2008); Gudmundsson, J., et al. Nat Genet. 40:281-3 (2008); Stacey, S.N., et al., Nat Genet. 39:865-69 (2007); Helgadottir, A., et al., Science 316:1491-93 (2007); Steinthorsdottir, V., et al., Nat Genet. 39:770-75 (2007); Gudmundsson, J., et al., Nat Genet 39:631-37 (2007); Frayling, TM, Nature Reviews Genet 8:657-662 (2007); Amundadottir, L.T., et al., Nat Genet. 38:652-58 (2006); Grant, S.F., et al., Nat Genet. 38:320-23 (2006)). Thus, genetic findings in the Icelandic population have in general been replicated in other populations, including populations from Africa and Asia.

It is thus believed that the markers described herein to be associated with risk of Bladder Cancer will show similar association in other human populations. Particular embodiments comprising individual human populations are thus also contemplated and within the scope of the invention. Such embodiments relate to human subjects that are from one or more human population including, but not limited to, Caucasian populations, European populations, American populations, Eurasian populations, Asian populations, Central/South Asian populations, East Asian populations, Middle

The racial contribution in individual subjects may also be determined by genetic analysis. Genetic analysis of ancestry may be carried out using unlinked microsatellite markers such as those set out in Smith et al. (Am J Hum Genet 74, 1001-13 (2004)).

In certain embodiments, the invention relates to markers identified in specific populations, as described in the above. The person skilled in the art will appreciate that measures of linkage disequilibrium (LD) may give different results when applied to different populations. This is due to different population history of different human populations as well as differential selective pressures that may have led to differences in LD in specific genomic regions. It is also well known to the person skilled in the art that certain markers, *e.g.* SNP markers, have different population frequency in different populations, or are polymorphic in one population but not in another. The person skilled in the art will however apply the methods available and as taught herein to practice the present invention in any given human population. This may include assessment of polymorphic markers in the LD region of the present invention, so as to identify those markers that give strongest association within the specific population. Thus, the at-risk variants of the present invention may reside on different haplotype background and in different frequencies in various human populations. However, utilizing methods known in the art and the markers of the present invention, the invention can be practiced in any given human population.

*Screening Methods*

The invention also provides a method of screening candidate markers for assessing susceptibility to Bladder Cancer. The invention also provides a method of identification of a marker for use in assessing susceptibility to Bladder Cancer. The method may comprise analyzing the frequency of at least one allele of a polymorphic marker in a population of human individuals diagnosed with Bladder Cancer, wherein a significant difference in frequency of the at least one allele in the population of human individuals diagnosed with Bladder Cancer as compared to the frequency of the at least one allele in a control population of human individuals is indicative of the allele as a marker of the Bladder Cancer. In certain embodiments, the candidate marker is a marker in linkage disequilibrium with rs1058396.

In one embodiment, the method comprises (i) identifying at least one polymorphic marker within the human *SLC14A1* gene; (ii) obtaining sequence information about the at least one polymorphic marker in a group of individuals diagnosed with Bladder Cancer; and (iii) obtaining sequence information about the at least one polymorphic marker in a group of control individuals; wherein determination of a significant difference in frequency of at least one allele in the at least one polymorphism in individuals diagnosed with Bladder Cancer as compared with the frequency of the at least one allele in the control group is indicative of the at least one polymorphism being useful for assessing susceptibility to Bladder Cancer. In certain embodiments, the marker is in linkage disequilibrium with rs1058396.

In one embodiment, an increase in frequency of the at least one allele in the at least one polymorphism in individuals diagnosed with Bladder Cancer, as compared with the frequency of the at least one allele in the control group, is indicative of the at least one polymorphism being useful for assessing increased susceptibility to Bladder Cancer. In another embodiment, a decrease in frequency of the at least one allele in the at least one polymorphism in individuals diagnosed with Bladder Cancer, as compared with the frequency of the at least one allele in the control group, is indicative of the at least one polymorphism being useful for assessing decreased susceptibility to, or protection against, Bladder Cancer.

*Utility of Genetic Testing*

The person skilled in the art will appreciate and understand that the variants described herein in general do not, by themselves, provide an absolute identification of individuals who will develop urinary bladder cancer. The variants described herein do however indicate increased and/or decreased likelihood that individuals carrying the at-risk or protective variants of the invention will develop UBC, or symptoms associated with UBC. This information is however extremely valuable in itself, as outlined in more detail in the below, as it can be used to, for example, initiate preventive measures at an early stage, perform regular physical exams to monitor the progress and/or appearance of symptoms, or to schedule exams at a regular interval to identify early symptoms, so as to be able to apply treatment at an early stage.

Bladder cancer is a disease with a high prevalence and potential for improved survival with early detection. Understanding of the genetic factors contributing to the residual genetic risk for bladder cancer is very limited. No universally successful method for the prevention or treatment of bladder cancer is currently available. Management of the disease currently relies on a combination of early diagnosis, appropriate treatments and secondary prevention. There are clear clinical imperatives for integrating genetic testing into all aspects of these management areas. Identification of cancer susceptibility genes may also reveal key molecular pathways that may be manipulated (e.g., using small or large molecular weight drugs) and may lead to more effective treatments.

A screening program that would result in detection of bladder cancer at an earlier stage, prior to muscle invasion or metastasis, could render a significant improvement in patient morbidity and overall survival. In order for bladder cancer screening to become a reality, first a high incidence population has to be identified and secondly a cost-effective marker with good performance characteristics has to be available. Individuals with many environmental risk factors, such as older male smokers and who have the high-risk genetic profile may benefit from periodic screening. Clinical screening for bladder cancer is mainly performed by urine cytology, cystoscopy or Hematuria tests.

Home urine dipstick to assess for hematuria is convenient, inexpensive, and noninvasive. However, utility of widespread screening with hematuria testing is limited due to the low positive predictive value (PPV) of the test. The PPV for hematuria dipstick for screening ranges between 5 and 8.3% resulting in many unnecessary workups with their attendant patient anxiety and cost. Due to the relatively low sensitivity and low PPV of the reagent strip for hemoglobin as well as cytology, multiple urine-based bladder markers have been developed to try to assist in detecting bladder cancer non-invasively (Lotan Y, Roehrborn CG (2003) Urology 61(1):109-118). These include the NMP22 BladderChek Test (Matritech Inc., Newton, MA, USA) and UroVysion (Vysis Downer's Grove, IL, USA) (Grossman, HB et al. JAMA 293:810-816, 2005).

Genetic variants described herein can be used alone or in combination, as well as in combination with other factors, including other genetic risk factors or biomarkers, for risk assessment of an individual for UBC. In certain embodiments, the variants described herein may be included in genetic screening programs for UBC that also include other risk factors for UBC, including variants on chromosome 3q (*e.g,* rs710521), chromosome 4p (*e.g.,* rs798766), chromosome 5q (*e.g.,* rs4446484) and chromosome 8q (*e.g.,* rs9642880). Other factors known to affect the predisposition of an individual towards developing risk of developing UBC are also known to the person skilled in the art and can be utilized in such assessment. These include, but are not limited to, age, gender, smoking status and/or smoking history, family history of cancer, and of UBC in particular. Methods known in the art can be used for such assessment, including multivariate analyses or logistic regression.

*Diagnostic Methods*

Polymorphic markers associated with increased susceptibility of Bladder Cancer and related conditions are useful in diagnostic methods. While methods of diagnosing such conditions are known in the art, the detection of one or more alleles of the specific polymorphic markers advantageously may be useful for detection of these conditions at their early stages and may also reduce the occurrence of mis-diagnosis. In this regard, the invention further provides methods of diagnosing these conditions comprising obtaining sequence data identifying at least one allele of at least one polymorphic marker of a specified group, in conjunction with carrying out one or more steps, e.g., clinical diagnostic steps, such as any of those described herein.

The present invention pertains in some embodiments to methods of clinical applications of diagnosis, *e.g.,* diagnosis performed by a medical professional. In other embodiments, the invention pertains to methods of diagnosis or methods of determination of a susceptibility performed by a layman. The layman can be the customer of a sequencing or genotyping service. The layman may also be a genotype or sequencing service provider, who performs analysis on a DNA sample from an individual, in order to provide service related to genetic risk factors for particular traits or diseases, based on the genotype status of the individual (*i.e.,* the customer). Sequencing methods include for example those discussed in the above, but in general any suitable sequencing method may be used in the methods described and claimed herein. Recent technological advances in genotyping technologies, including high-throughput genotyping of SNP markers, such as Molecular Inversion Probe array technology (*e.g.,* Affymetrix GeneChip), and BeadArray Technologies (*e.g.,* Illumina GoldenGate and Infinium assays) have made it possible for individuals to have their own genome assessed for up to one million SNPs simultaneously, at relatively little cost. The resulting genotype information, which can be made available to the individual, can be compared to information about disease or trait risk associated with various SNPs, including information from public literature and scientific publications.

The application of disease-associated alleles as described herein, can thus for example be performed by the individual, through analysis of his/her genotype data, by a health professional based on results of a clinical test, or by a third party, including the genotype or sequencing service provider. The third party may also be service provider who interprets genotype or sequence information from the customer to provide service related to specific genetic risk factors, including the genetic markers described herein. In other words, the diagnosis or determination of a susceptibility of genetic risk can be made by health professionals, genetic counselors, third parties providing genotyping and/or sequencing service, third parties providing risk assessment service or by the layman (*e.g.,* the individual), based on information about the genotype status of an individual and knowledge about the risk conferred by particular genetic risk factors (*e.g.,* particular SNPs). In the present context, the term "diagnosing", "diagnose a susceptibility" and "determine a susceptibility" is meant to refer to any available method for determining a susceptibility or risk of disease, including those mentioned above.

In certain embodiments, a sample containing genomic DNA from an individual is collected. Such sample can for example be a buccal swab, a saliva sample, a blood sample, or other suitable samples containing genomic DNA, as described further herein. In certain embodiments, the sample is obtained by non-invasive means (*e.g.,* for obtaining a buccal sample, saliva sample, hair sample or skin sample). In certain embodiments, the sample is obtained by non-surgical means, *i.e.* in the absence of a surgical intervention on the individual that puts the individual at substantial health risk. Such embodiments may, in addition to non-invasive means also include obtaining sample by extracting a blood sample (*e.g.,* a venous blood sample). The genomic DNA obtained from the individual is then analyzed using any common technique available to the skilled person, such as high-throughput technologies for genotyping and/or sequencing. Results from such methods are stored in a convenient data storage unit, such as a data carrier, including computer databases, data storage disks, or by other convenient data storage means. In certain embodiments, the computer database is an object database, a relational database or a post-relational database. The genotype data is subsequently analyzed for the presence of certain variants known to be susceptibility variants for a particular human condition, such as the genetic variants described herein associated with risk of Bladder Cancer. Genotype and/or sequencing data can be retrieved from the data storage unit using any convenient data query method. Calculating risk conferred by a particular genotype for the individual can be based on comparing the genotype of the individual to previously determined risk (expressed as a relative risk (RR) or and odds ratio (OR), for example) for the genotype, for example for an heterozygous carrier of an at-risk variant. The calculated risk for the individual can be the relative risk for a person, or for a specific genotype of a person, compared to the average population with matched gender and ethnicity. The average population risk can be expressed as a weighted average of the risks of different genotypes, using results from a reference population, and the appropriate calculations to calculate the risk of a genotype group relative to the population can then be performed. Alternatively, the risk for an individual is based on a comparison of particular genotypes, for example heterozygous carriers of an at-risk allele of a marker compared with non-carriers of the at-risk allele. The calculated risk estimated can be made available to the customer via a website, preferably a secure website.

In certain embodiments, a service provider will include in the provided service all of the steps of isolating genomic DNA from a sample provided by the customer, performing genotyping or sequencing of the isolated DNA, calculating genetic risk based on the genotype or sequence data, and report the risk to the customer. In some other embodiments, the service provider will include in the service the interpretation of genotype data for the individual, *i.e.,* risk estimates for particular genetic variants based on the genotype data for the individual. In some other embodiments, the service provider may include service that includes genotyping and/or sequencing service and interpretation of the resulting sequence data, starting from a sample of isolated DNA from the individual.

Decreased susceptibility is in general determined based on the absence of particular at-risk alleles and/or the presence of protective alleles. As discussed in more detail herein, for biallelic markers such as SNPs, the alternate allele of an at-risk allele is, by definition, a protective allele. Determinations of its presence, in particular for homozygous individuals, is thus indicative of a decreased susceptibility.

*Kits*

Kits useful in the methods of the invention comprise components useful in any of the methods described herein, including for example, primers for nucleic acid amplification, hybridization probes, restriction enzymes (*e.g.,* for RFLP analysis), allele-specific oligonucleotides, antibodies that bind to specific polypeptides encoded by a nucleic acid of the invention (*e.g.,* N280D, R4W, M167 and/or K44E SLC14A1 polypeptides), means for amplification of a nucleic acids, means for analyzing the nucleic acid sequence of a nucleic acids, means for analyzing the amino acid sequence of a polypeptide, etc. The kits can for example include necessary buffers, nucleic acid primers for amplifying nucleic acids of the invention (*e.g.,* a nucleic acid segment comprising one or more of the polymorphic markers as described herein), and reagents for allele-specific detection of the fragments amplified using such primers and necessary enzymes (*e.g.,* DNA polymerase). Additionally, kits can provide reagents for assays to be used in combination with other diagnostic assays for UBC.

Disclosed is a kit for assaying a sample from a subject to detect a susceptibility to UBC in a subject, wherein the kit comprises reagents necessary for selectively detecting at least one allele of at least one polymorphism of the present invention in the genome of the individual. In a particular embodiment, the reagents comprise at least one contiguous oligonucleotide that hybridizes to a fragment of the genome of the individual comprising at least one polymorphism of the present invention. In another embodiment, the reagents comprise at least one pair of oligonucleotides that hybridize to opposite strands of a genomic segment obtained from a subject, wherein each oligonucleotide primer pair is designed to selectively amplify a fragment of the genome of the individual that includes at least one polymorphism associated with UBC risk. In one such embodiment, the polymorphism is selected from the polymorphic markers rs1058396, rs11877062, rs2298719 and rs2298720, and markers in linkage disequilibrium therewith. In yet another embodiment, the fragment is at least 20 base pairs in size. In another embodiment, the fragment is no more than 200 base pairs in size. Such oligonucleotides or nucleic acids (*e.g.,* oligonucleotide primers) can be designed using portions of the nucleic acid sequence flanking the polymorphic site. In another embodiment, the kit comprises one or more labeled nucleic acids capable of allele-specific detection of one or more specific polymorphic markers or haplotypes, and reagents for detection of the label. Suitable labels include, *e.g.,* a radioisotope, a fluorescent label, an enzyme label, an enzyme co-factor label, a magnetic label, a spin label, an epitope label.

In a preferred embodiment, the DNA template containing the SNP polymorphism is amplified by Polymerase Chain Reaction (PCR) prior to detection, and primers for such amplification are included in the reagent kit. In such an embodiment, the amplified DNA serves as the template for the detection probe and the enhancer probe.

In one embodiment, the DNA template is amplified by means of Whole Genome Amplification (WGA) methods, prior to assessment for the presence of specific polymorphic markers as described herein. Standard methods well known to the skilled person for performing WGA may be utilized, and are within scope of the invention. In one such embodiment, reagents for performing WGA are included in the reagent kit.

In certain embodiments, the kit further comprises a collection of data comprising correlation data between the polymorphic markers assessed by the kit and susceptibility to urinary bladder cancer.

Also disclosed is a pharmaceutical pack (kit), the pack comprising a therapeutic agent and a set of instructions for administration of the therapeutic agent to humans diagnostically tested for one or more variants of the present invention, as disclosed herein. The therapeutic agent can be a small molecule drug, an antibody, a peptide, an antisense or RNAi molecule, or other therapeutic molecules. In one embodiment, an individual identified as a carrier of at least one variant of the present invention is instructed to take a prescribed dose of the therapeutic agent. In one such embodiment, an individual identified as a homozygous carrier of at least one variant of the present invention is instructed to take a prescribed dose of the therapeutic agent. In another embodiment, an individual identified as a non-carrier of at least one variant of the present invention is instructed to take a prescribed dose of the therapeutic agent.

In certain embodiments, the kit further comprises a set of instructions for using the reagents comprising the kit.

*Antisense agents*

The nucleic acids and/or variants described herein, or nucleic acids comprising their complementary sequence, may be used as antisense constructs to control gene expression in cells, tissues or organs. The methodology associated with antisense techniques is well known to the skilled artisan, and is for example described and reviewed in AntisenseDrug Technology: Principles, Strategies, and Applications, Crooke, ed., Marcel Dekker Inc., New York (2001).

In general, antisense agents (antisense oligonucleotides) are comprised of single stranded oligonucleotides (RNA or DNA) that are capable of binding to a complimentary nucleotide segment. By binding the appropriate target sequence, an RNA-RNA, DNA-DNA or RNA-DNA duplex is formed. The antisense oligonucleotides are complementary to the sense or coding strand of a gene. It is also possible to form a triple helix, where the antisense oligonucleotide binds to duplex DNA.

Several classes of antisense oligonucleotide are known to those skilled in the art, including cleavers and blockers. The former bind to target RNA sites, activate intracellular nucleases (*e.g.,* RnaseH or Rnase L), that cleave the target RNA. Blockers bind to target RNA, inhibit protein translation by steric hindrance of the ribosomes. Examples of blockers include nucleic acids, morpholino compounds, locked nucleic acids and methylphosphonates (Thompson, Drug Discovery Today, 7:912-917 (2002)). Antisense oligonucleotides are useful directly as therapeutic agents, and are also useful for determining and validating gene function, for example by gene knock-out or gene knock-down experiments. Antisense technology is further described in Lavery et al., Curr. Opin. Drug Discov. Devel. 6:561-569 (2003), Stephens et al., Curr. Opin. Mol. Ther. 5:118-122 (2003), Kurreck, Eur. J. Biochem. 270:1628-44 (2003), Dias et al., Mol. Cancer Ter. 1:347-55 (2002), Chen, Methods Mol. Med. 75:621-636 (2003), Wang et al., Curr. Cancer Drug Targets 1:177-96 (2001), and Bennett, Antisense Nucleic Acid Drug.Dev. 12:215-24 (2002).

In certain embodiments, the antisense agent is an oligonucleotide that is capable of binding to a particular nucleotide segment. In certain embodiments, the nucleotide segment comprises all or a portion of the human *SLC14A1* gene. In certain other embodiments, the antisense nucleotide is capable of binding to a nucleotide segment of the human *SLC14A1* as set forth in SEQ ID NO: 134. Antisense nucleotides can be from 5-400 nucleotides in length, including 5-200 nucleotides, 5-100 nucleotides, 10-50 nucleotides, and 10-30 nucleotides. In certain preferred embodiments, the antisense nucleotides are from 14-50 nucleotides in length, including 14-40 nucleotides and 14-30 nucleotides..

The variants described herein can also be used for the selection and design of antisense reagents that are specific for particular variants. Using information about the variants described herein, antisense oligonucleotides or other antisense molecules that specifically target mRNA molecules that contain one or more variants of the invention can be designed. In this manner, expression of mRNA molecules that contain one or more variant of the present invention (*e.g.* at risk marker alleles, such as rs1058396 allele G, rs11877062 allele C, rs2298720 allele G and rs2298719 allele A) can be inhibited or blocked. In one embodiment, the antisense molecules are designed to specifically bind a particular allelic form (*e.g.,* an at-risk variant) of the target nucleic acid, thereby inhibiting translation of a product originating from this specific allele or haplotype, but which do not bind other or alternate variants at the specific polymorphic sites of the target nucleic acid molecule. As antisense molecules can be used to inactivate mRNA so as to inhibit gene expression, and thus protein expression, the molecules can be used for disease treatment. The methodology can involve cleavage by means of ribozymes containing nucleotide sequences complementary to one or more regions in the mRNA that attenuate the ability of the mRNA to be translated. Such mRNA regions include, for example, protein-coding regions, in particular protein-coding regions corresponding to catalytic activity, substrate and/or ligand binding sites, or other functional domains of a protein.

The phenomenon of RNA interference (RNAi) has been actively studied for the last decade, since its original discovery in C. *elegans* (Fire et al.,Nature 391:806-11 (1998)), and in recent years its potential use in treatment of human disease has been actively pursued (reviewed in Kim & Rossi, Nature Rev. Genet. 8:173-204 (2007)). RNA interference (RNAi), also called gene silencing, is based on using double-stranded RNA molecules (dsRNA) to turn off specific genes. In the cell, cytoplasmic double-stranded RNA molecules (dsRNA) are processed by cellular complexes into small interfering RNA (siRNA). The siRNA guide the targeting of a protein-RNA complex to specific sites on a target mRNA, leading to cleavage of the mRNA (Thompson, Drug Discovery Today, 7:912-917 (2002)). The siRNA molecules are typically about 20, 21, 22 or 23 nucleotides in length. Thus, one aspect of the invention relates to isolated nucleic acid molecules, and the use of those molecules for RNA interference, i.e. as small interfering RNA molecules (siRNA). In one embodiment, the isolated nucleic acid molecules are 18-26 nucleotides in length, preferably 19-25 nucleotides in length, more preferably 20-24 nucleotides in length, and more preferably 21, 22 or 23 nucleotides in length.

Another pathway for RNAi-mediated gene silencing originates in endogenously encoded primary microRNA (pri-miRNA) transcripts, which are processed in the cell to generate precursor miRNA (pre-miRNA). These miRNA molecules are exported from the nucleus to the cytoplasm, where they undergo processing to generate mature miRNA molecules (miRNA), which direct translational inhibition by recognizing target sites in the 3' untranslated regions of mRNAs, and subsequent mRNA degradation by processing P-bodies (reviewed in Kim & Rossi, Nature Rev. Genet. 8:173-204 (2007)).

Clinical applications of RNAi include the incorporation of synthetic siRNA duplexes, which preferably are approximately 20-23 nucleotides in size, and preferably have 3' overlaps of 2 nucleotides. Knockdown of gene expression is established by sequence-specific design for the target mRNA. Several commercial sites for optimal design and synthesis of such molecules are known to those skilled in the art.

Other applications provide longer siRNA molecules (typically 25-30 nucleotides in length, preferably about 27 nucleotides), as well as small hairpin RNAs (shRNAs; typically about 29 nucleotides in length). The latter are naturally expressed, as described in Amarzguioui et al. (FEBS Lett. 579:5974-81 (2005)). Chemically synthetic siRNAs and shRNAs are substrates for *in vivo* processing, and in some cases provide more potent gene-silencing than shorter designs (Kim et al., Nature Biotechnol. 23:222-226 (2005); Siolas et al., Nature Biotechnol. 23:227-231 (2005)). In general siRNAs provide for transient silencing of gene expression, because their intracellular concentration is diluted by subsequent cell divisions. By contrast, expressed shRNAs mediate long-term, stable knockdown of target transcripts, for as long as transcription of the shRNA takes place (Marques et al., Nature Biotechnol. 23:559-565 (2006); Brummelkamp et al., Science 296: 550-553 (2002)).

Since RNAi molecules, including siRNA, miRNA and shRNA, act in a sequence-dependent manner, the variants presented herein can be used to design RNAi reagents that recognize specific nucleic acid molecules comprising specific alleles and/or haplotypes (*e.g.,* the alleles and/or haplotypes of the present invention), while not recognizing nucleic acid molecules comprising other alleles or haplotypes. These RNAi reagents can thus recognize and destroy the target nucleic acid molecules. As with antisense reagents, RNAi reagents can be useful as therapeutic agents (i.e., for turning off disease-associated genes or disease-associated gene variants), but may also be useful for characterizing and validating gene function (*e.g.,* by gene knock-out or gene knock-down experiments).

Delivery of RNAi may be performed by a range of methodologies known to those skilled in the art. Methods utilizing non-viral delivery include cholesterol, stable nucleic acid-lipid particle (SNALP), heavy-chain antibody fragment (Fab), aptamers and nanoparticles. Viral delivery methods include use of lentivirus, adenovirus and adeno-associated virus. The siRNA molecules are in some embodiments chemically modified to increase their stability. This can include modifications at the 2' position of the ribose, including 2'-O-methylpurines and 2'-fluoropyrimidines, which provide resistance to Rnase activity. Other chemical modifications are possible and known to those skilled in the art.

The following references provide a further summary of RNAi, and possibilities for targeting specific genes using RNAi: Kim & Rossi, Nat. Rev. Genet. 8:173-184 (2007), Chen & Rajewsky, Nat. Rev. Genet. 8: 93-103 (2007), Reynolds, et al., Nat. Biotechnol. 22:326-330 (2004), Chi et al., Proc. Natl. Acad. Sci. USA 100:6343-6346 (2003), Vickers et al., J. Biol. Chem. 278:7108-7118 (2003), Agami, Curr. Opin. Chem. Biol. 6:829-834 (2002), Lavery, et al., Curr. Opin. Drug Discov. Devel. 6:561-569 (2003), Shi, Trends Genet. 19:9-12 (2003), Shuey et al., Drug Discov. Today 7:1040-46 (2002), McManus et al., Nat. Rev. Genet. 3:737-747 (2002), Xia et al., Nat. Biotechnol. 20:1006-10 (2002), Plasterk et al., curr. Opin. Genet. Dev. 10:562-7 (2000), Bosher et al., Nat. Cell Biol. 2:E31-6 (2000), and Hunter, Curr. Biol. 9:R440-442 (1999)

*Methods of assessing probability of response to therapeutic agents, methods of monitoring progress of treatment and methods of treatment*

As is known in the art, individuals can have differential responses to a particular therapy (*e.g.,* a therapeutic agent or therapeutic method). Pharmacogenomics addresses the issue of how genetic variations (*e.g.,* the variants (markers and/or haplotypes) of the present invention) affect drug response, due to altered drug disposition and/or abnormal or altered action of the drug. Thus, the basis of the differential response may be genetically determined in part. Clinical outcomes due to genetic variations affecting drug response may result in toxicity of the drug in certain individuals (*e.g.,* carriers or non-carriers of the genetic variants of the present invention), or therapeutic failure of the drug. Therefore, the variants of the present invention may determine the manner in which a therapeutic agent and/or method acts on the body, or the way in which the body metabolizes the therapeutic agent.

Accordingly, in one embodiment, the presence of a particular allele at a polymorphic site is indicative of a different response, *e.g.* a different response rate, to a particular treatment modality. This means that a patient diagnosed with UBC, and carrying a certain allele at a polymorphic site described herein (*e.g.,* the at-risk and protective alleles of the invention) would respond better to, or worse to, a specific therapeutic, drug and/or other therapy used to treat the disease. Therefore, the identity of a marker allele could aid in deciding what treatment should be used for a the patient. For example, for a newly diagnosed patient, the presence of an at-risk marker allele of the present invention may be assessed (*e.g.,* through testing DNA derived from a blood sample, as described herein). If the patient is positive for the marker allele, then the physician recommends one particular therapy, while if the patient is negative for the at least one allele of a marker, or a haplotype, then a different course of therapy may be recommended. Thus, the patient's carrier status could be used to help determine whether a particular treatment modality should be administered.

As described above, current clinical treatment options for UBC include different surgical procedures, depending on the severity of the cases, e.g. whether the cancer is invasive into the muscle wall of the bladder. Treatment options also include radiation therapy, for which a proportion of patients experience adverse symptoms. The markers of the invention, as described herein, may be used to assess response to these therapeutic options, or to predict the progress of therapy using any one of these treatment options. Thus, genetic profiling can be used to select the appropriate treatment strategy based on the genetic status of the individual, or it may be used to predict the outcome of the particular treatment option, and thus be useful in the strategic selection of treatment options or a combination of available treatment options. Again, such profiling and classification of individuals is supported further by first analysing known groups of patients for marker and/or haplotype status, as described further herein.

The present invention also relates to methods of monitoring progress or effectiveness of a treatment for urinary bladder cancer. This can be done based on the genotype status of the markers described herein, i.e., by assessing the absence or presence of at least one allele of at least one polymorphic marker as disclosed herein, or by monitoring expression of genes that are associated with the variants (markers and haplotypes) described herein (*e.g.,* the *SLC14A1* gene). The risk gene mRNA or the encoded polypeptide can be measured in a tissue sample (*e.g.,* a peripheral blood sample, or a biopsy sample). Expression levels and/or mRNA levels can thus be determined before and during treatment to monitor its effectiveness. Alternatively, or concomitantly, the genotype status of at least one risk variant for UBC as presented herein is determined before and during treatment to monitor its effectiveness.

In a further aspect, the markers of the present invention can be used to increase power and effectiveness of clinical trials. Thus, individuals who are carriers of at-risk variants described herein may be more likely to respond favorably to a particular treatment modality for Bladder Cancer. In one embodiment, individuals who carry an at-risk variant are more likely to be responders to the treatment. In another embodiment, individuals who carry at-risk variants of a gene, which expression and/or function is altered by the at-risk variant (*e.g.,* the at-risk missense variants in the *SLC14A1* described herein), are more likely to be responders to a treatment modality targeting that gene, its expression or its gene product. This application can improve the safety of clinical trials, but can also enhance the chance that a clinical trial will demonstrate statistically significant efficacy, which may be limited to a certain sub-group of the population. Thus, one possible outcome of such a trial is that carriers of certain genetic variants, *e.g.,* at-risk markers described herein, are statistically significantly likely to show positive response to the therapeutic agent, i.e. experience alleviation of symptoms associated with Bladder Cancer when taking the therapeutic agent or drug as prescribed.

*Computer-implemented aspects*

As understood by those of ordinary skill in the art, the methods and information described herein may be implemented, in all or in part, as computer executable instructions on known computer readable media. For example, the methods described herein may be implemented in hardware. Alternatively, the method may be implemented in software stored in, for example, one or more memories or other computer readable medium and implemented on one or more processors. As is known, the processors may be associated with one or more controllers, calculation units and/or other units of a computer system, or implanted in firmware as desired. If implemented in software, the routines may be stored in any computer readable memory such as in RAM, ROM, flash memory, a magnetic disk, a laser disk, or other storage medium, as is also known. Likewise, this software may be delivered to a computing device via any known delivery method including, for example, over a communication channel such as a telephone line, the Internet, a wireless connection, etc., or via a transportable medium, such as a computer readable disk, flash drive, etc.

More generally, and as understood by those of ordinary skill in the art, the various steps described above may be implemented as various blocks, operations, tools, modules and techniques which, in turn, may be implemented in hardware, firmware, software, or any combination of hardware, firmware, and/or software. When implemented in hardware, some or all of the blocks, operations, techniques, etc. may be implemented in, for example, a custom integrated circuit (IC), an application specific integrated circuit (ASIC), a field programmable logic array (FPGA), a programmable logic array (PLA), etc.

When implemented in software, the software may be stored in any known computer readable medium such as on a magnetic disk, an optical disk, or other storage medium, in a RAM or ROM or flash memory of a computer, processor, hard disk drive, optical disk drive, tape drive, etc. Likewise, the software may be delivered to a user or a computing system via any known delivery method including, for example, on a computer readable disk or other transportable computer storage mechanism.

Thus, another aspect of the invention is a system that is capable of carrying out a part or all of a method of the invention, or carrying out a variation of a method of the invention as described herein in greater detail. Exemplary systems include, as one or more components, computing systems, environments, and/or configurations that may be suitable for use with the methods and include, but are not limited to, personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, and the like. In some variations, a system of the invention includes one or more machines used for analysis of biological material (e.g., genetic material), as described herein. In some variations, this analysis of the biological material involves a chemical analysis and/or a nucleic acid amplification.

Fig. 1 illustrates an example of a suitable computing system environment 100 on which a system for the steps of the claimed method and apparatus may be implemented. The computing system environment 100 is only one example of a suitable computing environment and is not intended to suggest any limitation as to the scope of use or functionality of the method or apparatus of the claims. Neither should the computing environment 100 be interpreted as having any dependency or requirement relating to any one or combination of components illustrated in the exemplary operating environment 100.

The steps of the claimed method and system are operational with numerous other general purpose or special purpose computing system environments or configurations. Examples of well known computing systems, environments, and/or configurations that may be suitable for use with the methods or system of the claims include, but are not limited to, personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, and the like.

The steps of the claimed method and system may be described in the general context of computer-executable instructions, such as program modules, being executed by a computer. Generally, program modules include routines, programs, objects, components, data structures, etc. that perform particular tasks or implement particular abstract data types. The methods and apparatus may also be practiced in distributed computing environments where tasks are performed by remote processing devices that are linked through a communications network. In both integrated and distributed computing environments, program modules may be located in both local and remote computer storage media including memory storage devices.

With reference to Fig. 1, an exemplary system for implementing the steps of the claimed method and system includes a general purpose computing device in the form of a computer 110. Components of computer 110 may include, but are not limited to, a processing unit 120, a system memory 130, and a system bus 121 that couples various system components including the system memory to the processing unit 120. The system bus 121 may be any of several types of bus structures including a memory bus or memory controller, a peripheral bus, and a local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus also known as Mezzanine bus.

Computer 110 typically includes a variety of computer readable media. Computer readable media can be any available media that can be accessed by computer 110 and includes both volatile and nonvolatile media, removable and non-removable media. By way of example, and not limitation, computer readable media may comprise computer storage media and communication media. Computer storage media includes both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can accessed by computer 110. Communication media typically embodies computer readable instructions, data structures, program modules or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. Combinations of the any of the above should also be included within the scope of computer readable media.

The system memory 130 includes computer storage media in the form of volatile and/or nonvolatile memory such as read only memory (ROM) 131 and random access memory (RAM) 132. A basic input/output system 133 (BIOS), containing the basic routines that help to transfer information between elements within computer 110, such as during start-up, is typically stored in ROM 131. RAM 132 typically contains data and/or program modules that are immediately accessible to and/or presently being operated on by processing unit 120. By way of example, and not limitation, Fig. 1 illustrates operating system 134, application programs 135, other program modules 136, and program data 137.

The computer 110 may also include other removable/non-removable, volatile/nonvolatile computer storage media. By way of example only, Fig. 1 illustrates a hard disk drive 140 that reads from or writes to non-removable, nonvolatile magnetic media, a magnetic disk drive 151 that reads from or writes to a removable, nonvolatile magnetic disk 152, and an optical disk drive 155 that reads from or writes to a removable, nonvolatile optical disk 156 such as a CD ROM or other optical media. Other removable/non-removable, volatile/nonvolatile computer storage media that can be used in the exemplary operating environment include, but are not limited to, magnetic tape cassettes, flash memory cards, digital versatile disks, digital video tape, solid state RAM, solid state ROM, and the like. The hard disk drive 141 is typically connected to the system bus 121 through a non-removable memory interface such as interface 140, and magnetic disk drive 151 and optical disk drive 155 are typically connected to the system bus 121 by a removable memory interface, such as interface 150.

The drives and their associated computer storage media discussed above and illustrated in Fig. 1, provide storage of computer readable instructions, data structures, program modules and other data for the computer 110. In Fig. 1, for example, hard disk drive 141 is illustrated as storing operating system 144, application programs 145, other program modules 146, and program data 147. Note that these components can either be the same as or different from operating system 134, application programs 135, other program modules 136, and program data 137. Operating system 144, application programs 145, other program modules 146, and program data 147 are given different numbers here to illustrate that, at a minimum, they are different copies. A user may enter commands and information into the computer 20 through input devices such as a keyboard 162 and pointing device 161, commonly referred to as a mouse, trackball or touch pad. Other input devices (not shown) may include a microphone, joystick, game pad, satellite dish, scanner, or the like. These and other input devices are often connected to the processing unit 120 through a user input interface 160 that is coupled to the system bus, but may be connected by other interface and bus structures, such as a parallel port, game port or a universal serial bus (USB). A monitor 191 or other type of display device is also connected to the system bus 121 via an interface, such as a video interface 190. In addition to the monitor, computers may also include other peripheral output devices such as speakers 197 and printer 196, which may be connected through an output peripheral interface 190.

The computer 110 may operate in a networked environment using logical connections to one or more remote computers, such as a remote computer 180. The remote computer 180 may be a personal computer, a server, a router, a network PC, a peer device or other common network node, and typically includes many or all of the elements described above relative to the computer 110, although only a memory storage device 181 has been illustrated in Fig. 1. The logical connections depicted in Fig. 1 include a local area network (LAN) 171 and a wide area network (WAN) 173, but may also include other networks. Such networking environments are commonplace in offices, enterprise-wide computer networks, intranets and the Internet.

When used in a LAN networking environment, the computer 110 is connected to the LAN 171 through a network interface or adapter 170. When used in a WAN networking environment, the computer 110 typically includes a modem 172 or other means for establishing communications over the WAN 173, such as the Internet. The modem 172, which may be internal or external, may be connected to the system bus 121 via the user input interface 160, or other appropriate mechanism. In a networked environment, program modules depicted relative to the computer 110, or portions thereof, may be stored in the remote memory storage device. By way of example, and not limitation, Fig. 1 illustrates remote application programs 185 as residing on memory device 181. It will be appreciated that the network connections shown are exemplary and other means of establishing a communications link between the computers may be used.

While the risk evaluation system and method, and other elements, have been described as preferably being implemented in software, they may be implemented in hardware, firmware, etc., and may be implemented by any other processor. Thus, the elements described herein may be implemented in a standard multi-purpose CPU or on specifically designed hardware or firmware such as an application-specific integrated circuit (ASIC) or other hard-wired device as desired, including, but not limited to, the computer 110 of Fig. 1. When implemented in software, the software routine may be stored in any computer readable memory such as on a magnetic disk, a laser disk, or other storage medium, in a RAM or ROM of a computer or processor, in any database, etc. Likewise, this software may be delivered to a user or a diagnostic system via any known or desired delivery method including, for example, on a computer readable disk or other transportable computer storage mechanism or over a communication channel such as a telephone line, the internet, wireless communication, etc. (which are viewed as being the same as or interchangeable with providing such software via a transportable storage medium).

Thus, many modifications and variations may be made in the techniques and structures described and illustrated herein without departing from the spirit and scope of the present invention. Thus, it should be understood that the methods and apparatus described herein are illustrative only and are not limiting upon the scope of the invention.

Accordingly, certain aspects of the invention relate to computer-implemented applications using the polymorphic markers and haplotypes described herein, and genotype and/or disease-association data derived therefrom. Such applications can be useful for storing, manipulating or otherwise analyzing genotype data that is useful in the methods of the invention. One example pertains to storing genotype and/or sequence data derived from an individual on readable media, so as to be able to provide the data to a third party (*e.g.,* the individual, a guardian of the individual, a health care provider or genetic analysis service provider), or for deriving information from the data, *e.g.,* by comparing the data to information about genetic risk factors contributing to increased susceptibility to Bladder Cancer, and reporting results based on such comparison.

In certain embodiments, computer-readable media suitably comprise capabilities of storing (i) identifier information for at least one polymorphic marker (*e.g*, marker names), as described herein; (ii) an indicator of the identity (*e.g.,* presence or absence) of at least one allele of said at least one marker in individuals with the disease (*e.g.*, rs1058396, rs11877062, rs2298720 or rs2298719, or the encoded protein variants); and (iii) an indicator of the risk associated with a particular marker allele (*e.g.,* the G allele of rs1058396). The media may also be suitably comprise capabilities of storing protein sequence data.

In one embodiment, the invention provides a computer-readable medium having computer executable instructions for determining susceptibility to Bladder Cancer in a human individual, the computer readable medium comprising (i) sequence data identifying at least one allele of at least one polymorphic marker in the individual; and (ii) a routine stored on the computer readable medium and adapted to be executed by a processor to determine risk of developing Bladder Cancer for the at least one polymorphic marker; wherein the at least one polymorphic marker is a marker in the human *SLC14A1* gene, or an amino acid substitution in an encoded SLC14A1 protein, that is predictive of susceptibility of Bladder Cancer in humans. In one embodiment, the at least one polymorphic marker is selected from the group consisting of rs1058396, ras11877062, rs2298720 and rs2298719. In another embodiment, the amino acid substitution is a substitution selected from the group consisting of N280D, R4W, K44E and M167V.

In certain embodiments, a report is prepared, which contains results of a determination of susceptibility of bladder cancer. The report may suitably be written in any computer readable medium, printed on paper, or displayed on a visual display.

With reference to Fig. 2, a second exemplary system of the invention, which may be used to implement one or more steps of methods of the invention, includes a computing device in the form of a computer 110. Components shown in dashed outline are not technically part of the computer 110, but are used to illustrate the exemplary embodiment of Fig. 2. Components of computer 110 may include, but are not limited to, a processor 120, a system memory 130, a memory/graphics interface 121, also known as a Northbridge chip, and an I/O interface 122, also known as a Southbridge chip. The system memory 130 and a graphics processor 190 may be coupled to the memory/graphics interface 121. A monitor 191 or other graphic output device may be coupled to the graphics processor 190.

A series of system busses may couple various system components including a high speed system bus 123 between the processor 120, the memory/graphics interface 121 and the I/O interface 122, a front-side bus 124 between the memory/graphics interface 121 and the system memory 130, and an advanced graphics processing (AGP) bus 125 between the memory/graphics interface 121 and the graphics processor 190. The system bus 123 may be any of several types of bus structures including, by way of example, and not limitation, such architectures include Industry Standard Architecture (ISA) bus, Micro Channel Architecture (MCA) bus and Enhanced ISA (EISA) bus. As system architectures evolve, other bus architectures and chip sets may be used but often generally follow this pattern. For example, companies such as Intel and AMD support the Intel Hub Architecture (IHA) and the Hypertransport™ architecture, respectively.

The computer 110 typically includes a variety of computer-readable media. Computer-readable media can be any available media that can be accessed by computer 110 and includes both volatile and nonvolatile media, removable and non-removable media. By way of example, and not limitation, computer readable media may comprise computer storage media. Computer storage media includes both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other physical medium which can be used to store the desired information and which can accessed by computer 110.

The system memory 130 includes computer storage media in the form of volatile and/or nonvolatile memory such as read only memory (ROM) 131 and random access memory (RAM) 132. The system ROM 131 may contain permanent system data 143, such as identifying and manufacturing information. In some embodiments, a basic input/output system (BIOS) may also be stored in system ROM 131. RAM 132 typically contains data and/or program modules that are immediately accessible to and/or presently being operated on by processor 120. By way of example, and not limitation, Fig. 5 illustrates operating system 134, application programs 135, other program modules 136, and program data 137.

The I/O interface 122 may couple the system bus 123 with a number of other busses 126, 127 and 128 that couple a variety of internal and external devices to the computer 110. A serial peripheral interface (SPI) bus 126 may connect to a basic input/output system (BIOS) memory 133 containing the basic routines that help to transfer information between elements within computer 110, such as during start-up.

A super input/output chip 160 may be used to connect to a number of 'legacy' peripherals, such as floppy disk 152, keyboard/mouse 162, and printer 196, as examples. The super I/O chip 160 may be connected to the I/O interface 122 with a bus 127, such as a low pin count (LPC) bus, in some embodiments. Various embodiments of the super I/O chip 160 are widely available in the commercial marketplace.

In one embodiment, bus 128 may be a Peripheral Component Interconnect (PCI) bus, or a variation thereof, may be used to connect higher speed peripherals to the I/O interface 122. A PCI bus may also be known as a Mezzanine bus. Variations of the PCI bus include the Peripheral Component Interconnect-Express (PCI-E) and the Peripheral Component Interconnect - Extended (PCI-X) busses, the former having a serial interface and the latter being a backward compatible parallel interface. In other embodiments, bus 128 may be an advanced technology attachment (ATA) bus, in the form of a serial ATA bus (SATA) or parallel ATA (PATA).

The computer 110 may also include other removable/non-removable, volatile/nonvolatile computer storage media. By way of example only, Fig. 2 illustrates a hard disk drive 140 that reads from or writes to non-removable, nonvolatile magnetic media. The hard disk drive 140 may be a conventional hard disk drive..

Removable media, such as a universal serial bus (USB) memory 153, firewire (IEEE 1394), or CD/DVD drive 156 may be connected to the PCI bus 128 directly or through an interface 150. A storage media 154 may coupled through interface 150. Other removable/non-removable, volatile/nonvolatile computer storage media that can be used in the exemplary operating environment include, but are not limited to, magnetic tape cassettes, flash memory cards, digital versatile disks, digital video tape, solid state RAM, solid state ROM, and the like.

The drives and their associated computer storage media discussed above and illustrated in Fig. 2, provide storage of computer readable instructions, data structures, program modules and other data for the computer 110. In Fig. 2, for example, hard disk drive 140 is illustrated as storing operating system 144, application programs 145, other program modules 146, and program data 147. Note that these components can either be the same as or different from operating system 134, application programs 135, other program modules 136, and program data 137. Operating system 144, application programs 145, other program modules 146, and program data 147 are given different numbers here to illustrate that, at a minimum, they are different copies. A user may enter commands and information into the computer 20 through input devices such as a mouse/keyboard 162 or other input device combination. Other input devices (not shown) may include a microphone, joystick, game pad, satellite dish, scanner, or the like. These and other input devices are often connected to the processor 120 through one of the I/O interface busses, such as the SPI 126, the LPC 127, or the PCI 128, but other busses may be used. In some embodiments, other devices may be coupled to parallel ports, infrared interfaces, game ports, and the like (not depicted), via the super I/O chip 160.

The computer 110 may operate in a networked environment using logical connections to one or more remote computers, such as a remote computer 180 via a network interface controller (NIC) 170. The remote computer 180 may be a personal computer, a server, a router, a network PC, a peer device or other common network node, and typically includes many or all of the elements described above relative to the computer 110. The logical connection between the NIC 170 and the remote computer 180 depicted in Fig. 2 may include a local area network (LAN), a wide area network (WAN), or both, but may also include other networks. Such networking environments are commonplace in offices, enterprise-wide computer networks, intranets, and the Internet. The remote computer 180 may also represent a web server supporting interactive sessions with the computer 110, or in the specific case of location-based applications may be a location server or an application server.

In some embodiments, the network interface may use a modem (not depicted) when a broadband connection is not available or is not used. It will be appreciated that the network connection shown is exemplary and other means of establishing a communications link between the computers may be used.

In some variations, the invention is a system for identifying susceptibility to bladder cancer in a human subject. For example, in one variation, the system includes tools for performing at least one step, preferably two or more steps, and in some aspects all steps of a method of the invention, where the tools are operably linked to each other. Operable linkage describes a linkage through which components can function with each other to perform their purpose.

In some variations, a system of the invention is a system for identifying susceptibility to bladder cancer in a human subject, and comprises:

(a) at least one processor;

(b) at least one computer-readable medium;

(c) a susceptibility database operatively coupled to a computer-readable medium of the system and containing population information correlating the presence or absence of one or more alleles of the human SLC14A1 gene and susceptibility to bladder cancer in a population of humans;

(d) a measurement tool that receives an input about the human subject and generates information from the input about the presence or absence of at least one mutant SLC14A1 allele in the human subject; and

(e) an analysis tool or routine that:

(i) is operatively coupled to the susceptibility database and the information generated by the measurement tool,

(ii) is stored on a computer-readable medium of the system,

(iii) is adapted to be executed on a processor of the system, to compare the information about the human subject with the population information in the susceptibility database and generate a conclusion with respect to susceptibility to the condition for the human subject.

Exemplary processors (processing units) include all variety of microprocessors and other processing units used in computing devices. Exemplary computer-readable media are described above. When two or more components of the system involve a processor or a computer-readable medium, the system generally can be created where a single processor and/or computer readable medium is dedicated to a single component of the system; or where two or more functions share a single processor and/or share a single computer readable medium, such that the system contains as few as one processor and/or one computer readable medium. In some variations, it is advantageous to use multiple processors or media, for example, where it is convenient to have components of the system at different locations. For instance, some components of a system may be located at a testing laboratory dedicated to laboratory or data analysis, whereas other components, including components (optional) for supplying input information or obtaining an output communication, may be located at a medical treatment or counseling facility (e.g., doctor's office, health clinic, HMO, pharmacist, geneticist, hospital) and/or at the home or business of the human subject (patient) for whom the testing service is performed.

Referring to Figure 3, an exemplary system includes a susceptibility database 208 that is operatively coupled to a computer-readable medium of the system and that contains population information correlating the presence or absence of one or more alleles of the human SLC14A1 gene and susceptibility to bladder cancer in a population of humans. For example, the one or more alleles of the SLC14A1 gene include mutant alleles that cause, or are indicative of, a SLC14A1 defect such as reduced or lost function, as described elsewhere herein.

In a simple variation, the susceptibility database contains 208 data relating to the frequency that a particular allele of SLC14A1 has been observed in a population of humans with bladder cancer and a population of humans free of bladder cancer. Such data provides an indication as to the relative risk or odds ratio of developing bladder cancer for a human subject that is identified as having the allele in question. In another variation, the susceptibility database includes similar data with respect to two or more alleles of SLC14A1, thereby providing a useful reference if the human subject has any of the two or more alleles. In still another variation, the susceptibility database includes additional quantitative personal, medical, or genetic information about the individuals in the database diagnosed with bladder cancer or free of bladder cancer. Such information includes, but is not limited to, information about parameters such as age, sex, ethnicity, race, medical history, weight, diabetes status, blood pressure, family history of bladder cancer, smoking history, and alcohol use in humans and impact of the at least one parameter on susceptibility to bladder cancer. The information also can include information about other genetic risk factors for bladder cancer besides SLC14A1 alleles. These more robust susceptibility databases can be used by an analysis routine 210 to calculate a combined score with respect to susceptibility or risk for developing bladder cancer.

In addition to the susceptibility database 208, the system further includes a measurement tool 206 programmed to receive an input 204 from or about the human subject and generate an output that contains information about the presence or absence of the at least one SLC14A1 allele of interest. (The input 204 is not part of the system per se but is illustrated in the schematic Figure 3.) Thus, the input 204 will contain a specimen or contain data from which the presence or absence of the at least one SLC14A1 allele can be directly read, or analytically determined. In a simple variation, the input contains annotated information about genotypes or allele counts for SLC14A1 in the genome of the human subject, in which case no further processing by the measurement tool 206 is required, except possibly transformation of the relevant information about the presence/absence of the SLC14A1 allele into a format compatible for use by the analysis routine 210 of the system.

In another variation, the input 204 from the human subject contains data that is unannotated or insufficiently annotated with respect to SLC14A1, requiring analysis by the measurement tool 206.

For example, the input can be genetic sequence of a chromosomal region or chromosome on which SLC14A1 resides, or whole genome sequence information, or unannotated information from a gene chip analysis of a variable loci in the human subject's genome. In such variations of the invention, the measurement tool 206 comprises a tool, preferably stored on a computer-readable medium of the system and adapted to be executed on a processor of the system, to receive a data input about a subject and determine information about the presence or absence of the at least one mutant SLC14A1 allele in a human subject from the data. For example, the measurement tool 206 contains instructions, preferably executable on a processor of the system, for analyzing the unannotated input data and determining the presence or absence of the SLC14A1 allele of interest in the human subject. Where the input data is genomic sequence information, and the measurement tool optionally comprises a sequence analysis tool stored on a computer readable medium of the system and executable by a processor of the system with instructions for determining the presence or absence of the at least one mutant SLC14A1 allele from the genomic sequence information.

In yet another variation, the input 204 from the human subject comprises a biological sample, such as a fluid (e.g., blood) or tissue sample that contains genetic material that can be analyzed to determine the presence or absence of the SLC14A1 allele of interest. In this variation, an exemplary measurement tool 206 includes laboratory equipment for processing and analyzing the sample to determine the presence or absence (or identity) of the SLC14A1 allele(s) in the human subject. For instance, in one variation, the measurement tool includes: an oligonucleotide microarray (e.g., "gene chip") containing a plurality of oligonucleotide probes attached to a solid support; a detector for measuring interaction between nucleic acid obtained from or amplified from the biological sample and one or more oligonucleotides on the oligonucleotide microarray to generate detection data; and an analysis tool stored on a computer-readable medium of the system and adapted to be executed on a processor of the system, to determine the presence or absence of the at least one SLC14A1 allele of interest based on the detection data.

To provide another example, in some variations the measurement tool 206 includes: a nucleotide sequencer (e.g., an automated DNA sequencer) that is capable of determining nucleotide sequence information from nucleic acid obtained from or amplified from the biological sample; and an analysis tool stored on a computer-readable medium of the system and adapted to be executed on a processor of the system, to determine the presence or absence of the at least one mutant SLC14A1 allele based on the nucleotide sequence information.

In some variations, the measurement tool 206 further includes additional equipment and/or chemical reagents for processing the biological sample to purify and/or amplify nucleic acid of the human subject for further analysis using a sequencer, gene chip, or other analytical equipment.

The exemplary system further includes an analysis tool or routine 210 that: is operatively coupled to the susceptibility database 208 and operatively coupled to the measurement tool 206, is stored on a computer-readable medium of the system, is adapted to be executed on a processor of the system to compare the information about the human subject with the population information in the susceptibility database 208 and generate a conclusion with respect to susceptibility to bladder cancer for the human subject. In simple terms, the analysis tool 210 looks at the SLC14A1 alleles identified by the measurement tool 206 for the human subject, and compares this information to the susceptibility database 208, to determine a susceptibility to bladder cancer for the subject. The susceptibility can be based on the single parameter (the identity of one or more SLC14A1 alleles), or can involve a calculation based on other genetic and non-genetic data, as described above, that is collected and included as part of the input 204 from the human subject, and that also is stored in the susceptibility database 208 with respect to a population of other humans. Generally speaking, each parameter of interest is weighted to provide a conclusion with respect to susceptibility to bladder cancer. Such a conclusion is expressed in the conclusion in any statistically useful form, for example, as an odds ratio, a relative risk, or a lifetime risk for subject developing the condition.

In some variations of the invention, the system as just described further includes a communication tool 212. For example, the communication tool is operatively connected to the analysis routine 210 and comprises a routine stored on a computer-readable medium of the system and adapted to be executed on a processor of the system, to: generate a communication containing the conclusion; and to transmit the communication to the human subject 200 or the medical practitioner 202, and/or enable the subject or medical practitioner to access the communication. (The subject and medical practitioner are depicted in the schematic Fig. 3, but are not part of the system per se, though they may be considered users of the system. The communication tool 212 provides an interface for communicating to the subject, or to a medical practitioner for the subject (e.g., doctor, nurse, genetic counselor), the conclusion generated by the analysis tool 210 with respect to susceptibility to the condition for the subject. Usually, if the communication is obtained by or delivered to the medical practitioner 202, the medical practitioner will share the communication with the human subject 200 and/or counsel the human subject about the medical significance of the communication. In some variations, the communication is provided in a tangible form, such as a printed report or report stored on a computer readable medium such as a flash drive or optical disk. In some variations, the communication is provided electronically with an output that is visible on a video display or audio output (e.g., speaker). In some variations, the communication is transmitted to the subject or the medical practitioner, e.g., electronically or through the mail. In some variations, the system is designed to permit the subject or medical practitioner to access the communication, e.g., by telephone or computer. For instance, the system may include software residing on a memory and executed by a processor of a computer used by the human subject or the medical practitioner, with which the subject or practitioner can access the communication, preferably securely, over the internet or other network connection. In some variations of the system, this computer will be located remotely from other components of the system, e.g., at a location of the human subject's or medical practitioner's choosing.

In some variations of the invention, the system as described (including embodiments with or without the communication tool) further includes components that add a treatment or prophylaxis utility to the system. For instance, value is added to a determination of susceptibility to bladder cancer when a medical practitioner can prescribe or administer a standard of care that can reduce susceptibility to bladder cancer; and/or delay onset of bladder cancer; and/or increase the likelihood of detecting the cancer at an early stage. Exemplary lifestyle change protocols include loss of weight, increase in exercise, cessation of unhealthy behaviors such as smoking, and change of diet. Exemplary medicinal and surgical intervention protocols include administration of pharmaceutical agents for prophylaxis; and surgery.

For example, in some variations, the system further includes a medical protocol database 214 operatively connected to a computer-readable medium of the system and containing information correlating the presence or absence of the at least one SLC14A1 allele of interest and medical protocols for human subjects at risk for the cancer. Such medical protocols include any variety of medicines, lifestyle changes, diagnostic tests, increased frequencies of diagnostic tests, and the like that are designed to achieve one of the aforementioned goals. The information correlating a SLC14A1 allele with protocols could include, for example, information about the success with which the cancer is avoided or delayed, or success with which the cancer is detected early and treated, if a subject has a SLC14A1 susceptibility allele and follows a protocol.

The system of this embodiment further includes a medical protocol tool or routine 216, operatively connected to the medical protocol database 214 and to the analysis tool or routine 210. The medical protocol tool or routine 216 preferably is stored on a computer-readable medium of the system, and adapted to be executed on a processor of the system, to: (i) compare (or correlate) the conclusion that is obtained from the analysis routine 210 (with respect to susceptibility to bladder cancer for the subject) and the medical protocol database 214, and (ii) generate a protocol report with respect to the probability that one or more medical protocols in the medical protocol database will achieve one or more of the goals of reducing susceptibility to the cancer; delaying onset of the cancer; and increasing the likelihood of detecting the cancer at an early stage to facilitate early treatment. The probability can be based on empirical evidence collected from a population of humans and expressed either in absolute terms (e.g., compared to making no intervention), or expressed in relative terms, to highlight the comparative or additive benefits of two or more protocols.

Some variations of the system include the communication tool 212. In some examples, the communication tool generates a communication that includes the protocol report in addition to, or instead of, the conclusion with respect to susceptibility.

Information about SLC14A1 allele status not only can provide useful information about identifying or quantifying susceptibility to the cancer; it can also provide useful information about possible causative factors for a human subject identified with the cancer, and useful information about therapies for the patient. In some variations, systems of the invention are useful for these purposes.

For instance, in some variations the invention is a system for assessing or selecting a treatment protocol for a subject diagnosed with bladder cancer. An exemplary system, schematically depicted in Figure 4, comprises:

(a) at least one processor;

(b) at least one computer-readable medium;

(c) a medical treatment database 308 operatively connected to a computer-readable medium of the system and containing information correlating the presence or absence of at least one SLC14A1 allele and efficacy of treatment regimens for bladder cancer;

(d) a measurement tool 306 to receive an input (304, depicted in Fig. 4 but not part of the system per se) about the human subject and generate information from the input 304 about the presence or absence of the at least one SLC14A1 allele in a human subject diagnosed with bladder cancer; and

(e) a medical protocol routine or tool 310 operatively coupled to the medical treatment database 308 and the measurement tool 306, stored on a computer-readable medium of the system, and adapted to be executed on a processor of the system, to compare the information with respect to presence or absence of the at least one SLC14A1 allele for the subject and the medical treatment database, and generate a conclusion with respect to at least one of:

(i) the probability that one or more medical treatments will be efficacious for treatment of bladder cancer for the patient; and

(ii) which of two or more medical treatments for bladder cancer will be more efficacious for the patient.

Preferably, such a system further includes a communication tool 312 operatively connected to the medical protocol tool or routine 310 for communicating the conclusion to the subject 300, or to a medical practitioner for the subject 302 (both depicted in the schematic of Fig. 4, but not part of the system per se). An exemplary communication tool comprises a routine stored on a computer-readable medium of the system and adapted to be executed on a processor of the system, to generate a communication containing the conclusion; and transmit the communication to the subject or the medical practitioner, or enable the subject or medical practitioner to access the communication.

In a further embodiment, the invention provides a computer-readable medium having computer executable instructions for determining susceptibility to bladder cancer in a human individual, the computer readable medium comprising (i) sequence data identifying at least one allele of at least one polymorphic marker in the individual; and (ii) a routine stored on the computer readable medium and adapted to be executed by a processor to determine risk of developing bladder cancer for the at least one polymorphic marker; wherein the at least one polymorphic marker is a marker in the human *SLC14A1* gene, or an amino acid substitution in an encoded SLC14A1 protein, that is predictive of susceptibility of bladder cancer in humans. In one embodiment, the at least one polymorphic marker is selected from the group consisting of rs1058396, rs11877062, rs2298720 and rs2298719. In one preferred embodiment, the polymorphic marker is rs1058396. In another embodiment, the amino acid substitution is selected from the group consisting of an N280D substitution, a R4W substitution, a K44E substitution and a M167V substitution. In one preferred embodiment, the amino acid substitution is a N280D substitution.

In certain embodiments, a report is prepared, which contains results of a determination of susceptibility of bladder cancer. The report may suitably be written in any computer readable medium, printed on paper, or displayed on a visual display.

*Nucleic acids and polypeptides*

The nucleic acids and polypeptides described herein can be used in methods and kits of the present invention. An "isolated" nucleic acid molecule, as used herein, is one that is separated from nucleic acids that normally flank the gene or nucleotide sequence (as in genomic sequences) and/or has been completely or partially purified from other transcribed sequences (e.g., as in an RNA library). For example, an isolated nucleic acid of the invention can be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material can be purified to essential homogeneity, for example as determined by polyacrylamide gel electrophoresis (PAGE) or column chromatography *(e.g.,* HPLC). An isolated nucleic acid molecule of the invention can comprise at least about 50%, at least about 80% or at least about 90% (on a molar basis) of all macromolecular species present. With regard to genomic DNA, the term "isolated" also can refer to nucleic acid molecules that are separated from the chromosome with which the genomic DNA is naturally associated. For example, the isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of the nucleotides that flank the nucleic acid molecule in the genomic DNA of the cell from which the nucleic acid molecule is derived.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells or heterologous organisms, as well as partially or substantially purified DNA molecules in solution. "Isolated" nucleic acid molecules also encompass *in vivo* and *in vitro* RNA transcripts of the DNA molecules of the present invention. An isolated nucleic acid molecule or nucleotide sequence can include a nucleic acid molecule or nucleotide sequence that is synthesized chemically or by recombinant means. Such isolated nucleotide sequences are useful, for example, in the manufacture of the encoded polypeptide, as probes for isolating homologous sequences (e.g., from other mammalian species), for gene mapping (e.g., by *in situ* hybridization with chromosomes), or for detecting expression of the gene in tissue (e.g., human tissue), such as by Northern blot analysis or other hybridization techniques.

Also disclosed are nucleic acid molecules that hybridize under high stringency hybridization conditions, such as for selective hybridization, to a nucleotide sequence described herein (*e.g.*, nucleic acid molecules that specifically hybridize to a nucleotide sequence containing a polymorphic site associated with a marker or haplotype described herein). Such nucleic acid molecules can be detected and/or isolated by allele- or sequence-specific hybridization (*e.g.*, under high stringency conditions). Stringency conditions and methods for nucleic acid hybridizations are well known to the skilled person (see, *e.g.,* Current Protocols in Molecular Biology, Ausubel, F. et al, John Wiley & Sons, (1998), and Kraus, M. and Aaronson, S., Methods Enzymol., 200:546-556 (1991).

The percent identity of two nucleotide or amino acid sequences can be determined by aligning the sequences for optimal comparison purposes (*e.g.*, gaps can be introduced in the sequence of a first sequence). The nucleotides or amino acids at corresponding positions are then compared, and the percent identity between the two sequences is a function of the number of identical positions shared by the sequences (i.e., % identity = # of identical positions/total # of positions x 100). In certain embodiments, the length of a sequence aligned for comparison purposes is at least 30%, at least 40%, at least 50%, at least 60%, at least 70%, at least 80%, at least 90%, or at least 95%, of the length of the reference sequence. The actual comparison of the two sequences can be accomplished by well-known methods, for example, using a mathematical algorithm. A non-limiting example of such a mathematical algorithm is described in Karlin, S. and Altschul, S., Proc. Natl. Acad. Sci. USA, 90:5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, the default parameters of the respective programs (*e.g.*, NBLAST) can be used. See the website on the world wide web at ncbi.nlm.nih.gov. In one embodiment, parameters for sequence comparison can be set at score=100, wordlength=12, or can be varied (*e.g.*, W=5 or W=20). Another example of an algorithm is BLAT (Kent, W.J. Genome Res. 12:656-64 (2002)).

Other examples include the algorithm of Myers and Miller, CABIOS (1989), ADVANCE and ADAM as described in Torellis, A. and Robotti, C., Comput. Appl. Biosci. 10:3-5 (1994); and FASTA described in Pearson, W. and Lipman, D., Proc. Natl. Acad. Sci. USA, 85:2444-48 (1988). In another embodiment, the percent identity between two amino acid sequences can be accomplished using the GAP program in the GCG software package (Accelrys, Cambridge, UK).

Also provided are nucleic acid molecules that contain a fragment or portion that hybridizes under highly stringent conditions to a nucleic acid that comprises, or consists of, the nucleotide sequence of the human *SLC14A1* gene as set forth in SEQ ID NO: 134, any one of SEQ ID NO: 1-132, or a nucleotide sequence comprising, or consisting of, the complement of the nucleotide sequence of the human *SLC14A1* gene as set forth in SEQ ID NO:134 or any one of SEQ ID NO:1-132. The nucleic acid fragments of the invention are at least about 15, at least about 18, 20, 23 or 25 nucleotides, and can be 30, 40, 50, 100, 200, 500, 1000, 10,000 or more nucleotides in length.

The nucleic acid fragments are used as probes or primers in assays such as those described herein. "Probes" or "primers" are oligonucleotides that hybridize in a base-specific manner to a complementary strand of a nucleic acid molecule. In addition to DNA and RNA, such probes and primers include polypeptide nucleic acids (PNA), as described in Nielsen, P. et al., Science 254:1497-1500 (1991). A probe or primer comprises a region of nucleotide sequence that hybridizes to at least about 15, typically about 20-25, and in certain embodiments about 40, 50 or 75, consecutive nucleotides of a nucleic acid molecule. In one embodiment, the probe or primer comprises at least one allele of at least one polymorphic marker or at least one haplotype described herein, or the complement thereof. In particular embodiments, a probe or primer can comprise 100 or fewer nucleotides; for example, in certain embodiments from 6 to 50 nucleotides, or, for example, from 12 to 30 nucleotides. In other embodiments, the probe or primer is at least 70% identical, at least 80% identical, at least 85% identical, at least 90% identical, or at least 95% identical, to the contiguous nucleotide sequence or to the complement of the contiguous nucleotide sequence. In another embodiment, the probe or primer is capable of selectively hybridizing to the contiguous nucleotide sequence or to the complement of the contiguous nucleotide sequence. Often, the probe or primer further comprises a label, *e.g.,* a radioisotope, a fluorescent label, an enzyme label, an enzyme co-factor label, a magnetic label, a spin label, an epitope label.

The nucleic acid molecules, such as those described above, can be identified and isolated using standard molecular biology techniques well known to the skilled person. The amplified DNA can be labeled (e.g., radiolabeled, fluorescently labeled) and used as a probe for screening a cDNA library derived from human cells. The cDNA can be derived from mRNA and contained in a suitable vector. Corresponding clones can be isolated, DNA obtained following *in vivo* excision, and the cloned insert can be sequenced in either or both orientations by art-recognized methods to identify the correct reading frame encoding a polypeptide of the appropriate molecular weight. Using these or similar methods, the polypeptide and the DNA encoding the polypeptide can be isolated, sequenced and further characterized.

*Antibodies*

The invention also provides antibodies which bind to an epitope comprising either a variant amino acid sequence (e.g., comprising an amino acid substitution) encoded by a variant allele or the reference amino acid sequence encoded by the corresponding non-variant or wild-type allele. The term "antibody" as used herein refers to immunoglobulin molecules and immunologically active portions of immunoglobulin molecules, *i.e.,* molecules that contain antigen-binding sites that specifically bind an antigen. A molecule that specifically binds to a polypeptide of the invention is a molecule that binds to that polypeptide or a fragment thereof, but does not substantially bind other molecules in a sample, *e.g.,* a biological sample, which naturally contains the polypeptide. Examples of immunologically active portions of immunoglobulin molecules include F(ab) and F(ab')$_2$ fragments which can be generated by treating the antibody with an enzyme such as pepsin. The invention provides polyclonal and monoclonal antibodies that bind to a polypeptide of the invention. The term "monoclonal antibody" or "monoclonal antibody composition", as used herein, refers to a population of antibody molecules that contain only one species of an antigen binding site capable of immunoreacting with a particular epitope of a polypeptide of the invention. A monoclonal antibody composition thus typically displays a single binding affinity for a particular polypeptide of the invention with which it immunoreacts.

Polyclonal antibodies can be prepared as described above by immunizing a suitable subject with a desired immunogen, e.g., polypeptide of the invention or a fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (e.g., from the blood) and further purified by well-known techniques, such as protein A chromatography to obtain the IgG fraction. At an appropriate time after immunization, *e.g.,* when the antibody titers are highest, antibody-producing cells can be obtained from the subject and used to prepare monoclonal antibodies by standard techniques, such as the hybridoma technique originally described by Kohler and Milstein, Nature 256:495-497 (1975), the human B cell hybridoma technique (Kozbor et al., Immunol. Today 4: 72 (1983)), the EBV-hybridoma technique (Cole et al., Monoclonal Antibodies and Cancer Therapy, Alan R. Liss,1985, Inc., pp. 77-96) or trioma techniques. The technology for producing hybridomas is well known (see generally Current Protocols in Immunology (1994) Coligan et al., (eds.) John Wiley & Sons, Inc., New York, NY). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide of the invention.

Any of the many well known protocols used for fusing lymphocytes and immortalized cell lines can be applied for the purpose of generating a monoclonal antibody to a polypeptide of the invention (see, *e.g., Current Protocols in Immunology, supra;* Galfre et al., Nature 266:55052 (1977); R.H. Kenneth, in Monoclonal Antibodies: A New Dimension In Biological Analyses, Plenum Publishing Corp., New York, New York (1980); and Lerner, Yale J. Biol. Med. 54:387-402 (1981)). Moreover, the ordinarily skilled worker will appreciate that there are many variations of such methods that also would be useful.

Alternative to preparing monoclonal antibody-secreting hybridomas, a monoclonal antibody to a polypeptide of the invention can be identified and isolated by screening a recombinant combinatorial immunoglobulin library (e.g., an antibody phage display library) with the polypeptide to thereby isolate immunoglobulin library members that bind the polypeptide. Kits for generating and screening phage display libraries are commercially available *(e.g.,* the Pharmacia *Recombinant Phage Antibody System,* Catalog No. 27-9400-01; and the Stratagene *Surf*ZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., Bio/Technology 9: 1370-1372 (1991); Hay et al., Hum. Antibod. Hybridomas 3:81-85 (1992); Huse et al., Science 246: 1275-1281 (1989); and Griffiths et al., EMBO J. 12:725-734 (1993).

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

In general, antibodies of the invention (*e.g*., a monoclonal antibody) can be used to isolate a polypeptide of the invention by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide of the invention can be used to detect the polypeptide (*e.g*., in a cellular lysate, cell supernatant, or tissue sample) in order to evaluate the abundance and pattern of expression of the polypeptide. Antibodies can be used diagnostically to monitor protein levels in tissue as part of a clinical testing procedure, *e.g*., to, for example, determine the efficacy of a given treatment regimen. The antibody can be coupled to a detectable substance to facilitate its detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include

$^{125}I$, $^{131}I$, $^{35}S$ or $^{3}H$.

Antibodies may also be useful in pharmacogenomic analysis. In such embodiments, antibodies against variant proteins encoded by nucleic acids according to the invention, such as variant proteins that are encoded by nucleic acids that contain at least one polymorpic marker of the invention, can be used to identify individuals that require modified treatment modalities.

Antibodies can furthermore be useful for assessing expression of variant proteins in disease states, such as in active stages of a disease, or in an individual with a predisposition to a disease related to the function of the protein, in particular urinary bladder cancer. Antibodies specific for a variant protein of the present invention can be used to screen for the presence of the variant protein, for example to screen for a predisposition to Bladder Cancer as indicated by the presence of the variant protein.

Antibodies can be used in other methods. Thus, antibodies are useful as diagnostic tools for evaluating proteins, such as variant proteins described herein, in conjunction with analysis by electrophoretic mobility, isoelectric point, tryptic or other protease digest, or for use in other physical assays known to those skilled in the art. Antibodies may also be used in tissue typing. In one such embodiment, a specific variant protein has been correlated with expression in a specific tissue type, and antibodies specific for the variant protein can then be used to identify the specific tissue type.

Subcellular localization of proteins, including variant proteins, can also be determined using antibodies, and can be applied to assess aberrant subcellular localization of the protein in cells in various tissues. Such use can be applied in genetic testing, but also in monitoring a particular treatment modality. In the case where treatment is aimed at correcting the expression level or presence of the variant protein or aberrant tissue distribution or developmental expression of the variant protein, antibodies specific for the variant protein or fragments thereof can be used to monitor therapeutic efficacy.

Antibodies are further useful for inhibiting variant protein function, for example by blocking the binding of a variant protein to a binding molecule or partner. Such uses can also be applied in a therapeutic context in which treatment involves inhibiting a variant protein's function. An antibody can be for example be used to block or competitively inhibit binding, thereby modulating (i.e., agonizing or antagonizing) the activity of the protein. Antibodies can be prepared against specific protein fragments containing sites required for specific function or against an intact protein that is associated with a cell or cell membrane. For administration *in vivo,* an antibody may be linked with an additional therapeutic payload, such as radionuclide, an enzyme, an immunogenic epitope, or a cytotoxic agent, including bacterial toxins (diphtheria or plant toxins, such as ricin). The *in vivo* half-life of an antibody or a fragment thereof may be increased by pegylation through conjugation to polyethylene glycol.

The present invention further relates to kits for using antibodies in the methods described herein. This includes, but is not limited to, kits for detecting the presence of a variant protein in a test sample. One preferred embodiment comprises antibodies such as a labelled or labelable antibody and a compound or agent for detecting variant proteins in a biological sample, means for determining the amount or the presence and/or absence of variant protein in the sample, and means for comparing the amount of variant protein in the sample with a standard, as well as instructions for use of the kit.

The present invention will now be exemplified by the following non-limiting example.

## EXAMPLE 1

Genetic association results on a total of 595 Icelandic Bladder Cancer cases and 37,075 Icelandic controls and close to 1,600 Dutch Bladder Cancer cases and 1,800 Dutch controls were analyzed together. The data analysed included a total of 2.5 million SNPs, including SNPs from the HumanHap 370DUO bead chip and SNPs imputed from the HapMap data. Using this dataset as a starting point, a focused analysis of the association between bladder cancer and about 20,000 non-synonymous SNPs was conducted. Based on study groups from Iceland and the Netherlands, this analysis yielded an association signal on chromosome 18q12.3, with the strongest signal observed for marker rs1058396. This SNP is located in the *SLC14A1* gene where it causes an amino acid variation, N280D (D conferring increased risk).

Association of rs1058396 was further confirmed by analysis (Centaurus genotyping) of additional samples from, Belgium, Germany, Eastern Europe, Italy (Brescia), Italy (Torino), Sweden and UK, giving an overall result of OR = 0.90 and a p-value of $3.7 \times 10^{-5}$ (Table 3).

In addition to rs1058396, a second non-synonymous SNP in the SLC14A1 gene, rs11877062 (causes amino acid change R4W), showed association with bladder cancer in the combined analysis of the Icelandic and Dutch sample sets (Table 4). The association between rs11877062 and bladder cancer was found to have an OR of 1.14 for the C allele (encoding W at position 4 in the encoded SLC14A1 polypeptide) and a P value of $7.1 \times 10^{-4}$.

Further association data for the missense variants rs2298719 (encoding M167V) and rs2298720 (encoding K44E) are shown in Table 5 (Iceland data only) and Table 6 (Iceland and Holland data).

**Table 2**. Study groups.

| *Discovery groups (GWA)* | #cases | #controls | Average age at diagnosis (range) | % males (cases) | Study type |
|---|---|---|---|---|---|
| Iceland | 595 | 37,075 | 68 (20-95) | 76 | Population based |
| Holland discovery qroup | 1,600 | 1,800 | 62 (25-93) | 81 | Population based |
| **Total** | 2195 | 38,875 | | | |
| **Follow up groups** | | | | | |
| Belgium, Leuven | 191 | 375 | 68 (40-93) | 86 | Population based |
| Germany, Lutherstadt Wittenberg | 213 | 194 | 65 (20-91) | 86 | Hospital-based |
| Eastern Europe (Hungary, Romania, Slovakia) | 200 | 526 | 65 (36-90) | 83 | Hospital-based |
| Italy, Brescia | 181 | 192 | 63 (22-80) | 100 | Hospital-based |
| Italy, Torino | 323 | 378 | 63 (40-75) | 100 | Hospital-based |
| Sweden, Stockholm | 344 | 1,224 | 69 (32-97) | 67 | Population based |
| UK, Leeds | 724 | 534 | 73 (30-101) | 71 | Hospital-based |
| **Total** | 2,176 | 3,423 | | | |

**Table** 3. Association between Urinary Bladder Cancer and rs1058396 (N280D in transcript 1 (SEQ ID NO:209; position 336 in transcript 2 (SEQ ID NO:133)). OR-values are reported for the A allele of rs1058396, the risk allele (with OR equalling 1/OR as listed in the table) is G, encoding an N to D change in the encoded SLC14A1 polypeptide.

| Discovery group | OR | P-value | 95%CI |
|---|---|---|---|
| Iceland chip | 0.84 | 0.0036 | (0.75,0.94) |
| Holland chip | 0.88 | 0.0034 | (0.81,0.96) |
| **Ice/Hol com bined** | **0.87** | **4.70E-05** | **(0.81,0.93)** |

| Follow up groups | OR | P-value | # affected | Freq affected | # controls | Freq controls |
|---|---|---|---|---|---|---|
| BEL | 1.1475 | 0.285541 | 191 | 0.515707 | 375 | 0.481333 |
| GERMANY | 0.8128 | 0.159806 | 213 | 0.448357 | 194 | 0.5 |
| EASTEUR | 0.8437 | 0.158602 | 200 | 0.4775 | 526 | 0.519962 |
| ITABR | 0.7919 | 0.123846 | 181 | 0.483425 | 192 | 0.541667 |
| ITATO | 1.0043 | 1 | 323 | 0.489164 | 378 | 0.488095 |
| SWE | 0.9372 | 0.484814 | 334 | 0.476048 | 1224 | 0.492239 |
| UK | 0.9638 | 0.655682 | 724 | 0.444061 | 534 | 0.453184 |
| **Overall*** | **OR** | **P-value** | **95%CI** | | | |
| | 0.90 | 3.70E-05 | (0.85,0.94) | | | |

*all cohorts combined

**Table 4.** Association between Uriary Bladder Cancer and allele T of rs11877062 in discovery cohorts of Icelandic and Dutch and combined groups. The risk allele C confers a risk of 1/0.88 = 1.14.

| Discovery group | P-value | OR | 95%CI |
|---|---|---|---|
| Iceland chip | 0.0011 | 0.82 | (0.73,0.92) |
| Holland chip* | 0.097 | 0.92 | |
| **Combined** | **0.00071** | **0.88** | |

*Using 1200 cases

**Table 5.** Association of missense markers within the *SLC14A1* gene on Chr 18 with Urinary Bladder Cancer, based on Icelandic imputed genotypes. The indicated risk allele is for the OR as shown; thus the C allele of rs11877062 is indicative of increased risk of bladder cancer, while the G allele of e.g. rs1058396 is indicative of increased risk of bladder cancer (OR value indicated in table is for the other allele of the marker (A), which thus has a risk less than unity, i.e. it confers decreased risk of bladder cancer.

| Marker | Pos NCBI B36 | Corr P-value | OR | Freq cases | Freq Ctl | No of imp gt | Freq | Info | Risk allele | Other allele | Coding effect* | Seq ID NO |
|---|---|---|---|---|---|---|---|---|---|---|---|---|
| rs11877062 | 41561244 | 0.000379915 | 1.246563 | 0.546494 | 0.495582 | 38082 | 0.496377 | 0.919451 | C | T | R4W | 130 |
| rs2298720 | 41564413 | 0.204434 | 0.842984 | 0.052479 | 0.061077 | 38082 | 0.060942 | 0.843395 | A | G | E44K | 131 |
| rs2298719 | 41570447 | 0.177335 | 1.927272 | 0.996657 | 0.993917 | 38082 | 0.99396 | 0.529603 | A | G | M/START167V | 132 |
| rs1058396 | 41573517 | 0.00424147 | 0.842842 | 0.467748 | 0.510193 | 38082 | 0.50953 | 0.988212 | A | G | D280N | 4 |

*The positions indicated refer to long transcript 2 as identified in SEQ ID NO: 133 for R4W, while the locations of the other three variants refer to their locations in the shorter transcript set forth in SEQ ID NO:209. The locations in SEQ ID NO: 133 for those variants is as follows: E44K corresponds to E100K in SEQ ID NO: 133, M167V corresponds to M223V in SEQ ID NO: 133 and D280N corresponds to D336N in SEQ ID NO: 133.

**Table** 6. Association of missense markers within the SLC14A1- gene on Chr 18 with Urinary Bladder Cancer, based on Icelandic and Dutch imputed genotypes from the 1000 Genomes project (http://www.1000genomes.org).

| Marker | Risk allele | OR Ice | (95%CI) | P-val Ice | OR Holl | (95%CI) | P-val Holl | OR comb | (95%CI) | P-val comb | Seq ID NO |
|---|---|---|---|---|---|---|---|---|---|---|---|
| rs1058396 | G | 0.84 | (0.75,0.94) | 0.0036 | 0.88 | (0.81,0.96) | 0.0034 | 0.87 | (0.81,0.93) | 4.70E-05 | 4 |
| rs11877062 | T | 0.82 | (0.73,0.92) | 0.0011 | 0.9 | (0.83,0.98) | 0.017 | 0.87 | (0.82,0.94) | 0.00012 | 130 |
| rs2298720 | A | 0.8 | (0.62,1.05) | 0.11 | 1.07 | (0.90,1.27) | 0.42 | 0.99 | (0.85,1.14) | 0.86 | 131 |

## Methods

The study was approved by the Data Protection Commission of Iceland and the National Bioethics Committee of Iceland. Written informed consent was obtained from all study participants. Personal identifiers associated with medical information and blood samples were encrypted with a third-party encryption system as provided by the Data Protection Commission of Iceland.

**Illumina genome-wide genotyping.** The Icelandic chip typed samples were assayed with the Illumina HumanHap300 or HumanHap CNV370 bead chips at deCODE genetics. The bead chips contained 317,503 and 370,404 haplotype tagging SNPs derived from phase I of the International HapMap project. Only SNPs present on both chips were included in the analysis and SNPs were excluded if they had (i) yield lower than 95% in cases or controls, (ii) minor allele frequency less than 1% in the population, or (iii) showed significant deviation from Hardy-Weinberg equilibrium in the controls ($P < 0.001$). All samples with a call rate below 98% were excluded from the analysis. The final analysis was based on direct genotyping of 289,658 autosomal SNPs.

**Single SNP genotyping.** Single SNP genotyping was carried out by deCODE genetics applying the Centaurus (Nanogen) platform (Kutyavin, I.V., et al. Nucleic Acids Res 34:e128 (2006)). The quality of each Centaurus SNP assay was evaluated by genotyping each assay on the CEU samples and comparing the results with the HapMap data. All assays had mismatch rate < 0.5%. Additionally, all markers were genotyped again on more than 10% of samples typed with the Illumina platform, resulting in an observed mismatch in less than 0.5% of samples.

**Whole-genome sequencing.** *Sample preparation:* Paired-end libraries for sequencing were prepared according to manufacturer's instructions (Illumina). In short, approximately 5 micrograms of genomic DNA, isolated from frozen blood samples, was fragmented to a mean target size of 300 basepairs (bp) using a Covaris E210 instrument. The resulting fragmented DNA was end-repaired using T4 and Klenow polymerases and T4 polynucleotide kinase with 10 mM dNTP's followed by addition of an "A" base at the ends using Klenow exo fragment (3' to 5'-exo minus) and dATP (1 mM). Sequencing adaptors containing "T" overhangs were ligated to the DNA products followed by agarose (2%) gel electrophoresis. Fragments of about 400 bp were isolated from the gels (Qiagen Gel Extraction Kit) and the adaptor-modified DNA fragments were PCR enriched for 10-cycles using Phusion DNA polymerase (Finnzymes Oy) and PCR primers PE 1.0 and PE 2.0 (Illumina). Enriched libraries were further purified using agarose (2%) gel electrophoresis as described above. The quality and concentration of the libraries was assessed with the Agilent 2100 Bioanalyzer using the DNA 1000 LabChip (Agilent). Barcoded libraries were stored at -20°C. All steps in the workflow were monitored using an in-house laboratory information management system with barcode tracking of all samples and reagents. *DNA sequencing:* Template DNA fragments were hybridized to the surface of flow cells (Illumina PE flowcell, v4) and amplified to form clusters using the Illumina cBot. In brief, DNA (8-10 pM) was denatured followed by hybridization to grafted adaptors on the flowcell. Isothermal bridge amplification using Phusion polymerase was then followed by linearization of the bridged DNA, denaturation, blocking of 3'-ends and hybridization of the sequencing primer. Sequencing-by-synthesis was performed on Illumina GAIIx instruments equipped with paired-end modules. Paired-end libraries were sequenced using 2 x 101 cycles of incorporation and imaging with Illumina sequencing kits, v4. Each library/sample was initially run on a single lane for validation followed by further sequencing of ≥4 lanes with targeted cluster densities of 250-300K/$mm^2$. Imaging and analysis of the data was performed using the SCS 2.6 and RTA 1.6 software packages from Illumina, respectively. RTA analysis involved conversion of image data to base-calling in real-time. *Alignment:* For each lane in the DNA sequencing output, the resulting qseq files were converted into fastq files using an inhouse script. All output from sequencing was converted and the Illumina quality filtering flag was retained in the output. The fastq files were then aligned against Build 36 of the human reference sequence using bwa version 0.5.7 (Li, H. & Durbin, R., Bioinformatics 25:1754-60 (2009)). *BAM file generation:* SAM file output from the alignment was converted into BAM format using samtools version 0.1.8 (Li, H., et al. Bioinformatics 25:2078-9 (2009) and an inhouse script was used to carry the Illumina quality filter flag over to the BAM file. The BAM files for each sample were then merged into a single BAM file using samtools. Finally, Picard version 1.17 (see http://picard.sourceforge.net/) was used to mark duplicates in the resulting sample BAM files.

**SNP calling and genotyping in whole-genome sequencing.** A two step approach was applied to SNP genotyping the whole-genome sequencing data. First, a SNP detection step where sequence positions where at least one individual could be determined to be different from the reference sequence with confidence (quality threshold of 20) based on the SNP calling feature of the pileup tool of samtools (Li, H. & Durbin, R., Bioinformatics 25:1754-60 (2009)). SNPs that were always heterozygous, or always homozygous different from the reference were removed. Second, all positions that were flagged as polymorphic were then genotyped using the pileup tool, but since sequencing depth varies and hence the certainty of genotype calls, genotype likelihoods were calculated rather than deterministic calls.

**Genotype imputation.** We impute the SNPs identified and genotyped through sequencing into all Icelanders that have been phased using long range phasing using the model used by IMPUTE (Marchini, J. et al. Nat Genet 39:906-13 (2007)). The genotype data from sequencing can be ambiguous due to low sequencing coverage and is not phased. In order phase the sequencing genotypes an iterative algorithm was applied for each SNP with alleles 0 and 1. Let $H$ be the long range phased haplotypes of the sequenced individuals and follow:

1. For each haplotype $h$ in $H$, use the hidden Markov model of IMPUTE to calculate $\gamma_{h,k}$ for every other $k$ in $H$, a measure of how likely $h$ is to have the same ancestral source as $k$.

2. For every $h$ in $H$ initialize the parameter $\theta_h$ which specifies how likely the 1 allele of the SNP is to occur on the background of $h$ from the genotype likelihoods obtained from sequencing. If $L_0$, $L_1$ and $L_2$ are the likelihoods of the genotypes 0, 1 and 2 in the individual that carries h, then set $\theta_h = \frac{L_2 + \frac{1}{2}L_1}{L_2 + L_1 + L_0}$.

3. For every pair of haplotypes $h$ and $k$ in H that are carried by the same individual use the other haplotypes in H to predict thep genotype of the SNP on the backgrounds of $h$ and $k$: $\tau_h = \sum_{l \in H \setminus \{h\}} \gamma_{h,l}\theta_l$ and $\tau_k = \sum_{l \in H \setminus \{k\}} \gamma_{k,l}\theta_l$. Combining these predictions with the genotype likelihoods from sequencing gives un-normalized updated phased genotype probabilities: $P_{00} = (1 - \tau_h)(1 - \tau_k)L_0$, $P_{01} = (1-\tau_h)\tau_k \frac{1}{2} L_1$, $P_{10} = \tau_h(1-\tau_k)\frac{1}{2}L_1$ and $P_{11} = \tau_h\tau_k L_2$. Now use these values to update $\theta_h$ and $\theta_k$ to $\frac{P_{10} + P_{11}}{P_{00} + P_{01} + P_{10} + P_{11}}$ and $\frac{P_{01} + P_{11}}{P_{00} + P_{01} + P_{10} + P_{11}}$, respectively.

4. Repeat step 3 while the maximum difference between iterations is greater than $\varepsilon$. We used $\varepsilon = 10^{-7}$.

Given the long range phased haplotypes and $\theta$ the allele of the SNP on a new haplotype h, not in H, is imputed as $\sum_{l \in H} \gamma_{h,l}\theta_l$.

The above algorithm can easily be extended to handle simple family structures such as parent offspring pairs and triads by letting the P distribution run over all founder haplotypes in the family structure. The algorithm also extends trivially to the X-chromosome. If source genotype data is only ambiguous in phase, such as chip genotype data, then the algorithm is still applied but all but one of the $L$s will be 0.

**Association testing.** Logistic regression was used to test for association between SNPs and disease, treating disease status as the response and expected genotype counts from imputation or allele counts from direct genotyping as covariates. Testing was performed using the likelihood ratio statistic.

*Subjects:*

***Icelandic study population.*** Records of all urinary bladder cancer diagnoses were obtained from the Icelandic Cancer Registry (ICR) (http://www.krabbameinsskra.is). The ICR contains all cancer diagnoses in Iceland from January 1, 1955. The ICR contained records of 1,845 Icelandic Bladder Cancer patients diagnosed until December 31, 2009, and all prevalent cases were eligible to participate. The mean participation rate for newly diagnosed cases was 65%. Patients were recruited by trained nurses on behalf of the patients' treating physicians, through special recruitment clinics. Participants in the study donated a blood sample and answered a lifestyle questionnaire. A total of 611 patients (76% males; diagnosed from December 1974 to December 2008) were included in a genome-wide SNP genotyping effort, using the Infinium II assay method and either the Sentrix HumanHap 300 or HumanCNV370-duo BeadChip (Illumina). The median age at diagnosis for all consenting cases was 68 years (range 22-95 years) as compared to 68.5 years for all Bladder Cancer patients in the ICR. The 37,478 controls (41% males; mean age 61 years; SD = 21) used in this study consisted of individuals from other ongoing genome-wide association studies at deCODE and represent over 15% of the adult population of Iceland. No individual disease group is represented by more than 10% of the total control group. Cancer patients (prostate, breast, colorectal and lung) were analyzed separately, and the frequency of the sequence variants studied did not differ from other controls. The study was approved by the Data Protection Authority of Iceland and the National Bioethics Committee. Written informed consent was obtained from all patients, relatives and controls. Personal identifiers associated with medical information and blood samples were encrypted with a third-party encryption system in which the Data Protection Authority maintains the code.

***Dutch population***

The Netherlands, discovery population. The Dutch patients were recruited for the Nijmegen Bladder Cancer Study (see http://dceg.cancer.gov/icbc/membership.html). The Nijmegen Bladder Cancer Study identified patients through the population-based regional cancer registry held by the Comprehensive Cancer Centre East, Nijmegen that serves a region of 1.3 million inhabitants in the eastern part of the Netherlands (www.ikcnet.nl). Patients diagnosed between 1995 and 2009 under the age of 75 years were selected and their vital status and current addresses updated through the hospital information systems of the 7 community hospitals and one university hospital (Radboud University Nijmegen Medical Centre, RUNMC) that are covered by the cancer registry. All patients were invited to the study by the Comprehensive Cancer Center on behalf of the patients' treating physicians. In case of consent, patients were sent a lifestyle questionnaire to fill out and blood samples were collected by Thrombosis Service centers which hold offices in all the communities in the region. The number of participating patients was increased with a non-overlapping series of 376 bladder cancer patients who were recruited previously for a study on gene-environment interactions in three hospitals (RUNMC, Canisius Wilhelmina Hospital, Nijmegen, and Streekziekenhuis Midden-Twente, Hengelo, the Netherlands). All the patients that were selected for the analyses were of self-reported European descent. The median age at diagnosis was 62 (range 25-93) years. 82% of the participants were males. Data on tumor stage and grade were obtained through the cancer registry. The 1,832 control individuals (46% males) were cancer free and frequency-matched for age with the cases. They were recruited within a project entitled "Nijmegen Biomedical Study" (NBS). The details of this study were reported previously (Wetzels, J.F., et al. Kidney Int 72:632-7 (2007)). Briefly, this is a population-based survey conducted by the Department of Epidemiology and Biostatistics and the Department of Clinical Chemistry of the Radboud University Nijmegen Medical Centre (RUNMC), in which 9,371 individuals participated from a total of 22,500, age and sex stratified, randomly selected inhabitants of Nijmegen. Control individuals from the Nijmegen Biomedical Study were invited to participate in a study on gene-environment interactions in multifactorial diseases, such as cancer. All the 1,832 participants in the present study are of self-reported European descent and were fully informed about the goals and the procedures of the study. The study protocols of the Nijmegen Bladder Cancer Study and the Nijmegen Biomedical Study were approved by the Institutional Review Board of the RUNMC and all study subjects gave written informed consent.

***Leeds Bladder Cancer Study, United Kingdom.*** Details of the Leeds Bladder Cancer Study have been reported previously (Sak, S.C., et al. Br J Cancer 92:2262-5 (2005)). In brief, patients from the urology department of St James's University Hospital, Leeds were recruited from August 2002 to March 2006. All those patients attending for cystoscopy or transurethral resection of a bladder tumor (TURBT) who had previously been found, or were subsequently shown, to have urothelial cell carcinoma of the bladder were included. Exclusion criteria were significant mental impairment or a blood transfusion in the past month. All non-Caucasians were excluded from the study leaving 764 patients. The median age at diagnosis of the patients was 73 years (range 30-101). 71% of the patients were male and 36% of all the patients had a low risk tumor (pTaG1/2). The controls were recruited from the otolaryngology outpatients and ophthalmology inpatient and outpatient departments at St James's Hospital, Leeds, from August 2002 to March 2006. All controls of appropriate age for frequency matching with the cases were approached and recruited if they gave their informed consent. As for the cases, exclusion criteria for the controls were significant mental impairment or a blood transfusion in the past month. Also, controls were excluded if they had symptoms suggestive of bladder cancer, such as haematuria. 2.8% of the controls were non-Caucasian leaving 530 Caucasian controls for the study. 71% of the controls were male. Data were collected by a health questionnaire on smoking habits and smoking history (non- ex- or current smoker, smoking dose in pack-years), occupational exposure history (to plastics, rubber, laboratories, printing, dyes and paints, diesel fumes), family history of bladder cancer, ethnicity and place of birth, and places of birth of parents. The response rate of cases was approximately 99%, that among the controls approximately 80%. Ethical approval for the study was obtained from Leeds (East) Local Research Ethics Committee, project number 02/192.

***Torino Bladder Cancer Case Control Study, Italy.*** The source of cases for the Torino bladder cancer study are two urology departments of the main hospital in Torino, the San Giovanni Battista Hospital (Matullo, G., et al. Cancer Epidemiol Biomarkers Prev 14:2569-78 (2005)). Cases are all Caucasian men, aged 40 to 75 years (median 63 years) and living in the Torino metropolitan area. They were newly diagnosed between 1994 and 2006 with a histologically confirmed, invasive or in situ, bladder cancer. Of all the patients with information on stage and grade, 56% were low risk (pTaG1/2). The sources of controls are urology, medical and surgical departments of the same hospital in Torino. All controls are Caucasian men resident in the Torino metropolitan area. They were diagnosed and treated between 1994 and 2006 for benign diseases (such as prostatic hyperplasia, cystitis, hernias, heart failure, asthma, and benign ear diseases). Controls with cancer, liver or renal diseases and smoking related conditions were excluded. The median age of the controls was 57 years (range 40 to 74). Data were collected by a professional interviewer who used a structured questionnaire to interview both cases and controls face-to-face. Data collected included demographics (age, sex, ethnicity, region and education) and smoking. For cases, additional data were collected on tumor histology, tumor site, size, stage, grade, and treatment of the primary tumor. The response rates were 90% for cases and 75% for controls resulting in 328 cases and 389 controls. Ethical approval for the study was obtained from Comitato Etico Interaziendale, A.O.U. San Giovanni Batista - A.). C.T.O./ Maria Adelaide.

***The Brescia bladder cancer study, Italy.*** The Brescia bladder cancer study is a hospital-based case-control study. The study was reported in detail previously (Shen, M. et al., Cancer Epidemiol Biomarkers Prev 12:1234-40 (2003)). In short, the catchment area of the cases and controls was the Province of Brescia, a highly industrialized area in Northern Italy (mainly metal and mechanical industry, construction, transport, textiles) but also with relevant agricultural areas. Cases and controls were enrolled in 1997 to 2000 from the two main city hospitals. The total number of eligible subjects was 216 cases and 220 controls. The response rate (enrolled/eligible) was 93% (N=201) for cases and 97% (N=214) for controls. Only males were included. All cases and controls had Italian nationality and were of Caucasian ethnicity. All cases had to be residents of the Province of Brescia, aged between 20 and 80, and newly diagnosed with histologically confirmed bladder cancer. The median age of the patients was 63 years (range 22-80). 29% of all the patients with known stage and grade had a low risk tumor (pTaG1/2). Controls were patients admitted for various urological non-neoplastic diseases and were frequency matched to cases on age, hospital and period of admission. The study was formally approved by the ethical committee of the hospital where the majority of subjects were recruited. A written informed consent was obtained from all participants. Data were collected from clinical charts (tumor histology, site, grade, stage, treatments, etc.) and by means of face-to-face interviews during hospital admission, using a standardized semi-structured questionnaire. The questionnaire included data on demographics (age, ethnicity, region, education, residence, etc.), and smoking. ISCO and ISIC codes and expert assessments were used for occupational coding. Blood samples were collected from cases and controls for genotyping and DNA adducts analyses.

***The Belgian Case Control Study of Bladder Cancer.*** The Belgian study has been reported in detail (Kellen, E., et al. Int J Cancer 118:2572-8 (2006)). In brief, cases were selected from the Limburg Cancer Registry (LIKAR) and were approached through urologists and general practitioners. All cases were diagnosed with histologically confirmed urothelial cell carcinoma of the bladder between 1999 and 2004, and were Caucasian inhabitants of the Belgian province of Limburg. The median age of the patients was 68 years. 86% of all the patients were males. For the recruitment of controls, a request was made to the "Kruispuntbank" of the social security for simple random sampling, stratified by municipality and socio-economic status, among all citizens above 50 years of age of the province. The median age of the controls was 64 years; 59% of the controls were males. Three trained interviewers visited cases and controls at home. Information was collected through a structured interview and a standardized food frequency questionnaire. In addition, biological samples were collected. Data collected included medical history, lifetime smoking history, family history of bladder cancer and a lifetime occupational history. Informed consent was obtained from all participants and the study was approved by the ethical review board of the Medical School of the Catholic University of Leuven, Belgium.

***The Eastern Europe study population.*** The details of this study have been described previously[8]. Cases and controls were recruited as part of a study designed to evaluate the risk of various cancers due to environmental arsenic exposure in Hungary, Romania and Slovakia between 2002 and 2004. The recruitment was carried out in the counties of Bacs, Bekes, Csongrad and Jasz-Nagykun-Szolnok in Hungary; Bihor and Arad in Romania; and Banska Bystrica and Nitra in Slovakia. The cases (N=214) and controls (N=533) selected were of Hungarian, Romanian and Slovak nationalities. Bladder cancer patients were invited on the basis of histopathological examinations by pathologists. Hospital-based controls were included in the study, subject to fulfillment of a set of criteria. All general hospitals in the study areas were involved in the process of control recruitment. The controls were frequency matched with cases for age, gender, country of residence and ethnicity. Controls included general surgery, orthopedic and trauma patients aged 30-79 years. Patients with malignant tumors, diabetes and cardiovascular diseases were excluded as controls. The median age for the bladder cancer patients was 65 years (range 36-90). 83% of the patients were males. The median age for the controls was 61 years (range 28-83). 51% of the controls were males. The response rates among cases and controls were ~70%. Of all the patients with known stage and grade information, 28% had a low risk tumor (pTaG1/2). Clinicians took venous blood and other biological samples from cases and controls after consent forms had been signed. Cases and controls recruited to the study were interviewed by trained personnel and completed a general lifestyle questionnaire. Ethnic background for cases and controls was recorded along with other characteristics of the study population. Local ethical boards approved the study plan and design.

***The Swedish Bladder Cancer Study.*** The Swedish patients come from a population-based study of urinary bladder cancer patients diagnosed in the Stockholm region in 1995-1996 (Larsson, P. et al. Scand J Urol Nephrol 37:195-201 (2003)). Blood samples from 352 patients were available out of a collection of 538 patients with primary urothelial carcinoma of the bladder. The average age at onset for these patients is 69 years (range 32-97 years) and 67% of the patients are males. Clinical data, including age at onset, grade and stage of tumor, were prospectively obtained from hospitals and urology units in the region. The control samples came from blood donors in the Stockholm region and were from cancer free individuals of both genders. The regional ethical committee approved of the study and all participants gave informed consent.

***Lutherstadt Wittenberg bladder cancer study, Germany.*** Details of the bladder cancer cases of this study have been reported previously (Golka, K. et al., J Toxicol Environ Helath A 71:881-6 (2008)); Golka, K. et al., Pharmacogenet Genomics (2009)). In brief, 221 patients with a confirmed bladder cancer from the Department of Urology, Paul Gerhardt Foundation, Lutherstadt Wittenberg, Germany, were included. Patients were enrolled from December 1995 to January 1999. Exclusion criterion was a missing written informed consent into the study. The median age of the patients was 65 years (range 20-91); 86% of the patients were males. A total of 214 controls were from the same department of urology, but were admitted for treatment of benign urological diseases. Exclusion criteria were malignant disease in the medical history or a missing written informed consent. The median age of the controls was 68 years (range 29-91); 84% of the controls were males. Data were collected from July 2000 to May 2005. All cases and controls were Caucasians, which was confirmed by questionnaire-based documentation of nationality. Cases and controls were matched for age. Data collected in cases and controls include age, gender, a complete documentation of occupational activities performed at least for 6 months, documentation of work places with known bladder cancer risk over the entire working life, exposures to known or suspected occupational bladder carcinogens, lifetime smoking habits, family history of bladder cancer, numbers of urinary infections treated by drugs during the previous 10 years, place of birth and places of residency for more than 10 years. For bladder cancer cases, data on tumor staging, grading and treatment were taken from the records. First diagnosis of bladder cancer was recorded from July 1979 to January 1999. The local ethics committee approved the study plan and design.

## Claims

1. A method of determining a susceptibility to Bladder Cancer, the method comprising:

analyzing nucleic acid sequence data from a human individual for at least one polymorphic marker in the human *SLC14A1* gene;
wherein different alleles of the at least one polymorphic marker are associated with different susceptibilities to Bladder Cancer in humans, and
determining a susceptibility to Bladder Cancer from the nucleic acid sequence data.

2. The method of claim 1, wherein the nucleic acid sequence data is obtained from a biological sample containing nucleic acid from the human individual.

3. The method of claim 2, wherein the nucleic acid sequence data is obtained using a method that comprises at least one procedure selected from:

(i) amplification of nucleic acid from the biological sample;
(ii) hybridization assay using a nucleic acid probe and nucleic acid from the biological sample;
(iii) hybridization assay using a nucleic acid probe and nucleic acid obtained by amplification of the biological sample,
(iv) high-throughput sequencing, and

wherein the analyzing comprises determining the presence or absence of at least one at-risk allele for Bladder Cancer of the polymorphic marker.

4. The method of any one of the preceding claims, wherein the at least one polymorphic marker encodes a missense substitution, a nonsense substitution, or a truncation in a SLC14A1 protein with sequence as set forth in SEQ ID NO:133,
wherein the at least one polymorphic marker in the *SLC14A1* gene is selected from the group consisting of rs1058396, rs11877062, rs2298720 and rs2298719, and markers in linkage disequilibrium with rs1058396 selected from the group consisting of the markers set forth in Table 1.

5. The method of claim 3, wherein the at least one at-risk allele is selected from the group consisting of the G allele of marker rs1058936, the C allele of marker rs11877062, the G allele of marker rs2298720, and the A allele of marker rs2298719.

6. A method of determining a susceptibility to Bladder Cancer, the method comprising:

obtaining amino acid sequence data about at least one encoded SLC14A1 protein in a human individual; wherein the amino acid sequence data is obtained from a biological sample containing SLC14A1 protein from the human individual, and
analyzing the amino acid sequence data to determine whether at least one amino acid substitution predictive of increased susceptibility of Bladder Cancer is present;
wherein a determination of the presence of the at least one amino acid substitution is indicative of increased susceptibility of Bladder Cancer for the individual, and wherein
a determination of the absence of the at least one amino acid substitution is indicative of the individual not having the increased susceptibility.

7. The method of claim 6, wherein the presence of an amino acid selected from the group consisting of:

Aspartic acid at position 336;
Tryptophan at position 4;
Glutamic acid at position 100; and
Valine at position 223;

in an SLC14A1 protein with sequence as set forth in SEQ ID NO:133 is indicative of an increased risk of bladder cancer for the human individual.

8. An apparatus for determining a susceptibility to Bladder Cancer in a human individual, comprising:

a processor;
a computer readable memory having computer executable instructions adapted to be executed on the processor to analyze information for at least one human individual with respect to at least one marker in the human *SLC14A1* gene that is predictive of susceptibility to Bladder Cancer in humans, or at least one amino acid variation in an encoded SLC14A1 protein, and generate an output based on the marker or amino acid information, wherein the output comprises at least one measure of susceptibility to Bladder Cancer for the human individual.

**9.** The apparatus of claim 8, wherein the marker information comprises nucleic acid sequence data identifying at least one allele of the at least one marker in the genome of the individual, and wherein the computer readable memory optionally further comprises data indicative of the risk of developing Bladder Cancer associated with at least one allele of at least one polymorphic marker, and wherein a risk measure for the human individual is based on a comparison of the marker information for the human individual to the risk of Bladder Cancer associated with the at least one allele of the at least one polymorphic marker.

**10.** The apparatus according to claim 8 or claim 9, wherein the at least one marker is selected from the group consisting rs1058396, rs11877062, rs2298720 and rs2298719, and markers in linkage disequilibrium with rs1058396 selected from the group consisting of the markers set forth in Table 1, and
wherein the amino acid variation is a variation in a protein with sequence as set forth in SEQ ID NO:133, selected from the group consisting of:

an arginine to tryptophan variation at position 4;
a lysine to glutamic acid variation at position 100;
a methionine to valine variation at position 223; and
an asparagine to aspartic acid variation at position 336.

**11.** A system for identifying susceptibility to bladder cancer in a human subject, the system comprising:

at least one processor;
at least one computer-readable medium;
a susceptibility database operatively coupled to a computer-readable medium of the system and containing population information correlating the presence or absence of one or more alleles of the human SLC14A1 gene and susceptibility to bladder cancer in a population of humans;
a measurement tool that receives an input about the human subject and generates information from the input about the presence or absence of the at least one allele in the human subject; and
an analysis tool that:

is operatively coupled to the susceptibility database and the measurement tool,
is stored on a computer-readable medium of the system,
is adapted to be executed on a processor of the system, to compare the information about the human subject with the population information in the susceptibility database and generate a conclusion with respect to susceptibility to bladder cancer for the human subject,

wherein the at least one allele is indicative of an amino acid substitution in a protein with sequence as set forth in SEQ ID NO:133, selected from the group consisting of:

an arginine to tryptophan substitution at position 4;
a lysine to glutamic acid substitution at position 100;
a methionine to valine substitution at position 223; and
an asparagine to aspartic acid substitution at position 336, and

wherein the at least one allele is selected from the group consisting of :

the G allele of marker rs1058396;
the C allele of marker rs11877062;
the G allele of marker rs2298720; and
the A allele of marker rs2298719.

**12.** The system according to claims 11, further including:

a communication tool operatively coupled to the analysis tool, stored on a computer-readable medium of the system and adapted to be executed on a processor of the system to communicate to the subject, or to a medical practitioner for the subject, the conclusion with respect to susceptibility to bladder cancer for the subject.

**13.** The system according to claim 11 or claim 12, wherein the measurement tool comprises a tool stored on a computer-readable medium of the system and adapted to be executed by a processor of the system to receive a data input about a subject and determine information about the presence or absence of the at least one allele in a human subject from the data,
wherein the data is optionally genomic sequence information, and the measurement tool comprises a sequence analysis tool stored on a computer readable medium of the system and adapted to be executed by a processor of the system to determine the presence or absence of the at least one allele from the genomic sequence information,
wherein the input about the human subject optionally is a biological sample from the human subject, and wherein the measurement tool comprises a tool to identify the presence or absence of the at least one allele in the biological sample, thereby generating information about the presence or absence of the at least one allele in a human subject,
wherein the measurement tool optionally includes:

an oligonucleotide microarray containing a plurality of oligonucleotide probes attached to a solid support;
a detector for measuring interaction between nucleic acid obtained from or amplified from the biological sample and one or more oligonucleotides on the oligonucleotide microarray to generate detection data; and
an analysis tool stored on a computer-readable medium of the system and adapted to be executed on a processor of the system, to determine the presence or absence of the at least one allele based on the detection data, and

wherein the measurement tool further optionally includes:

a nucleotide sequencer capable of determining nucleotide sequence information from nucleic acid obtained from or amplified from the biological sample; and
an analysis tool stored on a computer-readable medium of the system and adapted to be executed on a processor of the system, to determine the presence or absence of the at least one allele based on the nucleotide sequence information.

**14.** The system according to any one of claims 11-13, further comprising:

a medical protocol database operatively connected to a computer-readable medium of the system and containing information correlating the presence or absence of the at least one allele and medical protocols for human subjects at risk for bladder cancer; and
a medical protocol routine, operatively connected to the medical protocol database and the analysis routine, stored on a computer-readable medium of the system, and adapted to be executed on a processor of the system, to compare the conclusion from the analysis routine with respect to susceptibility to bladder cancer for the subject and the medical protocol database, and generate a protocol report with respect to the probability that one or more medical protocols in the database will:

reduce susceptibility to bladder cancer; or
delay onset of bladder cancer; or
increase the likelihood of detecting bladder cancer at an early stage to facilitate early treatment.

**15.** The system according to any one of claims11 to 14, wherein the communication tool is operatively connected to the analysis routine and comprises a routine stored on a computer-readable medium of the system and adapted to be executed on a processor of the system, to:

generate a communication containing the conclusion; and
transmit the communication to the subject or the medical practitioner, or enable the subject or medical practitioner to access the communication,

wherein the communication further includes the protocol report.

**Patentansprüche**

**1.** Verfahren zur Bestimmung einer Anfälligkeit für Blasenkrebs, das Folgendes umfasst:

Analysieren von Nukleinsäuresequenzdaten von einem menschlichen Individuum auf wenigstens einen polymorphen Marker im menschlichen *SLC14A1*-Gen;

wobei unterschiedliche Allele des wenigstens einen polymorphen Markers mit unterschiedlichen Anfälligkeiten für Blasenkrebs beim Menschen assoziiert sind, und
Bestimmen einer Anfälligkeit für Blasenkrebs anhand der Nukleinsäuresequenzdaten.

**2.** Verfahren nach Anspruch 1, wobei die Nukleinsäuresequenzdaten aus einer biologischen Probe erhalten werden, die Nukleinsäure von dem menschlichen Individuum enthält.

**3.** Verfahren nach Anspruch 2, wobei die Nukleinsäuresequenzdaten unter Verwendung eines Verfahrens erhalten werden, das wenigstens eine aus Folgendem ausgewählte Vorgehensweise umfasst:

(i) Amplifikation von Nukleinsäure aus der biologischen Probe;
(ii) Hybridisierungstest unter Verwendung einer Nukleinsäuresonde und von Nukleinsäure aus der biologischen Probe;
(iii) Hybridisierungstest unter Verwendung einer Nukleinsäuresonde und von durch Amplifikation der biologischen Probe erhaltener Nukleinsäure,
(iv) Sequenzieren mit hohem Durchsatz, und

wobei das Analysieren Bestimmen des Vorliegens oder Fehlens wenigstens eines Blasenkrebs-At-risk-Allels des polymorphen Markers umfasst.

**4.** Verfahren nach einem der vorhergehenden Ansprüche, wobei der wenigstens eine polymorphe Marker eine Missense-Substitution, eine Nonsense-Substitution oder eine Verkürzung bei einem SLC14A1-Protein mit einer Sequenz gemäß SEQ ID NO:133 codiert,
wobei der wenigstens eine polymorphe Marker im *SLC14A1*-Gen aus der aus rs1058396, rs11877062, rs2298720 und rs2298719 und Markern im Kopplungsungleichgewicht mit rs1058396, die aus der aus den Markern gemäß Tabelle 1 bestehenden Gruppe ausgewählt sind, bestehenden Gruppe ausgewählt ist.

**5.** Verfahren nach Anspruch 3, wobei das wenigstens eine At-risk-Allel aus der aus dem G-Allel von Marker rs1058936, dem C-Allel von Marker rs11877062, dem G-Allel von Marker rs2298720 und dem A-Allel von Marker rs2298719 bestehenden Gruppe ausgewählt ist.

**6.** Verfahren zur Bestimmung einer Anfälligkeit für Blasenkrebs, das Folgendes umfasst:

Gewinnen von Aminosäuresequenzdaten über wenigstens ein codiertes SLC14A1-Protein bei einem menschlichen Individuum; wobei die Aminosäuresequenzdaten aus einer biologischen Probe erhalten werden, die SLC14A1-Protein von dem menschlichen Individuum enthält, und
Analysieren der Aminosäuresequenzdaten, um zu bestimmen, ob wenigstens eine eine erhöhte Anfälligkeit für Blasenkrebs vorhersagende Aminosäuresubstitution vorhanden ist;
wobei eine Bestimmung des Vorliegens der wenigstens einen Aminosäuresubstitution eine erhöhte Anfälligkeit für Blasenkrebs für das Individuum anzeigt und wobei eine Bestimmung des Fehlens der wenigstens einen Aminosäuresubstitution anzeigt,
dass das Individuum nicht die erhöhte Anfälligkeit aufweiset.

**7.** Verfahren nach Anspruch 6, wobei das Vorliegen einer aus der aus

Asparaginsäure an Position 336;
Tryptophan an Position 4;
Glutaminsäure an Position 100; und
Valin an Position 223;

bestehenden Gruppe ausgewählten Aminosäure in einem SLC14A1-Protein mit einer Sequenz gemäß SEQ ID NO:133 ein erhöhtes Blasenkrebsrisiko für das menschliche Individuum anzeigt.

**8.** Vorrichtung zur Bestimmung einer Anfälligkeit für Blasenkrebs bei einem menschlichen Individuum, umfassend:

einen Prozessor,
einen computerlesbaren Speicher mit durch einen Computer ausführbaren Anweisungen, die zur Ausführung auf dem Prozessor adaptiert sind, um Informationen für wenigstens ein menschliches Individuum in Bezug auf wenigstens einen Marker im menschlichen *SLC14A1*-Gen*,* der eine Anfälligkeit für Blasenkrebs beim Menschen vorhersagt, oder
wenigstens eine Aminosäurevariation in einem codierten SLC14A1-Protein zu analysieren und einen auf den Marker- bzw. Aminosäure-Informationen basierenden Output zu erzeugen, wobei der Output wenigstens ein Maß der Anfälligkeit für Blasenkrebs für das menschliche Individuum umfasst.

**9.** Vorrichtung nach Anspruch 8, wobei die Marker-Informationen Nukleinsäuresequenzdaten umfassen, die wenigstens ein Allel des wenigstens einen Markers im Genom des Individuums identifizierten, und wobei der computerlesbare Speicher ferner gegebenenfalls Daten umfasst, die das mit wenigstens einem Allel wenigstens eines polyamorphen Markers assoziierte Risiko, Blasenkrebs zu entwickeln, anzeigen und wobei ein Risixomaß für das menschlichen Individuum auf einem Vergleich der Marker-Informationen für das menschliche Individuum mit dem mit dem wenigstens einen Allele des wenigstens einen polymorphen Markers assoziierten Blasenkrebsrisiko beruht.

**10.** Vorrichtung gemäß Anspruch 8 oder Anspruch 9, wobei der wenigstens eine Marker aus der aus rs1058396, rs11877062, rs2298720 und rs2298719 und Markern im Kopplungsungleichgewicht mit rs1058396, die aus der aus den Markern gemäß Tabelle 1 bestehenden Gruppe ausgewählt sind, bestehenden Gruppe ausgewählt ist und
wobei es sich bei der Aminosäurevariation um eine Variation in einem Protein mit einer Sequenz gemäß SEQ ID NO:133 handelt, die aus der aus

einer Arginin-zu-Tryptophan-Variation an Position 4;
einer Lysin-zu-Glutaminsäure-Variation an Position 100;
einer Methionin-zu-Valin-Variation an Position 223; und
einer Asparagin-zu-Asparaginsäure-Variation an Position 336

bestehenden Gruppe ausgewählt ist.

**11.** System zur Identifizierung einer Anfälligkeit für Blasenkrebs bei einem menschlichen Patienten, das Folgendes umfasst:

wenigstens einen Prozessor;
wenigstens einen computerlesbaren Datenträger;
eine Anfälligkeits-Datenbank in operativer Kopplung an einen computerlesbaren Datenträger des Systems, die Populationsinformationen enthält, die das Vorliegen oder Fehlen eines oder mehrerer Allele des menschlichen SLC14A1-Gens und Anfälligkeit für Blasenkrebs in einer Population von Menschen korrelieren;
ein Messwerkzeug, das eine Eingabe über den menschlichen Patienten empfängt und anhand der Eingabe Informationen über das Vorliegen oder
Fehlen des wenigstens einen Allels bei dem menschlichen Patienten erzeugt; und

ein Analysewerkzeug, das:

in operativer Kopplung an die Anfälligkeits-Datenbank und das Messwerkzeug steht,
auf einem computerlesbaren Datenträger des Systems gespeichert ist,
so adaptiert ist, dass es auf einem Prozessor des Systems ausgeführt wird, um die Informationen über den menschlichen Patienten mit den Populationsinformationen in der Anfälligkeits-Datenbank zu vergleichen und eine Schlussfolgerung in Bezug auf Anfälligkeit für Blasenkrebs für den menschlichen Patienten zu erzeugen,
wobei das wenigstens eine Allel eine Aminosäuresubstitution in einem Protein mit einer Sequenz gemäß SEQ ID NO:133 anzeigt, die aus der aus

einer Arginin-zu-Tryptophan-Substitution an Position 4;
einer Lysin-zu-Glutaminsäure-Substitution an Position 100;
einer Methionin-zu-Valin-Substitution an Position 223; und
einer Asparagin-zu-Asparaginsäure-Substitution an Position 336

bestehenden Gruppe ausgewählt ist, und wobei das wenigstens eine Allel aus der aus

dem G-Allel von Marker rs1058396;
dem C-Allel von Marker rs11877062;
dem G-Allel von Marker rs2298720; und

dem A-Allel von Marker rs2298719
bestehenden Gruppe ausgewählt ist.

**12.** System gemäß Anspruch 11, ferner enthaltend:

ein Kommunikationswerkzeug in operativer Kopplung an das Analysewerkzeug, auf einem computerlesbaren Datenträger des Systems gespeichert und so adaptiert, dass es auf einem Prozessor des Systems ausgeführt wird, um dem Patienten, oder einem Arzt für den Patienten, die Schlussfolgerung in Bezug auf Anfälligkeit für Blasenkrebs für den Patienten mitzuteilen.

**13.** System gemäß Anspruch 11 oder Anspruch 12, wobei das Messwerkzeug ein Werkzeug umfasst, das auf einem computerlesbaren Datenträger des Systems gespeichert und so adaptiert ist, dass es von einem Prozessor des Systems ausgeführt wird, um eine Dateneingabe über einen Patienten zu empfangen und aus den Daten Informationen über das Vorliegen oder Fehlen des wenigstens einen Allels bei einem menschlichen Patienten zu bestimmen, wobei es sich bei den Daten gegebenenfalls um genomische Sequenzinformationen handelt und das Messwerkzeug ein Sequenzanalysenwerkzeug umfasst, das auf einem computerlesbaren Datenträger des Systems gespeichert und so adaptiert ist, dass es von einem Prozessor des Systems ausgeführt wird, um anhand der genomischen Sequenzinformationen das Vorliegen oder Fehlen des wenigstens einen Allels zu bestimmten, wobei es sich bei der Eingabe über den menschlichen Patienten gegebenenfalls um eine biologische Probe vom menschlichen Patienten handelt und wobei das Messwerkzeug ein Werkzeug zum Identifizieren des Vorliegens oder Fehlens des wenigstens einen Allels in der biologischen Probe umfasst, wodurch Informationen über das Vorliegen oder Fehlen des wenigstens einen Allels bei einem menschlichen Patienten erzeugt werden,
wobei das Messwerkzeug gegebenenfalls Folgendes enthält:

ein Oligonukleotid-Mikroarray, das mehrere an einen festen Träger gebundene Oligonukleotidsonden enthält;
einen Detektor zum Messen einer Wechselwirkung zwischen aus der biologischen Probe erhaltener oder amplifizierter Nukleinsäure und einem oder mehreren Oligonukleotiden auf dem Oligonucleotid-Mikroarray, so dass Nachweisdaten erzeugt werden; und
ein Analysewerkzeug, das auf einem computerlesbaren Datenträger des Systems gespeichert und so adaptiert ist, dass es auf einem Prozessor des Systems ausgeführt wird, um bezogen auf die Nachweisdaten das Vorliegen oder Fehlen des wenigstens einen Allels zu bestimmen, und

wobei das Messwerkzeug ferner gegebenenfalls Folgendes enthält:

einen Nukleotidsequenzierer, mit dem Nukleotidsequenzinformationen aus aus der biologischen Probe erhaltener oder amplifizierten Nukleinsäure bestimmt werden können; und
ein Analysewerkzeug, das auf einem computerlesbaren Datenträger des Systems gespeichert und so adaptiert ist, dass es auf einem Prozessor des Systems ausgeführt wird, um bezogen auf die Nukleotidsequenzinformationen das Vorliegen oder Fehlen des wenigstens einen Allels zu bestimmen.

**14.** System gemäß einem der Ansprüche 11 bis 13, ferner umfassend:

eine Datenbank für medizinische Dokumentation in operativer Verbindung mit einem computerlesbaren Datenträger des Systems, die Informationen enthält, die das Vorliegen oder Fehlen des wenigstens einen Allels und medizinische Dokumentation für menschliche Patienten mit einem Blasenkrebsrisiko korrelieren; und
eine Routine für medizinische Dokumentation, die in operativer Verbindung mit der Datenbank für medizinische Dokumentation und der Analyseroutine steht, auf einem computerlesbaren Datenträger des Systems gespeichert ist und so adaptiert ist, dass sie auf einem Prozessor des Systems ausgeführt wird, um die Schlussfolgerung aus der Analyseroutine in Bezug auf Anfälligkeit für Blasenkrebs für den Patienten und die Datenbank für medizinische Dokumentation zu vergleichen und einen Protokollbericht in Bezug auf die Wahrscheinlichkeit zu erzeugen, dass bei einer oder mehreren medizinischen Dokumentationen in der Datenbank:

eine Anfälligkeit für Blasenkrebs reduziert ist; oder
der Beginn einer Blasenkrebserkrankung verzögert ist; oder
die Wahrscheinlichkeit des Nachweises von Blasenkrebs in einem Frühstadium erhöht ist, um eine frühe Behandlung zu erleichtern.

**15.** System gemäß einem der Ansprüche 11 bis 14, wobei das Kommunikationswerkzeug in operativer Verbindung mit der Analyseroutine steht und eine Routine umfasst, die auf einem computerlesbaren Datenträger des Systems gespeichert ist und so adaptiert ist, dass sie auf einem Prozessor des Systems ausgeführt wird, um:

eine die Schlussfolgerung enthaltende Mitteilung zu erzeugen; und
die Mitteilung dem Patienten oder dem Arzt zu übermitteln oder dem Patienten bzw. Arzt Zugang zu der Mitteilung zu ermöglichen,
wobei die Mitteilung ferner den Protokollbericht enthält.

## Revendications

**1.** Procédé de détermination d'une susceptibilité à un cancer de la vessie, le procédé comprenant les étapes consistant à :

analyser des données de séquences d'acide nucléique provenant d'un individu humain pour au moins un marqueur polymorphe dans le gène *SLC14A1* humain ;
dans lequel différents allèles du au moins un marqueur polymorphe sont associés à différentes susceptibilités à un cancer de la vessie chez les êtres humains, et
déterminer une susceptibilité à un cancer de la vessie à partir des données de séquences d'acide nucléique.

**2.** Procédé selon la revendication 1, dans laquelle les données de séquences d'acide nucléique sont obtenues à partir d'un échantillon biologique contenant un acide nucléique qui provient de l'individu humain.

**3.** Procédé selon la revendication 2, dans laquelle les données de séquences d'acide nucléique sont obtenues en utilisant un procédé qui comprend au moins une procédure choisie parmi :

(i) une amplification d'acide nucléique à partir de l'échantillon biologique ;
(ii) un dosage d'hybridation utilisant une sonde d'acide nucléique et un acide nucléique provenant de l'échantillon biologique ;
(iii) un dosage d'hybridation utilisant une sonde d'acide nucléique et un acide nucléique obtenu par amplification de l'échantillon biologique,
(iv) un séquençage à haut débit, et

dans lequel l'analyse comprend la détermination de la présence ou l'absence d'au moins un allèle du marqueur polymorphe à risque pour un cancer de la vessie.

**4.** Procédé selon l'une quelconque des revendications précédentes, dans laquelle l'au moins un marqueur polymorphe code pour une substitution faux-sens, une substitution non-sens ou une troncature dans une protéine SLC14A1, avec une séquence telle qu'indiquée dans la SEQ ID n° : 133,
dans laquelle l'au moins un marqueur polymorphe dans le gène *SLC14A1* est choisi dans le groupe constitué par les rs1058396, rs11877062, rs2298720 et rs2298719, ainsi que des marqueurs en déséquilibre de liaison avec un rs1058396, choisis dans le groupe constitué par les marqueurs indiqués dans le Tableau 1.

**5.** Procédé selon la revendication 3, dans laquelle l'au moins un allèle à risque est choisi dans le groupe constitué par l'allèle G du marqueur rs1058936, l'allèle C du marqueur rs11877062, l'allèle G du marqueur rs2298720 et l'allèle A du marqueur rs2298719.

**6.** Procédé de détermination d'une susceptibilité à un cancer de la vessie, le procédé comprenant les étapes consistant à :

obtenir des données de séquences d'acides aminés concernant au moins une protéine SLC14A1 codée chez un individu humain ; dans laquelle les données de séquences d'acides aminés sont obtenues à partir d'un échantillon biologique contenant une protéine SLC14A1 provenant de l'individu humain, et
analyser les données de séquences d'acides aminés pour déterminer si au moins une substitution d'acide aminé, prédictive d'une susceptibilité accrue à un cancer de la vessie, est présente ;
dans lequel une détermination de la présence de l'au moins une substitution d'acides aminés est indicative d'une susceptibilité accrue à un cancer de la vessie pour l'individu, et dans lequel une détermination de l'absence de l'au moins une substitution d'acides aminés est une indication que l'individu n'a pas la susceptibilité accrue.

**7.** Procédé selon la revendication 6, dans laquelle la présence d'un acide aminé choisi dans le groupe constitué par :

un acide aspartique en position 336 ;
un tryptophane en position 4 ;
un acide glutamique en position 100 ; et
une valine en position 223 ;

dans une protéine SLC14A1, avec une séquence telle qu'indiquée dans la SEQ ID n° : 133, est indicative d'un risque accru à un cancer de la vessie pour l'individu humain.

**8.** Appareil de détermination d'une susceptibilité à un cancer de la vessie chez un individu humain, comprenant :

un processeur ;
une mémoire lisible par ordinateur ayant des instructions exécutables par ordinateur étant adaptées à être exécutées sur le processeur pour analyser une information pour au moins un individu humain, en ce qui concerne l'au moins un marqueur dans le gène *SLC14A1* humain qui est prédictif d'une susceptibilité à un cancer de la vessie chez les êtres humains, ou au moins une variation d'acides aminés dans une protéine SLC14A1 humaine codée, et générer un résultat basé sur le marqueur ou l'information d'acides aminés, dans lequel le résultat comprend au moins une mesure de la susceptibilité à un cancer de la vessie pour l'individu humain.

**9.** Appareil selon la revendication 8, dans laquelle l'information sur le marqueur comprend des données de séquences d'acide nucléique identifiant au moins un allèle du au moins un marqueur dans le génome de l'individu, et dans laquelle la mémoire lisible par ordinateur comprend facultativement en outre des données indicatives du risque de développer un cancer de la vessie associé à au moins un allèle d'au moins un marqueur polymorphe, et dans laquelle une mesure de risque pour l'individu humain est basée sur une comparaison de l'information sur le marqueur pour l'individu humain par rapport au risque d'un cancer de la vessie associé à au moins un allèle de l'au moins un marqueur polymorphe.

**10.** Appareil selon la revendication 8 ou la 9, dans laquelle l'au moins un marqueur est choisi dans le groupe constitué par les rs1058396, rs11877062, rs2298720 et rs2298719, ainsi que des marqueurs en déséquilibre de liaison avec un rs1058396, choisis dans le groupe constitué par les marqueurs indiqués dans le Tableau 1, et
dans laquelle la variation d'acides aminés est une variation dans une protéine avec une séquence telle qu'indiquée dans la SEQ ID n° : 133, choisie dans le groupe constitué par :

une variation d'une arginine en un tryptophane à la position 4 ;
une variation d'une lysine en un acide glutamique à la position 100 ;
une variation d'une méthionine en une valine à la position 223 ; et
une variation d'une asparagine en un acide aspartique à la position 336.

**11.** Système pour identifier la susceptibilité à un cancer de la vessie chez un sujet humain, le système comprenant :

au moins un processeur :

au moins un support lisible par ordinateur :
une base de données de susceptibilité étant couplée, de manière opérationnelle, avec un support lisible par ordinateur du système et contenant une information sur la population étant en corrélation avec la présence ou l'absence d'un ou plusieurs allèle(s) du gène SLC14A1 humain ainsi que la susceptibilité à un cancer de la vessie dans une population d'êtres humains ;
un outil de mesure qui reçoit une entrée concernant le sujet humain et qui génère une information à partir de l'entrée sur la présence ou l'absence du au moins un allèle dans le sujet humain ; et

un outil d'analyse qui :

est couplé, de manière opérationnelle, à la base de données de susceptibilité et l'outil de mesure,
est stocké sur un support lisible par ordinateur du système,
est adapté à être exécuté sur un processeur du système, pour comparer l'information concernant le sujet humain avec l'information sur la population dans la base de données de susceptibilité et générer une conclusion concernant la susceptibilité à un cancer de la vessie pour le sujet humain,

dans lequel l'au moins un allèle est indicateur d'une substitution d'acide aminé dans une protéine avec une séquence telle qu'indiquée dans la SEQ ID n° : 133, choisie dans le groupe constitué par :

une substitution d'une arginine en un tryptophane à la position 4 ;
une substitution d'une lysine en un acide glutamique à la position 100 ;
une substitution d'une méthionine en une valine à la position 223 ; et
une substitution d'une asparagine en un acide aspartique à la position 336, et

dans lequel l'au moins un allèle est choisi dans le groupe constitué par :

l'allèle G du marqueur rs1058396 ;
l'allèle C du marqueur rs11877062 ;
l'allèle G du marqueur rs2298720 ; et
l'allèle A du marqueur rs2298719.

**12.** Système, selon la revendication 11, comprenant en outre :

un outil de communication étant couplé, de manière opérationnelle, à l'outil d'analyse, stocké sur un support lisible par ordinateur du système et adapté à être exécuté sur un processeur du système pour communiquer au sujet, ou à un praticien médical destiné au sujet, la conclusion concernant la susceptibilité à un cancer de la vessie pour le sujet.

**13.** Système selon la revendication 11 ou la revendication 12, dans laquelle l'outil de mesure comprend un outil stocké sur un support lisible par ordinateur du système et adapté à être exécuté par un processeur du système pour recevoir une entrée de données sur un sujet et déterminer l'information au sujet de la présence ou l'absence du au moins un allèle chez un sujet humain à partir des données,
dans laquelle les données sont facultativement des informations de séquences génomiques, et l'outil de mesure comprend un outil d'analyse de séquence stocké sur un support lisible par ordinateur du système et adapté à être exécuté par un processeur du système pour déterminer la présence ou l'absence du au moins un allèle provenant de l'information de séquence génomique, dans laquelle l'entrée sur le sujet humain est, en option, un échantillon biologique provenant du sujet humain, et dans laquelle l'outil de mesure comprend un outil pour identifier la présence ou l'absence du au moins un allèle dans l'échantillon biologique, en générant de ce fait une information sur la présence ou l'absence du au moins un allèle chez un sujet humain,
dans laquelle l'outil de mesure comprend, en option :

un micro-arrangement d'oligonucléotides contenant une pluralité de sondes d'oligonucléotides fixées à un support solide ;
un détecteur pour mesurer l'interaction entre un acide nucléique, obtenu à partir de l'échantillon biologique ou amplifié à partir de celui-ci, et un ou plusieurs oligonucléotide(s) sur le micro-arrangement d'oligonucléotides pour générer des données de détection ; et
un outil d'analyse stocké sur un support lisible par ordinateur du système et adapté à être exécuté sur un processeur du système, pour déterminer la présence ou l'absence du au moins un allèle basé sur les données de détection, et
dans laquelle l'outil de mesure comprend facultativement en outre :

un séquenceur nucléotidique étant capable de déterminer l'information de séquence nucléotidique à partir d'un acide nucléique obtenu à partir de l'échantillon biologique ou amplifié à partir de celui-ci ; et
un outil d'analyse, stocké sur un support lisible par ordinateur du système et adapté à être exécuté sur un processeur du système, pour déterminer la présence ou l'absence du au moins un allèle basé sur l'information de séquence nucléotidique.

**14.** Système, selon l'une quelconque des revendications 11 à 13, comprenant en outre :

une base de données de protocoles médicaux étant reliée, de manière opérationnelle, à un support lisible par ordinateur du système et contenant des informations en corrélation avec la présence ou l'absence du au moins un allèle et des protocoles médicaux pour les sujets humains étant à risque d'un cancer de la vessie ; et
une routine de protocole médical étant reliée, de manière opérationnelle, à la base de données de protocoles médicaux et à routine d'analyse, stockée sur un support lisible par ordinateur du système, et adaptée à être exécutée sur un processeur du système, pour comparer la conclusion provenant de la routine d'analyse, eu égard à la susceptibilité à un cancer de la vessie pour le sujet et la base de données de protocoles médicaux, et générer un rapport de protocole concernant la probabilité qu'un ou plusieurs protocole(s) dans la base de données :

réduise la susceptibilité à un cancer de la vessie ; ou retarde l'apparition d'un cancer de la vessie ; ou augmente la vraisemblance de la détection d'un cancer de la vessie à un stade précoce pour faciliter un traitement précoce.

**15.** Système selon l'une quelconque des revendications 11 à 14, dans laquelle l'outil de communication est relié, de manière opérationnelle, à la routine d'analyse et comprend une routine stockée sur un support lisible par ordinateur du système et étant adapté pour être exécutée sur un processeur du système, pour :

générer une communication contenant la conclusion ; et
transmettre la communication au sujet ou au praticien médical, ou bien permettre au sujet ou au praticien médical d'avoir accès à la communication,

dans laquelle la communication comprend en outre le rapport de protocole.

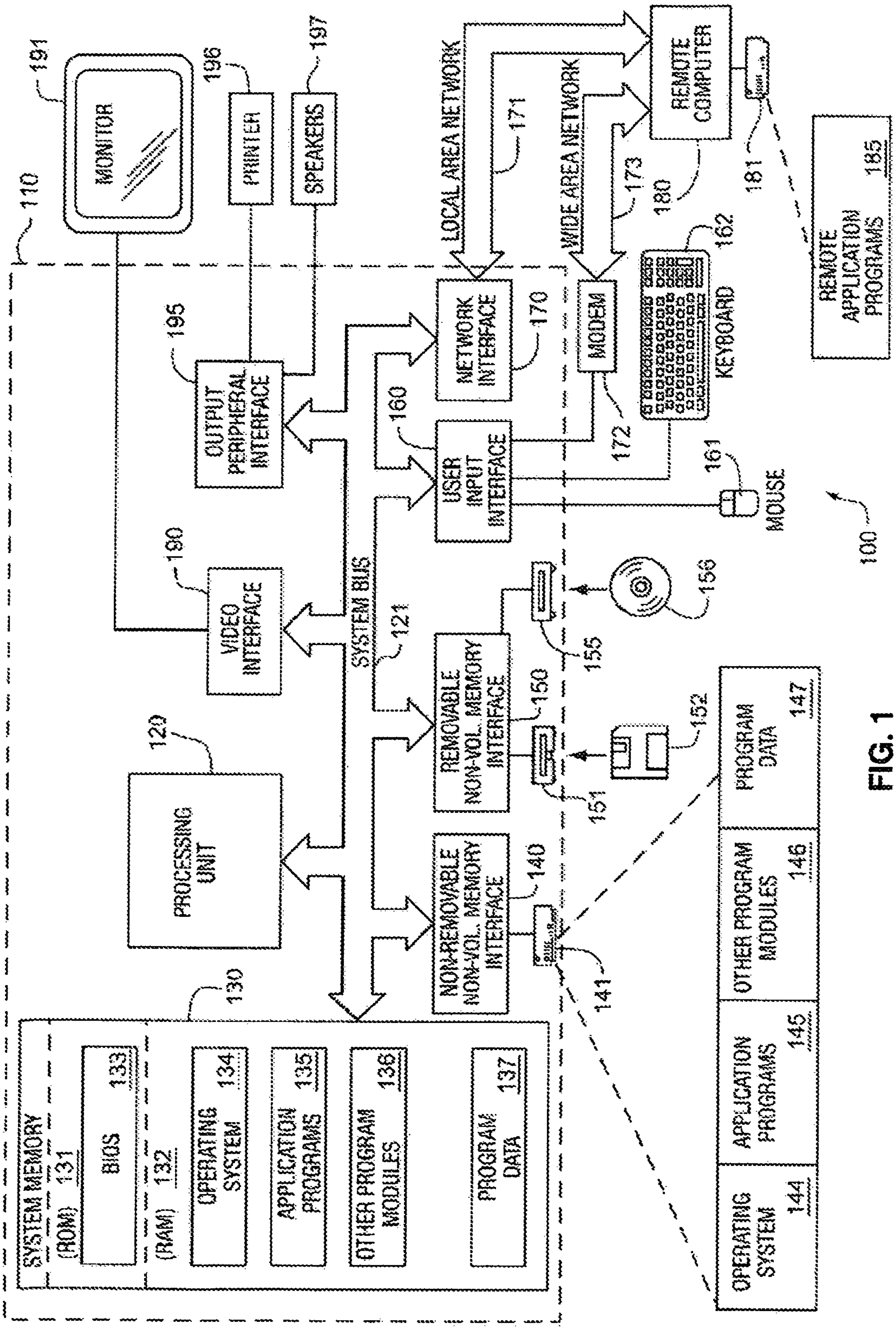

SYSTEM MEMORY
(ROM) 131
BIOS 133
(RAM) 132
OPERATING SYSTEM 134
APPLICATION PROGRAMS 135
OTHER PROGRAM MODULES 136
PROGRAM DATA 137
130
PROCESSING UNIT
120
SYSTEM BUS
121
NON-REMOVABLE NON-VOL. MEMORY INTERFACE
140
141
REMOVABLE NON-VOL. MEMORY INTERFACE
150
151
152
155
156
VIDEO INTERFACE
190
OUTPUT PERIPHERAL INTERFACE
195
USER INPUT INTERFACE
160
NETWORK INTERFACE
170
MODEM
172
KEYBOARD
162
MOUSE
161
MONITOR
191
PRINTER
196
SPEAKERS
197
110
LOCAL AREA NETWORK
171
WIDE AREA NETWORK
173
REMOTE COMPUTER
180
181
REMOTE APPLICATION PROGRAMS 185
OPERATING SYSTEM 144
APPLICATION PROGRAMS 145
OTHER PROGRAM MODULES 146
PROGRAM DATA 147
100

FIG. 1

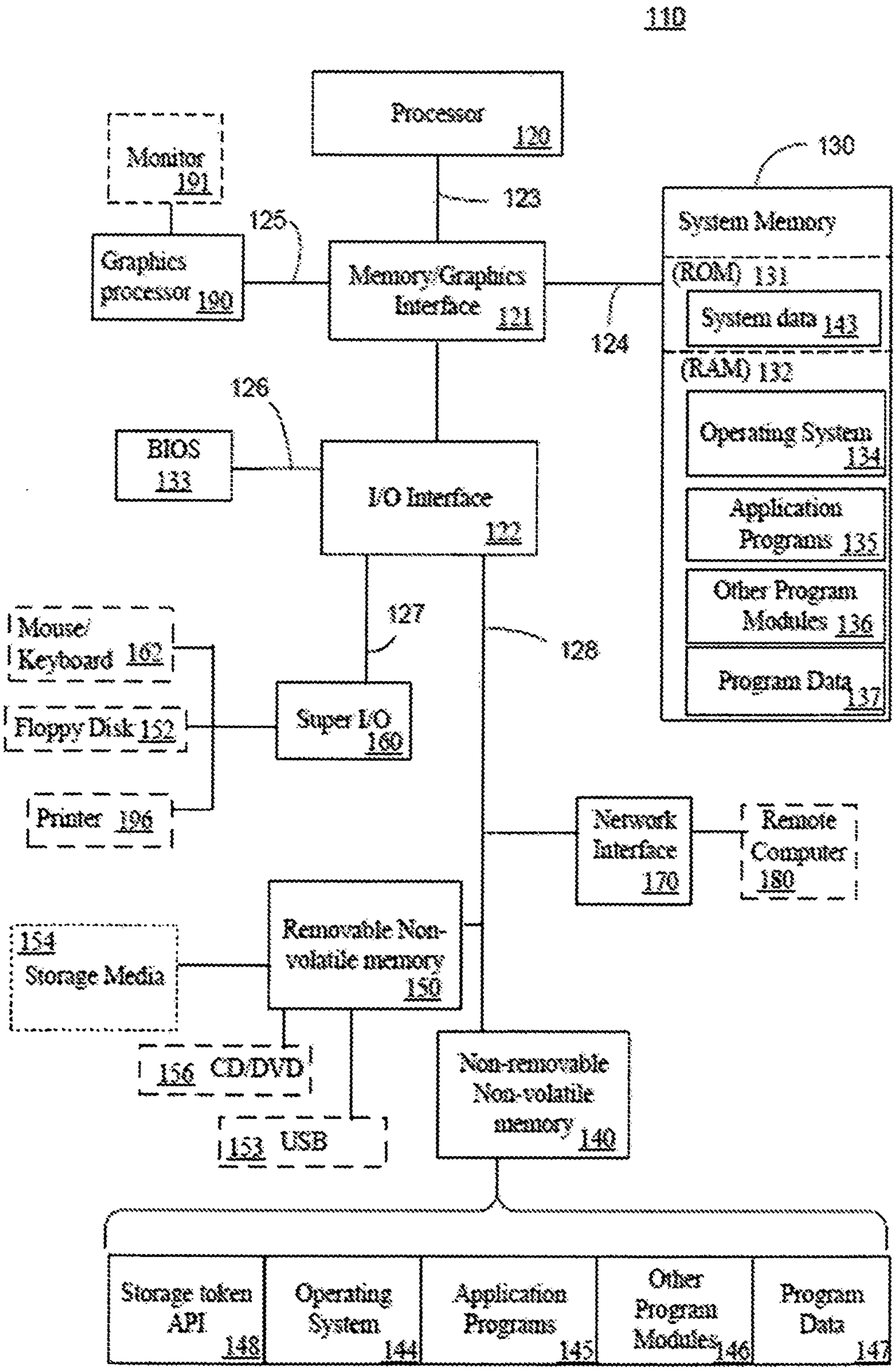
110
Processor 120
Monitor 191
123
125
130
System Memory
Graphics processor 190
Memory/Graphics Interface 121
(ROM) 131
System data 143
124
(RAM) 132
126
Operating System 134
BIOS 133
I/O Interface 122
Application Programs 135
Other Program Modules 136
127
128
Mouse/ Keyboard 162
Program Data 137
Floppy Disk 152
Super I/O 160
Printer 196
Network Interface 170
Remote Computer 180
154 Storage Media
Removable Non-volatile memory 150
156 CD/DVD
153 USB
Non-removable Non-volatile memory 140
Storage token API 148
Operating System 144
Application Programs 145
Other Program Modules 146
Program Data 147

FIG. 2

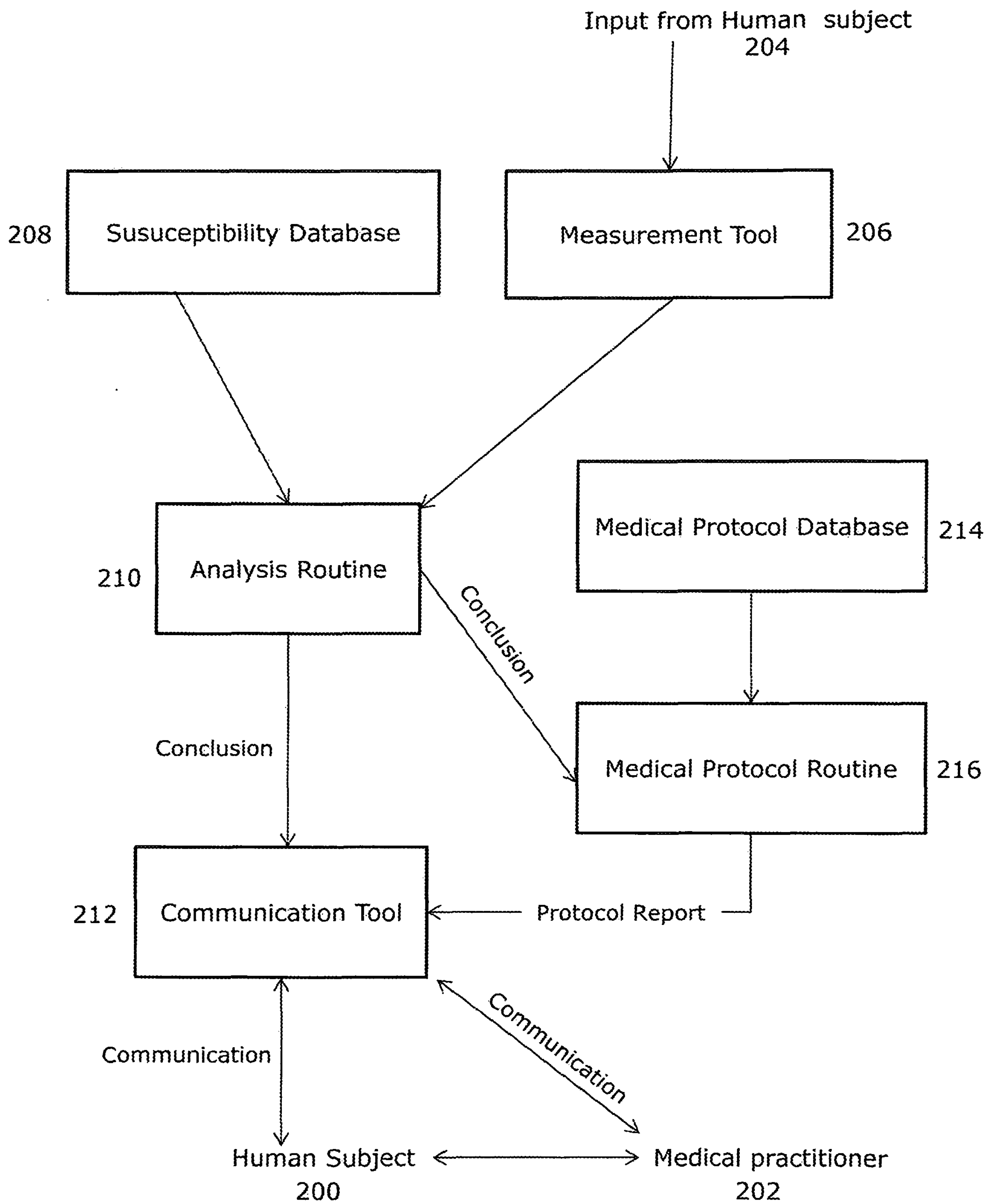
Input from Human subject
204
208
Susuceptibility Database
Measurement Tool
206
Medical Protocol Database
214
210
Analysis Routine
Conclusion
Conclusion
Medical Protocol Routine
216
212
Communication Tool
Protocol Report
Communication
Communication
Human Subject
200
Medical practitioner
202

FIG.3

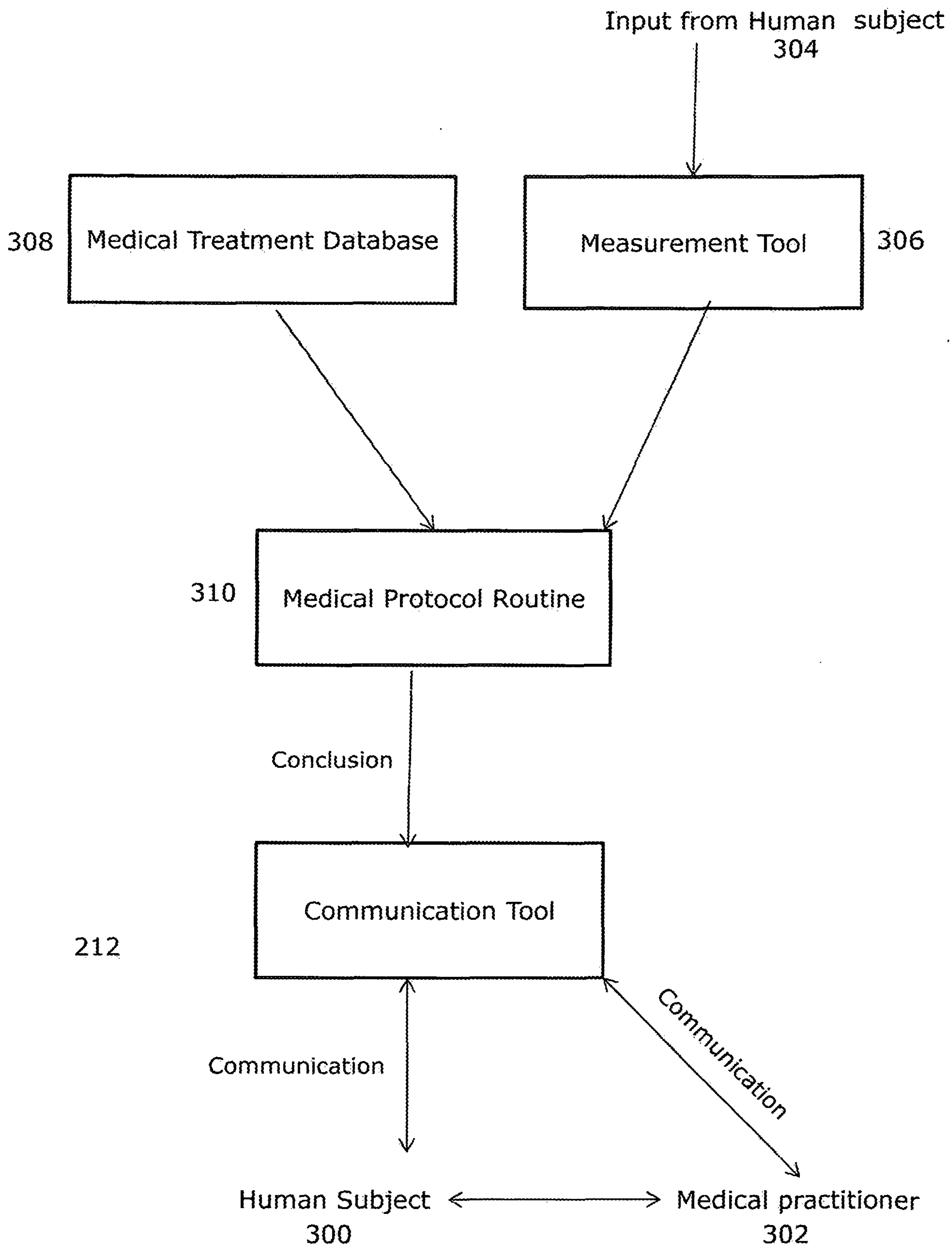
Input from Human subject
304
308
Medical Treatment Database
Measurement Tool
306
310
Medical Protocol Routine
Conclusion
Communication Tool
212
Communication
Communication
Human Subject
300
Medical practitioner
302

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5288644 A, Beavis
- WO 9322456 A
- US 6858394 B
- US 6429027 B
- US 5445934 A
- US 5700637 A
- US 5744305 A
- US 5945334 A
- US 6054270 A
- US 6300063 B
- US 6733977 B
- US 7364858 B
- EP 619321 A
- EP 373203 A
- US 5223409 A
- WO 9218619 A
- WO 9117271 A
- WO 9220791 A
- WO 9215679 A
- WO 9301288 A
- WO 9201047 A
- WO 9209690 A
- WO 9002809 A


### Non-patent literature cited in the description


- **ABEN, K.K. et al.** Familial aggregation of urothelial cell carcinoma. *Int J Cancer,* 2002, vol. 98, 274-8
- **AMUNDADOTTIR, L.T. et al.** Cancer as a Complex Phenotype: Pattern of Cancer Distribution within and beyond the Nuclear Family. *PLoS Med 1,* e65
- **MURTA-NASCIMENTO, C. et al.** Risk of bladder cancer associated with family history of cancer: do low-penetrance polymorphisms account for the increase in risk?. *Cancer Epidemiol Biomarkers Prev,* 2007, vol. 16, 1595-600
- **ABEN, K.K. et al.** Segregation analysis of urothelial cell carcinoma. *Eur J Cancer,* 2006, vol. 42, 1428-33
- **SANDERSON, S. et al.** Joint effects of the N-acetyltransferase 1 and 2 (NAT1 and NAT2) genes and smoking on bladder carcinogenesis: a literature-based systematic HuGE review and evidence synthesis. *Am J Epidemiol,* 2007, vol. 166, 741-51
- **KAUFMAN, D.S.** *Ann Oncol,* 2006, vol. 17, 106-112
- **LOKESHWAR, V.B. et al.** *Urology,* 2005, vol. 66, 35-63
- **FRIEDRICH, M.G. et al.** *BJU Int,* 2003, vol. 92, 389-92
- **RAMAKUMAR, S. et al.** *J Urol,* 1999, vol. 161
- **SOZEN, S. et al.** *Eur Urol,* 1999, vol. 36, 225-9
- **HEICAPPELL, R. et al.** *Urol Int,* 2000, vol. 65, 181-4
- **A. HIRVONEN.** *IARC Sci Publ,* 1999, vol. 148, 251-270
- **CASCORBI I et al.** *Cancer Res,* vol. 61, 5051-6
- **WU, X. et al.** *Front Biosci,* 2007, vol. 12, 192-213
- **KIEMENEY, LA et al.** *Nat Genet,* 2008, vol. 40, 1307-12
- **WU, X. et al.** *Nat Genet,* 2009, vol. 41, 991-5
- **RAFNAR, T. et al.** *Nat Genet,* 2009, vol. 41, 221-7
- **KIEMENEY, LA et al.** *Nat Genet,* 2010, vol. 42, 415-419
- **LI et al.** *Nucleic Acids Research,* 2000, vol. 28, 2
- **LIU et al.** *Biochem Cell Bio,* 2000, vol. 80, 17-22
- **BURCZAK et al.** Polymorphism Detection and Analysis. Eaton Publishing, 2000
- **SHEFFIELD et al.** *Proc. Natl. Acad. Sci. USA,* 1989, vol. 86, 232-236
- **ORITA et al.** *Proc. Natl. Acad. Sci. USA,* 1989, vol. 86, 2766-2770
- **FLAVELL et al.** *Cell,* 1978, vol. 15, 25-41
- **GEEVER et al.** *Proc. Narl. Acad. Sci. USA,* 1981, vol. 78, 5081-5085
- **COTTON et al.** *Proc. Natl. Acad. Sci. USA,* 1985, vol. 85, 4397-4401
- **MYERS et al.** *Science,* 1985, vol. 230, 1242-1246
- **CHURCH ; GILBERT.** *Proc. Natl. Acad. Sci. USA,* 1988, vol. 81, 1991-1995
- **SANGER et al.** *Proc. Natl. Acad. Sci. USA,* 1977, vol. 74, 5463-5467

- **NYREN, P. et al.** *Anal Biochem,* 1993, vol. 208, 171-75
- **BENTLEY, D.R.** *Curr Opin Genet Dev,* 2006, vol. 16, 545-52
- **STRAUSBERG, R.L. et al.** *Drug Disc Today,* 2008, vol. 13, 569-77
- Current Protocols in Molecular Biology. John Wiley & Sons
- **NIELSEN et al.** *Bioconjug. Chem.,* 1994, vol. 5, 3-7
- **GIBBS et al.** *Nucleic Acids Res.,* 1989, vol. 17, 2437-2448
- **BIER et al.** *Adv Biochem Eng Biotechnol,* 2008, vol. 109, 433-53
- **HOHEISEL.** *Nat Rev Genet,* 2006, vol. 7, 200-10
- **FAN et al.** *Methods Enzymol,* 2006, vol. 410, 57-73
- **RAQOUSSIS ; ELVIDGE.** *Expert Rev Mol Diagn,* 2006, vol. 6, 145-52
- **MOCKLER et al.** *Genomics,* 2005, vol. 85, 1-15
- **CHEN et al.** *Genome Res.,* 1999, vol. 9 (5), 492-98
- **KUTYAVIN et al.** *Nucleic Acid Res.,* 2006, vol. 34, e128
- **SAMBROOK et al.** Molecular Cloning: A Laboratory Manual. Cold Spring Harbor Laboratory Press, 2001
- **DEVLIN, B. ; RISCH, N.** *Genomics,* 1995, vol. 29, 311-22
- **LEWONTIN, R.** *Genetics,* 1964, vol. 49, 49-67
- **HILL, W.G. ; ROBERTSON, A.** *Theor. Appl. Genet.,* 1968, vol. 22, 226-231
- The International HapMap Consortium. *Nature,* 2003, vol. 426, 789-796
- **RISCH, N. ; MERKIANGAS, K.** *Science,* 1996, vol. 273, 1516-1517
- **MANIATIS, N. et al.** *Proc Natl Acad Sci USA,* 2002, vol. 99, 2228-2233
- **REICH, DE et al.** *Nature,* 2001, vol. 411, 199-204
- **WALL., J.D. ; PRITCHARD, J.K.** *Nature Reviews Genetics,* 2003, vol. 4, 587-597
- **DALY, M. et al.** *Nature Genet.,* 2001, vol. 29, 229-232
- **GABRIEL, S.B.** *Science,* 2002, vol. 296, 2225-2229
- **PATIL, N. et al.** *Science,* 2001, vol. 294, 1719-1723
- **DAWSON, E. et al.** *Nature,* 2002, vol. 418, 544-548
- **PHILLIPS, M.S. et al.** *Nature Genet.,* 2003, vol. 33, 382-387
- **RISCH, N. ; TENG, J.** *Genome Res.,* 1998, vol. 8, 1273-1288
- **DEVLIN, B. ; ROEDER, K.** *Biometrics,* 1999, vol. 55, 997
- **TERWILLIGER, J.D. ; OTT, J.** *Hum. Hered.,* 1992, vol. 42, 337-46
- **FALK, C.T. ; RUBINSTEIN, P.** *Ann. Hum. Genet.,* 1987, vol. 51, 227-33
- **MANTEL ; HAENSZEL.** *J Natl Cancer Inst,* 1959, vol. 22, 719-48
- **SULEM, P. et al.** *Nat Genet,* 17 May 2009
- **RAFNAR, T. et al.** *Nat,* 2009, vol. 41, 221-7
- **GRETARSDOTTIR, S. et al.** *Ann Neurol,* 2008, vol. 64, 402-9
- **STACEY, S.N. et al.** *Nat Genet,* 2008, vol. 40, 1313-18
- **GUDBJARTSSON, D.F. et al.** *Nat Genet,* 2008, vol. 40, 886-91
- **STYRKARSDOTTIR, U. et al.** *N Engl J Med,* 2008, vol. 358, 2355-65
- **THORGEIRSSON, T. et al.** *Nature,* 2008, vol. 452, 638-42
- **GUDMUNDSSON, J. et al.** *Nat Genet.,* 2008, vol. 40, 281-3
- **STACEY, S.N. et al.** *Nat Genet.,* 2007, vol. 39, 865-69
- **HELGADOTTIR, A. et al.** *Science,* 2007, vol. 316, 1491-93
- **STEINTHORSDOTTIR, V. et al.** *Nat Genet.,* 2007, vol. 39, 770-75
- **GUDMUNDSSON, J. et al.** *Nat Genet,* 2007, vol. 39, 631-37
- **FRAYLING, TM.** *Nature Reviews Genet,* 2007, vol. 8, 657-662
- **AMUNDADOTTIR, L.T. et al.** *Nat Genet.,* 2006, vol. 38, 652-58
- **GRANT, S.F. et al.** *Nat Genet.,* 2006, vol. 38, 320-23
- **SMITH et al.** *Am J Hum Genet,* 2004, vol. 74, 1001-13
- **LOTAN Y ; ROEHRBORN CG.** *Urology,* 2003, vol. 61 (1), 109-118
- **GROSSMAN, HB et al.** *JAMA,* 2005, vol. 293, 810-816
- AntisenseDrug Technology: Principles, Strategies, and Applications. Marcel Dekker Inc, 2001
- **THOMPSON.** *Drug Discovery Today,* 2002, vol. 7, 912-917
- **LAVERY et al.** *Curr. Opin. Drug Discov. Devel.,* 2003, vol. 6, 561-569
- **STEPHENS et al.** *Curr. Opin. Mol. Ther.,* 2003, vol. 5, 118-122
- **KURRECK.** *Eur. J. Biochem.,* 2003, vol. 270, 1628-44
- **DIAS et al.** *Mol. Cancer Ter.,* 2002, vol. 1, 347-55
- **CHEN.** *Methods Mol. Med.,* 2003, vol. 75, 621-636
- **WANG et al.** *Curr. Cancer Drug Targets,* 2001, vol. 1, 177-96
- **BENNETT.** *Antisense Nucleic Acid Drug.Dev.,* 2002, vol. 12, 215-24

- **FIRE et al.** *Nature,* 1998, vol. 391, 806-11
- **KIM ; ROSSI.** *Nature Rev. Genet.,* 2007, vol. 8, 173-204
- **AMARZGUIOUI et al.** *FEBS Lett.,* 2005, vol. 579, 5974-81
- **KIM et al.** *Nature Biotechnol.,* 2005, vol. 23, 222-226
- **SIOLAS et al.** *Nature Biotechnol.,* 2005, vol. 23, 227-231
- **MARQUES et al.** *Nature Biotechnol.,* 2006, vol. 23, 559-565
- **BRUMMELKAMP et al.** *Science,* 2002, vol. 296, 550-553
- **KIM ; ROSSI.** *Nat. Rev. Genet.,* 2007, vol. 8, 173-184
- **CHEN ; RAJEWSKY.** *Nat. Rev. Genet.,* 2007, vol. 8, 93-103
- **REYNOLDS et al.** *Nat. Biotechnol.,* 2004, vol. 22, 326-330
- **CHI et al.** *Proc. Natl. Acad. Sci. USA,* 2003, vol. 100, 6343-6346
- **VICKERS et al.** *J. Biol. Chem.,* 2003, vol. 278, 7108-7118
- **AGAMI.** *Curr. Opin. Chem. Biol.,* 2002, vol. 6, 829-834
- **LAVERY et al.** *Opin. Drug Discov. Devel.,* 2003, vol. 6, 561-569
- **SHI.** *Trends Genet.,* 2003, vol. 19, 9-12
- **SHUEY et al.** *Drug Discov. Today,* 2002, vol. 7, 1040-46
- **MCMANUS et al.** *Nat. Rev. Genet.,* 2002, vol. 3, 737-747
- **XIA et al.** *Nat. Biotechnol.,* 2002, vol. 20, 1006-10
- **PLASTERK et al.** *curr. Opin. Genet. Dev.,* 2000, vol. 10, 562-7
- **BOSHER et al.** *Nat. Cell Biol.,* 2000, vol. 2, E31-6
- **HUNTER.** *Curr. Biol.,* 1999, vol. 9, R440-442
- **AUSUBEL, F. et al.** Current Protocols in Molecular Biology. John Wiley & Sons, 1998
- **KRAUS, M. ; AARONSON, S.** *Methods Enzymol.,* 1991, vol. 200, 546-556
- **KARLIN, S. ; ALTSCHUL, S.** *Proc. Natl. Acad. Sci. USA,* 1993, vol. 90, 5873-5877
- **ALTSCHUL, S. et al.** *Nucleic Acids Res.,* 1997, vol. 25, 3389-3402
- **KENT, W.J.** *Genome Res.,* 2002, vol. 12, 656-64
- **MYERS ; MILLER.** *CABIOS,* 1989
- **TORELLIS, A. ; ROBOTTI, C.** *Comput. Appl. Biosci.,* 1994, vol. 10, 3-5
- **PEARSON, W. ; LIPMAN, D.** *Proc. Natl. Acad. Sci. USA,* 1988, vol. 85, 2444-48
- **NIELSEN, P. et al.** *Science,* 1991, vol. 254, 1497-1500
- **KOHLER ; MILSTEIN.** *Nature,* 1975, vol. 256, 495-497
- **KOZBOR et al.** *Immunol. Today,* 1983, vol. 4, 72
- **COLE et al.** Monoclonal Antibodies and Cancer Therapy. Alan R. Liss, 1985, 77-96
- Current Protocols in Immunology. John Wiley & Sons, Inc, 1994
- **GALFRE et al.** *Nature,* 1977, vol. 266, 55052
- **R.H. KENNETH.** Monoclonal Antibodies: A New Dimension In Biological Analyses. Plenum Publishing Corp, 1980
- **LERNER.** *Yale J. Biol. Med.,* 1981, vol. 54, 387-402
- **FUCHS et al.** *Bio/Technology,* 1991, vol. 9, 1370-1372
- **HAY et al.** *Hum. Antibod. Hybridomas,* 1992, vol. 3, 81-85
- **HUSE et al.** *Science,* 1989, vol. 246, 1275-1281
- **GRIFFITHS et al.** *EMBO J.,* 1993, vol. 12, 725-734
- **KUTYAVIN, I.V. et al.** *Nucleic Acids Res,* 2006, vol. 34, e128
- **LI, H. ; DURBIN, R.** *Bioinformatics,* 2009, vol. 25, 1754-60
- **LI, H. et al.** *Bioinformatics,* 2009, vol. 25, 2078-9
- **MARCHINI, J. et al.** *Nat Genet,* 2007, vol. 39, 906-13
- **WETZELS, J.F. et al.** *Kidney Int,* 2007, vol. 72, 632-7
- **SAK, S.C. et al.** *Br J Cancer,* 2005, vol. 92, 2262-5
- **MATULLO, G. et al.** *Cancer Epidemiol Biomarkers Prev,* 2005, vol. 14, 2569-78
- **SHEN, M. et al.** *Cancer Epidemiol Biomarkers Prev,* 2003, vol. 12, 1234-40
- **KELLEN, E. et al.** *Int J Cancer,* 2006, vol. 118, 2572-8
- **LARSSON, P. et al.** *Scand J Urol Nephrol,* 2003, vol. 37, 195-201
- **GOLKA, K. et al.** *J Toxicol Environ Helath A,* 2008, vol. 71, 881-6
- **GOLKA, K. et al.** *Pharmacogenet Genomics,* 2009

SEQUENCE LISTING

<110> deCODE Genetics ehf

<120> GENETIC VARIANTS AS MARKERS FOR USE IN URINARY BLADDER CANCER RISK ASSESSMENT, DIAGNOSIS, PROGNOSIS AND TREATMENT

<130> 2594-PC00

<160> 209

<210> 1
<211> 401
<212> DNA
<213> Homo sapiens

<400> 1

```
ctggcatgat gacttatgcc tgtaatccca gtgttttggg aggctaaggt gggaggatca     60
cttgaggcca ggaattcaaa accagcctgg acaatacaat gagactttgt ctctaaaaaa    120
aaataaaata aattaaaata aacacagctg gatgtggtgg cacaggaaaa aaaaatacca    180
tttaggagtc tcttaaaggc rgcttgtgaa tgcttacaaa gcgtggctag tatcttatta    240
cagaaaacag agcccacatc atgcatcctt cttctcacat ttcataaaca aggccaaggg    300
aaactgctgt ggggcaacct gttgctttgg tgttggtccc caagatgcag ccctcacaat    360
ctgcccccaa acgtgtcaga acatgaaccc cctcctcccc c                        401
```

<210> 2
<211> 401
<212> DNA
<213> Homo sapiens

<400> 2

```
ttgtgttggc acatttgcca cagaaagaaa acttgggata aatcctatta tagaggcagt     60
aaatattaca taaagtaaaa aatcaactct cagcttattt ggcttttgaa aaattctact    120
ctggggttgc tcagagtgag ctatatattg agattttaaa aatatttttg aagtacttat    180
gacatacaaa ataaaatatt rtctacatag ttaaagtccc ctttgtccct ccacttgctc    240
ttatgtccct ccctccccac tagaggcaac cctgattctg atttggcaga tattatttct    300
ttaatatacc tatttttgga aaagagaaac aatcgttcaa ggatgcaggt agtgtgagct    360
ggaggaaaat gatatcacag ttggtggggc agagtgtgtg t                        401
```

<210> 3
<211> 401
<212> DNA

<213> Homo sapiens

<400> 3

```
ggaggtccca tttgctgagc atggaattca atgcacttga gaaaattggg ctaaaaaaag      60
aacagagcaa aagaatgtgc atgtgacaaa gcaggctgac acacggaatc gctatactct     120
ggaggggctt tgtttcacct gccaacaggc cccttacgtg ttgtgttggc acatttgcca     180
cagaaagaaa acttgggata ratcctatta tagaggcagt aaatattaca taaagtaaaa     240
aatcaactct cagcttattt ggcttttgaa aaattctact ctggggttgc tcagagtgag     300
ctatatattg agattttaaa aatatttttg aagtacttat gacatacaaa ataaaatatt     360
atctacatag ttaaagtccc ctttgtccct ccacttgctc t                          401
```

<210> 4
<211> 401
<212> DNA
<213> Homo sapiens

<400> 4

```
cccatgcatt gcctcaggca tcttctgtgc tccagatctt ccttgagatc ttggcttcct      60
agggaccaat gggagttccc gggatgcttc ctgctaactt tcaatcccac cctcagtttc     120
cttccagaac atcctgcctt tagtcctgag ttctgacccc tcctgtctta acaggactca     180

gtctttcagc cccatttgag racatctact ttggactctg gggtttcaac agctctctgg     240
cctgcattgc aatgggagga atgttcatgg cgctcacctg gcaaacccac ctcctggctc     300
ttggctgtgg tgagtctccc acgcccctgg gggagggctg ctcatgacta caggatctca     360
atcaaggata agcagtaaaa acggactgca tgaaaaatca g                          401
```

<210> 5
<211> 401
<212> DNA
<213> Homo sapiens

<400> 5

```
ggttgattac atttacccct ttgagtgagc atcacagtaa cccagccatt ctaaaacttc      60
agaatgcatc agaatcacct gaaagacttg ttaaaacaca aatcgctggg ccccctcctc     120
agtctgattc agcgtcagag ataaggggaa gaatatttct ttttttattt ttctaaaaaa     180
cagtctcatt ctgagccaag rtcgcgccac tgcacttcag cctgggcaac agagcaagac     240
ttcatctcaa aaaaaaaaaa aaaagagaaa agaaaaaaaa agaaaaaggg tctcattctg     300
ttgcccaggc tggagtgcgg tggtgtgaac acagctcact gcagcctcaa cctcctgggc     360
tcaagcaatc ctgcagcctc agcctcccaa gtaaagtagc t                          401
```

<210> 6
<211> 401
<212> DNA
<213> Homo sapiens

<400> 6

atttttctaa aaaacagtct cattctgagc caagatcgcg ccactgcact tcagcctggg 60
caacagagca agacttcatc tcaaaaaaaa aaaaaaaaga gaaaagaaaa aaaaagaaaa 120
agggtctcat tctgttgccc aggctggagt gcggtggtgt gaacacagct cactgcagcc 180
tcaacctcct gggctcaagc ratcctgcag cctcagcctc ccaagtaaag tagctaggac 240
cacaggcgtg ccaccatgcc tggttaattt tttatttttt atagagatgg ggtctcccta 300
tgttacccag gctgatcttg aattcccggg ctcaagcaat cctcccgcct ccacctccca 360
aagtgctggg attacaggca taagccacca tgccggcaga a 401

<210> 7
<211> 401
<212> DNA
<213> Homo sapiens

<400> 7

aaaaagaaaa agggtctcat tctgttgccc aggctggagt gcggtggtgt gaacacagct 60
cactgcagcc tcaacctcct gggctcaagc aatcctgcag cctcagcctc ccaagtaaag 120
tagctaggac cacaggcgtg ccaccatgcc tggttaattt tttatttttt atagagatgg 180
ggtctcccta tgttacccag sctgatcttg aattcccggg ctcaagcaat cctcccgcct 240
ccacctccca aagtgctggg attacaggca taagccacca tgccggcaga atttccactt 300
ctaacaagtt ctcagggggt gctgatgctg ttgctctcag gatcacattt caagaactgc 360
tgtattaatc ctttctgact cccagtgttc tagccagact c 401

<210> 8
<211> 401
<212> DNA
<213> Homo sapiens

<400> 8

gctcactgca gcctcaacct cctgggctca agcaatcctg cagcctcagc ctcccaagta 60
aagtagctag gaccacaggc gtgccaccat gcctggttaa ttttttattt tttatagaga 120
tggggtctcc ctatgttacc caggctgatc ttgaattccc gggctcaagc aatcctcccg 180
cctccacctc ccaaagtgct kggattacag gcataagcca ccatgccggc agaatttcca 240
cttctaacaa gttctcaggg ggtgctgatg ctgttgctct caggatcaca tttcaagaac 300
tgctgtatta atcctttctg actcccagtg ttctagccag actcagcctg tcagagcgag 360
aaggcatcct gagacctcta ctccatcctt cttactttac t 401

<210> 9
<211> 401
<212> DNA
<213> Homo sapiens

<400> 9

gggggtgctg atgctgttgc tctcaggatc acatttcaag aactgctgta ttaatccttt 60
ctgactccca gtgttctagc cagactcagc ctgtcagagc gagaaggcat cctgagacct 120
ctactccatc cttcttactt tactgttggg gtcctgaggc cagagaggct aagggatgtg 180
ccgcagggaa tctggacagc ratgggtaaa tccacccccg gaacccacac ttaccatcca 240
cctccagagt tatcccaccg cactcctctg cttccctttt atagcattca ggccctcacg 300
gcaacctctt aggtgaaaac agactgcatg tgatttggat ctgaaaagct aatagatccc 360
aggtggattt tgagtggagg ctcattcacc catagcctct g 401

<210> 10
<211> 401
<212> DNA
<213> Homo sapiens

<400> 10

```
ctccagagtt atcccaccgc actcctctgc ttccctttta tagcattcag gccctcacgg     60
caacctctta ggtgaaaaca gactgcatgt gatttggatc tgaaaagcta atagatccca    120
ggtggatttt gagtggaggc tcattcaccc atagcctctg gcatgcctaa ttcaatcaaa    180
gtataagcat ttaagataat rttctagagt ggagagaatg agatttgctt gggaacaaaa    240
aggaggaggg atagtgtaat gtggagaaat tatgtctaat ctagtggaaa tatatgtcta    300
gaatcagttt atcaccagat taatcaagcc aaggtatcta aacagttatg aaaacagtgg    360
gccatgtatc aggcgggttt agaatagatt tctgcactgg c                        401
```

<210> 11
<211> 401
<212> DNA
<213> Homo sapiens

<400> 11

```
ttcacccata gcctctggca tgcctaattc aatcaaagta taagcattta agataatatt     60
ctagagtgga gagaatgaga tttgcttggg aacaaaaagg aggagggata gtgtaatgtg    120
gagaaattat gtctaatcta gtggaaatat atgtctagaa tcagtttatc accagattaa    180
tcaagccaag gtatctaaac rgttatgaaa acagtgggcc atgtatcagg cgggtttaga    240
atagatttct gcactggcag aaaatgggat ggtaccaacg gtttctaaag acccattcca    300
ttttgattcg atgctatagc aagggtaaca taactcaggt tgctgtgatg tagccatgta    360
gatgtcattt tgtcaaattc tttactatta ctcagctatt t                        401
```

<210> 12
<211> 401
<212> DNA
<213> Homo sapiens

<400> 12

```
ccatagcctc tggcatgcct aattcaatca aagtataagc atttaagata atattctaga     60
gtggagagaa tgagatttgc ttgggaacaa aaaggaggag ggatagtgta atgtggagaa    120
attatgtcta atctagtgga aatatatgtc tagaatcagt ttatcaccag attaatcaag    180
ccaaggtatc taaacagtta ygaaaacagt gggccatgta tcaggcgggt ttagaataga    240
tttctgcact ggcagaaaat gggatggtac caacggtttc taaagaccca ttccattttg    300
attcgatgct atagcaaggg taacataact caggttgctg tgatgtagcc atgtagatgt    360
cattttgtca aattctttac tattactcag ctatttcacc t                        401
```

<210> 13
<211> 401
<212> DNA
<213> Homo sapiens

<400> 13

```
aatcaaagta taagcattta agataatatt ctagagtgga gagaatgaga tttgcttggg     60
aacaaaaagg aggagggata gtgtaatgtg gagaaattat gtctaatcta gtggaaatat    120
atgtctagaa tcagtttatc accagattaa tcaagccaag gtatctaaac agttatgaaa    180

acagtgggcc atgtatcagg ygggtttaga atagatttct gcactggcag aaaatgggat    240
ggtaccaacg gtttctaaag acccattcca ttttgattcg atgctatagc aagggtaaca    300
taactcaggt tgctgtgatg tagccatgta gatgtcattt tgtcaaattc tttactatta    360
ctcagctatt tcacctagct gttctgttga aatgttgaac t                        401
```

<210> 14
<211> 401
<212> DNA
<213> Homo sapiens

<400> 14

```
tgtctagaat cagtttatca ccagattaat caagccaagg tatctaaaca gttatgaaaa     60
cagtgggcca tgtatcaggc gggtttagaa tagatttctg cactggcaga aaatgggatg    120
gtaccaacgg tttctaaaga cccattccat tttgattcga tgctatagca agggtaacat    180
aactcaggtt gctgtgatgt rgccatgtag atgtcatttt gtcaaattct ttactattac    240
tcagctattt cacctagctg ttctgttgaa atgttgaact ccttctccat attcgttcac    300
aaggataaag gagaggatta cagacaggtg ctgtagccac ctgagttcag ctgggttgga    360
atgtttatcc tacaaccttt cagctttatt ctgagattgg t                        401
```

<210> 15
<211> 401
<212> DNA
<213> Homo sapiens

<400> 15

```
gtagccatgt agatgtcatt ttgtcaaatt ctttactatt actcagctat ttcacctagc     60
tgttctgttg aaatgttgaa ctccttctcc atattcgttc acaaggataa aggagaggat    120
tacagacagg tgctgtagcc acctgagttc agctgggttg gaatgtttat cctacaacct    180
ttcagcttta ttctgagatt sgttaggggt ttccacctga gttcagctgg gttagaatgt    240
ttatcctaca acctttcagc tttattctga gattggttag ggtttcaaa cctttatttg    300
ggatgcatac ctttattttt ctggaggaag tagccacaaa tatgtattaa acacacatga    360
tacaaaagac agtaccagga agagcaaggg gtttagaagc t                        401
```

<210> 16
<211> 401
<212> DNA
<213> Homo sapiens

<400> 16

```
gggacagcct gagcatgtcc ttcaagatca aggagaaggc attttgagca caggagatgg     60
cgacgaggtt tttgtttttc tgggtttttt gttgtttttt gtttttttggt tttttttttt    120
tttttttga cagagtcttg ctctgttgcc aggctggaat gcagtggcac agtggcacga    180
tcttggctca ctgcaacctc ygactccctg gttcaagcgg ttctcctgcc tcagcctccc    240
aagtagctgg gcttacaggc acgcaccatc acgcctagct aatttttgta tttttagtag    300
agacggggtt tcaccatgtt ggccaggatg gtctcaatct tctgacctca tgatctgtcc    360
accccggcct cccaaagtgc tgggattaca agtatgagcc a                        401
```

<210> 17
<211> 401
<212> DNA
<213> Homo sapiens

<400> 17

```
ttctgggttt tttgttgttt tttgtttttt ggtttttttt tttttttttt tgacagagtc     60
ttgctctgtt gccaggctgg aatgcagtgg cacagtggca cgatcttggc tcactgcaac    120
ctccgactcc ctggttcaag cggttctcct gcctcagcct cccaagtagc tgggcttaca    180
ggcacgcacc atcacgccta sctaattttt gtatttttag tagagacggg gtttcaccat    240
gttggccagg atggtctcaa tcttctgacc tcatgatctg tccaccccgg cctcccaaag    300
tgctgggatt acaagtatga gccaccgcac ctggcgggtg ctgagttttt tgttttatgt    360
tgttgttgtt gtttgagatg gactcttgct ctgtagctca g                        401
```

<210> 18
<211> 401
<212> DNA
<213> Homo sapiens

<400> 18

```
tgggccttct cagaggaagt aaaggcaggc ttgggtatca gtcatatttg gggagaagat     60
acagatgttg ataaacttaa gcaattgaga ggagaaaata aacaagcttg attcttaata    120
agttatgcaa atccaccaga aaaactgttt ttccagactt ttaaatagtg ctgagcgagt    180
gctagacact atttgttcta ygagtttcag acatgcatta actaacttaa tcatcagaac    240
aactgtatga attaaatatg gtaacttcca ttttctaaga tggggaaact gaggcataga    300
aaggttaagt aatttgctta cattcataag caaatacgct agtaagtagc agacctgctg    360
gaattgaatc caggctgtct ggcaccagag tctgagctct t                        401
```

<210> 19
<211> 401
<212> DNA
<213> Homo sapiens

<400> 19

```
ttttattttg gatttgtcgt aagtataagt ttttgttttg ggtacttgct tatttaggca     60
actgtaaact ttattaactt gcttattcac tctgacttag ttcatattaa ccttctgtac    120
tttttttttt ttgagacaga gtctcactct gttccccagg ctggagtgca gtggcacaat    180
ctcagctcac tgcagcctcc rcctcctggg ttcaagcgat tcctatgcct cagactccca    240
agtagctggg attacagaca tgcaccacca tgcccagcta atttttgta ctttttgtag    300
agacagggtt ttgccatgtt ggccaggctg gtctcaaact cctgacctca agtgatccac    360
ctgcctcggc ctcccaaagt gctaggatta ctggtggatt a                        401
```

<210> 20
<211> 401
<212> DNA
<213> Homo sapiens

<400> 20

```
cagcactttg ggaggccaag gcgggtggat cacaaggtca agagattgag accatcctgg     60
ctaacacggt gaaaccccat ctctactaaa aatacaaaaa ttagctgggc gtggtagcac    120
gcgcctatag tcccagctac tcaggaggct gaggcaggag aaccacttga actcggaagg    180
cagagctgca gtgagctgag rtcatgccac tgcactccag cctgggtgac agagagagac    240
tctgtctcaa agaaaaaatt atcgactgta ggttgttcag tttgttgtcc ttcttttatg    300
gtatttgctc tcctgggatg tcccctttcc ttgtcctggg agctcacgtt tccctcggga    360
taccagctgt ttgggtgagt ctctgggcag agatggaagc c                        401
```

<210> 21
<211> 401
<212> DNA
<213> Homo sapiens

<400> 21

```
acatgaagac gagatggtga gtgtgtctca cggtgagctc cggtggccca agtggctgtg     60
tggccattat atgaaggtca ttcttcaggc tgtccccatg aaacctgagg gcttccctga    120
gcctctgtga gccttctctt caaccaaaac tgaggaatag ataattagct ggttgagatc    180
tttgcttttg ttgttttaca ytgaaagtca cccatatact cgaattactg attctacaat    240
tttttggcca ctcaaagcaa ataaaaacat aagacgttgg ctgggcgcgg tggctcatgc    300
ctgtaatccc agcactttgg gaggccgaga cgggcagatg acaaggtcag gagattgaga    360
ccatcctggt taacatggtg aaaccccgtc tctactaaca a                        401
```

<210> 22
<211> 401
<212> DNA
<213> Homo sapiens

<400> 22

```
caacaccatg aaaaatatat tcctcttact tccattgtac aggtgaggaa atggaggctt     60
aaaacagagc ccatggagct cctaagtgat ggagccagga tttgaaccca ggactgctga    120
ctttaggctc atgcttgtaa tcagggcact gtgcattcca ggtgatttat attggaaggc    180

agcctttcct gtgattaaaa rtgcatctac gaagcattgt tctttccctc cttttttttt    240
ctgtagccct gttcacggcc tatcttggag tcggcatggc aaactttatg gctgaggtga    300
gtttgcttta gtctcacttt tcattagcgt aattgaccag cttacaacta tatgggaaat    360
gctcctgaag tccactgggc tggcatccag tggcaggatc c                        401
```

<210> 23
<211> 401
<212> DNA
<213> Homo sapiens

<400> 23

```
tgctgctgcc aaaagggcta gatactgcct tgactcctta gcagccatga ccacatcaac     60
atggactaag ctatcagaaa ccggccctgc atcacctgca atggaatatc tggctttctg    120
tttaagtaaa agatatccta aagtctactt cttatcactt agagttagac tctgaacttg    180
ataatatgtt atacaaccta ygtttcattt ttttttaaac caagcaatct gatacttgta    240
acaactgggt gattgtggtt tggactaaag tttgctgtga tgctgaaaga aaggccaggg    300
agctccagga gaagggagag cagtgcttct catggtcatg gatcctgcca ctggatgcca    360
gcccagtgga cttcaggagc atttcccata tagttgtaag c                        401
```

<210> 24
<211> 401
<212> DNA
<213> Homo sapiens

<400> 24

```
tgaagggccc catgtgctct gatgtactct ccatcatgca tgtgtgtaat gggactttgg     60
ctttccacca gaaagacact gtgtctcctt aaggaccctc ccagataaaa tctccatgct    120
gctgccaaaa gggctagata ctgccttgac tccttagcag ccatgaccac atcaacatgg    180
actaagctat cagaaaccgg scctgcatca cctgcaatgg aatatctggc tttctgttta    240
agtaaaagat atcctaaagt ctacttctta tcacttagag ttagactctg aacttgataa    300
tatgttatac aacctacgtt tcattttttt ttaaaccaag caatctgata cttgtaacaa    360
ctgggtgatt gtggtttgga ctaaagtttg ctgtgatgct g                        401
```

<210> 25
<211> 401
<212> DNA
<213> Homo sapiens

<400> 25

```
ggcaagctgc acaccagagt cagctaggaa gacagaaaaa tatggagcct taggccctgt     60
cctttggtat ttctgataga gtaggtcttg tatgatgctt gaacatctgt gttttttttt    120
aactccccca gatgattctg atgtgcagtc agattagggt acccctacac tccatcacac    180
cccagggagg tccatgcatc rggtcagagc taaccaatgg tgtatgctca gaattgtgtg    240
agtttccatg agcagcacaa agaggaccta ccctcaagga acttagagtc tatttgggag    300
acagaatgga aagaaacaaa gcaagtcaag tctaagatct agaccaggca gaagtcaagg    360
tcagagaggt cactgtgggc tggactaatc agagaaggcc t                        401
```

<210> 26
<211> 401
<212> DNA
<213> Homo sapiens

<400> 26

```
ttctctccct ctcatatatt cagacttcct gcaccagatg tgccaagaga acatttgaca     60
gagatgacac ttgcaaactg caaatggccc ctgaccagtc ttcatgtcca caaggccttc    120
tctgattagt ccagcccaca gtgacctctc tgaccttgac ttctgcctgg tctagatctt    180
agacttgact tgctttgttt ytttccattc tgtctcccaa atagactcta agttccttga    240
gggtaggtcc tctttgtgct gctcatggaa actcacacaa ttctgagcat acaccattgg    300
ttagctctga cctgatgcat ggacctccct ggggtgtgat ggagtgtagg ggtaccctaa    360
tctgactgca catcagaatc atctggggga gttaaaaaaa a                        401
```

<210> 27
<211> 401
<212> DNA
<213> Homo sapiens

<400> 27

```
caatagcgtg gccaaacaga agggccaggt acaagctggc aatccaacct ggggaagaag     60
agaaagccac acgctccttt cagatcccat ctgacagctg agctgcacaa ggcatccccc    120
tctacttgca ttcagcccag ggggatgaac cactgggggc attgctctgg attctgtgca    180
tgctcgtgga gactgaattt mcaatctcag ggcattgagt tgttggaatg ttgacatcca    240
ttctttaggg cagccactta tgtctctatc ttgtatgtct ttctctccct ctcatatatt    300
cagacttcct gcaccagatg tgccaagaga acatttgaca gagatgacac ttgcaaactg    360
caaatggccc ctgaccagtc ttcatgtcca caaggccttc t                        401
```

<210> 28
<211> 401
<212> DNA
<213> Homo sapiens

<400> 28

```
aacctgttct tacatattaa agaaaagtta cttactgtat ttatgaaata ctcagcttag      60
gcatttttac tttaacccct aaattgattt tgtaaatgcc acaaatgcat agaattgtta     120
ccaacctcca aagggctctt taaaatcata ttttttattc atttgaggat gtcttataaa     180
gactgaaggc aaaggtcaga wtgcttacgg gtgttatttt tataagttgt tgaattcctt     240
aatttaaaaa agctcattat tttttgcaca ctcacaatat tctctctcag aaatcaatgg     300
catttgaacc accaaaaaga aataaagggc tgagtgcggt ggctcacgcc tgtaatccca     360
gcactttggg gagcccaggc gggcagattg cttgaaccca g                         401
```

<210> 29
<211> 401
<212> DNA
<213> Homo sapiens

<400> 29

```
ttgcttgaac ccaggagttc aagaccagcc tgggcagcat ggtgaaaccc tgtatctaca      60
aaaaatacaa aaattagcca ggcatggtgg tgggtgcctg tagttccagc tacttgggag     120
gctgaggtgg gaaaatgact tgagcccagg aggaggaggc tgcagtgagc taagattgca     180
ccactgcact ccaacctggg ygacaagagt gaaactgtgt ctctcaaaaa aaaaaaaaaa     240
caaacaaaaa caaaaacaaa acaaaacaaa acaaaacaaa acaggtaagg attcccctgt     300
tttcctctct ttaattttaa agttatcagt tccgtaaagt ctctgtaacc aaacatactg     360
aagacagcaa cagaagtcac gttcagggac tggctcacac c                         401
```

<210> 30
<211> 401
<212> DNA
<213> Homo sapiens

<400> 30

```
tgggcaacat agcaagactc catctcttaa aaaataaaaa tagtaacatt agccaggtgt      60
agcagcacac atctgcagca gctactcagg aggctgaggt ggaaagatcg cttgtgcaca     120
gaagttcgag gctgcagtga gctatatgat catgtcactg cactccagcc tgtgtgaccg     180
agcaagaccc tatctcaaaa waattaatta attaattaat taattaattt aaaaaggaag     240
tcatgttcat ttactttcca cttcagtgtg tatcgtgtag tattttggag gttggaaagt     300
gaaacgtagg aatcctgaag atttttttcca cttctagttt gcagtgctca gtgcacaata     360
tacattttgc tgaatgaata aacagaaata gggaagtaaa c                         401
```

<210> 31
<211> 401
<212> DNA
<213> Homo sapiens

<400> 31

```
tgcatagaaa tttgttatac agacattagg aaaaagtata agtgctccct gcaaatattc      60
aattttagga aaatagtaac aaaaatagct tgagaaaatt aatatcattt aaattcgaca     120
atgtacaatg ttcccattta cagatttgtg tgagcacaag aagctcctgc atggtagatc     180
cctgttctga agatcgactc rggtccattt tagaggcctc tggttctctc agccacagcc     240
tagattcttc actgctgatg atctttggtt atgcttgctt ttttttcccc acaattcttc     300
tttcacacgt tccctaacaa gaaagggttc agttcaaaca ctgaaaattg agcttctcca     360
cagcaatatc cataaaccta tgacagttgc aaattcatag t                         401
```

<210> 32
<211> 401

<212> DNA
<213> Homo sapiens

<400> 32

```
taccatgcag gagcttcttg tgctcacaca aatctgtaaa tgggaacatt gtacattgtc     60
gaatttaaat gatattaatt ttctcaagct atttttgtta ctattttcct aaaattgaat    120
atttgcaggg agcacttata ctttttccta atgtctgtat aacaaatttc tatgcaagta    180
catgaataaa ttatgctcac mgctcagttt tgtataatgt gccatttata gccatcatgt    240
atatgaacaa tcacaggcat aagtaggtat cgcttatgtt gcacgtgcta gaatccttat    300
tcctttctta cacttggtaa tagcaccaga tgtatagttg agtaaaccct tttcaatttc    360
agattggcag gcccatcccc ttgggtatcc tctcaactat c                        401
```

<210> 33
<211> 401
<212> DNA
<213> Homo sapiens

<400> 33

```
aagatgagaa atttacttca acaagtttga atgataagtt tcaaagcagc cctaaaagga     60
aactaaagaa gccatagtgg ggtctcccta ttgaagtgcc ctctgtcata acaaaacttc    120
tcctacctat ggtttcaagg taaattcaaa taaattctcc agagggatct gggcattcta    180
tgtcttcttt tatctggggt rgaaacgaca ggttgtgagg ctcttatgct aaggacactc    240
tatacccttta ggagctaaag gggccaactt gcctgttacc catgatacag gattagcacg    300
gaatatgtca agatcaaagc taagttttac aagaaaccag gaatttctaa ggaggcttct    360
tattaacaat tttgcaagaa tatttttgtg gttagtaaac a                        401
```

<210> 34
<211> 401
<212> DNA
<213> Homo sapiens

<400> 34

```
ctaaaagaga aaattgctcc caggactgac ctaatcttat ttaatattaa cctggtaggt     60
gtgaacatat ctaaagcaag cttaggtcca cctgatgggg tagcaagtca accactgagg    120
ctcaggaaga ggaaaaccta ggaagggggt cttcaatatt ggaacttggg gtgggacagg    180
tgtcaagggt gagctctgtg rcggaaatgg cactggggta ttatggttta gtaaactgaa    240
ctatatagac caagcaacta atgctccttg tccaatcctg tactcacaag ctatttggct    300
tcctatgtag acttgtctta cagggtaaaa acacatgtgt ccacactgag gaagggaggt    360
ttaggcagag atctaaggaa taggcaggag gtggaggaag g                        401
```

<210> 35
<211> 401
<212> DNA
<213> Homo sapiens

<400> 35

```
gcgaggaaga gtgtgcagca tgagaggaga agtgatcctt cagtgctcca tcgatggtgt     60
ggcaccacca ttcaccttgt agcccaagct acaaatcagg aagccatcct tgacacctcc    120
ccctccctca ccctgcaaat caactcatca cagagttctg gtgattctgc ctcctgtctc    180
tcatatccat gaacgtctct mcatcaccac tgaagtccaa gccaccatca cttctctcct    240
gcactactgc aatggcctcc cagttggctt gctgaattca ctccagcacc ctttcattca    300
ccataatcca gccacagcaa tcttctcact ccttgaaagc accaatctcc ttgtttcaag    360
gcctttatcc acaagtttcc ctttgccaag ggtactgttc c                        401
```

<210> 36
<211> 401
<212> DNA
<213> Homo sapiens

<400> 36

```
cttcaactcc ctacagggac tgttccaaaa gcacttcctc aaactcaaca tatccaggac      60
aaaactcatc ttcttcaact attcttctct ccctcccatg ttgactcatg gagaaaggta     120
gcaccttcca cccgaatggg aaagccagaa acaggatgcc atccttgacc tcattctctc     180
acctccagga aatagttagc yaagggctga gggttccacc tccttgatag cccccattca     240
tccatttatt gaatgactgt ttactacata acaggaactg tgactacaat agtgaaccag     300
acggccatag tctcttccct cacaaaaact ttatgaggtg aactgacaag taaaggagta     360
atataatata gtgcagtgat gataatgagt aagagacaac c                          401
```

<210> 37
<211> 401
<212> DNA
<213> Homo sapiens

<400> 37

```
acagggactg ttccaaaagc acttcctcaa actcaacata tccaggacaa aactcatctt      60
cttcaactat tcttctctcc ctcccatgtt gactcatgga gaaaggtagc accttccacc     120
cgaatgggaa agccagaaac aggatgccat ccttgacctc attctctcac ctccaggaaa     180
tagttagcca agggctgagg kttccacctc cttgatagcc cccattcatc catttattga     240
atgactgttt actacataac aggaactgtg actacaatag tgaaccagac ggccatagtc     300
tcttccctca caaaaacttt atgaggtgaa ctgacaagta aaggagtaat ataatatagt     360
gcagtgatga taatgagtaa gagacaacca gttaacaagt t                          401
```

<210> 38
<211> 401
<212> DNA
<213> Homo sapiens

<400> 38

```
aactcaacat atccaggaca aaactcatct tcttcaacta ttcttctctc cctcccatgt      60
tgactcatgg agaaaggtag caccttccac ccgaatggga aagccagaaa caggatgcca     120
tccttgacct cattctctca cctccaggaa atagttagcc aagggctgag ggttccacct     180
ccttgatagc cccccattcat ycatttattg aatgactgtt tactacataa caggaactgt    240
gactacaata gtgaaccaga cggccatagt ctcttccctc acaaaaactt tatgaggtga     300
actgacaagt aaaggagtaa tataatatag tgcagtgatg ataatgagta agagacaacc     360
agttaacaag tttgagggaa gagtatgcca gtgacctctt t                          401
```

<210> 39
<211> 401
<212> DNA
<213> Homo sapiens

<400> 39

agttaggatg cctttgcctg caagtaacaa aatacctgac agaaagtggt taaaacaaca 60
ggaatttcat tcctaaaagt aggaagttcc agattttgtg ctcaatgatg tcaccaaggg 120
gtgcaatttc tttccatctt cctcatcatt ctcagcatcc tcagaaggct ccactttcat 180
cctagagctt gtagcctcat wgttacaaca tggctgccct ggttacaaac atcacaacca 240
catcaagaag aaggggggca aatagagttg caagagggcc attctctcat gtttctcttc 300
tttatcaggg agggaagtct ttcttagaag ctcctctaaa ataatttcac ttgtgtcttt 360
ttggcagaac tgggttgtat ggccattcat ggctgcctaa a 401

<210> 40
<211> 401
<212> DNA
<213> Homo sapiens

<400> 40

ctcttgtaac aggtgaactc tgccatattg gagcaaaagc aagaggaatg gcaattggac 60
aggcagccaa catggctgct acagtggtat atagtaccac catatagcat gtagttccta 120
tgtgcccaca caagagagag acatagtgat aggttagact gtctattgtg agattcacaa 180

aggggtttgg aacagaaatc yagaccaaga cctttacac aagagattct ttgtggaata 240
cttgctggaa tattcccaga gtattttggg ataccccaga ctcatacaag gttgtataac 300
ctctaactaa caagattcta agcaaaggat gagacgtgat catgctcagg taacgaaaac 360
ttagggtcaa ttcatgttct aattagttct cttgatctaa c 401

<210> 41
<211> 401
<212> DNA
<213> Homo sapiens

<400> 41

aagacctttt acacaagaga ttctttgtgg aatacttgct ggaatattcc cagagtattt 60
tgggataccc cagactcata caaggttgta taacctctaa ctaacaagat tctaagcaaa 120
ggatgagacg tgatcatgct caggtaacga aaacttaggg tcaattcatg ttctaattag 180
ttctcttgat ctaacagtta yggcttataa atcattccat gtcggaagcc ccaccggaaa 240
tagttgaggt tcaattcagt tacagctcaa tgaagtctga aaacaagtgt ccaacatttt 300
ctggttcatg gtttaaaata ggtttcaaat aaacaatgag gaagccagtt tcctgtttgg 360
gttgggtcca ttggatccta gcccatcaaa gctttgaatt a 401

<210> 42
<211> 401
<212> DNA
<213> Homo sapiens

<400> 42

tgaggttcaa ttcagttaca gctcaatgaa gtctgaaaac aagtgtccaa cattttctgg 60
ttcatggttt aaaataggtt tcaaataaac aatgaggaag ccagtttcct gtttgggttg 120
ggtccattgg atcctagccc atcaaagctt tgaattatat tacaatgaca ggcaaggact 180
agagggggaa gaactgaaac rcagagaaaa gttggcacag tgccaggaaa cctggctaaa 240
attaagtccc tcagtccaaa gaaaacaatg gcagctagga catgagtcaa tggtccaagt 300
gcaagtgcat ttgggggcaa caagagcaca cacaggcagg ccatggaact ggccaaccca 360
acaagaaggt ctttccacat tctgagatcc cccctaatgc t 401

<210> 43
<211> 401

<212> DNA
<213> Homo sapiens

<400> 43

```
ctccagaagt ttgagttcca acagctcagt gccatctaag ttgctatgga gactttgtaa    60
accactaaga atcctgggct ggggaactgt ctagcccagt gctgttgaat agggatacaa   120
tgtgagccac aaatgcaagt tatttgtata attttggatt ttcaaaagtg gccatcttaa   180
aaatgtattt aaaaaggtga rattaatttt aacaatatat tttattgtca tttcaaaatt   240
taatatagac aattattgag atattttttc cttttttgtt ataagtgttg ggaacccagt   300
gtgcaattta catgtagaac acatctcaat ttggaccagc tgcactgcaa gtgctgcacc   360
atggatagtg gctactgtat tcaatagtac agatctaccc t                       401
```

<210> 44
<211> 401
<212> DNA
<213> Homo sapiens

<400> 44

```
tacaatgttt cacaaccagg ggaatttatc agaattatgc ctaagggcct ttgaaaaact    60
tcagttattc ccatctcttc tcagacctcc tgaatctgaa tttctaaagt ttcgggggac   120
ctgccccgaa aatcacgtag gttcttttct attttcctaa gtgtcggctg gcttgagaaa   180
taaagggaca gagtacaaaa kagagaaatt ttaatttctg ggcgtccggg ggagacatca   240
cacgttggta ggatccatga tgccccacaa gccacaaaac caagcaagtt tttattaggg   300
attttcaaaa ggggagggag tgtgcgaata ggtgtgggtg acagacatca agtacctaac   360
agggtaatag aatatcacaa ggcaagtgga ggcagggcga g                       401
```

<210> 45
<211> 401
<212> DNA
<213> Homo sapiens

<400> 45

```
attaaaattg ctaatggagt ttcgggcacc attgtcattg ataacatctc atcaggagac    60
agggttttga gatcaaccgg tctgaccaaa atttattagg cgggaatttc gtcttcctaa   120
taagcctggg agcgctatgg gagactggag tctatctcac ctctgcaatc tcgaccataa   180
gagacaggta cgccccagga rggccagttc agagacctac ccctaggtgc gcattctctt   240
tctcagggac gttccatgct gagaaaaaga attcagcgat atttctccca tttgcttttg   300
aaagaagaga aatatggctc tgttctgcct ggctcactgg cagtcagagt ttaagtttat   360
ctctcatatt ccctgaacaa ttgctgttat cctgttcttt t                       401
```

<210> 46
<211> 401
<212> DNA
<213> Homo sapiens

<400> 46

```
agaaattata aaagtattaa tttggggaac taataaatgt ccataaaatc ttcacaatcc    60
acgttcttct gtcatggctt cagctggtcc ctctgtttgg ggtctctgac ttcccgcaac   120
actaaaggta ctacagatga ttcgaaaagt gaaaggaaaa taaatctcga gaccccaaaa   180
tcattaagcc aaagggaaaa rttaagctgg gaactgggtc acgcaaaact gcctcccctt   240
tggttcctaa attggatggc tacaagatga aaagctacac actgccctca tattttgccc   300
acagggaaat ccctagtgaa ctccaagatc tttaaagtgt ttctgttaaa attcaccatg   360
gtgcccaggc acagtggctc acgcctgtaa tcccaggact t                       401
```

<210> 47
<211> 401
<212> DNA
<213> Homo sapiens

<400> 47

```
ttaagctggg aactgggtca cgcaaaactg cctccccttt ggttcctaaa ttggatggct      60
acaagatgaa aagctacaca ctgccctcat attttgccca cagggaaatc cctagtgaac     120
tccaagatct ttaaagtgtt tctgttaaaa ttcaccatgg tgcccaggca cagtggctca     180
cgcctgtaat cccaggactt ygggaggcca aggtgggcgg atcatgaggt caggagattg     240
agaccatcct ggctaacacg gtgaaacccc ttctctacta aaactacaaa aaattagccg     300
ggtgtggtgg caggtgcctg tagtcccagc tactcgggag gctgagacag gagaatcact     360
tgaacctggg agggggaggt tgcagtgagc caagatcgca c                         401
```

<210> 48
<211> 401
<212> DNA
<213> Homo sapiens

<400> 48

```
tgggaggcca aggtgggcgg atcatgaggt caggagattg agaccatcct ggctaacacg      60
gtgaaacccc ttctctacta aaactacaaa aaattagccg ggtgtggtgg caggtgcctg     120
tagtcccagc tactcgggag gctgagacag gagaatcact tgaacctggg agggggaggt     180
tgcagtgagc caagatcgca ycactgcact ccagcctggg tgacagagcg agactctgtc     240
tcaacagaaa aaaaaaaaaa ttaactatgg caatgtaaat gataacttat ctttacaggt     300
gcaatcaccc ctcttcccac ctgatacaaa tgcatatctg attattccca cccacccacc     360
ccccaccgcc ccatttgtct gttaatctta tgtaaaagtg c                         401
```

<210> 49
<211> 401
<212> DNA
<213> Homo sapiens

<400> 49

```
catcctggct aacacggtga aaccccttct ctactaaaac tacaaaaaat tagccgggtg      60
tggtggcagg tgcctgtagt cccagctact cgggaggctg agacaggaga atcacttgaa     120
cctgggaggg ggaggttgca gtgagccaag atcgcaccac tgcactccag cctgggtgac     180

agagcgagac tctgtctcaa magaaaaaaa aaaaaattaa ctatggcaat gtaaatgata     240
acttatcttt acaggtgcaa tcacccctct tcccacctga tacaaatgca tatctgatta     300
ttcccaccca cccacccccc accgccccat ttgtctgtta atcttatgta aaagtgcaga     360
ttccttgcat ttcccctgtc ccatgtggcc atgttacata a                         401
```

<210> 50
<211> 401
<212> DNA
<213> Homo sapiens

<400> 50

```
atcgcaccac tgcactccag cctgggtgac agagcgagac tctgtctcaa cagaaaaaaa      60
aaaaaattaa ctatggcaat gtaaatgata acttatcttt acaggtgcaa tcacccctct     120
tcccacctga tacaaatgca tatctgatta ttcccaccca cccacccccc accgccccat     180
ttgtctgtta atcttatgta raagtgcaga ttccttgcat ttcccctgtc ccatgtggcc     240
atgttacata aaaatgcaga ttcactgagc tagacaaaga catgaatatt ttctccctac     300
ccacctcttg catgaaaatt gtgtacttct cactatcctg ccctttcctc tttaaatctg     360
gagccctcaa aatcatcttc agagaaaagc atagacctgt c                         401
```

<210> 51
<211> 401
<212> DNA
<213> Homo sapiens

<400> 51

```
ccccccttcct gcacacctcg aattgaccac cactgtctgc tcatcctagg aacctccaaa     60
ttcaggccct ttcaaccctc aggactccag attattcttc cctataccca catctaggac    120
tgacccactg ctgattcaca ccagtgcaga cattcaaatt attaaaatat tcagttcttt    180
tgcacacttt gtggtaagta rccactgccc tgagccctgt cacttgagtc tctctatgac    240
cttcaggaat ttctccacaa tccagcaact aaagggttaa cacaaagcac agctgtgagt    300
tccagcaatc gttctgattg gtcagtcccc ttgccttacc aggtgattaa ggttttaaat    360
ttcactgtgg caccacacgg cagataaatt ttgtatgcct a                        401
```

<210> 52
<211> 401
<212> DNA
<213> Homo sapiens

<400> 52

```
ctcacacctc atttgactaa cgagtcaaat ccttgctgac tggcttgtct cttcactgca     60
atatgttcag aggaacacga ctttaaatga tgcattgtcc tgggtttcct cttgtacacc    120
cctcttatgc ctcattcctt tcctttgcat gatactcccc tagttctgga tggccacctt    180
ccttggattt cccatgaaaa staatctttc tagagtcttt ttcctatagg actttctcta    240
tttttttatt ttattttatt tatttatttt ttttgagacg gagtctctct ctgtcaccca    300
ggctaaagtg caggggtgtg atcttggatc actgcagcct ctgcctccca ggttcaagca    360
attgtcctgc ctgagcctcc tgagtaactg ggattacagg t                        401
```

<210> 53
<211> 401
<212> DNA
<213> Homo sapiens

<400> 53

```
gagacaagcc agtcagcaag gatttgactc gttagtcaaa tgaggtgtga gcccaggcta     60
agaagcagct tccctcctcc atgccattta cgtggttact tcctcttctt gtaatcttca    120
aatcccagct tgcctgctct aatggccatt tgttgttttt gtcttccccg catgcttccc    180
accttcttct agtaacagcg mctccccttc cttaagggat ctcctccttc cccatccatg    240
gggtcctctg aggctgctgg cctcaatacc ataatctcaa acacagtgga gtcagtgact    300
cagctgtaat caatcatagt atctcaatcc cctggccatg gggattggtc caagggacag    360
ccacctaacc caaactgagg caataagatt cttccctgca g                        401
```

<210> 54
<211> 401
<212> DNA
<213> Homo sapiens

<400> 54

```
tgtgtactta gatttcaaat tcctgcaggg aagaatctta ttgcctcagt ttgggttagg      60
tggctgtccc ttggaccaat ccccatggcc aggggattga gatactatga ttgattacag     120
ctgagtcact gactccactg tgtttgagat tatggtattg aggccagcag cctcagagga     180
ccccatggat ggggaaggag sagatccctt aaggaagggg agtcgctgtt actagaagaa     240
ggtgggaagc atgcggggaa gacaaaaaca acaaatggcc attagagcag gcaagctggg     300
atttgaagat tacaagaaga ggaagtaacc acgtaaatgg catggaggag ggaagctgct     360
tcttagcctg ggctcacacc tcatttgact aacgagtcaa a                         401
```

<210> 55
<211> 401
<212> DNA
<213> Homo sapiens

<400> 55

```
cacctaaccc aaactgaggc aataagattc ttccctgcag gaatttgaaa tctaagtaca      60
cagacatcca ggatgaaggg aaatcgaggc tgggtctgag aatagtgctc taagagaaag     120
gccatgagat acccagagcc atcctggttt ctttccctcc caagcccagg ttactattac     180
cttgatgtaa taacaacatt ractatcccc cagccccata tctttccaat aaactgaatt     240
ggtacttaag ttagaatctg tttgtgtcat ttcaaacaca accaaaattg atacagaata     300
ttacaagtga catctctaga agatggaata ggcagagtca agaaggggcg gggatctgac     360
agttcttgct acatggttaa atgattagct gatacatatt g                         401
```

<210> 56
<211> 401
<212> DNA
<213> Homo sapiens

<400> 56

```
gcaagcacca ctatgccctg ctaatttttc atatttttag tagagacagg gtttcacctt      60
gttggccagg ctggtctcaa actcctgacc tcaggtgatc caccagcctc ggcctcccaa     120
agtgctggga ttacaggcat gaaccaccat gcctggcttt tttttttttt tttttttttt     180
aagacagagt ctcactctgt ygccaaggct ggaatgcatg gatgcgatct cggctcactg     240
tagcctccgc cttcccagtt caagtgattc tcctgcctca gcctccctag tagctgggat     300
tacaggtgtc caccacattt ttgtattttt agtagatacg ggttttcacc atgttggcca     360
ggctggtctt gaactcctga cctcaggtca tctgcctgct t                         401
```

<210> 57
<211> 401
<212> DNA
<213> Homo sapiens

<400> 57

```
gggtttcacc ttgttggcca ggctggtctc aaactcctga cctcaggtga tccaccagcc      60
tcggcctccc aaagtgctgg gattacaggc atgaaccacc atgcctggct tttttttttt     120
tttttttttt ttaagacaga gtctcactct gttgccaagg ctggaatgca tggatgcgat     180
ctcggctcac tgtagcctcc rccttcccag ttcaagtgat tctcctgcct cagcctccct     240
agtagctggg attacaggtg tccaccacat ttttgtattt ttagtagata cgggttttca     300
ccatgttggc caggctggtc ttgaactcct gacctcaggt catctgcctg cttcggcctc     360
ccaaagtgct gaaattacag gcatgagcca ccatgcccag c                         401
```

<210> 58
<211> 401

<212> DNA
<213> Homo sapiens

<400> 58

```
ttcaccttgt tggccaggct ggtctcaaac tcctgacctc aggtgatcca ccagcctcgg     60
cctcccaaag tgctgggatt acaggcatga accaccatgc ctggcttttt tttttttttt    120
ttttttttaa gacagagtct cactctgttg ccaaggctgg aatgcatgga tgcgatctcg    180
gctcactgta gcctccgcct ycccagttca agtgattctc ctgcctcagc ctccctagta    240
gctgggatta caggtgtcca ccacattttt gtatttttag tagatacggg ttttcaccat    300
gttggccagg ctggtcttga actcctgacc tcaggtcatc tgcctgcttc ggcctcccaa    360
agtgctgaaa ttacaggcat gagccaccat gcccagccca a                         401
```

<210> 59
<211> 401
<212> DNA
<213> Homo sapiens

<400> 59

```
ccaaagtgct gggattacag gcatgaacca ccatgcctgg cttttttttt tttttttttt     60
ttttaagaca gagtctcact ctgttgccaa ggctggaatg catggatgcg atctcggctc    120
actgtagcct ccgccttccc agttcaagtg attctcctgc ctcagcctcc ctagtagctg    180
ggattacagg tgtccaccac rtttttgtat ttttagtaga tacgggtttt caccatgttg    240
gccaggctgg tcttgaactc ctgacctcag gtcatctgcc tgcttcggcc tcccaaagtg    300
ctgaaattac aggcatgagc caccatgccc agcccaaccc actgttttcc tttgcctgtt    360
ggattctgcc aataggatga aatacagaga gaatggaggg a                         401
```

<210> 60
<211> 401
<212> DNA
<213> Homo sapiens

<400> 60

```
gatgcgatct cggctcactg tagcctccgc cttcccagtt caagtgattc tcctgcctca     60
gcctccctag tagctgggat tacaggtgtc caccacattt ttgtattttt agtagatacg    120
ggttttcacc atgttggcca ggctggtctt gaactcctga cctcaggtca tctgcctgct    180
tcggcctccc aaagtgctga rattacaggc atgagccacc atgcccagcc caacccactg    240
ttttcctttg cctgttggat tctgccaata ggatgaaata cagagagaat ggagggaaga    300
gggatttgcc cctttcagca gtagtttcca gctccttgtc actttgccac aacctgaccc    360
atcaggcccc cttaaagggt gcagcaccgc taagtcattc a                         401
```

<210> 61
<211> 401
<212> DNA
<213> Homo sapiens

<400> 61

```
aacccactgt tttcctttgc ctgttggatt ctgccaatag gatgaaatac agagagaatg     60
gagggaagag ggatttgccc ctttcagcag tagtttccag ctccttgtca ctttgccaca    120
acctgaccca tcaggccccc ttaaagggtg cagcaccgct aagtcattca ttccttcctc    180
agtgagttct gtccctggcc staattaaaa ttaatgtcct gactaggaag tgactacccg    240
ggaggattaa aagaaatggg caagtctgat cccaagcacc ttctattatc aatgttttct    300
ttgccaaaaa aaaaaaaaaa aatgctagca attcctggcc acaattaagt gtcactcact    360
tgggaagagc ctggggacag aggccgtact gaccttatac c                        401
```

<210> 62
<211> 401
<212> DNA
<213> Homo sapiens

<400> 62

```
agaaagcaag ctctaggcca tctggggagg gcattctgtt gtcctaggca cttggagcat     60
ggtccagaag agagtcccag gctccaggtg gacatatcct cttgggccca tgcgcttctt    120
gtccataggg aaggggcaga ggaatggcta gagggaggtc agtttgggac attttaaagc    180
attgaggggt cagtataggg yccttctagc ataggtataa ggtcagtacg gcctctgtcc    240
ccaggctctt cccaagtgag tgacacttaa ttgtggccag gaattgctag catttttttt    300
tttttttttg gcaaagaaaa cattgataat agaaggtgct tgggatcaga cttgcccatt    360
tcttttaatc ctcccgggta gtcacttcct agtcaggaca t                        401
```

<210> 63
<211> 401
<212> DNA
<213> Homo sapiens

<400> 63

```
acaacagaat gccctcccca gatggcctag agcttgcttt ctgggcccat gaggcccccct    60
tccctgagtt ctccttctaa aaggggattg ggtttccagg ttacacaccc cttagcctga    120
ggtgctacta agagatgctg atggaggctg ggggtgaatg gagcttggac atgcccactg    180
agttgtccat atgcttgcat rgggccccac taagcagaac agagtccagt ggaaagagaa    240
gatggggtgg gcaagggact tctttttgtg ttcttgcccc agcctcacaa agaggctcag    300
agagggcctc acaggaagtg agtaagttcc aggccctgga tctaaagcaa aggccaacaa    360
gatcattaag ccgagatcta aggaaatgag ctaagagaca g                        401
```

<210> 64
<211> 401
<212> DNA
<213> Homo sapiens

<400> 64

```
ggctcagaga gggcctcaca ggaagtgagt aagttccagg ccctggatct aaagcaaagg     60
ccaacaagat cattaagccg agatctaagg aaatgagcta agagacagaa ctgcaaggct    120
gggcacagat tcaaccaatg aaatgagtga gagttttatg aggctgtatt gccagagaaa    180
ccttaagctg aacatatagg ygcttatgat tgtaaacccc aagcaccaga acccatgcca    240
gaagggagcg ggcctcaagg cagggccacc ccagcagcaa aatcttccca ggtgctcctc    300
aggagtgtcc aaggtgagcc ctggatggga gaccccagca acagggaagc catactcact    360
gaccacagac tcacccacaa ccaggccgtg agtcaagaaa c                        401
```

<210> 65
<211> 401
<212> DNA
<213> Homo sapiens

<400> 65

```
attcaaccta ttacacatat ataatatgtc ctaccattgc ccacttgaga gggcttggtt      60
gttttacatg ccaaaagcag tgagcatacc taccactaag agcgtggctt ctaaatgcca     120
ttcttaaaac ctgcacattc tccacatgaa tcccagaact taaagtaaca acaacaacaa     180
aaaagaacca aatagtgatc kaagaatgat cggggaaaac tgaaaaagac acagaagtca     240
gtttgaaaga acttccactg gctaaatttg ggacagtttg agcatgaaaa taaataataa     300
cagtaatatg tcctctcaaa taatatagaa aaccatgaat caatacttat atacctaaat     360
acatacatac aaacaaacat acatacatgt gctaattgaa a                         401
```

<210> 66
<211> 401
<212> DNA
<213> Homo sapiens

<400> 66

```
tcatttaagt gttctattac tgattcaagt taattccaat gctttactat tttaatagta      60
atgcaatcca tttttttattt tataatattt tagactcagg gggtacatgt gcatgtttgt     120
tacatgagta tattttgtat ttgtggggat tggcttcta ctgtaccctt tacccaaata     180
gtgaacattg tactcaatgg ktaattttc aaccctcgcc ctcctcccaa cctccccct     240
tcaggagtcc ccactgtcta ttatttctat ctttgtgttc atgtttaccc attgtttatc     300
tcccacttat gagtgaaaac acgtggtatt tgatttcctg agttagttca ctaaggatac     360
tgccctccaa ttccatccat attgctgcaa aggacatgat t                         401
```

<210> 67
<211> 401
<212> DNA
<213> Homo sapiens

<400> 67

```
ctattactga ttcaagttaa ttccaatgct ttactatttt aatagtaatg caatccattt      60
tttattttat aatattttag actcaggggg tacatgtgca tgtttgttac atgagtatat     120
tttgtatttg tggggattgg gcttctactg taccctttac ccaaatagtg aacattgtac     180
```

```
tcaatgggta atttttcaac mctcgccctc ctcccaacct cccccttca ggagtcccca     240
ctgtctatta tttctatctt tgtgttcatg tttacccatt gtttatctcc cacttatgag     300
tgaaaacacg tggtatttga tttcctgagt tagttcacta aggatactgc cctccaattc     360
catccatatt gctgcaaagg acatgatttc atttgttatg g                         401
```

<210> 68
<211> 401
<212> DNA
<213> Homo sapiens

<400> 68

```
aatgctttac tattttaata gtaatgcaat ccatttttta ttttataata ttttagactc      60
aggggggtaca tgtgcatgtt tgttacatga gtatattttg tatttgtggg gattgggctt     120
ctactgtacc ctttacccaa atagtgaaca ttgtactcaa tgggtaattt ttcaaccctc     180
gccctcctcc caacctcccc ycttcaggag tccccactgt ctattatttc tatctttgtg     240
ttcatgttta cccattgttt atctcccact tatgagtgaa aacacgtggt atttgatttc     300
ctgagttagt tcactaagga tactgccctc caattccatc catattgctg caaaggacat     360
gatttcattt gttatggctg ccgaataaat tctattgagc t                         401
```

<210> 69
<211> 401
<212> DNA
<213> Homo sapiens

<400> 69

```
tcctatttgg aaaatctgga ggcaggctgt ctgtggctgg tgccccacca agtagttgag      60
gacccaggct tttccctct ttcccaagag gacctcccag tctaagatgg ttctggatca      120
gtggctcttc atcagggata atttcccccc taaaggacag ttgacaatgt ctggagacat      180
ttttattgtc ataatgggga ygaggtgcaa tggcatcaaa aaagtagagg ccaggggtgc      240
tgctaaatgt cctacaaccc ctggcaacaa ggaattatct aagcccaaaa tgggaacggt      300
gtggaggttg agaaatcccg ttctcactcc atctatcaca cctgtgatcc aagaagcagg      360
tcagcataca gaagaaaggg gcaaaaggca tatgccagct g                         401
```

<210> 70
<211> 401
<212> DNA
<213> Homo sapiens

<400> 70

```
cagtctaaga tggttctgga tcagtggctc ttcatcaggg ataatttccc ccctaaagga      60
cagttgacaa tgtctggaga catttttatt gtcataatgg ggacgaggtg caatggcatc      120
aaaaaagtag aggccagggg tgctgctaaa tgtcctacaa cccctggcaa caaggaatta      180
tctaagccca aaatgggaac rgtgtggagg ttgagaaatc ccgttctcac tccatctatc      240
acacctgtga tccaagaagc aggtcagcat acagaagaaa ggggcaaaag gcatatgcca      300
gctgacttaa gaaaagtttc tagaagctgc cacacaattc cacttatatt ccattcacca      360
aaacttaatc acatggccac acataattgg aaaatatagt c                         401
```

<210> 71
<211> 401
<212> DNA
<213> Homo sapiens

<400> 71

```
tcttcaattt ttccttcatc tccaagcttt catctgggat cttctcttta cccaaagaat      60
atctgtcaga atttccttta gtgcaggtct cttggtggca gcttctttca gtttttattt      120
cactgacttt attttaccat gattactgaa aaatattttt actaggtgta gaattctaga      180
ttggccaaat tgaaaatatc rttctactat tttcttattt ctgttatcat ttttgagaag      240
tctgctgtca ttctttttgt tcttggagat gaggtctcca cagtgttgcc caggttggag      300
tgccatggct agtcacaggt gcaatcatag ctcactgcag cctcgaactc ctggcctcaa      360
gcaatcctcc cacccgcctc agcctcttga gtagcttgga c                         401
```

<210> 72
<211> 401
<212> DNA
<213> Homo sapiens

<400> 72

```
atatttttaa ttagagtgtt tctagaggtt ctctatcaaa tgtggacaat tactttttat     60
agcttccaat ttccaatcct tgccaacaaa tttcaagttt ggcctttact ttctttgttt    120
gtttttgttt ttgtttttga gacggagtct tgctctgttg cccaggctag agtgcaatgg    180
cacaatctct cctcaccgca rgcttcacct cccgggttca agcgattcca ttcccctgcc    240
tcagcttcct gagtaactgg gattacaggt gcctgccacc acacctggct aatttttgta    300
tttttagtag aggtggggtt tcaccatact gaccaggttg gtctcgaact cctgacctca    360
ggtgatccgc ccacctcggc ctcccaaagt gctgggatta c                        401
```

<210> 73
<211> 401
<212> DNA
<213> Homo sapiens

<400> 73

```
gtgcagccct tcggggcccc aactctgagt gccaagtagt ttaccagggt ctccaccatg     60
gaaaagaaag gaagcagtag attcgaactt atgggcacct agccacccac agttttcaaa    120
aacatggctc atctctctgt cttctcttta gatgagccag tgccccctgg gcaaaagtag    180
ccctaaatgc caagcttgcc yctctagagc ctcattgtct cctagatctt ggcttgcttc    240
ctcactatct tattagcttt tgatatttta gggaattttt tttaaatgtt ttctctgaat    300
ttgttagttg ttttcagagg gaaggttggc ccatattagc tagtctgcct ctattagaag    360
cagaagtctg atatggactt tggatagaaa tgaggatcag a                        401
```

<210> 74
<211> 401
<212> DNA
<213> Homo sapiens

<400> 74

```
attgtctcct agatcttggc ttgcttcctc actatcttat tagcttttga tattttaggg     60
aatttttttt aaatgttttc tctgaatttg ttagttgttt tcagagggaa ggttggccca    120
tattagctag tctgcctcta ttagaagcag aagtctgata tggactttgg atagaaatga    180
ggatcagaag tttttaagaa yagagggaca tggccaggca cggtggctca tgcctgtaat    240
cccaacactt tgggaggcca aggcgggtgg atcacgaggt caggagattg aaaccatcct    300
tgctaacatg gtgaaacccc gtctctacta aaaatacaaa aaataaaaat aaaaaattta    360
gtcgggcatg gtggcaggca tctgtagtcc cagctactcg g                        401
```

<210> 75
<211> 401
<212> DNA
<213> Homo sapiens

<400> 75

```
agggcatcca tagccaggac tcttcgtctg tggtctgctg tactccgatt ccttatgatc     60
cccactccta ctcctcactc agccctgttc ctttacctct tatccaaaca cttccataag    120
atttcctatc agtcactgac ccctcagtct agtatagctc caagggacta gattctccta    180
tctctggttc tagagaaaaa stttatcctt cctaaaatga gttttaggct ctgaggcaca    240
ggagacggtg agactttgta tagtgcttat gacagtgctt acttgaggga attcttccct    300
ggattcctga gattcctgag tattactgcc ttcttcacag aagcccaacc ctgattcttt    360
cagatggact tatccctatt cctcacatcc aagcagtatc a                        401
```

<210> 76
<211> 401
<212> DNA
<213> Homo sapiens

<400> 76

```
aaaataaatt tagatgcatc aagctggcag atggagatgg gaaaggggcc tccccaagaa     60
gtcaagttct gctaaagctg cagtgcctaa taaggtatta tcttgcctct ctgatcattt    120
aagttagaaa agggaggcaa taacaagagg aagttgtatg gtttctcagt gtttatatct    180

gagtgaaaag aaagaaccaa mcagagtcat gaaagtgaca tccttgggag gcagtgattt    240
ctcccctgct gttgaaagtg gattttattt atttatttat ttttgagatg gagtcccact    300
ctgtcaccca ggctggagta cagtggcgcg atctcggctc attgcaacct ccacctcctg    360
ggttcaagca attctcttgc ctcagcctcc caagtagctg g                        401
```

<210> 77
<211> 401
<212> DNA
<213> Homo sapiens

<400> 77

```
tttgggaggc cgaggcgggc agatcacgag gtcaggagat cgagaccatc ctggctaaca     60
cggtgaaatc ccgtctctac taaaaataca aaaaattagc cgggcgtggt ggcgggtgcc    120
tgtggtcaca gctactcggg aggctgaggc aggagaatgg cgtgaatcca ggaggcggag    180
cttgcagtca gccgagatcg ygccactgca ctccagcctg ggagacagag caagactccg    240
tctcaaaaaa aaaaaaatgc aaatccagta ggttaagaac acactagaag tcaagagcat    300
gacacacacc tgtagtccca gatactgaag aggctaaggt gggaggatgg cttgagctca    360
ggagatagaa gctggagtga attatgatca cacctgtgaa t                        401
```

<210> 78
<211> 401
<212> DNA
<213> Homo sapiens

<400> 78

```
catcacaaat ggaagggatc tgagtcccca cctcattgct cgggattgac tgtcccacac     60
atggtaagac tgattgaaaa ctaagcctct cttgtgctgg ctactgaggc tttggagttg    120
cttgttacct taagtagtac tcattcctgt attacagaaa tgcaataaag atctcacaaa    180
gagaatctag tagacaatac rttattggct gagtgtttgt gtaccacctc caaattcata    240
tgttaaagca ccaaccccaa gtgtaatggc agcaggagac ggggcctttg agaggtgatt    300
agggtcatat gaggccacaa gggtgaggcc ttcatgatta gattaatgtc tttataagaa    360
gaagaagaga taccagagca ttcatgagct ctctcactcc c                        401
```

<210> 79
<211> 401
<212> DNA
<213> Homo sapiens

<400> 79

```
caataaagat ctcacaaaga gaatctagta gacaatacgt tattggctga gtgtttgtgt     60
accacctcca aattcatatg ttaaagcacc aaccccaagt gtaatggcag caggagacgg    120
ggcctttgag aggtgattag ggtcatatga ggccacaagg gtgaggcctt catgattaga    180
ttaatgtctt tataagaaga rgaagagata ccagagcatt catgagctct ctcactccct    240
cactcacttt ctccctcttt ctctcactct tcctttgtct ccgctatgtg aggacacagc    300
aagaaggtgg ccatctgcaa accaagaaga gagccctcac cagaaactga aattgccagc    360
accttgatct tggacatccc agcctccaga atggtgagaa a                        401
```

<210> 80
<211> 401
<212> DNA
<213> Homo sapiens

<400> 80

```
ccgagtctgt agtgttttgt aatgacaaac caagcagact aagacatgat gtatatgaaa    60
tagcagagaa ggtcttcata actaagactg agaactagaa gctatttttt aaaaaaaaag   120
ataggcatat tttgactgtt aaaaattaca aggaaaacaa gaaaattttg tacagtaaaa   180
gattccataa acaaactata kttgtccctt agtctacgca ggggattggt ttcaggaccc   240
cctcttatac caaaagtcag tcctgcagaa ctggcatata tgaaaagttg gccctttgta   300
tatggaattt ttgcattcca taagtactgt attttcaatc cacttttggt tgaaaaaaat   360
ctacatataa gtggagccat gcagttaaaa cccatattat t                       401
```

<210> 81
<211> 401
<212> DNA
<213> Homo sapiens

<400> 81

```
gcgccactgc actctagctt gggtgacaga gcaagactcc atctcaaaaa aacaaacgaa    60
caaaacaaaa caaaacaaaa actgtatgcc ctttggccta acaattccac ttctgggaat   120
ccatcttata aaaataaaat tgccagtaca tgaggatata tgcgtgaggg tgtttcttac   180
aggattgttc atagggggggg raaatgaaaa caaatgaaat taccattaat agagacatag   240
ctaaataaat gtgggtatac ccacaccatg aaatattatg ccattattta aaagaatgaa   300
taagataaca aatggcttaa agaagtcttc agcaaacatc gttgagtaag aaaagcaaac   360
gacagtcaaa tgttcataac tcaatccatt ttaataagtt a                       401
```

<210> 82
<211> 401
<212> DNA
<213> Homo sapiens

<400> 82

```
ggccactcgt cagctggtaa tgtcagccat gtggtggtgg caggggcaca aaagatgcct    60
gagggactcc agctctgagg gccacaatgc tgtccctcct ggaggcgtgg tcttcctatg   120
gtgactgccc acttggttcc atccttccct taccccctca aaatccgtgt cacataaagc   180
ccaccccagc agacctaatt yttgttaaag tgccattctg taagggcacc ccctctggcc   240
attattccac cacgactacc acgggactca ctttcctgtc tgtgacaccc tggagagggt   300
ctttaattcc aggcattagt aaaacatcca cagagagcaa gtgacccgtg ctccgccatg   360
aaaccgacaa gcagctcagc tttctctcta gttaacagaa g                       401
```

<210> 83
<211> 401
<212> DNA
<213> Homo sapiens

<400> 83

```
agggcggggga acatcacaca ccagggcttg tggggggtgg agggtggggg actgggggag     60
ggatagcatt aggagaaata cctaatgcaa atgataagtt gacgggtgca gcaaaccaac     120
atggcacatg tatacctatg taacaaacct gcaggttgtg cacatgtacc ctagaagtta     180
aagtataata aaaatttttt waaaaaaaag aaattatggt ttaggagtca tgacgatgga     240
ggctgcaaga ttctgaccct ccctaaactg ctcccaagat cagtgcctga catatcttgc     300
agacactgca gttgatggat cagctggcac cacctggatc aataaactgg ttcatcttat     360
cttgtggccc ccacccagga actgactcag tacaagagga c                         401
```

<210> 84
<211> 401
<212> DNA
<213> Homo sapiens

<400> 84

```
gtttttttat tattcaaatc agtctcccca aacatttgga aatcagagtt tttaaggata     60
atttggtggg tggaggaagt cctgtgagtc gagagtgctg actggttggg ttggagatga     120
aatcatggga agttgaaggt gtcctcttgt actgagtcag ttcctgggtg ggggccacaa     180
gataagatga accagtttat ygatccaggt ggtgccagct gatccatcaa ctgcagtgtc     240
tgcaagatat gtcaggcact gatcttggga gcagtttagg gagggtcaga atcttgcagc     300
ctccatcgtc atgactccta aaccataatt tctttttttt aaaaaaattt ttattatact     360
ttaacttcta gggtacatgt gcacaacctg caggtttgtt a                         401
```

<210> 85
<211> 401
<212> DNA
<213> Homo sapiens

<400> 85

```
aaaactttcc aaaatttctg tcccaatgac ctggtctagg ctgcagggcc ctcaggactg     60
ggtttccctg aattgagata gtgcagctac tctactgctc tgaaaaacaa gacattttca     120
acttaatatc cttggatatc taacctccca caaacataaa tttaatagct gcaaaaatat     180

gattgtatat atactcatct stgtgcaatt tccccccctt tattgttttt tgatttgctc     240
tttattcctc ctttttttccc ttcctcttgt aacagctggt ctgtgtcttg cctgctgcct    300
agacagagct gacttatcaa gacaggggaa ttgcaataga gaaagagtaa ttcacacaga     360
aagcgggctg tggaggagat tggagttttt ttattattca a                         401
```

<210> 86
<211> 401
<212> DNA
<213> Homo sapiens

<400> 86

```
gacaggctca ggagactggg cctgatggga ggtcagggag atgtgtgact ttccttgtga     60
tcacagcaac agaagggttt ttccagcttg caaatgacct ctcaaaataa cctgaatttc     120
cacccagggc tggtgggaat gtaagctgat acaaacttcg gggaaataat gaggtggaag     180
atagtaactg gcttaagaac rtaaagggca gaggagtgga ggttgagact cagcgtccta     240
ggattaagag aaacctgtcc tggtcatccc actcctgaaa gtctgtccta aaggacttct     300
acaccaaaat ggtcatttca gatttactta tagtaactta aaagtaaggt agagttggaa     360
acaactcaaa tatgcaaaaa gtaagcaaat ttgttaagca a                         401
```

<210> 87
<211> 401

<212> DNA
<213> Homo sapiens

<400> 87

```
cagtctatca ttgttggata tttgggttgg ctccaagtct ttgctattgt gaataatgcc      60
gcaataaaca tacatgtgca tgtgtcttta tagcagcatg atttataatc atttgggtat     120
atacccagta ataggatggc tgggtcatat ggaatttcta gttctagatc cctgaggaat     180
cgccacactg acttccacaa kggttgaact agtttacagt cccaccaaca gtgtaaaagt     240
gttcctattt ctccacatct tcttcagcac ctgttgtttc ctgacttttt aatgatcgcc     300
attctaactg gtgtgagatg gtatctcatt gtggttttga tttgcatttc tttgatggcc     360
agtcatgatg agcatttttt catgcagttt ttggctgcat a                         401
```

<210> 88
<211> 401
<212> DNA
<213> Homo sapiens

<400> 88

```
tttgcatttc tttgatggcc agtcatgatg agcatttttt catgcagttt ttggctgcat      60
aaatgtcttc ttttgagaag tgtctgttca tatcctttgc ccactttttg atggggttgt     120
ttgttttttt cttgtaaatt tgtttgagtt cattgtagat tctgtatatt agccctttgt     180
cagatgagta gattgcaaaa wttttctccc attctgtggg ttgcctgttc actctgatgg     240
tagtttcttt tgctgtgcag aagctcttta gtttaattag atcccacttg tcaattttgg     300
cttttgttgc cattgctttt ggtgttttag gcatgaagtc cttgcccatg cctgtgtcct     360
gaatggtatt gcctaggttt tcttctaggg tttttatggt t                         401
```

<210> 89
<211> 401
<212> DNA
<213> Homo sapiens

<400> 89

```
taaggaaggg atccagtttc agctttctac atatggctag ccagttttcc catcaccatt      60
tattaaatag ggaatccttt ccccattgct tgtttttgtc aggtttgtca aagatcagat     120
agttgtagat atgcggcatt atttctgagg gctctgttct gttccattgg tctatatctc     180
tgttttggta ccagtaccat rctgttttgc ttactgtagc cttgtagtat agtttgaagt     240
caggtagcat gatgcctcca gctttgttct tttggcttag gattgacttg gcaatctggg     300
ctctttttttg gttccataag aactttaaag tagtttttc caattctgtg aagaaagtca     360
ttggcagctt gatggggatg gcattgaatc tgtaaattac c                         401
```

<210> 90
<211> 401
<212> DNA
<213> Homo sapiens

<400> 90

```
gagagagggc atccctgtct tgtgccagtt ttcaaaggga atgcttccag tttttgtcca      60
ttgagtatga tattggctgt gggtttgtca tagatagctc ttattttgag atacgtccca     120
tcaataccca atttattgag agatttagc atgaaggttg ttgaattttg tcaaaggcct     180
tttctgcatc tattgagata wtcatgtggt ttttgtcttt ggttctgttt atatgctgga     240
ttatgtttat tgatttgcgt atgctgaacc agccttgcat cccagggatg aagtccactt     300
gattatggtg gataagcttt ttgatgagct gctggattcg gtttgccagt attttattga     360
ggatttttgc atcaatgttc atcggggtta ttggtctaaa a                         401
```

<210> 91
<211> 401
<212> DNA
<213> Homo sapiens

<400> 91

```
agggcatccc tgtcttgtgc cagttttcaa agggaatgct tccagttttt gtccattgag     60
tatgatattg gctgtgggtt tgtcatagat agctcttatt ttgagatacg tcccatcaat    120
acccaattta ttgagagatt ttagcatgaa ggttgttgaa ttttgtcaaa ggccttttct    180
gcatctattg agatattcat rtggttttg tctttggttc tgtttatatg ctggattatg    240
tttattgatt tgcgtatgct gaaccagcct tgcatcccag ggatgaagtc cacttgatta    300
tggtggataa gctttttgat gagctgctgg attcggtttg ccagtatttt attgaggatt    360
tttgcatcaa tgttcatcgg ggttattggt ctaaaattct c                        401
```

<210> 92
<211> 401
<212> DNA
<213> Homo sapiens

<400> 92

```
ggtatcagga tgatgctggc ctcataaaat gagttaggga ggattccctc tttttctatt     60
gattggaata gtttcagaag gaatggtacc agctcctcct tgtgcctctg gtagaattcg    120
gctgtgaatt catctggtct tggacttttt ttggttggta agctattaat tattgcctca    180
atttcagagc ctgttattgg yctattcaga gattcaactt cttcctggtt tagtcttggg    240
agggagtatg tgtcgaggaa tttatccatt tcttctagat tttctagttt atttgcatag    300
aggtgtttat attattctct gatggtagtt tgtatttctg tgggattggt ggtgatatcc    360
ccttcgtcat tgtttattgc gtctatttga ttcttctctc t                        401
```

<210> 93
<211> 401
<212> DNA
<213> Homo sapiens

<400> 93

```
cactgtggtc tgagagacag tttgttataa tttctgttct tttacattag ctgaggagtg     60
ctttacttcc aactatgtgg tcaattttgg aataggtgtg gtgtggtgct gaaaagaatg    120
tatattctgt tgatttgggg tggagagttc tgtagatgtc tattaggtcc acttggtgca    180
gagctgagtt caattcctgg rtatccttgt taactttctg tctcattgat ccatctaatg    240
ttgacagtgg ggtgttaaag tctcccatta ttattgtgtg ggagtctaag tctctttgta    300
ggtcactcag gacttgcttt atgaatctgg gtgctcatgt attgggtgca tatatattta    360
ggatagttag ctcttcttgt tgaattgatc cctttaccat t                        401
```

<210> 94
<211> 401
<212> DNA
<213> Homo sapiens

<400> 94

```
cctggatatc cttgttaact ttctgtctca ttgatccatc taatgttgac agtggggtgt     60
taaagtctcc cattattatt gtgtgggagt ctaagtctct ttgtaggtca ctcaggactt    120
gctttatgaa tctgggtgct catgtattgg gtgcatatat atttaggata gttagctctt    180
```

```
cttgttgaat tgatcccttt mccattatgt aatggccttc tttgtctctt ttgatcttag      240
ttgatttaaa gtctgttttt tcagagacta gggttgcaac acctgccttt ttttgttttc      300
cacttgcttg gtagatcttc cttcatccct ttattttgag cctatgtgtg tctctgcatg      360
tgagatgggt ttcctgaata cagcacactg atgggtcttg a                          401
```

<210> 95
<211> 401
<212> DNA
<213> Homo sapiens

<400> 95

```
ttcccatctt tgtggtttta tctacctttg gtctttgatg atggtgacgt acagatgggt      60
ttttggtgtg gatgtccttt ctgtttgtta attttccttc taacagtcag gaccctcagc      120
tgcaggtctg ttggagtttg ctggacgtcc actccagacc ctgtttgcct gggtatcagc      180
agcggtggct gcagaacagc rgatattgga gaaccgcaaa tgctgctgcc tgatccttcc      240
tctggaagtt ttgtctcaga ggtgcaccca gtcatgtgaa gtgtcagtcc gcccctactg      300
gggggtgcct cccagttagg ctactcaggg gtcagggacc cacttgagga ggcagtctgc      360
ctgttctcag atctcaagct gcgtgctggg agaacgacta c                          401
```

<210> 96
<211> 401
<212> DNA
<213> Homo sapiens

<400> 96

```
ggatgtcctt tctgtttgtt aattttcctt ctaacagtca ggaccctcag ctgcaggtct      60
gttggagttt gctggacgtc cactccagac cctgtttgcc tgggtatcag cagcggtggc      120
tgcagaacag cagatattgg agaaccgcaa atgctgctgc ctgatccttc ctctggaagt      180
tttgtctcag aggtgcaccc rgtcatgtga agtgtcagtc cgcccctact ggggggtgcc      240
tcccagttag gctactcagg ggtcagggac ccacttgagg aggcagtctg cctgttctca      300
gatctcaagc tgcgtgctgg gagaacgact actctcttca aagctgtcag acagggacat      360
ttaagtctgc agaggttact gctgactttt gtttgtatgt g                          401
```

<210> 97
<211> 401
<212> DNA
<213> Homo sapiens

<400> 97

```
tgggcatagg accctccaag ccatgtgcgg gatatcatct cctggtgtgc catttgttaa      60
gcccgttgga aaagcacagt attagggtgg gagtgacccg attttccagg tgccatctgt      120
cacccctttc tttgactagg aaagggaatt ccctgacccc ttgcgcttcc caggtgaggt      180
gatgcctcgc cctgcttcgg ytcatgcatg gtgtgctgca cccactgtcc acccactgtc      240
cagcactccc cagtgagatg aacccggtac ctcagttgga aatgcagaaa tcacccgtct      300
tctgcatcgc ttacactggg agctgtagac tggagctgct gctattcggc catcttggct      360
ccacctccac agagttttaa tgtcatggga aaattctatt g                          401
```

<210> 98
<211> 401
<212> DNA
<213> Homo sapiens

<400> 98

gatattggag cttcttcctc ctgtgctgtc ttctctgcct ggggaaattt gtgtccctca 60
gctaggccac ccaggggaat cacatgatga tcctgaacat tccatcccat caggctgtgc 120
acaggactgg ctgcatctga gatcaaacga tgttcaaaga aagttcagac tccagcactc 180
cagttagggc aagcctctta raaggtgagc gtcttgatac ctgtttggaa ggagggaaca 240
ctggttgcag gaaattcagg tgagcatttt tgaaagaggt ggtgtataga ctccaaagca 300
tcttcctcct actttcttaa atttttttgt tttgtgtgtt tgggtttttg tgttttgaat 360
gggggttgt ttttcggttt ttttgagatg gagtctcgct t 401

<210> 99
<211> 401
<212> DNA
<213> Homo sapiens

<400> 99

tgctaggatt acaggcgtga gccaccacat ccggccctgt gtgtttgttt tttgagacag 60
ggtctcacta tgttgtccag gctggtctcg aactcctgag ctcaagcaat cctcctccct 120
tggccttccc agtaattggc ttttaaacat ttgatggagg gtgctggtga gcaaaacttg 180
acagttatga aacaggaaac sagcaggaaa aggacctgcc caaacttgga ggagaaggta 240
gtgccaggga agaagggatg gcactgagct tagtgtgcaa cccagaattt gtgacaattg 300
cacaacacac cctcttgtca ttcctgtcag ttttattcat tcccctccct cccttccatt 360
cctttcccag tctttaatcc ccctgttcct tcccttctcc t 401

<210> 100
<211> 401
<212> DNA
<213> Homo sapiens

<400> 100

tttttgaga caggtctca ctatgttgtc caggctggtc tcgaactcct gagctcaagc 60
aatcctcctc ccttggcctt cccagtaatt ggcttttaaa catttgatgg agggtgctgg 120
tgagcaaaac ttgacagtta tgaaacagga aaccagcagg aaaaggacct gcccaaactt 180
ggaggagaag gtagtgccag rgaagaaggg atggcactga gcttagtgtg caacccagaa 240
tttgtgacaa ttgcacaaca caccctcttg tcattcctgt cagttttatt cattcccctc 300
cctcccttcc attcctttcc cagtctttaa tccccctgtt ccttcccttc tcctactccc 360
ttaatcactt acagtcccac tcccaggagg aaggggtaca g 401

<210> 101
<211> 401
<212> DNA
<213> Homo sapiens

<400> 101

ctttccccac actgtcctac tgcctctcct caccttctgc cccaccctca ctctgcatga 60
ctgaatgact taattcaaat tgcatttatt tgctcatttc taatcacatc ctaggatctc 120
atgctctctt gatggtaaat aaaagtgaac agaaaatgtc ttctaatgac ctggtaaatc 180
aagtttatac ctttcccaaa kcagggtcaa tttgctctaa atctagtcaa gtcagaaccc 240
agcattagta ggtggcctga ccacatggcc ccttccccca gtctcttaag gcatagtgag 300
tcactcacag aggccagctt agcccttcat gagtcagtat ctcctgttta tgtacagagt 360
cattactttc tctataggaa aaaaaggagg tggtggtgga g 401

<210> 102
<211> 401
<212> DNA
<213> Homo sapiens

<400> 102

```
agtgagtcac tcacagaggc cagcttagcc cttcatgagt cagtatctcc tgtttatgta      60
cagagtcatt actttctcta taggaaaaaa aggaggtggt ggtggaggtg cttgtctata     120
gatggactgg gcattgtaat ttgaaaagca ttgagaaaag tccctatctc caaagacatt     180
atcagcctta cataatatct raaaacctat catttgagac atttctcaga gcaatgatcc     240
tttataaaat atgggatgag gccagaagag ccattttact atgtgacagt cagtacaact     300
cagtggctag gaatcaggat ctggatcgag ttccacttct ggtatggctg agtaaacgct     360
tactgtactg agcttcctgc caataacaac tataaactca g                         401
```

<210> 103
<211> 401
<212> DNA
<213> Homo sapiens

<400> 103

```
ctggcctgaa gaactggcag acagagtttg agggcaaccc cagctgctag aaagtgaggg      60
agaatcctca aagggagaga gccagagaaa gaaaatccca aattccatgt ataaagtctg     120
ccccaatttt tgcctgaccc ccaaacaccc atgtacagca gagattgtaa gcaccctatc     180

taagggtgaa agaacaaaaa rcagatatga ggggccactc agaaaaaaac agaatttgca     240
gtttgaacca taacaagtta atttctcatt aaaatgaaaa attcaaaact ctttgaaaga     300
atacaactga atccagtctc cacaatatga cattcacaat atccagagta caatccaaaa     360
tttcttgaca tattgagaga caggaagatg tgatgtattc t                         401
```

<210> 104
<211> 401
<212> DNA
<213> Homo sapiens

<400> 104

```
tataatgatt aaaacatagt tagtatcatc acctaaatag agacgcctaa aattataatc      60
aaatggaaat tctataacta aaaaatacag tatctaattt taaaaaatta actggatggg     120
gcttaacata attttttaaa acagatgaaa gagtaggtga atctaaagag agatcaacag     180
aaaatatcca atctgaaaga yagagataaa aatgatagta cctaaagggt ggtattgaaa     240
ggtctaacat acctacaatt gaactcctag aagaacagag gacatggggt agaaaaaaat     300
tatttgaata aataatggcc aaaaagtccc caaatttggc aaaagacata aatttacaaa     360
tctaagaagc tcagcaaatc ccaatcaagt taaataaaag a                         401
```

<210> 105
<211> 401
<212> DNA
<213> Homo sapiens

<400> 105

```
acattagtac actactatta attaagtgcc agactttatt cagatttctt tgtttcttca      60
caaataaccc ctttttcctg tttcaggagc caatctaggg taccatattg catttagtgc     120
cttttagaaa ttattatttg gacatgtttt agaattccag tttaatttac atattaatgt     180
tttttctata tattattgta ktttttaccc aatgtccgct ctaagaatca cgatatacat     240
tcttaacttt ttataatctg atttgagttt acattgtccc actttgcata aaatatgcaa     300
accttgcaat cgtataggtc ctcttatacc tgcccccagc attcctttgt gttatgatta     360
ccatatgaca tttatataca ttatgcatcc tacaatgcaa g                         401
```

<210> 106
<211> 401
<212> DNA
<213> Homo sapiens

<400> 106

```
taaactcaaa tcagattata aaaagttaag aatgtatatc gtgattctta gagcggacat     60
tgggtaaaaa ctacaataat atatagaaaa aacattaata tgtaaattaa actggaattc    120
taaaacatgt ccaaataata atttctaaaa ggcactaaat gcaatatggt accctagatt    180
ggctcctgaa acaggaaaaa rgggttattt gtgaagaaac aaagaaatct gaataaagtc    240
tggcacttaa ttaatagtag tgtactaatg ttagtttctt agttgtgatg aatgtactgt    300
gataatgtaa gatgttaact tcagcagaaa ctgggtgaga ggtatacagg aactctctgt    360
actatttttg caacttttat gtaaatctaa aatttaaaca a                        401
```

<210> 107
<211> 401
<212> DNA
<213> Homo sapiens

<400> 107

```
acaccatttt atttgttttc ttttttgccc ctctgatttt tgttcttctg ttacttcttt     60
cttgtctttt tttttttttt tacagaagct ctttattttg tttaaatttt agatttacat    120
aaaagttgca aaaatagtac agagagttcc tgtatacctc tcacccagtt tctgctgaag    180
ttaacatctt acattatcac rgtacattca tcacaactaa gaaactaaca ttagtacact    240
actattaatt aagtgccaga ctttattcag atttctttgt ttcttcacaa ataacccctt    300
tttcctgttt caggagccaa tctagggtac catattgcat ttagtgcctt ttagaaatta    360
ttatttggac atgttttaga attccagttt aatttacata t                        401
```

<210> 108
<211> 401
<212> DNA
<213> Homo sapiens

<400> 108

```
ttcctacaag gattatagat gtctttactg tgtcatgcag gttgtcagag cagtgcagga     60
aagccagcag ttacaggtct cacccagctc ccacataatc cgaagggctg gtctcacttt    120
cactgtgccc cctgcccccc gcctgccaat agcactgagt ctgtttccag gcaatgggcg    180
agcagggctg agaacttgcc scacgatacc tgcctcccag ctgcagtagc aagtatgtct    240
ttccttcttc ccctgcctgt ggagtctgca caccagattc atgccctccc ctgagttctg    300
accaggagag ttctccacca gttcaaattg tttcaaagtt cagctgaaga tgtccttctc    360
tctgtggcct tttcccagtg cctctggctg cccttctgaa g                        401
```

<210> 109
<211> 401
<212> DNA
<213> Homo sapiens

<400> 109

cctggaaaca gactcagtgc tattggcagg cggggggcag ggggcacagt gaaagtgaga 60
ccagcccttc ggattatgtg ggagctgggt gagacctgta actgctggct ttcctgcact 120
gctctgacaa cctgcatgac acagtaaaga catctataat ccttgtagga acatagctcc 180
attgacctgg gagcctcatc yccatccccc acagcagatg cagcaagacc tgcccaagga 240
gagtctgtgc ttagacatgc ctagccctgc ccccacacaa tggtccttcc ctatccaccc 300
tggtaattga agacaaaggg catatactct tgggagttct agggccctgc ccaccacctg 360
ttcttcccca tactactgca gctgatgctc tctggaaagc g 401

<210> 110
<211> 401
<212> DNA
<213> Homo sapiens

<400> 110

ccttctcatt tgggtaggtt ctgtcagagg gaaggtctac ggctgaagtc tgttgttcag 60
attttttttg tcccacaggg tgttcccttg atgtagaact ctctcccttt tcctatggat 120
gtggcttcct gagagccgag ctgtagtgat tgttttctct ctttaggacc ttgccaccca 180
gtaagtctac caggctctgg kctggtattg ggagttttct gcacagagtc ctgtgatgtg 240
aaccgtctgt gggtctctca gctgtggata ccagcacctg ttctagtgga ggtggcaggg 300
aggtgaaatg gactctgtga gggttcttag ctttggtggt ttaatgtagt atttttgtgc 360
tggttggcct cctgccagga ggtggcgctt tccagagagc a 401

<210> 111
<211> 401
<212> DNA
<213> Homo sapiens

<400> 111

tttgtcttaa ctttacataa cctgttgaca acgtgcatag gtatgatctt tttgtgatga 60
atttcccagg tgttttttgt gtttcttgta tttggatgtc tagatctcta gcaagtctgg 120
ggaagttttc cttgattatt cccccaaata ggttttccaa actttcaaat gtctgttctt 180
cctcaggaac actgattatt ytaggtttgg tcatttaata gaatcccaga ctgcttggag 240
gctttgttca tattttctta tctttttctt ttgtctttgt tagactgggt taatttgaag 300
accttgtctt caagctctga atttctttct tctacttgtt cgattctatt gctgagactt 360
tccagagcat tttgcatttc tataagtgtg tccattgttt c 401

<210> 112
<211> 401
<212> DNA
<213> Homo sapiens

<400> 112

tctgtatata tctgttaatt ccatttgttc tagggtatag tttaattcaa tttttttgt 60
tgcctttctg ccttgatgac ctctctaatc ctgttagtgt agtattgaag tcccccacta 120
ttattgtgtt gctgtctatc tcacttttta ggtctagtaa ttgttttata aatttgggag 180

ctccagtgtt agatgcatat rtatttagga ttgtgatatt ttcctgttgg acaaggcctt 240
ttatcattaa ataatgtcct tctgtctttt taaactgctg tcactttaaa gtttgtttga 300
atagctactc ctgctcactt ttggtgtcaa tttgcatgaa atgtcttttt ccaccccttt 360
accttaagtt tatgtgagtc cttatgtgtt aggtgagcct c 401

<210> 113
<211> 401

<212> DNA
<213> Homo sapiens

<400> 113

```
ttccactgtg gtctgagaga gtgcttgata tgatttccat tttcttaact ttattgaggc     60
tcattttgtg gcctatcata tggtctatct tggagaaggt tccatgtgct gttgaataga    120
atgtatagtc tgcagttgtt agatggaatg ttctgtatat atctgttaat tccatttgtt    180
ctagggtata gtttaattca wttttttttg ttgcctttct gccttgatga cctctctaat    240
cctgttagtg tagtattgaa gtcccccact attattgtgt tgctgtctat ctcacttttt    300
aggtctagta attgttttat aaatttggga gctccagtgt tagatgcata tatatttagg    360
attgtgatat tttcctgttg gacaaggcct tttatcatta a                        401
```

<210> 114
<211> 401
<212> DNA
<213> Homo sapiens

<400> 114

```
cctttgctgt atcccagagg ttttgatagg ttgtgtcact attgtcgttt agttcaaaga     60
actttttaat tttcatcttg atttcacttc tgacccaatg atcattcagg aacaggttat    120
ttaatttcca tgtatttgca tggttttgaa ggttcctttt ggagttgatt tccagtttta    180
ttccactgtg gtctgagaga rtgcttgata tgatttccat tttcttaact ttattgaggc    240
tcattttgtg gcctatcata tggtctatct tggagaaggt tccatgtgct gttgaataga    300
atgtatagtc tgcagttgtt agatggaatg ttctgtatat atctgttaat tccatttgtt    360
ctagggtata gtttaattca attttttttg ttgcctttct g                        401
```

<210> 115
<211> 401
<212> DNA
<213> Homo sapiens

<400> 115

```
tgctctaatc ttggttattt cttttcttct gttgggtttg ggtttggttt gttcttgttt     60
ctctagttct tcgaggtgtg aacttagatt atctgtttgt gctctttcag accttttgat    120
gtgggcattt agggctatga actttcctct tagcaccgcc tttgctgtat cccagaggtt    180
ttgataggtt gtgtcactat ygtcgtttag ttcaaagaac tttttaattt tcatcttgat    240
ttcacttctg acccaatgat cattcaggaa caggttattt aatttccatg tatttgcatg    300
gttttgaagg ttccttttgg agttgatttc cagttttatt ccactgtggt ctgagagagt    360
gcttgatatg atttccattt tcttaacttt attgaggctc a                        401
```

<210> 116
<211> 401
<212> DNA
<213> Homo sapiens

<400> 116

```
cattggggat attggtctgt agttttcttt tttggttttg tcctttcctg gttgtggtgt     60
taggatgatg ctggcttcat agaatgattt aggaagggtt ccttctttct ccatcttgtg    120
gaatagtgtc aataggattg gtaccaattc ttttttgaat gtctggtaga attctgctgt    180
gaatccgtct ggtcctggcc wtttttttgtt ggtaattttt aaattaccat ctcaatctca   240
ctgcttgata ttggtctgtg cagggtgttt aattcttcct gatttaagct aggagagttg    300
tatctttcta agaatttatc catctcttct aggttttcta gtttatgcac ataaagatgt    360
tcatagtatc cttgaatgat cttttgtatt tctgtggttt c                        401
```

<210> 117
<211> 401
<212> DNA
<213> Homo sapiens

<400> 117

```
tattgacttg catatgttaa accatcccta catccctgct atgaaatcca cttgatcatg     60
gatcatggag tattatcttt ttttttgttg ttgttagaca gagtcccact ctgtcaccta    120
ggctagggtg cagtggcact atttcggctc actgcaacct ccgcctccca ggttcaagtg    180
attctcctgt ctcagcttcc ygagtagctg ggattacagg tgtgcgccac aatgcctggc    240
taatttttgt atttttagta gagacagggt tttgccatgt tagccaggct agccttgggt    300
gatccaccca cctgggtctc ccaaagtgct gggattacaa gcatgagcta ctgagcccgg    360
ccggattatc tttttgatat gttgttggct ttggttagct a                        401
```

<210> 118
<211> 401
<212> DNA
<213> Homo sapiens

<400> 118

```
attgctgaga gttttaatta taaagggata ctggattttg tcaaacgctt tttctgcatc     60
tattgagatg attatgtgat ttttgttttt aattctgttt atgtgttgta tcacatttat    120
tgacttgcat atgttaaacc atccctacat ccctgctatg aaatccactt gatcatggat    180
catggagtat tatctttttt kttgttgttg ttagacagag tcccactctg tcacctaggc    240
tagggtgcag tggcactatt tcggctcact gcaacctccg cctcccaggt tcaagtgatt    300
ctcctgtctc agcttcctga gtagctggga ttacaggtgt gcgccacaat gcctggctaa    360
tttttgtatt tttagtagag acagggtttt gccatgttag c                        401
```

<210> 119
<211> 401
<212> DNA
<213> Homo sapiens

<400> 119

```
agggaggatt ccctctttct ctattgattg gaatagtttc agaaggaatg gtaccacctc     60
ctccttgtac ctctggtaga atttgaaact gctgaattct tttatcagtt ctaggagctt    120
tctagaggaa ttgttagggt tttctaggta aacaatcata tcatcagcaa agtgacagtt    180
tgacttcctc cttactgatt kggatgccct ttatttcttt ctcttgtctg actgctctgg    240
ctaggacttc cagtaatatg ttgaagagga gtggtgagag tgggcgtcct tatcttgttt    300
cagttctcag agggaatgct ttcaactttt tcccattcag tattatattg gctgtgggct    360
tgtcatagat ggcctttatt acattgaggt atgtctcttg t                        401
```

<210> 120
<211> 401
<212> DNA
<213> Homo sapiens

<400> 120

```
taatttacag attcaatgcc atccccatca agctaccaat gactttcttc acagaattgg      60
aaaaaactac tttaaagttc atatggaacc aaaaaaaggg ccctcattgc caagtcaatc     120
ctaagccaaa agaacaaagc tggaggcatc acactacctg acttcaaact atactacaag     180
gctacagtaa ccaaaacagc wtggtactgg taccaaaaca gagatataga ccaatggaac     240
agaacagggc cctcagaaat aatgtcgcat atctacaact gtctgatctt tgacaaacct     300
gacaaaaaca agcaatgggg aaaggattcc ctatttaata aatggtgatg ggaaaaccgg     360
ctagccatat gtagaaagct gaaactggat cccttcctta c                         401
```

<210> 121
<211> 401
<212> DNA
<213> Homo sapiens

<400> 121

```
aactacttta aagttcatat ggaaccaaaa aaagggccct cattgccaag tcaatcctaa      60
gccaaaagaa caaagctgga ggcatcacac tacctgactt caaactatac tacaaggcta     120
cagtaaccaa aacagcttgg tactggtacc aaaacagaga tatagaccaa tggaacagaa     180

cagggccctc agaaataatg ycgcatatct acaactgtct gatctttgac aaacctgaca     240
aaaacaagca atggggaaag gattccctat ttaataaatg gtgatgggaa aaccggctag     300
ccatatgtag aaagctgaaa ctggatccct tccttacacc ttatgcaaaa attaattcaa     360
gatggattaa agacataaaa gttagaccta aaaccataaa a                         401
```

<210> 122
<211> 401
<212> DNA
<213> Homo sapiens

<400> 122

```
cagtgtgtga tgttcccctt cctgtgtcca ggtgttctca ttgttcaatt cccacctatc      60
agtgagaaca tgcagtgttt ggttttttgt ccttgtgaga gtttgctgag aatgatggtt     120
tccaccttta tccacgtccc tacaaaggac ctgaactcat ccttttttatg gctgcatagt    180
attccatggt gtatatgtgc yacattttct taatccagtc tgtcattgct ggacatttgg     240
gttggttcca agtctttgct attgtgaata gtgctgcaat aaacatacgt gtgcatgtgt     300
ctttatagca gcatgattta taatcatttg ggtatatacc cagtaatggg atggctgggt     360
caaatggtgt ttctagctct agatccttga gaaatcgcca c                         401
```

<210> 123
<211> 401
<212> DNA
<213> Homo sapiens

<400> 123

```
gcacttttct ctttatccct aaatatttca ggttctaact ttctcttaca taataactgt      60
atagttatca aatttacaga atttaacatg tatgcattat tacataattt gcattccata     120
tacaaaatct gccaactgtc ccatggtgtt ctttagagga atttttactt cttatctcaa     180
gatccaatcc aggatcaaat ygatcccatg ttgcatttag ttatgtagtc tctttagtct     240
tctttaatct agaacatttc tcgagccttt atctctcatg aattaacatt ttggaagagt     300
acaagctagt tgtcttgcag aatgcccttc aactgggttt gtctcgtcct tcctcatgat     360
tagtgtatgt tacctgtccc ctctggaata ccataaaagt g                         401
```

<210> 124
<211> 401

<212> DNA
<213> Homo sapiens

<400> 124

```
tctagattaa agaagactaa agagactaca taactaaatg caacatggga tcaatttgat      60
cctggattgg atcttgagat aagaagtaaa aattcctcta aagaacacca tgggacagtt     120
ggcagatttt gtatatggaa tgcaaattat gtaataatgc atacatgtta aattctgtaa     180
atttgataac tatacagtta ytatgtaaga gaaagttaga acctgaaata tttagggata     240
aagagaaaag tgcatgatat ctgtgactca tcaaagaatc ataaagtgtt agaggttgaa     300
gggtccctag agagtcagta ggccaaaccc tactttcttg aaatgatgtg accaaaatcc     360
tgagaaattg aattatctgc ctaagaccac acatccagcc a                         401
```

<210> 125
<211> 401
<212> DNA
<213> Homo sapiens

<400> 125

```
tctatgtgcc tatttttgtg ctagcactat gctgtttttg tgactatggc cttatagtat      60
agtttgaaat caggtaatgt gatgcttcta gatttgttct ttttgcttag tcttgctttg     120
gctatgcggg ctcttttttg gttctatatg aatttcagaa ttgctttttc taattctgtg     180
aagaatgatg gtagtatcgt wgtgaattgt gttgaatttg tagattgctt ttggcagtat     240
ggtcattttc acaatattga ttgtatccat gcatgagcat gggatgtgtt tccatttgtt     300
tgtgtcatct atggtttctt tcagcaatgt tttgtagttt tccttgtaga ggtctttcac     360
ctccttggtt aggtagattc gtaagtattt tattttattt t                         401
```

<210> 126
<211> 401
<212> DNA
<213> Homo sapiens

<400> 126

```
cacacctatc ttctattaat taatgtgcat cctctgagga cctgggcaag tcttcattgt      60
tggtttagtt gttttgagaa gcaacacaat tgctttttaa aatacaatta tataatttag     120
atatctatat atttctgca ttctcagtgc tccctaaatc ctcaccccaa cttcttctca     180
ctccccagct gtgtttcctt kctggccttg aacttctcag ctgctgaact tttttcctcc     240
tctcagtact tcaacaattt cccttaaagg gacgcttcag atcaatgaaa caaagagagt     300
ttataaatag cctgccaagc agagggaaac atagcataag atatttcaaa ttattggaaa     360
gaagtggatt atttaataat tggtactggg aaaactattt g                         401
```

<210> 127
<211> 401
<212> DNA
<213> Homo sapiens

<400> 127

```
ggaacttgag tgaggtcagt gtaacaccag cttatatcac acttagattg ggaagaagga      60
cagaatgctt ggggtttttct aagtcattct ctatcagagt ccatgaggcc atgccctctt    120
gctcattctg gatggagata gtcattcttc tccatgcctt ctcttctctg gaaaacccgg     180
ttattttcag cttcccatgc ygtttttttcc ctctacccta tccatcacat gggaaaggcc    240
atattgtgtc ctggcaagag cacagtttct gcaccagatg ggggtgaaac tgggtcccag     300
ctttctcact aacaacagga tgactttggg caaatacata ttttatttcc tgtgcaaaat     360
gaggatgatg atggcctcct tatggtattg ctacaagaaa g                         401
```

<210> 128
<211> 401
<212> DNA
<213> Homo sapiens

<400> 128

```
tcctgtgttc tgttcctcct cctccctttg gccgtgcctt ggctcaggct gtatcccttc      60
tcatgggact ccttcaactt cttggtcact ctctgcaagc acagcgactt ttgaaaacaa     120
tgaaattgat aatttctctt ccctgcttaa aaccctttca tgacatttca ttgttctctt     180
cttaaagttc caactgtcac rcctgtaatc ccagcatgtt gggaggctga ggcgggcaga     240
tcacaaggtc aggagatcga gaccatcttg gctaacacgg tgaaacaccg tctctactaa     300
aaatacaaaa tattagctgg gtgtggtggt gggtgcctgt agtctcagct acttgggagg     360
ctgaggcagg agaatggtgt gaagccagga ggcggagctt g                         401
```

<210> 129
<211> 401
<212> DNA
<213> Homo sapiens

<400> 129

```
ggccactcgt cagctggtaa tgtcagccat gtggtggtgg caggggcaca aaagatgcct      60
gagggactcc agctctgagg gccacaatgc tgtccctcct ggaggcgtgg tcttcctatg     120
gtgactgccc acttggttcc atccttccct taccccctca aaatccgtgt cacataaagc     180
ccaccccagc agacctaatt yttgttaaag tgccattctg taagggcacc ccctctggcc     240
attattccac cacgactacc acgggactca ctttcctgtc tgtgacaccc tggagagggt     300
ctttaattcc aggcattagt aaaacatcca cagagagcaa gtgacccgtg ctccgccatg     360
aaaccgacaa gcagctcagc tttctctcta gttaacagaa g                         401
```

<210> 130
<211> 401
<212> DNA
<213> Homo sapiens

<400> 130

```
tttggtgaag gcaaagtcct ccttcttcat tagcggtctc ccatgtgggg ccacatcttc      60
cctcaccagg aacccagtgg gcgcgctcca gcccccctca gcttgccttt tgcgtggtca     120
ttagagctag ggcacacgtc atgctgattc acatattttt gccctttgtc atgtattgag     180

aaaaagtaag gatgaatgga yggtctttga ttggcggcgc tggtgacgcc cgtcatggtc     240
ctgtttggaa ggaccctttt ggaactaaag ctggtgacgc agcgcgcaga ggcatcgccc     300
ggctaagctt ggccctggca gatgggtcgc aggaacaggt atgcttcctt cgtgcagcct     360
ctggctcggg gaacctggga gcctgctcca aactctggtg t                         401
```

<210> 131
<211> 401
<212> DNA
<213> Homo sapiens

<400> 131

```
ctccataaga aaaaaaaatg gagaggtttg caggttaaaa gataaaagta gaaaaatggt      60
gagtaaagag atctataaaa agttggagaa tataactaaa ggtataagta gaagtaccag     120
gaaaccattt cccccaaagc tacttctcct gtctctccaa aatgtgaaag gataaatacc     180
tttaagctgg ttggcaagtt ytttcatgtc accggtgaca tagccaagag ctttggggaa     240
gcaccttctc ccttgacatg gcgaaacctg gttttcaccc ctaaccatag tggggctgtc     300
cactctaacc atagtggggc tgtcctccat ggctatctct tcctctggct ccttgaggga     360
aggacagagc ttataagtat ccctaaggca gagccctcca g                         401
```

<210> 132
<211> 401
<212> DNA
<213> Homo sapiens

<400> 132

```
gagccagggc gaatgtgaga agccagtgtc tcttacctcc agggcactga ggtcagacca      60
ggagatattt ggagctgtag ttataggtat gaccagtttg gctggaaaga atggattgta     120
atgtcctgtg gctgaaaggt acattgacaa cgccatgttg aaagggaggg tgaagacggg     180
gaggtcccat ttgctgagca yggaattcaa tgcacttgag aaaattgggc taaaaaaaga     240
acagagcaaa agaatgtgca tgtgacaaag caggctgaca cacggaatcg ctatactctg     300
gaggggcttt gtttcacctg ccaacaggcc ccttacgtgt tgtgttggca catttgccac     360
agaaagaaaa cttgggataa atcctattat agaggcagta a                         401
```

<210> 133
<211> 445
<212> PRT
<213> Homo sapiens

<400> 133

```
Met Asn Gly Arg Ser Leu Ile Gly Gly Ala Gly Asp Ala Arg His Gly
1               5                   10                  15
Pro Val Trp Lys Asp Pro Phe Gly Thr Lys Ala Gly Asp Ala Ala Arg
            20                  25                  30
Arg Gly Ile Ala Arg Leu Ser Leu Ala Leu Ala Asp Gly Ser Gln Glu
        35                  40                  45
Gln Glu Pro Glu Glu Glu Ile Ala Met Glu Asp Ser Pro Thr Met Val
    50                  55                  60
Arg Val Asp Ser Pro Thr Met Val Arg Gly Glu Asn Gln Val Ser Pro
65                  70                  75                  80
Cys Gln Gly Arg Arg Cys Phe Pro Lys Ala Leu Gly Tyr Val Thr Gly
                85                  90                  95
Asp Met Lys Glu Leu Ala Asn Gln Leu Lys Asp Lys Pro Val Val Leu
            100                 105                 110
Gln Phe Ile Asp Trp Ile Leu Arg Gly Ile Ser Gln Val Val Phe Val
        115                 120                 125
Asn Asn Pro Val Ser Gly Ile Leu Ile Leu Val Gly Leu Leu Val Gln
    130                 135                 140
Asn Pro Trp Trp Ala Leu Thr Gly Trp Leu Gly Thr Val Val Ser Thr
145                 150                 155                 160
Leu Met Ala Leu Leu Leu Ser Gln Asp Arg Ser Leu Ile Ala Ser Gly
                165                 170                 175
Leu Tyr Gly Tyr Asn Ala Thr Leu Val Gly Val Leu Met Ala Val Phe
            180                 185                 190
```

Ser Asp Lys Gly Asp Tyr Phe Trp Trp Leu Leu Leu Pro Val Cys Ala
195 200 205
Met Ser Met Thr Cys Pro Ile Phe Ser Ser Ala Leu Asn Ser Met Leu
210 215 220
Ser Lys Trp Asp Leu Pro Val Phe Thr Leu Pro Phe Asn Met Ala Leu
225 230 235 240
Ser Met Tyr Leu Ser Ala Thr Gly His Tyr Asn Pro Phe Phe Pro Ala
245 250 255
Lys Leu Val Ile Pro Ile Thr Thr Ala Pro Asn Ile Ser Trp Ser Asp
260 265 270
Leu Ser Ala Leu Glu Leu Leu Lys Ser Ile Pro Val Gly Val Gly Gln
275 280 285
Ile Tyr Gly Cys Asp Asn Pro Trp Thr Gly Gly Ile Phe Leu Gly Ala
290 295 300
Ile Leu Leu Ser Ser Pro Leu Met Cys Leu His Ala Ala Ile Gly Ser
305 310 315 320
Leu Leu Gly Ile Ala Ala Gly Leu Ser Leu Ser Ala Pro Phe Glu Asp
325 330 335
Ile Tyr Phe Gly Leu Trp Gly Phe Asn Ser Ser Leu Ala Cys Ile Ala
340 345 350
Met Gly Gly Met Phe Met Ala Leu Thr Trp Gln Thr His Leu Leu Ala
355 360 365
Leu Gly Cys Ala Leu Phe Thr Ala Tyr Leu Gly Val Gly Met Ala Asn
370 375 380
Phe Met Ala Glu Val Gly Leu Pro Ala Cys Thr Trp Pro Phe Cys Leu
385 390 395 400
Ala Thr Leu Leu Phe Leu Ile Met Thr Thr Lys Asn Ser Asn Ile Tyr
405 410 415
Lys Met Pro Leu Ser Lys Val Thr Tyr Pro Glu Glu Asn Arg Ile Phe
420 425 430
Tyr Leu Gln Ala Lys Lys Arg Met Val Glu Ser Pro Leu
435 440 445

<210> 134
<211> 4129
<212> DNA
<213> Homo sapiens

<400> 134

acacagagca gagtggggct ctgagtatat aactgttagg tgcctccctc cagcaccatc 60
tcctgagaag cactctccct tgtcgtggag gtgggcaaat ctttatcagc cactgccttc 120
tgctgccagg aagccagcta gagtggtctt taaagaaaac tgggcatctc ctgctactta 180
aaatcaaaaa ctacctaaaa taaagattat aaaaaagtaa ggatgaatgg acggtctttg 240
attggcggcg ctggtgacgc ccgtcatggt cctgtttgga aggacccttt tggaactaaa 300
gctggtgacg cagcgcgcag aggcatcgcc cggctaagct tggccctggc agatgggtcg 360
caggaacagg agccagagga agagatagcc atggaggaca gccccactat ggttagagtg 420
gacagcccca ctatggttag gggtgaaaac caggtttcgc catgtcaagg gagaaggtgc 480
ttccccaaag ctcttggcta tgtcaccggt gacatgaaag aacttgccaa ccagcttaaa 540
gacaaacccg tggtgctcca gttcattgac tggattctcc ggggcatatc ccaagtggtg 600
ttcgtcaaca accccgtcag tggaatcctg attctggtag gacttcttgt tcagaacccc 660
tggtgggctc tcactggctg gctgggaaca gtggtctcca ctctgatggc cctcttgctc 720
agccaggaca ggtcattaat agcatctggg ctctatggct acaatgccac cctggtggga 780
gtactcatgg ctgtcttttc ggacaaggga gactatttct ggtggctgtt actccctgta 840
tgtgctatgt ccatgacttg cccaatttc tcaagtgcat tgaattccat gctcagcaaa 900
tgggacctcc ccgtcttcac cctccctttc aacatggcgt tgtcaatgta cctttcagcc 960
acaggacatt acaatccatt ctttccagcc aaactggtca tacctataac tacagctcca 1020
aatatctcct ggtctgacct cagtgccctg gagttgttga aatctatacc agtgggagtt 1080
ggtcagatct atggctgtga taatccatgg acaggggca ttttcctggg agccatccta 1140
ctctcctccc cactcatgtg cctgcatgct gccataggat cattgctggg catagcagcg 1200
ggactcagtc tttcagcccc atttgaggac atctactttg gactctgggg tttcaacagc 1260
tctctggcct gcattgcaat gggaggaatg ttcatggcgc tcacctggca aacccacctc 1320
ctggctcttg gctgtgccct gttcacggcc tatcttggag tcggcatggc aaactttatg 1380

```
gctgaggttg gattgccagc ttgtacctgg cccttctgtt tggccacgct attgttcctc      1440
atcatgacca caaaaaattc caacatctac aagatgcccc tcagtaaagt tacttatcct      1500
gaagaaaacc gcatcttcta cctgcaagcc aagaaaagaa tggtggaaag ccctttgtga      1560
gaacaagccc catttgcagc catggtcacg agtcatttct gcctgactgc tccagctaac      1620
ttccagggtc tcagcaaact gctgtttttc acgagtatca actttcatac tgacgcgtct      1680
gtaatctgtt cttatgctca ttttgtattt tcctttcaac tccaggaata tccttgagca      1740
tatgagagtc acatccaggt gatgtgctct ggtatggaat ttgaaacccc aatggggcct      1800
tggcactaag actggaatgt atataaagtc aaagtgctcc aacagaagga ggaagtgaaa      1860
acaaactatt agtatttatt gatattcttg gtgtttagct ggctcgatga tgttaacagt      1920
attaaaaatt aaaccccata aaccaactaa gccttatgga attcacagtc acaaaatcga      1980
agttaatcca gaattctgtg ataagcagct tggctttttt tttaaatcaa tgcaagttac      2040
acattatagc cagaatctgt atcacagagg tgcaagctga cagcagagct cagtccccac      2100
ttcctgcaaa caatggcctg caccctatcc cttgtgtgtg tgacattctc tcatggaca      2160
atgttggggt ttttcagact gacaggactg caagagggag aaaggaattt tgtcaatcaa      2220
aattattctg tattgcaact tttctcagag attgcaaagg attttttagg tagagattat      2280
ttttccttat gaaaaatgat ctgttttaaa tgagataaaa taggagaagt tcctggctta      2340
acctgttctt acatattaaa gaaaagttac ttactgtatt tatgaaatac tcagcttagg      2400
catttttact ttaaccccta aattgatttt gtaaatgcca caaatgcata gaattgttac      2460
caacctccaa agggctcttt aaaatcatat tttttattca tttgaggatg tcttataaag      2520
actgaaggca aaggtcagat tgcttacggg tgttattttt ataagttgtt gaattcctta      2580
atttaaaaaa gctcattatt ttttgcacac tcacaatatt ctctctcaga aatcaatggc      2640
atttgaacca ccaaaaagaa ataaagggct gagtgcggtg gctcacgcct gtaatcccag      2700
cactttgggg agcccaggcg ggcagattgc ttgaacccag gagttcaaga ccagcctggg      2760
cagcatggtg aaaccctgta tctacaaaaa atacaaaaat tagccaggca tggtggtggg      2820
tgcctgtagt tccagctact tgggaggctg aggtgggaaa atgacttgag cccaggagga      2880
ggaggctgca gtgagctaag attgcaccac tgcactccaa cctgggcgac aagagtgaaa      2940
ctgtgtctct caaaaaaaaa aaaaaacaaa caaaaacaaa aacaaaacaa aacaaaacaa      3000
aacaaaacag gtaaggattc ccctgttttc ctctctttaa ttttaaagtt atcagttccg      3060
taaagtctct gtaaccaaac atactgaaga cagcaacaga agtcacgttc agggactggc      3120
tcacacctgt aatcccagca ctttgggaga tggaggtaaa aggatctctt gagcccagga      3180
gttcaagacc agcttgggca acatagcaag actccatctc ttaaaaaata aaaatagtaa      3240
cattagccag gtgtagcagc acacatctgc agcagctact caggaggctg aggtggaaag      3300
atcgcttgtg cacagaagtt cgaggctgca gtgagctata tgatcatgtc actgcactcc      3360
agcctgtgtg accgagcaag accctatctc aaaaaaatta attaattaat taattaatta      3420
atttaaaaag gaagtcatgt tcatttactt tccacttcag tgtgtatcgt gtagtatttt      3480
ggaggttgga aagtgaaacg taggaatcct gaagattttt tccacttcta gtttgcagtg      3540
ctcagtgcac aatatacatt ttgctgaatg aataaacaga aatagggaag taaacctaca      3600
aatattttag ggagaagctc acttcttcct tttctcagga aaccaagcaa gcaaacatat      3660
cgttccaatt ttaaaaccca gtgaccaaag cctttggaac tatgaatttg caactgtcat      3720
aggtttatgg atattgctgt ggagaagctc aattttcagt gtttgaactg aaccctttct      3780
tgttagggaa cgtgtgaaag aagaattgtg gggaaaaaaa agcaagcata accaaagatc      3840
atcagcagtg aagaatctag gctgtggctg agagaaccag aggcctctaa aatggacccg      3900
agtcgatctt cagaacaggg atctaccatg caggagcttc ttgtgctcac acaaatctgt      3960
aaatgggaac attgtacatt gtcgaattta aatgatatta attttctcaa gctatttttg      4020
ttactatttt cctaaaattg aatatttgca gggagcactt atactttttc ctaatgtctg      4080
tataacaaat ttctatgcaa gtacatgaat aaattatgct cacagctca                  4129
```

<210> 135
<211> 401
<212> DNA
<213> Homo sapiens

<400> 135

```
ggaaggatgt cccaagccgg caagaaccct gtggtctggc gggtgtccag cgggtggagc    60
cagagagctc ccagccatgg gccacctagc tgaaggctga taagtactat ctgcacatcc   120
aagcaggcag gagacccaga taaaagacct atgacatctg catggctggc tgtgggatta   180
catatatata ttatatatat wtttaaaagt atatgtatat atgtatatat attacatata   240
tacacacata tatatctaca catatgtaat atatgtatat atgtatattt attatatgta   300
tatatctaca tatatacatg tatgtatgta atatagatgt atatatgtta catatataca   360
cacatataca tatatacaca tatatttttt taaaattctg c                       401
```

<210> 136
<211> 401
<212> DNA
<213> Homo sapiens

<400> 136

```
gtcccaagcc ggcaagaacc ctgtggtctg gcgggtgtcc agcgggtgga gccagagagc    60
tcccagccat gggccaccta gctgaaggct gataagtact atctgcacat ccaagcaggc   120
aggagaccca gataaaagac ctatgacatc tgcatggctg gctgtgggat tacatatata   180
tattatatat atatttaaaa rtatatgtat atatgtatat atattacata tatacacaca   240
tatatatcta cacatatgta atatatgtat atatgtatat ttattatatg tatatatcta   300
catatataca tgtatgtatg taatatagat gtatatatgt tacatatata cacacatata   360
catatataca catatatttt tttaaaattc tgcactcata a                       401
```

<210> 137
<211> 401
<212> DNA
<213> Homo sapiens

<400> 137

```
agatatttta attcagtttt agatttccct gctatagaat gtttgcttcc tgacttttaa    60
agaggcagca gagtatgtcc aagaattctc acctgggaag acagacattc cagggaaaca   120
ctgatatttt tgtttctcc tcactgggaa aaaggcagct ggccttcaga aaacaacttg   180
catgtacctt atcttcccca mcctcccaac agcccctcag gcccttgctg gagtcttaca   240
tctgtgtaga agttgtgcca agtctaagaa aatttcaccc caggagcagt cgcttagctt   300
ccgagtccac acagcagcaa cctgccccat ggtttttagt ttgacaccgt gttgatcctt   360
ttcagttgta aatctttcca aaaatatgag taggggccag a                       401
```

<210> 138
<211> 401
<212> DNA
<213> Homo sapiens

<400> 138

```
aactgctgct tcacttgttt gttgacttga accgaacctt gggtggcatt aatgtgcctg    60
gcccaagact gaaaaattaa gaaccaccag agctgaccta ttccataaga cccagtctgc   120
ctgccacgta ctgagtgaat ctggatgatg cccactctga tccttggttt tctcttctat   180
aaaatgaagg cttgaactac rtggtctcta aaatcctacc tagctctcaa atttctcttg   240
gttctaggaa aatattgatg ttgagctcaa ggaaggggtt ctccaaggtg tgtgattttg   300
gtggtagagg aaaggccggt gccaggcagg ggcagaagga gacgctgtct acactgagaa   360
aatgtgacaa cccctgcttg tctctttttt cattcttcat t                       401
```

<210> 139
<211> 401
<212> DNA
<213> Homo sapiens

<400> 139

```
ctaaaaaaaa ataaaataaa ttaaaataaa cacagctgga tgtggtggca caggaaaaaa      60
aaataccatt taggagtctc ttaaaggcag cttgtgaatg cttacaaagc gtggctagta     120
tcttattaca gaaaacagag cccacatcat gcatccttct tctcacattt cataaacaag     180
gccaagggaa actgctgtgg rgcaacctgt tgctttggtg ttggtcccca agatgcagcc     240
ctcacaatct gcccccaaac gtgtcagaac atgaaccccc tcctccccct ctggaagaag     300
caacctcaga tccaacagca gagacacgca gcagaacaaa atctgggcat tggtccctgt     360
gtaggatggc ttcccgttat ttttttttta agcaaagtaa a                        401
```

<210> 140
<211> 401
<212> DNA
<213> Homo sapiens

<400> 140

```
ctcctccccc tctggaagaa gcaacctcag atccaacagc agagacacgc agcagaacaa      60
aatctgggca ttggtccctg tgtaggatgg cttcccgtta tttttttttt aagcaaagta     120
aatgaacatc aaatttccat agtcagctgc tgtctttctg cccactgaga gctctttggt     180

gaaggcaaag tcctccttct ycattagcgg tctcccatgt ggggccacat cttccctcac     240
caggaaccca gtgggcgcgc tccagccccc ctcagcttgc cttttgcgtg gtcattagag     300
ctagggcaca cgtcatgctg attcacatat ttttgccctt tgtcatgtat tgagaaaaag     360
taaggatgaa tggacggtct ttgattggcg gcgctggtga c                        401
```

<210> 141
<211> 401
<212> DNA
<213> Homo sapiens

<400> 141

```
cccccctcagc ttgccttttg cgtggtcatt agagctaggg cacacgtcat gctgattcac    60
atatttttgc cctttgtcat gtattgagaa aaagtaagga tgaatggacg gtctttgatt   120
ggcggcgctg gtgacgcccg tcatggtcct gtttggaagg accctttgg aactaaagct    180
ggtgacgcag cgcgcagagg matcgcccgg ctaagcttgg ccctggcaga tgggtcgcag   240
gaacaggtat gcttccttcg tgcagcctct ggctcgggga acctgggagc ctgctccaaa   300
ctctggtgta tcttttccgg gcagagcctg ggaagtgggg gttggctgtg agctaagcca   360
aaggcacagg gatcttggtc caaaaagccc catggcgctc a                       401
```

<210> 142
<211> 401
<212> DNA
<213> Homo sapiens

<400> 142

```
tcatgagaag acccctctct gcaggacatc ctagccctac aacccatccc aattatgttg    60
aaattagatt cacaaatggc aataagtctt ctatatgttg ggctgtcgat ttggagaaaa   120
ctagtttaat ctttacttaa ctttgggtgg ctcaacagga gactcgggcc gctcaggctc   180
tcaatcacgt ctggccagtt ytattatcag gtttcgaatc tgtatctcca aaatctctga   240
ggtgatggga tatttcaagc cctctaaaat aaataaatat atgctgggaa ttttgagaac   300
atgaatttgt ttattctgaa atggtccatg ttcctgcttt gggagttgat ggaaaatgcc   360
acttgagtgt tttcatttga tgctgccacc ttagggtttt a                       401
```

<210> 143
<211> 401
<212> DNA
<213> Homo sapiens

<400> 143

```
acccctctct gcaggacatc ctagccctac aacccatccc aattatgttg aaattagatt     60
cacaaatggc aataagtctt ctatatgttg ggctgtcgat ttggagaaaa ctagtttaat    120
ctttacttaa ctttgggtgg ctcaacagga gactcgggcc gctcaggctc tcaatcacgt    180
ctggccagtt ctattatcag ktttcgaatc tgtatctcca aaatctctga ggtgatggga    240
tatttcaagc cctctaaaat aaataaatat atgctgggaa ttttgagaac atgaatttgt    300
ttattctgaa atggtccatg ttcctgcttt gggagttgat ggaaaatgcc acttgagtgt    360
tttcatttga tgctgccacc ttagggtttt atagattcag t                        401
```

<210> 144
<211> 401
<212> DNA
<213> Homo sapiens

<400> 144

```
aatctttact taactttggg tggctcaaca ggagactcgg gccgctcagg ctctcaatca     60
cgtctggcca gttctattat caggtttcga atctgtatct ccaaaatctc tgaggtgatg    120
ggatatttca agccctctaa aataaataaa tatatgctgg gaattttgag aacatgaatt    180
tgtttattct gaaatggtcc rtgttcctgc tttgggagtt gatggaaaat gccacttgag    240
tgttttcatt tgatgctgcc accttagggt tttatagatt cagttccaga aactcaaggc    300
atttatctct ttgggctgct tgtccttgcc tgagctgaag cctgatgcct cccataagtt    360
ggtatggctt tgaaaatggg tcactacagc agaggcatgg g                        401
```

<210> 145
<211> 401
<212> DNA
<213> Homo sapiens

<400> 145

```
taccatagct aacaaatagc aatgaacaga tactaagtta tacctattaa aaaaagttga     60
actctagctt tggcttctat taatcccagt aaagaaatca atatgggtaa ctgatttatt    120
ggtagcaatt acatttcaat tgtattacct cactgcccaa ctaaccattc tgctttttaa    180
acaatgccaa actattaatc wttacacaaa aaagcagctt tttatacaat gcattccaga    240
ccaaggtcat tttatctcgg taaaacaatg ttagatacca acatctgtgt gacttgcaat    300
gaatcttgga ggtcaggaac caaaattaag tccatatgga gctgagttgg ctccatagtt    360
aaagataaaa aaccgtatga ataattaagc acttatgaaa a                        401
```

<210> 146
<211> 401
<212> DNA
<213> Homo sapiens

<400> 146

```
tggttcctga cctccaagat tcattgcaag tcacacagat gttggtatct aacattgttt     60
taccgagata aaatgacctt ggtctggaat gcattgtata aaaagctgct tttttgtgta    120
aagattaata gtttggcatt gtttaaaaag cagaatggtt agttgggcag tgaggtaata    180
caattgaaat gtaattgcta scaataaatc agttacccat attgatttct ttactgggat    240
taatagaagc caaagctaga gttcaacttt ttttaatagg tataacttag tatctgttca    300
ttgctatttg ttagctatgg taaatggaac aatgatgggg ccagaaatat ccatgaggac    360
catttgatca cagcctggca acacagagaa gacaggctgg t                        401
```

<210> 147
<211> 401
<212> DNA
<213> Homo sapiens

<400> 147

```
gataaaatga ccttggtctg gaatgcattg tataaaaagc tgcttttttg tgtaaagatt     60
aatagtttgg cattgtttaa aaagcagaat ggttagttgg gcagtgaggt aatacaattg    120
aaatgtaatt gctaccaata aatcagttac ccatattgat ttctttactg ggattaatag    180
aagccaaagc tagagttcaa ytttttttaa taggtataac ttagtatctg ttcattgcta    240
tttgttagct atggtaaatg gaacaatgat ggggccagaa atatccatga ggaccatttg    300
atcacagcct ggcaacacag agaagacagg ctggtttctc tatgtgggct ttcagtgttt    360
ctttggtagt gtcttatgtg gctgtggctt caacattcca c                        401
```

<210> 148
<211> 401
<212> DNA
<213> Homo sapiens

<400> 148

```
aagccaaagc tagagttcaa cttttttttaa taggtataac ttagtatctg ttcattgcta     60
tttgttagct atggtaaatg gaacaatgat ggggccagaa atatccatga ggaccatttg    120
atcacagcct ggcaacacag agaagacagg ctggtttctc tatgtgggct ttcagtgttt    180
ctttggtagt gtcttatgtg kctgtggctt caacattcca caattatgcc ttccagggtc    240
tgatgatttt ggcgtttccc tgcttcccaa ttgacctggc tgtgctgttg gctgttcttg    300
cacactcaag gtggttttgc cattggcttc ctccctcagc ctgcctctgg gattatgcca    360
ctgctattct tttttatcta ccatcagcac aatgaaatca t                        401
```

<210> 149
<211> 401
<212> DNA
<213> Homo sapiens

<400> 149

```
caattatgcc ttccagggtc tgatgatttt ggcgtttccc tgcttcccaa ttgacctggc     60
tgtgctgttg gctgttcttg cacactcaag gtggttttgc cattggcttc ctccctcagc    120
ctgcctctgg gattatgcca ctgctattct tttttatcta ccatcagcac aatgaaatca    180

tcatttttgt cttcaaggta scaaattctg gtgatattgg tgctttcttg cagctactta    240
tcatgagaag tgaatggtct catagtgaac acagtcatgg ttatagtgtt catacgttcc    300
agagacatgt ttcctataat tatgccctgc acatttttct atcatacaat ccttagatta    360
cagctctttg gttttcaaca gctttgtcca attccatctt t                        401
```

<210> 150
<211> 401

<212> DNA
<213> Homo sapiens

<400> 150

```
atccttagat tacagctctt tggttttcaa cagctttgtc caattccatc tttcccagtt      60
tctctacctt gatgaaatat ccttcttgcc tggttttaca tatttaaata acaaattcca     120
aaagtaaaga gtatctgagg cagtcacatg acataaggac aaattcaagc catcttggac     180
ttgcagaggg tggggagacc rtgtcaacac acacaatttt aaaaatttct tccctttcaa     240
tcttttaaaa acaaaacttt ttataaaata aaaatgtaat ttaaaaaggc tacctgtctt     300
ggcaagtagc tgatcagcct gcattggtga gcaggccatt ccataacctg gtttcttgct     360
ccttaattga cagcatggag ctaacgtact taatttcagc t                         401
```

<210> 151
<211> 401
<212> DNA
<213> Homo sapiens

<400> 151

```
aataacaaat tccaaaagta aagagtatct gaggcagtca catgacataa ggacaaattc      60
aagccatctt ggacttgcag agggtgggga gaccgtgtca acacacacaa ttttaaaaat     120
ttcttccctt tcaatctttt aaaaacaaaa ctttttataa aataaaaatg taatttaaaa     180
aggctacctg tcttggcaag yagctgatca gcctgcattg gtgagcaggc cattccataa     240
cctggtttct tgctccttaa ttgacagcat ggagctaacg tacttaattt cagctctttc     300
tacgtgattt gactcattct gttaacatta actgtttttc agtcttctca actagactga     360
actccttaag tgcaagaaat acacgcttag taaatgtttg t                         401
```

<210> 152
<211> 401
<212> DNA
<213> Homo sapiens

<400> 152

```
acacacaatt ttaaaaattt cttccctttc aatcttttaa aaacaaaact ttttataaaa      60
taaaaatgta atttaaaaag gctacctgtc ttggcaagta gctgatcagc ctgcattggt     120
gagcaggcca ttccataacc tggtttcttg ctccttaatt gacagcatgg agctaacgta     180
cttaatttca gctctttcta ygtgatttga ctcattctgt taacattaac tgtttttcag     240
tcttctcaac tagactgaac tccttaagtg caagaaatac acgcttagta aatgtttgtt     300
ggaccagaca ctgcacctta tgaaattaaa gaccagaaca ttctcatggt agcattacag     360
acactgatgg caaaggtact gtgggatttg ggtttggcta a                         401
```

<210> 153
<211> 401
<212> DNA
<213> Homo sapiens

<400> 153

```
atacacgctt agtaaatgtt tgttggacca gacactgcac cttatgaaat taaagaccag      60
aacattctca tggtagcatt acagacactg atggcaaagg tactgtggga tttgggtttg     120
gctaataagc tctgtggtgg tgtttcagaa ggaaaatggt gctctcttag ttctatggaa     180
catagtggtc cagatcttct rctgtaacca ggcccaaagc tggctaatct ggagggctct     240
gccttaggga tacttataag ctctgtcctt ccctcaagga gccagaggaa gagatagcca     300
tggaggacag ccccactatg gttagagtgg acagccccac tatggttagg ggtgaaaacc     360
aggtttcgcc atgtcaaggg agaaggtgct tccccaaagc t                         401
```

<210> 154
<211> 401
<212> DNA
<213> Homo sapiens

<400> 154

```
tgacgaacac cacttgggat atgccccgga gaatccagtc aatgaactgg agcaccacgg     60
gtttgtctgg aagggatgag gtaaagcaga gctaaggaag ctgccaagtg atactcttca    120
accacaggcc caccctgagc aaaatcagca aaacctcctg gaagtgaaac atttttgata    180
tttctttaat atttctctct saacccattg ttgcattagt ggcacccaga tccctgaaag    240
cctctatcta ctgtatgctc aacccacact gcccctccta gaatctaagc tcctgaaagc    300
atctaatgca cttattttca tgcttatagg tacaaattct gaagccaaag gaataaattt    360
aagttttaag tactgaaact acaggatgaa gccaggtgcc t                        401
```

<210> 155
<211> 401
<212> DNA
<213> Homo sapiens

<400> 155

```
ctggcttcat cctgtagttt cagtacttaa aacttaaatt tattcctttg gcttcagaat     60
ttgtacctat aagcatgaaa ataagtgcat tagatgcttt caggagctta gattctagga    120
ggggcagtgt gggttgagca tacagtagat agaggctttc agggatctgg gtgccactaa    180
tgcaacaatg ggttgagaga raaatattaa agaaatatca aaaatgtttc acttccagga    240
ggttttgctg attttgctca gggtgggcct gtggttgaag agtatcactt ggcagcttcc    300
ttagctctgc tttacctcat cccttccaga caaacccgtg gtgctccagt tcattgactg    360
gattctccgg ggcatatccc aagtggtgtt cgtcaacaac c                        401
```

<210> 156
<211> 401
<212> DNA
<213> Homo sapiens

<400> 156

```
cctttcaagc cttctcagct cccttctgag acacaggggc tgaccagtta ctgtgggcaa     60
cagtgataaa accacatcct tcccaggata aacaacattt agtccacaga actgtttata    120
tttgttttta gtcagaggtc agggaatcag ttacagtctc ttgctcttga tatctgaata    180
aatggctggt ctaaatgatg scagattctt gtggcattac gtgctaacca gaactaagct    240
acaagtattt ccctggagag gttctgaagg gatcttcttt aatgattgat aaaattattt    300
gtcgtcagca ttctatttgg gaaaaagtgc atatgaattc agaaaaagtt ttagtggctt    360
aataaccccc gttatatctt gttgctatga tgagtttagg a                        401
```

<210> 157
<211> 401
<212> DNA
<213> Homo sapiens

<400> 157

```
tcccttctga gacacagggg ctgaccagtt actgtgggca acagtgataa aaccacatcc      60
ttcccaggat aaacaacatt tagtccacag aactgtttat atttgttttt agtcagaggt     120
cagggaatca gttacagtct cttgctcttg atatctgaat aaatggctgg tctaaatgat     180
gccagattct tgtggcatta ygtgctaacc agaactaagc tacaagtatt tccctggaga     240
ggttctgaag ggatcttctt taatgattga taaaattatt tgtcgtcagc attctatttg     300
ggaaaaagtg catatgaatt cagaaaaagt tttagtggct taataacccc cgttatatct     360
tgttgctatg atgagtttag gaaactcatt cttcatagac a                          401
```

<210> 158
<211> 401
<212> DNA
<213> Homo sapiens

<400> 158

```
tggtctaaat gatgccagat tcttgtggca ttacgtgcta accagaacta agctacaagt      60
atttccctgg agaggttctg aagggatctt ctttaatgat tgataaaatt atttgtcgtc     120
agcattctat ttgggaaaaa gtgcatatga attcagaaaa agttttagtg gcttaataac     180

ccccgttata tcttgttgct rtgatgagtt taggaaactc attcttcata gacagtgcaa     240
aggtcagctc agctcctgga gaaaagaata accatgaatt ccaattgagt ggattctgac     300
ttaagaagcc ttagtgagtc ttctgatata ttgattagat taaaaatagc acacacttta     360
taaattgatc tgtcattgaa gaagtgatga gctgactctc a                          401
```

<210> 159
<211> 401
<212> DNA
<213> Homo sapiens

<400> 159

```
ttgggaaaaa gtgcatatga attcagaaaa agttttagtg gcttaataac ccccgttata      60
tcttgttgct atgatgagtt taggaaactc attcttcata gacagtgcaa aggtcagctc     120
agctcctgga gaaaagaata accatgaatt ccaattgagt ggattctgac ttaagaagcc     180
ttagtgagtc ttctgatata ytgattagat taaaaatagc acacacttta taaattgatc     240
tgtcattgaa gaagtgatga gctgactctc accagggcag tagatagctc cccactagcc     300
agttccttta gggagggaac cagtattcca ggtgtctgag atcaacgcat aatcccaatc     360
cccagtgtgg tcattacaca actaagctct tgtaacactg g                          401
```

<210> 160
<211> 401
<212> DNA
<213> Homo sapiens

<400> 160

```
gtgttagttt caaatgtttt actttccttg gtctgaaaag actgcattaa aatggaaatt      60
ctctgtttta agtaaatata tgtcttcctg tggctttaac tatggcattc cacaatttgt     120
agatgttgcc attaattttc cactgatcaa actcaagcat taacatctcc aagtcagttg     180
ttgagaggac aagtctgcat rgctctctac tgtcatgtgt agtcccagtc tctgagttgt     240
acctttgcaa attgtatcac ctcccatttg ccctcaagga ttatttaagg gaaacaaaga     300
acttttgaat agggaacccc acatttaatg ttcatctgga ttaatgtacg tgacatcatc     360
ttgcctgttg caatggtgcc tcctggccca gttagaaaca a                          401
```

<210> 161
<211> 401

<212> DNA
<213> Homo sapiens

<400> 161

```
gctttaacta tggcattcca caatttgtag atgttgccat taattttcca ctgatcaaac      60
tcaagcatta acatctccaa gtcagttgtt gagaggacaa gtctgcatgg ctctctactg     120
tcatgtgtag tcccagtctc tgagttgtac ctttgcaaat tgtatcacct cccatttgcc     180
ctcaaggatt atttaaggga racaaagaac ttttgaatag ggaaccccac atttaatgtt     240
catctggatt aatgtacgtg acatcatctt gcctgttgca atggtgcctc ctggcccagt     300
tagaaacaag ccaagaagca gctgtcacac tatcccttac cagcccctgc agtgtggctc     360
actggctata gcacctcctg ctcgagccca gcattaggcc t                         401
```

<210> 162
<211> 401
<212> DNA
<213> Homo sapiens

<400> 162

```
ctcaagcatt aacatctcca agtcagttgt tgagaggaca agtctgcatg gctctctact      60
gtcatgtgta gtcccagtct ctgagttgta cctttgcaaa ttgtatcacc tcccatttgc     120
cctcaaggat tatttaaggg aaacaaagaa cttttgaata gggaacccca catttaatgt     180
tcatctggat taatgtacgt racatcatct tgcctgttgc aatggtgcct cctggcccag     240
ttagaaacaa gccaagaagc agctgtcaca ctatccctta ccagcccctg cagtgtggct     300
cactggctat agcacctcct gctcgagccc agcattaggc ctcacctact cacttcacca     360
tctttactcc cccatccccc tacagacatc atccttgagt g                         401
```

<210> 163
<211> 401
<212> DNA
<213> Homo sapiens

<400> 163

```
tatcccttac cagcccctgc agtgtggctc actggctata gcacctcctg ctcgagccca      60
gcattaggcc tcacctactc acttcaccat ctttactccc ccatccccct acagacatca     120
tccttgagtg acaggccctt gggaagtgga tcctgtgcct ttcacggtgc cagacgttgc     180
caactctcag agctgtggga rtcctgcctt gtcaggtcaa tcaatctagg tgcccatcaa     240
tggtggatta tataaagaat atgtggtgca tatacaacac gaactactac atagccataa     300
aaaggattga aatcaagtcc tttgcagcag catggatgta tctggagacc aatatcctaa     360
gtgaattaat gtagtaacag aaaatcaaat accacacgtt t                         401
```

<210> 164
<211> 401
<212> DNA
<213> Homo sapiens

<400> 164

```
tggctatagc acctcctgct cgagcccagc attaggcctc acctactcac ttcaccatct      60
ttactccccc atccccctac agacatcatc cttgagtgac aggcccttgg gaagtggatc     120
ctgtgccttt cacggtgcca gacgttgcca actctcagag ctgtgggaat cctgccttgt     180
caggtcaatc aatctaggtg yccatcaatg gtggattata taaagaatat gtggtgcata     240
tacaacacga actactacat agccataaaa aggattgaaa tcaagtcctt tgcagcagca     300
tggatgtatc tggagaccaa tatcctaagt gaattaatgt agtaacagaa aatcaaatac     360
cacacgtttt cacttacaat taggagctaa acactgggta a                         401
```

<210> 165
<211> 401
<212> DNA
<213> Homo sapiens

<400> 165

```
aatacatcta tgtaacaaac ctgcacatgt accccctcaa tctaaagaag gagaagaaga     60
cggggaagaa atgagattga atactaagca aaaagtaacc tcagaaagaa ctgggtgctc    120
aacatgcaca taattaaatg ggatacttct ccaagtaaga gaaaagcaat tgttcttctt    180
tgcaataact ttgaaatgtg ygtttggaga caacaaaata gaagcatcag gacacaaaaa    240
tgtatactaa cctggaagat taatgttgat aagatcaaag acactgtgaa agtgaattta    300
catttcagga atcttatatc tctcaccaag aaatcaaact taagcaacag tttcatatgc    360
taaaagcgct cttcaagtca gaggctcttg atttaaaaga a                        401
```

<210> 166
<211> 401
<212> DNA
<213> Homo sapiens

<400> 166

```
gccaccctgg tgggagtact catggctgtc ttttcggaca agggagacta tttctggtgg     60
ctgttactcc ctgtatgtgc tatgtccatg acttggtaag ttacaattgg ttttcaaaat    120
gcctttttga aaaaaaaaac atggcagaag gagggaatgg gagttgttat atggcagagt    180
ttcagttttg caagatgaaa watgttctct gaatgtatag tggtgatggt tgtacaacaa    240
tgtgattgtc cttaatgtca ttgagctgca cacttaaaaa tggttagccg ggtgcggtgg    300
ttcttgtttg tagtccaaac tattcagaag gctgaggggg aaggatcact tgagcccagg    360
agttaggggc tgcagtgagc tatgattgcg tcaccgcact c                        401
```

<210> 167
<211> 401
<212> DNA
<213> Homo sapiens

<400> 167

```
gtatgtgcta tgtccatgac ttggtaagtt acaattggtt ttcaaaatgc ctttttgaaa     60
aaaaaaacat ggcagaagga gggaatggga gttgttatat ggcagagttt cagttttgca    120
agatgaaata tgttctctga atgtatagtg gtgatggttg tacaacaatg tgattgtcct    180

taatgtcatt gagctgcaca yttaaaaatg gttagccggg tgcggtggtt cttgtttgta    240
gtccaaacta ttcagaaggc tgagggggaa ggatcacttg agcccaggag ttaggggctg    300
cagtgagcta tgattgcgtc accgcactcc agttctccga acctccttgc ttgggctaag    360
tgaggaggag gaggaggagg agaaggatgg aaaggaggag g                        401
```

<210> 168
<211> 401
<212> DNA
<213> Homo sapiens

<400> 168

```
attggattcc agtagattct gtctattgga aacagaaaca accattttaa aagatgtata      60
tttccttaca accagttatt tggccttttg tctgatctgg ctacacatcc actaatacct     120
ctcaaccaga ggtggctgca cattgacact tccatgggga agggaaacag tgctgcaatg     180
aagatacgag tgcaggtgtc yttttggtag aaacacactg atgcacgtgg cccccacata     240
cacttgactc ctccctccca agactctact gtcattggtc tgcggtagcg cctgggcttt     300
gggagtttct aaagcttccc agatgactct aaagtatagc caaagttgag acccacttcc     360
tccatcattg cctctcaaac ttgagcaata tgagaatcac c                         401
```

<210> 169
<211> 401
<212> DNA
<213> Homo sapiens

<400> 169

```
ggtctccctt agaaaaaatt tttttgctga attccttttt tttcaaaccc aaatccttca      60
aactagtttt tatgttgaca atgtcttaca tccttttttct ggaaacaaag atttccttct     120
ttctatattg tagttaaata taaaatacta atatgcacat aaataagcac agcctgctgt     180
gggcagtgtc tgcagaaggg mtgcccaccc ttactgtacc cacgggtgtg tggacgagga     240
cctacctgta gagctaaact cttcaggaag taatttgggc cctgctctga agaataggtt     300
cgtgggaagg aggcctagcc tgtaagtgct caccacgctc ccttccacaa tccaggaaaa     360
tgggagttct ggtctttaag tgatggctct ttgattgggc c                         401
```

<210> 170
<211> 401
<212> DNA
<213> Homo sapiens

<400> 170

```
tgaaggcaga aaggccacca tagtcctgag catctaggag actctgacac ccgtggacag      60
ttgaccagga ggccattgcg tatcttgcag agccagggcg aatgtgagaa gccagtgtct     120
cttacctcca gggcactgag gtcagaccag gagatatttg gagctgtagt tataggtatg     180
accagtttgg ctggaaagaa yggattgtaa tgtcctgtgg ctgaaaggta cattgacaac     240
gccatgttga aagggagggt gaagacgggg aggtcccatt tgctgagcat ggaattcaat     300
gcacttgaga aaattgggct aaaaaaagaa cagagcaaaa gaatgtgcat gtgacaaagc     360
aggctgacac acggaatcgc tatactctgg aggggctttg t                         401
```

<210> 171
<211> 401
<212> DNA
<213> Homo sapiens

<400> 171

```
tccagagcat tagggtctaa gggatttttt aaaattacta tttagtcaag ctgatttttc      60
tgccttttcc cctaaacatc tacagtgcta accccagagt acagttccac tgggagtcac     120
tctatcgtaa gcttgggggt gggggtgatg ggagccagcc cttaaggcat gtggcctcca     180
gcctggtttt aaatcttcca yagtctactc cctccaatca aaaaactgga tgcttactct     240
tagagcttct gacagaacct ctctattctg cttttcctta tggcatagct catagaacat     300
ctacaataat ttagggttcc caagctttgg taggcatcag aatcacctgg ggagctttaa     360
atacccaaac aggcttcatc tcagaccctc taaatcacaa t                         401
```

<210> 172
<211> 401
<212> DNA
<213> Homo sapiens

<400> 172

```
catagctcat agaacatcta caataattta gggttcccaa gctttggtag gcatcagaat     60
cacctgggga gctttaaata cccaaacagg cttcatctca gaccctctaa atcacaatct    120
ctaagggtgg ggcctggaac ctgttttaac aaactcccca aattgtgatg cgggccagag    180
tttgagaacc actgtatcaa rgggtgaatc ctatgtatct ctttaaagat ggctataaag    240
agattctgta tttttaaaa cctggttaac ccaaatcaaa ttccagctct tcctgttggt    300
gtgtaataaa tatgtttaag gtttctggat tatcaagaac aagagaacac ctgaaattag    360
aagaaaacca aagaaacctt acctttttaa tgtgctctcc c                        401
```

<210> 173
<211> 401
<212> DNA
<213> Homo sapiens

<400> 173

```
ttcccaagct ttggtaggca tcagaatcac ctggggagct ttaaataccc aaacaggctt     60
catctcagac cctctaaatc acaatctcta agggtggggc ctggaacctg ttttaacaaa    120
ctccccaaat tgtgatgcgg gccagagttt gagaaccact gtatcaaggg gtgaatccta    180
tgtatctctt taaagatggc yataaagaga ttctgtattt tttaaaacct ggttaaccca    240
aatcaaattc cagctcttcc tgttggtgtg taataaatat gtttaaggtt tctggattat    300
caagaacaag agaacacctg aaattagaag aaaaccaaag aaaccttacc tttttaatgt    360
gctctcccac tgtcaggtta tgaaacgccc ttttgtcttc t                        401
```

<210> 174
<211> 401
<212> DNA
<213> Homo sapiens

<400> 174

```
attccagctc ttcctgttgg tgtgtaataa atatgtttaa ggtttctgga ttatcaagaa     60
caagagaaca cctgaaatta gaagaaaacc aaagaaacct taccttttta atgtgctctc    120
ccactgtcag gttatgaaac gcccttttgt cttctttgtt gagtgatcaa aacacacgag    180
gagctcaagt caccttctcc ytagcttctt gccagaaaac taaagggagc acctggaaat    240
aattcagaag gaaaaaatca aagattcatt agaactaccc atgaaaaata acagtataaa    300
atagcattaa tcgatctaga actgcactaa cacaggagcc tctagcccca tgtggctata    360
taaatttaga tgtagattag ttaaaaattg agttcctcaa c                        401
```

<210> 175
<211> 401
<212> DNA
<213> Homo sapiens

<400> 175

```
gtgtgtaata aatatgttta aggtttctgg attatcaaga acaagagaac acctgaaatt     60
agaagaaaac caaagaaacc ttaccttttt aatgtgctct cccactgtca ggttatgaaa    120
cgcccttttg tcttctttgt tgagtgatca aaacacacga ggagctcaag tcaccttctc    180
cctagcttct tgccagaaaa ytaaagggag cacctggaaa taattcagaa ggaaaaaatc    240
aaagattcat tagaactacc catgaaaaat aacagtataa aatagcatta atcgatctag    300
aactgcacta acacaggagc ctctagcccc atgtggctat ataaatttag atgtagatta    360
gttaaaaatt gagttcctca acctctctag ccacatctca g                        401
```

<210> 176
<211> 401
<212> DNA

<213> Homo sapiens

<400> 176

```
cctctagccc catgtggcta tataaattta gatgtagatt agttaaaaat tgagttcctc     60
aacctctcta gccacatctc aggtgcttga tagccacacg tggctaggac ccactgtatt    120
agacagcaca gatacagact attccatcat ctcggaaagt tatcctgcac agtgctgatc    180
tggggcaggg gaagccttgt scttctcact ctgaatgaac agcccatcct cagcaccaac    240
cccaacccta tggctacctg agagagagtt ctgcagccaa gtccaaaaac aaacaaacaa    300
acaaaaaaag catatgccat ctttgccaag ttccctggtc tagaaatagc aaaatgtcta    360
gacatgaaga ctcagcatgg gctggaagaa tttagagtcc a                        401
```

<210> 177
<211> 401
<212> DNA
<213> Homo sapiens

<400> 177

```
cactgtatta gacagcacag atacagacta ttccatcatc tcggaaagtt atcctgcaca     60
gtgctgatct ggggcagggg aagccttgtc cttctcactc tgaatgaaca gcccatcctc    120
agcaccaacc ccaaccctat ggctacctga gagagagttc tgcagccaag tccaaaaaca    180
aacaaacaaa caaaaaaagc rtatgccatc tttgccaagt tccctggtct agaaatagca    240
aaatgtctag acatgaagac tcagcatggg ctggaagaat ttagagtcca tcttagggta    300
gagtcaaact cacactatgg tctggtgccc ttagccaatg ttagactcag cctaatataa    360
gaggggagaa gacacttccc cttgtgccaa agctggggct c                        401
```

<210> 178
<211> 401
<212> DNA
<213> Homo sapiens

<400> 178

```
tctttgccaa gttccctggt ctagaaatag caaaatgtct agacatgaag actcagcatg     60
ggctggaaga atttagagtc catcttaggg tagagtcaaa ctcacactat ggtctggtgc    120
ccttagccaa tgttagactc agcctaatat aagaggggag aagacacttc cccttgtgcc    180
aaagctgggg ctccctctgg yagagtcact gcctccagaa ggtctttggt acatacacga    240
cctagcaatg gtggagaggg caagatggga actgaggaaa acatctttca gtaaatggcc    300
ttgctcaaaa gggacatgct atggctaatt atgcctatcc tagccctacc agaagttcag    360
ctgtaaagaa tgatcacttg ttaggttcag ttaaaccttg t                        401
```

<210> 179
<211> 401
<212> DNA
<213> Homo sapiens

<400> 179

atttagagtc catcttaggg tagagtcaaa ctcacactat ggtctggtgc ccttagccaa 60
tgttagactc agcctaatat aagaggggag aagacacttc cccttgtgcc aaagctgggg 120
ctccctctgg tagagtcact gcctccagaa ggtctttggt acatacacga cctagcaatg 180
gtggagaggg caagatggga rctgaggaaa acatctttca gtaaatggcc ttgctcaaaa 240
gggacatgct atggctaatt atgcctatcc tagccctacc agaagttcag ctgtaaagaa 300
tgatcacttg ttaggttcag ttaaaccttg ttcactcctg agaactgcaa ttctgtgaac 360
agaataacta aattcaggcc tcagccagaa agtagaatta t 401

<210> 180
<211> 401
<212> DNA
<213> Homo sapiens

<400> 180

aagtagaatt atgacatttc catgtatttt tgtgttttga gacctgcttg acagttgttc 60
ataactagaa taagctaaaa atatctttgt ttaaatgaat acatgttcca cttaatgaca 120
gaaaagtaaa ttcacaaact tgctaaaaat tacttctaaa ttgtggacaa gataacctgg 180
ctttgggtct ctggctttag ygtaagcatc caaattgcat agtgataata atctctattg 240
aacataggga tgcatggata gattaaatca ccctcaacac tgatggacat ttgaaagcaa 300
aagaagtgtc agctgtggtc cttgccatcc ccagtaggag gcaaggcaga tcctcatagc 360
caggagcagt gagtggcacc aagctgggag cttaacagtg a 401

<210> 181
<211> 401 <212> DNA
<213> Homo sapiens

<400> 181

acagttgttc ataactagaa taagctaaaa atatctttgt ttaaatgaat acatgttcca 60
cttaatgaca gaaaagtaaa ttcacaaact tgctaaaaat tacttctaaa ttgtggacaa 120
gataacctgg ctttgggtct ctggctttag tgtaagcatc caaattgcat agtgataata 180
atctctattg aacataggga wgcatggata gattaaatca ccctcaacac tgatggacat 240
ttgaaagcaa aagaagtgtc agctgtggtc cttgccatcc ccagtaggag gcaaggcaga 300
tcctcatagc caggagcagt gagtggcacc aagctgggag cttaacagtg accaaggcca 360
agtgtcagtg caagcaggag agcacagggg gagctttgag a 401

<210> 182
<211> 401
<212> DNA
<213> Homo sapiens

<400> 182

atgttccact taatgacaga aaagtaaatt cacaaacttg ctaaaaatta cttctaaatt 60
gtggacaaga taacctggct ttgggtctct ggctttagtg taagcatcca aattgcatag 120
tgataataat ctctattgaa catagggatg catggataga ttaaatcacc ctcaacactg 180
atggacattt gaaagcaaaa raagtgtcag ctgtggtcct tgccatcccc agtaggaggc 240
aaggcagatc ctcatagcca ggagcagtga gtggcaccaa gctgggagct taacagtgac 300
caaggccaag tgtcagtgca agcaggagag cacaggggga gctttgagaa ggcatgtgtt 360
gcatgcacca gggaagggct ggtgtatctc tggggataaa g 401

<210> 183
<211> 401
<212> DNA
<213> Homo sapiens

<400> 183

agtgcaagca ggagagcaca gggggagctt tgagaaggca tgtgttgcat gcaccaggga 60
agggctggtg tatctctggg gataaagctg aaggatgact gggattttc tgtaatcaaa 120
gagagagaat tttaaatggt attaacactg ttcttgaaag aggtaaggta tgtccaatct 180
aaaattacat tgtaggagtt ygtgggtgtc ctgtgggttt ctgttcagtt gttttggtag 240
cctcattttt cttaaatttc ttttgcagtt gttgaaatct ataccagtgg gagttggtca 300
gatctatggc tgtgataatc catggacagg gggcattttc ctgggagcca tcctactctc 360
ctccccactc atgtgcctgc atgctgccat aggatcattg c 401

<210> 184
<211> 401
<212> DNA
<213> Homo sapiens

<400> 184

ggtgaaaccc tgtatctaca aaaaatacaa aaattagcca ggcatggtgg tgggtgcctg 60
tagttccagc tacttgggag gctgaggtgg gaaaatgact tgagcccagg aggaggaggc 120
tgcagtgagc taagattgca ccactgcact ccaacctggg cgacaagagt gaaactgtgt 180
ctctcaaaaa aaaaaaaaaa maaacaaaaa caaaaacaaa acaaaacaaa acaaaacaaa 240
acaggtaagg attcccctgt tttcctctct ttaattttaa agttatcagt tccgtaaagt 300
ctctgtaacc aaacatactg aagacagcaa cagaagtcac gttcagggac tggctcacac 360
ctgtaatccc agcactttgg gagatggagg taaaaggatc t 401

<210> 185
<211> 401
<212> DNA
<213> Homo sapiens

<400> 185

agagtgtgtg tgttctagaa gcctcagttc tctcgttcta gtcccagagg tcaccagagt 60
ttcctgcaaa tccactttag gttctatgag cttccttacc ttttctactt aagctagtat 120
agaaagtata actgacactt tcaactaaga gtctcaacta attaaccaat ccttaacatt 180

tgagttaaac tttcttaaac ycatggttct ttaaaatact ttctcattct gtttatatat 240
cctcccaaca gaataagaga gagagagaga gagagagaga gagagagagt gtgtgtgtgt 300
gtgtgtgtgt gtgtggttta aaacaataga aatttattct ctcacagttc tggaggccag 360
aaatccaaaa ctaaggtatt gccagagttg tgtttttccc t 401

<210> 186
<211> 401
<212> DNA
<213> Homo sapiens

<400> 186

gcttccttac cttttctact taagctagta tagaaagtat aactgacact ttcaactaag 60
agtctcaact aattaaccaa tccttaacat ttgagttaaa ctttcttaaa cccatggttc 120
tttaaaatac tttctcattc tgtttatata tcctcccaac agaataagag agagagagag 180
agagagagag agagagagag wgtgtgtgtg tgtgtgtgtg tgtgtggttt aaaacaatag 240
aaatttattc tctcacagtt ctggaggcca gaaatccaaa actaaggtat tgccagagtt 300
gtgtttttcc ctgagaaaga agattccatg cctgtcaact ggctcctggt ggtagtcagc 360
aatccttgat gttccctggc atttcctggc ttgtagtggc a 401

<210> 187
<211> 401

<212> DNA
<213> Homo sapiens

<400> 187

ttccttacct tttctactta agctagtata gaaagtataa ctgacacttt caactaagag 60
tctcaactaa ttaaccaatc cttaacattt gagttaaact ttcttaaacc catggttctt 120
taaaatactt tctcattctg tttatatatc ctcccaacag aataagagag agagagagag 180
agagagagag agagagagtg wgtgtgtgtg tgtgtgtgtg tgtggtttaa aacaatagaa 240
atttattctc tcacagttct ggaggccaga aatccaaaac taaggtattg ccagagttgt 300
gtttttccct gagaaagaag attccatgcc tgtcaactgg ctcctggtgg tagtcagcaa 360
tccttgatgt tccctggcat ttcctggctt gtagtggcat c 401

<210> 188
<211> 401
<212> DNA
<213> Homo sapiens

<400> 188

cagaataaga gagagagaga gagagagaga gagagagaga gtgtgtgtgt gtgtgtgtgt 60
gtgtgtggtt taaaacaata gaaatttatt ctctcacagt tctggaggcc agaaatccaa 120
aactaaggta ttgccagagt tgtgtttttc cctgagaaag aagattccat gcctgtcaac 180
tggctcctgg tggtagtcag yaatccttga tgttccctgg catttcctgg cttgtagtgg 240
catcactcca acctttgtct atatcttcag atggttttct tctctttgcg tgtctgtggc 300
tctgtggctc catgttttcc tctcttttcc cttacaaaga tgccagtcat tggatgtagg 360
gcccacccta atccaacgtt acctcatctt aactagctac a 401

<210> 189
<211> 401
<212> DNA
<213> Homo sapiens

<400> 189

agagagagag agagagagag agagagtgtg tgtgtgtgtg tgtgtgtgtg tggtttaaaa 60
caatagaaat ttattctctc acagttctgg aggccagaaa tccaaaacta aggtattgcc 120
agagttgtgt ttttccctga gaaagaagat tccatgcctg tcaactggct cctggtggta 180
gtcagcaatc cttgatgttc yctggcattt cctggcttgt agtggcatca ctccaacctt 240
tgtctatatc ttcagatggt tttcttctct ttgcgtgtct gtggctctgt ggctccatgt 300
tttcctctct tttcccttac aaagatgcca gtcattggat gtagggccca ccctaatcca 360
acgttacctc atcttaacta gctacatctg caaaggctct g 401

<210> 190
<211> 401
<212> DNA
<213> Homo sapiens

<400> 190

gaaagaagat tccatgcctg tcaactggct cctggtggta gtcagcaatc cttgatgttc 60
cctggcattt cctggcttgt agtggcatca ctccaacctt tgtctatatc ttcagatggt 120
tttcttctct ttgcgtgtct gtggctctgt ggctccatgt tttcctctct tttcccttac 180
aaagatgcca gtcattggat ktagggccca ccctaatcca acgttacctc atcttaacta 240
gctacatctg caaaggctct gtttccaaat aaagtcacat tctgaggttc tggatagaca 300
ttagccttag ggggcacact attcaaccta ttacacatat ataatatgtc ctaccattgc 360
ccacttgaga gggcttggtt gttttacatg ccaaaagcag t 401

<210> 191
<211> 401
<212> DNA
<213> Homo sapiens

<400> 191

caactggctc ctggtggtag tcagcaatcc ttgatgttcc ctggcatttc ctggcttgta 60
gtggcatcac tccaaccttt gtctatatct tcagatggtt ttcttctctt tgcgtgtctg 120
tggctctgtg gctccatgtt ttcctctctt ttcccttaca aagatgccag tcattggatg 180
tagggcccac cctaatccaa ygttacctca tcttaactag ctacatctgc aaaggctctg 240
tttccaaata aagtcacatt ctgaggttct ggatagacat tagccttagg gggcacacta 300
ttcaacctat tacacatata taatatgtcc taccattgcc cacttgagag ggcttggttg 360
ttttacatgc caaaagcagt gagcatacct accactaaga g 401

<210> 192
<211> 401
<212> DNA
<213> Homo sapiens

<400> 192

cctttgtcta tatcttcaga tggttttctt ctctttgcgt gtctgtggct ctgtggctcc 60
atgttttcct ctcttttccc ttacaaagat gccagtcatt ggatgtaggg cccaccctaa 120
tccaacgtta cctcatctta actagctaca tctgcaaagg ctctgtttcc aaataaagtc 180
acattctgag gttctggata sacattagcc ttagggggca cactattcaa cctattacac 240
atatataata tgtcctacca ttgcccactt gagagggctt ggttgtttta catgccaaaa 300
gcagtgagca tacctaccac taagagcgtg gcttctaaat gccattctta aaacctgcac 360
attctccaca tgaatcccag aacttaaagt aacaacaaca a 401

<210> 193
<211> 401
<212> DNA
<213> Homo sapiens

<400> 193

ttacacatat ataatatgtc ctaccattgc ccacttgaga gggcttggtt gttttacatg 60
ccaaaagcag tgagcatacc taccactaag agcgtggctt ctaaatgcca ttcttaaaac 120
ctgcacattc tccacatgaa tcccagaact taaagtaaca acaacaacaa aaaagaacca 180
aatagtgatc gaagaatgat yggggaaaac tgaaaaagac acagaagtca gtttgaaaga 240
acttccactg gctaaatttg ggacagtttg agcatgaaaa taaataataa cagtaatatg 300
tcctctcaaa taatatagaa aaccatgaat caatacttat atacctaaat acatacatac 360
aaacaaacat acatacatgt gctaattgaa aatagagata t 401

<210> 194
<211> 401
<212> DNA
<213> Homo sapiens

<400> 194

aaaacctgca cattctccac atgaatccca gaacttaaag taacaacaac aacaaaaaag 60
aaccaaatag tgatcgaaga atgatcgggg aaaactgaaa aagacacaga agtcagtttg 120
aaagaacttc cactggctaa atttgggaca gtttgagcat gaaaataaat aataacagta 180

```
atatgtcctc tcaaataata kagaaaacca tgaatcaata cttatatacc taaatacata      240
catacaaaca aacatacata catgtgctaa ttgaaaatag agatatttgt cattcctttt      300
tcagcaaata taatttcatc ctatggatat atatggtagt tcatttaagt gttctattac      360
tgattcaagt taattccaat gctttactat tttaatagta a                          401
```

<210> 195
<211> 401
<212> DNA
<213> Homo sapiens

<400> 195

```
ctgcacattc tccacatgaa tcccagaact taaagtaaca acaacaacaa aaaagaacca      60
aatagtgatc gaagaatgat cggggaaaac tgaaaaagac acagaagtca gtttgaaaga      120
acttccactg gctaaatttg ggacagtttg agcatgaaaa taaataataa cagtaatatg      180
tcctctcaaa taatatagaa raccatgaat caatacttat atacctaaat acatacatac      240
aaacaaacat acatacatgt gctaattgaa aatagagata tttgtcattc ctttttcagc      300
aaatataatt tcatcctatg gatatatatg gtagttcatt taagtgttct attactgatt      360
caagttaatt ccaatgcttt actattttaa tagtaatgca a                          401
```

<210> 196
<211> 401
<212> DNA
<213> Homo sapiens

<400> 196

```
atcccagaac ttaaagtaac aacaacaaca aaaaagaacc aaatagtgat cgaagaatga      60
tcggggaaaa ctgaaaaaga cacagaagtc agtttgaaag aacttccact ggctaaattt      120
gggacagttt gagcatgaaa ataaataata acagtaatat gtcctctcaa ataatataga      180
aaaccatgaa tcaatactta yatacctaaa tacatacata caaacaaaca tacatacatg      240
tgctaattga aaatagagat atttgtcatt cctttttcag caaatataat ttcatcctat      300
ggatatatat ggtagttcat ttaagtgttc tattactgat tcaagttaat tccaatgctt      360
tactatttta atagtaatgc aatccatttt ttattttata a                          401
```

<210> 197
<211> 401
<212> DNA
<213> Homo sapiens

<400> 197

```
ctcagggggt acatgtgcat gtttgttaca tgagtatatt ttgtatttgt ggggattggg      60
cttctactgt accctttacc caaatagtga acattgtact caatgggtaa tttttcaacc      120
ctcgccctcc tcccaacctc ccccttcag gagtccccac tgtctattat ttctatcttt       180
gtgttcatgt ttacccattg yttatctccc acttatgagt gaaaacacgt ggtatttgat      240
ttcctgagtt agttcactaa ggatactgcc ctccaattcc atccatattg ctgcaaagga      300
catgatttca tttgttatgg ctgccgaata aattctattg agctttattt tgtatttcaa      360
aatacagaca atttaaccaa ttagaattaa gtatgactgc a                          401
```

<210> 198
<211> 401
<212> DNA
<213> Homo sapiens

<400> 198

aattaagtat gactgcaact agcatagact caaagtaata gtgggtaatt tctttctcct 60
atttggaaaa tctggaggca ggctgtctgt ggctggtgcc ccaccaagta gttgaggacc 120
caggcttttt ccctctttcc caagaggacc tcccagtcta agatggttct ggatcagtgg 180
ctcttcatca gggataattt ycccccctaaa ggacagttga caatgtctgg agacattttt 240
attgtcataa tggggacgag gtgcaatggc atcaaaaaag tagaggccag gggtgctgct 300
aaatgtccta caacccctgg caacaaggaa ttatctaagc ccaaaatggg aacggtgtgg 360
aggttgagaa atcccgttct cactccatct atcacacctg t 401

<210> 199
<211> 401
<212> DNA
<213> Homo sapiens

<400> 199

tggtttagtc ttgggaggga gtatgtgtcg aggaatttat ccatttcttc tagattttct 60
agtttatttg catagaggtg tttatattat tctctgatgg tagtttgtat ttctgtggga 120
ttggtggtga tatccccttc gtcattgttt attgcgtcta tttgattctt ctctcttttc 180
ttctttatta gtcttgctag yggtctatca attttgttga tcttttcaaa aaaccagctc 240
ctggattcat tgattttttg aagggttttt tgtgtctcta ttttcttcag ttctgctctg 300
atcttagtta tttcttgcct tctgctagct tttgaatgtg tttgctcttg cttctctagt 360
tcttttaatt gtgatgttag ggtgtcaatt ttagatattt c 401

<210> 200
<211> 401
<212> DNA
<213> Homo sapiens

<400> 200

ggtttagtct tgggagggag tatgtgtcga ggaatttatc catttcttct agattttcta 60
gtttatttgc atagaggtgt ttatattatt ctctgatggt agtttgtatt tctgtgggat 120
tggtggtgat atccccttcg tcattgttta ttgcgtctat ttgattcttc tctcttttct 180
tctttattag tcttgctagt rgtctatcaa ttttgttgat cttttcaaaa aaccagctcc 240
tggattcatt gattttttga agggtttttt gtgtctctat tttcttcagt tctgctctga 300
tcttagttat ttcttgcctt ctgctagctt ttgaatgtgt ttgctcttgc ttctctagtt 360
cttttaattg tgatgttagg gtgtcaattt tagatatttc c 401

<210> 201
<211> 401
<212> DNA
<213> Homo sapiens

<400> 201

ctagtggtct atcaattttg ttgatctttt caaaaaacca gctcctggat tcattgattt 60
tttgaagggt tttttgtgtc tctattttct tcagttctgc tctgatctta gttatttctt 120
gccttctgct agcttttgaa tgtgtttgct cttgcttctc tagttctttt aattgtgatg 180
ttagggtgtc aattttagat mtttcctgct ttctcttgtg ggcatttact gctgtaaatt 240
tccgtctaca cactgctttg aatgtgtccc agagattctg gtatgttgtg tctttgttct 300
cgttggtttc aaagaacatc tttatttctg ccttcatttc gttatgtacc cagtagtcat 360
tcaggagcag gttgttcagt ttccatgtag ttgagcggtt t 401

<210> 202
<211> 401
<212> DNA
<213> Homo sapiens

<400> 202

```
cagttctgct ctgatcttag ttatttcttg ccttctgcta gcttttgaat gtgtttgctc 60
ttgcttctct agttctttta attgtgatgt tagggtgtca attttagata tttcctgctt 120
tctcttgtgg gcatttactg ctgtaaattt ccgtctacac actgctttga atgtgtccca 180
gagattctgg tatgttgtgt stttgttctc gttggtttca aagaacatct ttatttctgc 240
cttcatttcg ttatgtaccc agtagtcatt caggagcagg ttgttcagtt tccatgtagt 300
tgagcggttt tgagtgagtt tcttaatcct gagttctagt ttgattgcac tgtggtctga 360
gagacagttt gttataattt ctgttctttt acattagctg a 401
```

<210> 203
<211> 401
<212> DNA
<213> Homo sapiens

<400> 203

```
tcattcccct ccctcccttc cattcctttc ccagtcttta atccccctgt tccttccctt 60
ctcctactcc cttaatcact tacagtccca ctcccaggag gaaggggtac aggaaactgc 120
atttctcctc ctgaaggtca ctgtgtgcag ctgaagccac ttagtccaca ggaaggaagg 180

ccagctggta ggagttcaga yggcagttac agccaccagg ggacagcttt ggcatccacc 240
cccttcaggg ctctcctgag agggaggagc agtgcccacc ctagggaggg ctgagcttta 300
tcacactctc tgagggctct ctgggtcctg cctcatcagc tctgatggcg gaagggttga 360
caggggggcag caaggatggt tttgtcctgt ggcacctcac c 401
```

<210> 204
<211> 401
<212> DNA
<213> Homo sapiens

<400> 204

```
cattcttata caccaataac agacaaacag agagccaaat catgagtgaa ctcccatttg 60
ccattgcttc acagagaata aaatacctag gaatccaact tataagggat atgaaggacc 120
tcttcaagga gaactacaaa ccactgctca acaaaacaaa agaggacaca aacaaatgga 180
agaacattcc atgctcatgg rtaggaagaa tcaatatcgt gaaaatggcc acactgtcca 240
aggtaattta cagattcaat gccatcccca tcaagctacc aatgactttc ttcacagaat 300
tggaaaaaac tactttaaag ttcatatgga accaaaaaaa gggccctcat tgccaagtca 360
atcctaagcc aaaagaacaa agctggaggc atcacactac c 401
```

<210> 205
<211> 401
<212> DNA
<213> Homo sapiens

<400> 205

```
tatataggca gccaccctga agccagagac atggagtgcc tgtctcaagt cacctgaaat 60
ccaagacaca gcactgattt gaacttctgg cttctggcac cacaggcccc aagcgcagct 120
cttcctcctc cacaggcctg gaggctctgc acacctgctt cctgccttcc aggggagact 180
gtacctaaga tgctttcagc rtctattctg tcccgagcat gggccagaga gagtagcaga 240
cactcacctg tcgggagaac ccacgccaag caggccagca gccagatgga cttttccatg 300
ctgtgagcat ctgaacaccc caggccagac ccgctctcgg ccacctcccc agtgagggcc 360
ctcccaggcg tggtggggga aggaaggcgg gcgggggtgg g 401
```

<210> 206
<211> 401
<212> DNA
<213> Homo sapiens

<400> 206

```
cccctcccatc tggcgtgtac tcagactgga gtccagctcg gggctggttt gggcagtaac     60
agcaaacctc tgcacagctg tcaccacatg ctgggcactt tagtaactca cataaggctg     120
aaaaccctat gagaaaggaa ctctgtttgt ttgtttgttt ctctttggag acagagtccc     180
actctgtcac ccaggctgga rtgcagtggc gcaatctcag ctcactgtaa actctgcctc     240
ccaagttcaa gtgattcttg tgcctcagcc tcccaagtag ctgggattac aagtgtgtgc     300
caccacgccc agctaatttt ttaaattttt agtagagaca gaattttgcc acgttgtcca     360
gcctggtctc gaactcctga cctcaagtga tcagcccgcc t                         401
```

<210> 207
<211> 401
<212> DNA
<213> Homo sapiens

<400> 207

```
cacataccta caggtgaggt cgacagccaa tgggagtgtc tctccttgcc ctcaaaatgc     60
cctctcctgg ccctccaggt gtcacaatgc cggaagtgcc acccccggag ccacactcct     120
ggagcaccct gttagcatgc ccaggtgccc tcaaggaggg tagcccctcc cagattgttg     180
ggctcctcat actggccact ygtcagctgg taatgtcagc catgtggtgg tggcaggggc     240
acaaaagatg cctgagggac tccagctctg agggccacaa tgctgtccct cctggaggcg     300
tggtcttcct atggtgactg cccacttggt tccatccttc ccttaccccc tcaaaatccg     360
tgtcacataa agcccacccc agcagaccta atttttgtta a                         401
```

<210> 208
<211> 401
<212> DNA
<213> Homo sapiens

<400> 208

```
ccagagagca agccccacta gagtgtggac gtaagacagg gacagtaggg gcagcccggg     60
agaggaaaag gcaagtagaa agactcaggt gaggcccaga aaggaggcag gcatttctgg     120
agggtggacg ggaggaaggc aggaaaggcg tgggggttat tgtgggtgca atgggaagtc     180
actgaatggt tttaagcgga ygcatggctc catcagaaca agctggccca ggagacttgg     240
ttgcaggccg ttggcaaggc tgaggctggc ttggctgttt ggacaaaggt gcaggggtgg     300
cggtggggaa gtgggtagac gggaggcagg gcttgcccaa gagcccagtg ggcagcacac     360
agtgtgagag ggagccaggc tgccggggtt gggagcctga g                         401
```

<210> 209
<211> 389
<212> PRT
<213> Homo sapiens

<400> 209

```
Met Glu Asp Ser Pro Thr Met Val Arg Val Asp Ser Pro Thr Met Val
 1               5                  10                  15
Arg Gly Glu Asn Gln Val Ser Pro Cys Gln Gly Arg Arg Cys Phe Pro
            20                  25                  30
Lys Ala Leu Gly Tyr Val Thr Gly Asp Met Lys Glu Leu Ala Asn Gln
        35                  40                  45
Leu Lys Asp Lys Pro Val Val Leu Gln Phe Ile Asp Trp Ile Leu Arg
    50                  55                  60
Gly Ile Ser Gln Val Val Phe Val Asn Asn Pro Val Ser Gly Ile Leu
65                  70                  75                  80
Ile Leu Val Gly Leu Leu Val Gln Asn Pro Trp Trp Ala Leu Thr Gly
                85                  90                  95
Trp Leu Gly Thr Val Val Ser Thr Leu Met Ala Leu Leu Leu Ser Gln
            100                 105                 110
Asp Arg Ser Leu Ile Ala Ser Gly Leu Tyr Gly Tyr Asn Ala Thr Leu
        115                 120                 125
Val Gly Val Leu Met Ala Val Phe Ser Asp Lys Gly Asp Tyr Phe Trp
    130                 135                 140
Trp Leu Leu Leu Pro Val Cys Ala Met Ser Met Thr Cys Pro Ile Phe
145                 150                 155                 160
Ser Ser Ala Leu Asn Ser Met Leu Ser Lys Trp Asp Leu Pro Val Phe
                165                 170                 175
Thr Leu Pro Phe Asn Met Ala Leu Ser Met Tyr Leu Ser Ala Thr Gly
            180                 185                 190
His Tyr Asn Pro Phe Phe Pro Ala Lys Leu Val Ile Pro Ile Thr Thr
        195                 200                 205
Ala Pro Asn Ile Ser Trp Ser Asp Leu Ser Ala Leu Glu Leu Leu Lys
    210                 215                 220
Ser Ile Pro Val Gly Val Gly Gln Ile Tyr Gly Cys Asp Asn Pro Trp
225                 230                 235                 240
Thr Gly Gly Ile Phe Leu Gly Ala Ile Leu Leu Ser Ser Pro Leu Met
                245                 250                 255
Cys Leu His Ala Ala Ile Gly Ser Leu Leu Gly Ile Ala Ala Gly Leu
            260                 265                 270
Ser Leu Ser Ala Pro Phe Glu Asp Ile Tyr Phe Gly Leu Trp Gly Phe
        275                 280                 285
Asn Ser Ser Leu Ala Cys Ile Ala Met Gly Gly Met Phe Met Ala Leu
    290                 295                 300
Thr Trp Gln Thr His Leu Leu Ala Leu Gly Cys Ala Leu Phe Thr Ala
305                 310                 315                 320
Tyr Leu Gly Val Gly Met Ala Asn Phe Met Ala Glu Val Gly Leu Pro
                325                 330                 335
Ala Cys Thr Trp Pro Phe Cys Leu Ala Thr Leu Leu Phe Leu Ile Met
            340                 345                 350
Thr Thr Lys Asn Ser Asn Ile Tyr Lys Met Pro Leu Ser Lys Val Thr


        355                 360                 365
Tyr Pro Glu Glu Asn Arg Ile Phe Tyr Leu Gln Ala Lys Lys Arg Met
    370                 375                 380
Val Glu Ser Pro Leu
385
```